(19) Europäisches Patentamt
European Patent Office
Office européen des brevets

(11) EP 4 198 060 A1

(12) # EUROPEAN PATENT APPLICATION
published in accordance with Art. 153(4) EPC

(43) Date of publication:
**21.06.2023 Bulletin 2023/25**

(21) Application number: **21855968.0**

(22) Date of filing: **10.08.2021**

(51) International Patent Classification (IPC):
***C08B 11/15*** (2006.01) ***A61K 9/06*** (2006.01)
***A61K 9/48*** (2006.01) ***A61K 45/00*** (2006.01)
***A61K 47/61*** (2017.01) ***C08B 37/02*** (2006.01)
***C08B 37/04*** (2006.01) ***C08B 37/08*** (2006.01)

(52) Cooperative Patent Classification (CPC):
**A61K 9/06; A61K 9/48; A61K 45/00; A61K 47/61; C08B 11/15; C08B 37/0021; C08B 37/003; C08B 37/0084**

(86) International application number:
**PCT/JP2021/029573**

(87) International publication number:
**WO 2022/034889 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2020 JP 2020137010**

(71) Applicants:
- **The University of Tokyo**
  **Bunkyo-ku, Tokyo 113-8654 (JP)**
- **Mochida Pharmaceutical Co., Ltd.**
  **Tokyo 160-8515 (JP)**

(72) Inventors:
- **ITO, Taichi**
  **Tokyo 113-8654 (JP)**
- **OHTA, Seiichi**
  **Tokyo 113-8654 (JP)**
- **QI, Pan**
  **Tokyo 113-8654 (JP)**
- **CHANDEL, Arvind Kumar Singh**
  **Tokyo 113-8654 (JP)**
- **ISAJI, Mitsuko**
  **Tokyo 160-8515 (JP)**

(74) Representative: **Hoffmann Eitle**
**Patent- und Rechtsanwälte PartmbB**
**Arabellastraße 30**
**81925 München (DE)**

(54) **POLYSACCHARIDE DERIVATIVE, POLYSACCHARIDE DERIVATIVE-DRUG CONJUGATE, AND METHOD FOR PRODUCING SAME**

(57) To provide a novel polysaccharide derivative which can be used to form a conjugate with a drug and can be used as a medical material, and a polysaccharide derivative-drug conjugate which uses the same. A polysaccharide derivative obtained by introducing a group represented by formula (A) to a polysaccharide which is acidic, basic or both, and a polysaccharide derivative-drug conjugate of said polysaccharide derivative and a drug.

EP 4 198 060 A1

## Description

[Technical Field]

The present invention relates to a polysaccharide derivative that can be used as a medical material such as a DDS (drug delivery system) carrier, drug, medical device or pharmaceutical additive, as a separation material, and as a food, supplement, food additive or the like, to a polysaccharide derivative-drug conjugate using the same, and to uses for these.

[Background Art]

Because polysaccharides such as hyaluronic acid and alginic acid generally exhibit superior water solubility and water retention and suitable viscosity, adhesiveness and biocompatibility, they are widely used as medical materials and food additives, as additives in cosmetics and everyday goods, and as thickening agents. For example, hyaluronic acid (HA) is a biodegradable and biocompatible natural polymer that is abundant in the body, and is widely used in pharmaceuticals for orthopedics, ophthalmology and the like, in medical devices and cosmetics, and as a material in health care products such as contact lenses. Alginic acid (AL) is a natural polysaccharide found in brown algae and the like, and because it has properties such as high biocompatibility and the ability to be crosslinked by polyvalent metal ions such as Ca, it is used in pharmaceuticals including hemostats and wound dressings, in medical devices and pharmaceutical additives, and in foods, supplements and food additives.

Recently research has being done into applying these polysaccharides as drug carrier materials in drug delivery systems (DDS). Because HA binds specifically to the CD44 receptor, is overexpressed on the surface of various tumor cells, and can be easily derivatized using its hydroxyl and carboxyl groups, it is attracting attention as a drug carrier material. For example, there have been multiple reports of HA-drug conjugates using Schiff bases (NPL 1: Materials Science and Engineering: C, 2014, 36: 287-293; NPL 2: Carbohydrate Polymers 134 (2015) 293-299; NPL 3: Carbohydrate Polymers 189 (2018) 273-279; NPL 4: Carbohydrate Polymers 216 (2019) 63-71). Schiff bases are imine group-containing compounds formed by condensation reactions between primary amines and reactive carbonyl compounds. Because Schiff bases exhibit reversible pH-responsive dissociation, they are expected to be able to selectively release drugs. However, Schiff bases formed by common aldehydes or ketones with amines are poorly stable in water due to rapid reverse reactions. Drug conjugates using Schiff bases therefore have rapid drug release rates, and often adequate performance has not been obtained. On the other hand, biocompatible in situ crosslinked hydrogels with hyaluronic acid or alginic acid frameworks have been achieved by using hydrazide groups or the like in place of amino groups to slow down the hydrolysis rate.

Because of its aromatic structure, benzaldehyde has reportedly been able to form stable Schiff bases with amine groups contained in various drugs (NPL 5: Materials Chemistry Frontiers, 2018, 2(10): 1765-1778). NPL 6 (J. Biomed. Nanotechnol. 2017, 13, 1647-1659) discloses conjugates of carboxymethyl chitosan and daunorubicin using Schiff base formation between the aldehyde groups of triazole benzylaldehyde and the amino groups of daunorubicin. Conjugate formation in NPL 6 is complex and involves multiple steps in which the drug is reacted with azido benzylaldehyde, and this is then reacted with alkyne-modified carboxymethyl chitosan to form triazole rings and bind the drug to the chitosan, and conjugate formation also requires that the drug and the azido benzylaldehyde be reacted together in advance. This reaction also uses a copper catalyst, which is a problem from a manufacturing and safety standpoint.

In addition, NPL 7 (Polymer Bulletin 31 (1993) 145-149) discloses dextran derivatives obtained by introducing benzaldehydes via ester bonds at the hydroxyl group position of dextran. Because these polysaccharide derivatives use the neutral polysaccharide dextran as the polysaccharide, it has been difficult to introduce hydrophobic benzaldehydes at a high modification rate. Furthermore, the resulting polysaccharide derivatives also have poor water solubility, which is a problem in terms of application. This literature also discloses derivatives obtained by introducing benzaldehydes into dextran which has been derivatized with epichlorohydrin or epoxy. With these derivatives the aim is to improve hydrophilicity by introducing hydroxy groups from the epichlorohydrin or epoxy into the side chains, but synthesis of these derivatives requires multiple reaction steps, and there is room for improvement from the standpoint of industrial manufacture and benzaldehyde modification rates.

There have been multiple reports of conjugates between drugs and hyaluronic acid without Schiff bases. For example, these include conjugates of HA and proteins (NPL 8: Carbohydrate Polymers 92 (2013) 2163-2170), an HA-IFN$\alpha$2a conjugate (NPL 9: Journal of Controlled Release 236 (2016) 79-89), and a conjugate of HA and pemetrexed (NPL 10: European Journal of Pharmaceutical Sciences 138 (2019) 105008).

[Citation List]

[Non Patent Literature]

[NPL 1] Materials Science and Engineering: C, 2014, 36: 287-293
[NPL 2] Carbohydrate Polymers 134 (2015) 293-299
[NPL 3] Carbohydrate Polymers 189 (2018) 273-279
[NPL 4] Carbohydrate Polymers 216 (2019) 63-71
[NPL 5] Materials Chemistry Frontiers 2 (2018) 1765-1778
[NPL 6] J. Biomed. Nanotechnol. 13 (2017) 1647-1659
[NPL 7] Polymer Bulletin 31 (1993) 145-149
[NPL 8] Carbohydrate Polymers 92 (2013) 2163-2170
[NPL 9] Journal of Controlled Release 236 (2016) 79-89
[NPL 10] European Journal of Pharmaceutical Sciences 138 (2019) 105008

[Summary of Invention]

Under these circumstances, there is demand for novel polysaccharide derivatives capable of forming conjugates with drugs.

For example, the present invention is as follows.

[1] A polysaccharide derivative obtained by introducing a group represented by formula (A) below into a polysaccharide which is acidic, basic or amphoteric:

[C1]

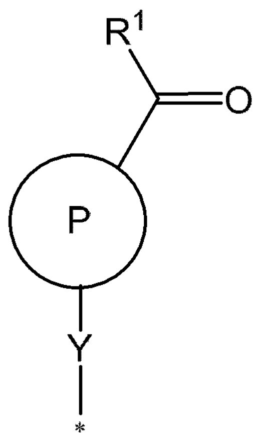

(A)

(in the formula, $R^1$ represents a hydrogen atom or $C_{1-4}$ alkyl,

ring P is a phenyl ring or pyridine ring, and the phenyl ring and pyridine ring are optionally substituted with one or more substituents independently selected from the halogen atoms and $-CF_3$, $-NO_2$, carboxyl and $-SO_3H$ groups,
Y represents a divalent group selected from the group consisting of -NH-, -C(=O)-, - S-, -O-, the $C_{1-6}$ alkylenes and $-(CH_2CH_2O)_n-$ and any combinations of these, with n being an integer from 1 to 9, and
* represents a linkage with the polysaccharide).

[2] The polysaccharide derivative according to [1], wherein the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives

thereof or salts of these, chitosan, derivatives thereof or salts of these, regenerated oxidized cellulose, derivatives thereof or salts of these, and pectic acid, derivatives thereof or salts of these.
[3] The polysaccharide derivative according to [1] or [2], wherein the group represented by formula (A) above is a group selected from formula (A-1) or (A-2) below:

[C2]

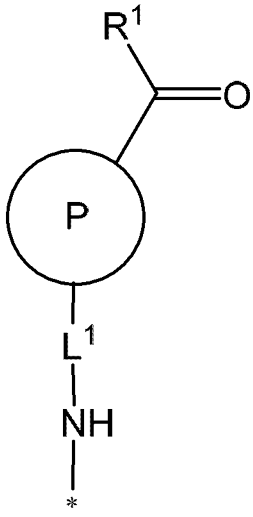

(A-1)

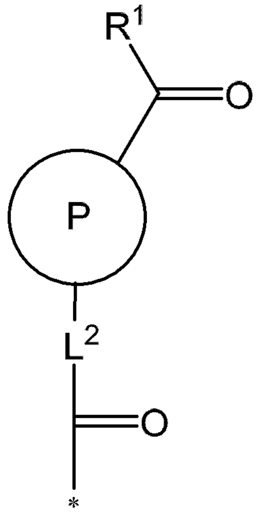

(A-2)

(in formulae (A-1) and (A-2), $R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
$L^1$ is a single bond or represents a divalent group selected from the group consisting of -C(=O)-, -S-, -O-, the $C_{1-6}$ alkylenes and $-(CH_2CH_2O)_n-$ and any combinations of these,
$L^2$ is a single bond or represents a divalent group selected from the group consisting of -NH-, -S-, -O-, the $C_{1-6}$ alkylenes and $-(CH_2CH_2O)_n-$ and any combinations of these,
n is an integer from 1 to 9, and
* represents a linkage with the polysaccharide).

[4] The polysaccharide derivative according to any of [1] to [3], wherein

the polysaccharide is a polysaccharide containing a carboxyl group and/or amino group, and
the group represented by the formula (A) is introduced into the polysaccharide by forming an amide bond with a carboxyl group or amino group of the polysaccharide.

[5] The polysaccharide derivative according to any of [1] to [4], wherein

the polysaccharide is a polysaccharide containing a carboxyl group,
the group represented by the formula (A) is a group represented by the formula (A-1), and
the group represented by the formula (A-1) is introduced into the polysaccharide by substitution for the -OH of the carboxyl group of the polysaccharide, forming an amide bond.

[6] The polysaccharide derivative according to any of [1] to [5], containing at least one structural unit selected from formulae (c11), (c12), (c13), (c14) and (c15) below:

[C3]

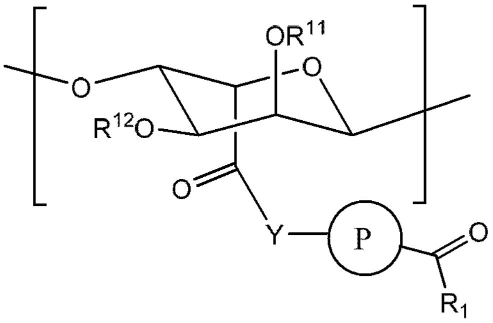

(c11)

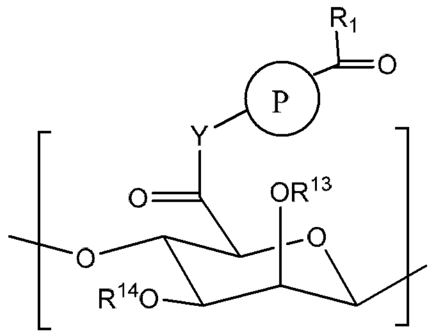

(c12)

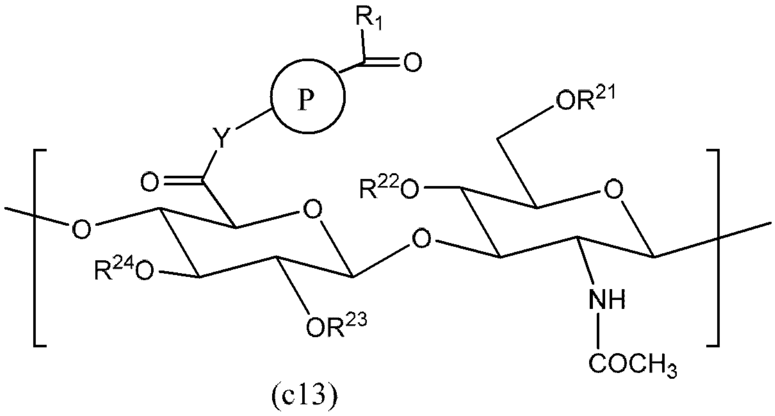

(c13)

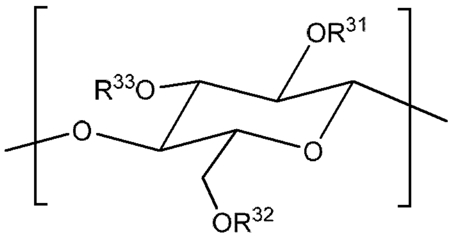

(c14)

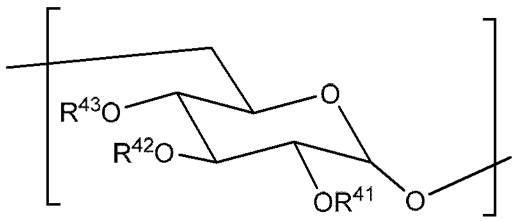

(c15)

(in the formulae, each of $R^{11}$, $R^{12}$, $R^{13}$, $R^{14}$, $R^{21}$, $R^{22}$, $R^{23}$ and $R^{24}$ is independently selected from a hydrogen atom, a $C_{1-6}$ alkyl and a -C(=O)-$C_{1-6}$ alkyl, and

1 to 3 of $R^{31}$, $R^{32}$ and $R^{33}$ represent:

[C4]

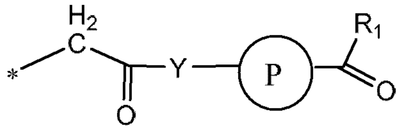

while each of the rest of $R^{31}$, $R^{32}$ and $R^{33}$ is independently selected from a hydrogen atom, a $C_{1-6}$ alkyl, a -C(=O)-$C_{1-6}$ alkyl and -$CH_2COOH$, and
1 to 3 of $R^{41}$, $R^{42}$ and $R^{43}$ represent:

[C5]

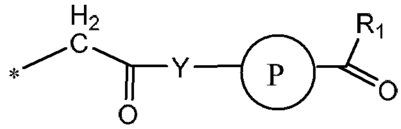

while each of the rest of $R^{41}$, $R^{42}$ and $R^{43}$ is independently selected from a hydrogen atom, a $C_{1-6}$ alkyl, a $-C(=O)-C_{1-6}$ alkyl and $-CH_2COOH$,
$R^1$ represents a hydrogen atom or $C_{1-4}$ alkyl,
ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
Y represents $-L^1-NH-$, in which $L^1$ is bound to ring P, and
$L^1$ is selected from a single bond, a $C_{1-6}$ alkylene and $-(CH_2CH_2O)_n-$, with n being an integer from 1 to 9).

[7] The polysaccharide derivative according to any of [1] to [4], wherein

the polysaccharide is a polysaccharide containing an amino group,
the group represented by the formula (A) is a group represented by the formula (A-2), and
the group represented by the formula (A-2) is introduced into the polysaccharide by substitution for a hydrogen atom of the amino group of the polysaccharide, forming an amide bond.

[8] The polysaccharide derivative according to any of [1] to [4] and [7], containing a structural unit represented by formula (c16) below:

[C6]

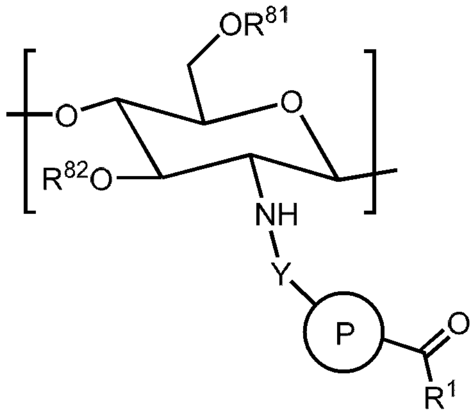

(c16)

(in the formula, each of $R^{81}$ and $R^{82}$ is independently selected from a hydrogen atom, a $C_{1-6}$ alkyl and a $-C(=O)-C_{1-6}$ alkyl,

$R^1$ represents a hydrogen atom or $C_{1-4}$ alkyl,
ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
Y represents $-L^2-C(=O)-$, in which $L^2$ is bound to ring P,
$L^2$ is selected from a single bond, a $C_{1-6}$ alkylene, $-(CH_2CH_2O)_n-$, $-(CH_2)_{m1}-(CH_2CH_2O)_n-(CH_2)_{m2}-$ and $-(CH_2)_{m1}-O-(CH_2CH_2O)_n-(CH_2)_{m2}$,
n is an integer from 1 to 9, and
each of m1 and m2 is independently an integer from 1 to 9).

[8-1] The polysaccharide derivative according to [3], wherein the group represented by the formula (A) is a group represented by the formula (A-1), the ring P is a phenyl ring, and the phenyl ring is optionally substituted with 1 to 4 substituents independently selected from the halogen atoms (F, Cl, Br and/or I) and $-CF_3$, $-NO_2$, carboxyl and $-SO_3H$ groups.

[8-2] The polysaccharide derivative according to [3], wherein the group represented by the formula (A) is a group represented by the formula (A-1), and the group represented by the formula (A-1) is a group selected from formulae (1), (2) and (3):

[C7]

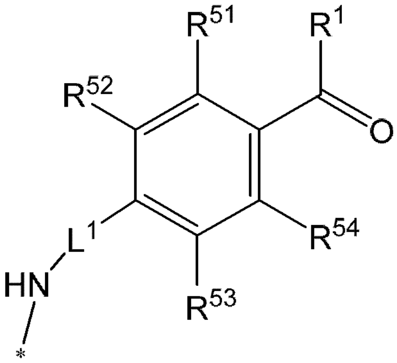

(1)

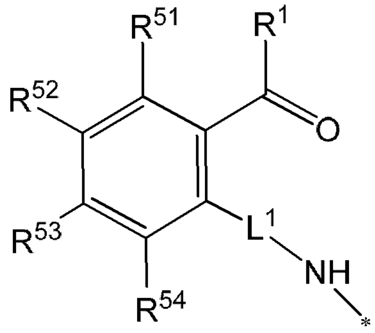

(2)

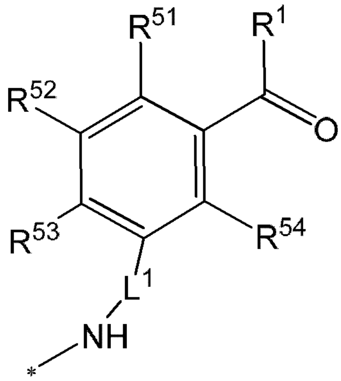

(3)

(in formulae (1) to (3), *, $R^1$ and $L^1$ are defined as in [3], and each of $R^{51}$, $R^{52}$, $R^{53}$ and $R^{54}$ is independently selected from a hydrogen atom, a halogen atom, and the $-CF_3$, $-NO_2$, carboxyl and $-SO_3H$ groups).

[8-3] The polysaccharide derivative according to [8-2], wherein $R^1$ in the formulae (1) to (3) is a hydrogen atom, $L^1$ is a single bond, and $R^{51}$, $R^{52}$, $R^{53}$ and $R^{54}$ are all hydrogen atoms, or are selected from a hydrogen atom and a halogen atom (preferably F).

[8-4] The polysaccharide derivative according to any of [1] to [7], in which the group represented by the formula (A) is a group represented by the following formula:

[C8]

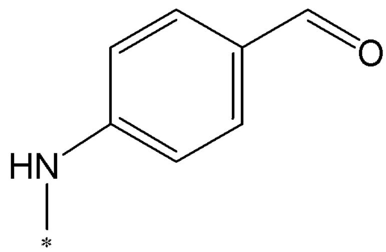

(in the formula, * represents a linkage with the polysaccharide).

[8-5] The polysaccharide derivative according to any of [1] to [7] and [8-1] to [8-4], wherein the polysaccharide is an acidic polysaccharide.

[8-6] The polysaccharide derivative according to [1] to [4], [7] or [8-1], wherein the polysaccharide is a basic polysaccharide.

[8-7] The polysaccharide derivative according to [1] to [5] or [8-1] to [8-4], wherein the polysaccharide is an amphoteric polysaccharide.

[8-8] The polysaccharide derivative according to any of [1] to [7] and [8-1] to [8-4], wherein the polysaccharide is alginic acid or a derivative thereof or a salt of these.

[8-9] The polysaccharide derivative according to any of [1] to [7] and [8-1] to [8-4], wherein the polysaccharide is hyaluronic acid or a derivative thereof or a salt of these.

[8-10] The polysaccharide derivative according to any of [1] to [7] and [8-1] to [8-4], wherein the polysaccharide is carboxymethyl cellulose or a derivative thereof or a salt of these.

[8-11] The polysaccharide derivative according to any of [1] to [7] and [8-1] to [8-4], wherein the polysaccharide is carboxymethyl starch or a derivative thereof or a salt of these.
[8-12] The polysaccharide derivative according to [1] to [4], [7] or [8], wherein the polysaccharide is chitosan or a derivative thereof or a salt of these.
[8-13] The polysaccharide derivative according to [1] to [4], [7] or [8], wherein the polysaccharide is chitosan or a derivative thereof or a salt of these,

the group represented by the formula (A) is a group represented by the formula (A-2), and
the group represented by the formula (A-2) is introduced into the polysaccharide by substitution for a hydrogen atom of an amino group of the polysaccharide, forming an amide bond.

[9] The polysaccharide derivative according to any of [1] to [8] and [8-1] to [8-13], wherein the modification rate by the group represented by the formula (A) in the polysaccharide derivative is 0.01 to 1.
[10] A polysaccharide derivative-drug conjugate between a drug containing a primary amino group and the polysaccharide derivative according to any of [1] to [9] and [8-1] to [8-13], wherein a structure represented by the following formula (D) is formed between the primary amino group in the drug and the group represented by formula (A) in the polysaccharide:

[C9]

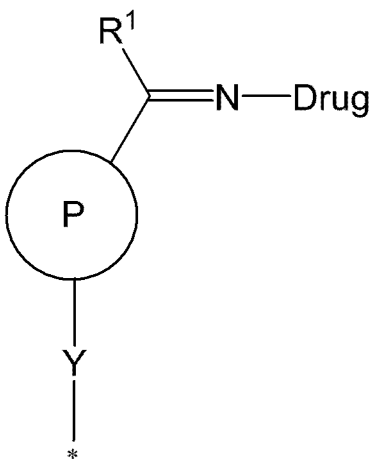

(D)

(in the formula, "Drug" represents the drug part excluding the primary amino group, and ring P, $R^1$ and * are defined as in [1]).
[11] The polysaccharide derivative-drug conjugate according to [10], wherein the drug is released from the polysaccharide derivative-drug conjugate under low pH conditions.
[12] The polysaccharide derivative-drug conjugate according to [10] or [11], wherein the drug is at least one selected from the low-molecular-weight compounds, middle-molecular-weight compounds, peptides, nucleic acids, nucleic acid derivatives, aptamers, vitamins, monoamines, amino acids, polyamines, antibodies, fluorescent dyes and contrast agents.
[13] A crosslinked structure containing the polysaccharide derivative according to any one of [1] to [9] and [8-1] to [8-13], wherein the polysaccharide derivative is crosslinked via a crosslinking group.
[14] A crosslinked structure containing the polysaccharide derivative according to any one of [1] to [9] and [8-1] to [8-13] together with at least one of an amino group-containing polymer and an amino group-containing low-molecular-weight compound containing two or more primary amino groups, hydrazide groups or aminooxy groups, wherein the crosslinked structure is crosslinked by covalent bonds formed via Schiff bases between the primary amino groups, hydrazide groups or aminooxy groups contained in the amino group-containing polymer and amino group-containing low-molecular-weight compound and the group represented by the formula (A) in the polysaccharide derivative.
[15] The crosslinked structure according to [14], wherein the amino group-containing polymer is at least one selected from linear, branched or dendritic polyamines such as the polyalkylene imines (for example, polyethyleneimine); polyalkylene glycols substituted with amino groups, hydrazide groups or aminooxy groups; polyallylamine; polyvinylamine; polyacrylamine; amino-group containing polysaccharides such as chitosan; amino group-containing proteins such as gelatins, collagens, fibrinogen and albumin; and polyamino acids such as polylysine.

[16] A crosslinked structure-drug conjugate between a drug containing a primary amino group and the crosslinked structure according to any of [13] to [15], wherein the primary amino group contained in the drug and the group represented by formula (A) in the crosslinked structure are crosslinked by covalent binding via a Schiff base.

[17] A composition containing the polysaccharide derivative according to any of [1] to [9] or [8-1] to [8-13], the polysaccharide derivative-drug conjugate according to any of [10] to [12], the crosslinked structure according to any of [13] to [15], or the crosslinked structure-drug conjugate according to [16].

[18] A gel, sponge, film or capsule containing the polysaccharide derivative according to any of [1] to [9] or [8-1] to [8-13], the polysaccharide derivative-drug conjugate according to any of [10] to [12], the crosslinked structure according to any of [13] to [15], the crosslinked structure-drug conjugate according to [16], or the composition according to [17].

[19] A tissue adhesive material containing the polysaccharide derivative according to any of [1] to [9] or [8-1] to [8-13], the polysaccharide derivative-drug conjugate according to any of [10] to [12], the crosslinked structure according to any of [13] to [15], or the crosslinked structure-drug conjugate according to [16].

[20] A drug delivery carrier or separation material containing the polysaccharide derivative according to any of [1] to [9] or [8-1] to [8-13] or the crosslinked structure according to any of [13] to [15].

[21] A method for manufacturing the polysaccharide derivative represented by formula (C1) below, wherein a polysaccharide containing carboxyl groups and a compound (a1) represented by formula (a1) below are subjected to a condensation reaction in an aqueous solvent:

[C10]

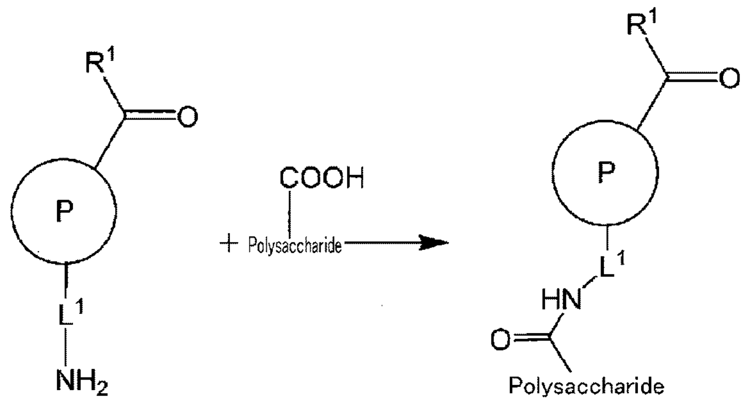


(a1) (C1)

(in the formula, $L^1$ is selected from a single bond, a $C_{1-6}$ alkylene and $-(CH_2CH_2O)_n-$, n is an integer from 1 to 9, and ring P and $R^1$ are defined as in [1]).

[22] The method according to [15], wherein the reaction between the compound (a1) and the polysaccharide is performed under conditions of pH 5 to 10 (preferably pH 7.5 to 8.0).

[23] A method for manufacturing the polysaccharide derivative-drug conjugate according to any of [8] to [10], wherein the polysaccharide derivative according to any of [1] to [9] or [8-1] to [8-13] and a drug containing a primary amino group are mixed in a solvent.

One aspect of the present invention provides a novel polysaccharide derivative capable of forming a Schiff base with a primary amino group. This polysaccharide derivative can form a conjugate by forming a Schiff base with a drug having a primary amino group. One aspect of the present invention provides a polysaccharide derivative-drug conjugate. This polysaccharide derivative-drug conjugate can efficiently release a drug under low pH conditions.

One aspect of the present invention provides a crosslinked structure comprising a polysaccharide derivative crosslinked via a crosslinking group.

One aspect of the present invention provides a crosslinked structure composed of a polysaccharide derivative and an amino group-containing polymer that contains two or more primary amino groups.

Another aspect of the present invention provides a crosslinked structure-drug conjugate.

In one aspect of the present invention, the polysaccharide derivative and crosslinked structure can be used in various applications such as tissue adhesive materials, drug delivery carriers and separation materials.

In one aspect of the present invention, the polysaccharide derivative, polysaccharide derivative-drug conjugate, crosslinked structure and crosslinked-structure drug conjugate can be used favorably as medical materials such as drug release devices and tissue adhesive materials.

[Brief Description of Drawings]

[Fig. 1]
Fig. 1A shows the $^1$H NMR spectra of a benzaldehyde-modified alginic acid (AL-ABA) synthesized in Example I-1 and alginic acid (AL), and Fig. 1B shows the sites corresponding to peaks b, c and d in Fig. 1A.
[Fig. 2]
Fig. 2 shows the UV-visible light absorption spectra (UV-vis) of the benzaldehyde-modified alginic acid (AL-ABA) synthesized in Example I-1, alginic acid (AL), and 4-aminobenzaldehyde (ABA).
[Fig. 3]
Fig. 3 shows the FT-IR spectra of the benzaldehyde-modified alginic acid (AL-ABA) synthesized in Example I-1 and alginic acid (AL).
[Fig. 4]
Fig. 4 shows cytotoxicity test results (WST assay results) for the benzaldehyde-modified alginic acid (AL-ABA) synthesized in Example I-1 and alginic acid (AL) at different concentrations (0.01 mg/mL, 0.1 mg/mL and 1 mg/mL) with respect to MeT-5A (human mesothelial cell line), NIH/3T3 (mouse embryo fibroblasts), HUVEC (human umbilical vein endothelial cells) and RAW264.7 cells (mouse macrophage-like cell line).
[Fig. 5]
Fig. 5 shows the $^1$H NMR spectrum of an AAP-modified alginic acid (AL-AAP) synthesized in Example I-3.
[Fig. 6]
Fig. 6 shows the FT-IR spectrum of the AAP-modified alginic acid (AL-AAP) synthesized in Example I-3.
[Fig. 7]
Fig. 7 shows the $^1$H NMR spectrum of an ADFBA-modified alginic acid (AL-ADFBA) synthesized in Example I-4.
[Fig. 8]
Fig. 8 shows the FT-IR spectrum of the ADFBA-modified alginic acid (AL-ADFBA) synthesized in Example I-4.
[Fig. 9]
Fig. 9 shows the $^1$H NMR spectrum of an AAP-modified alginic acid (AL-APCA) synthesized in Example I-5.
[Fig. 10]
Fig. 10 shows the FT-IR spectrum of the AAP-modified alginic acid (AL-APCA) synthesized in Example I-5.
[Fig. 11]
Fig. 11 shows the $^1$H NMR spectrum of an ANA-modified alginic acid (AL-ANA) synthesized in Example I-6.
[Fig. 12]
Fig. 12 shows the FT-IR spectrum of the ANA-modified alginic acid (AL-ANA) synthesized in Example I-6.
[Fig. 13]
Fig. 13 shows the $^1$H NMR spectra of a benzaldehyde-modified alginic acid-vancomycin conjugate (AL-ABA-Van) synthesized in Example I-7, the benzaldehyde-modified alginic acid (AL-ABA) synthesized in Example I-1, vancomycin (Van) and alginic acid (AL).
[Fig. 14]
Fig. 14 shows the UV-visible light absorption spectra (UV-vis) of the benzaldehyde-modified alginic acid-vancomycin conjugate (AL-ABA-Van) synthesized in Example I-7, the benzaldehyde-modified alginic acid (AL-ABA) synthesized in Example I-1 and vancomycin (Van).
[Fig. 15]
Fig. 15 shows the FT-IR spectra of the benzaldehyde-modified alginic acid-vancomycin conjugate (AL-ABA-Van) synthesized in Example I-7, the benzaldehyde-modified alginic acid (AL-ABA) synthesized in Example I-1, vancomycin (Van) and alginic acid (AL).
[Fig. 16A]
Fig. 16A shows the release behavior of vancomycin (Van) from a solution of the benzaldehyde-modified alginic acid-vancomycin conjugate (AL-ABA-Van) synthesized in Example I-7 under different pH conditions (pH = 5.0, 6.0, 7.4). The Van release rate (Cumulative Release %) shown on the vertical axis is the cumulative Van release rate (%), which was calculated from the absorbance of the UV-vis spectrum measurement.
[Fig. 16B]
Fig. 16B shows the release behavior of Van from a mixed solution (AL+Van) of sodium alginate and vancomycin under different pH conditions (pH = 5.0, 6.0, 7.4). The Van release rate (Cumulative Release %) shown on the

vertical axis is the cumulative Van release rate (%), which was calculated from the absorbance of the UV-vis spectrum measurement.

[Fig. 16C]

Fig. 16C shows the release behavior of Van from a vancomycin solution (Van only) under different pH conditions (pH = 5.0, 6.0, 7.4). The Van release rate (Cumulative Release %) shown on the vertical axis is the cumulative Van release rate (%), which is calculated from the absorbance of the UV-vis spectrum measurement.

[Fig. 17]

Fig. 17 shows confocal microscope images of an FTSC-loaded AL-ABA capsule prepared in Example I-8 and an FTSC-loaded AL capsule. The top row shows confocal microscope images of the AL capsule (Alg microcapsule) in FTSC-containing physiological saline, the middle row shows confocal microscope images of the AL-ABA capsule (Alg-ABA microcapsule) in FTSC-containing physiological saline, and the bottom row shows confocal microscope images of the AL-ABA capsule (Alg-ABA microcapsule) in FTSC-containing DMEM. In each row, the lefthand image is a transmitted image (Transmitted), the middle image is a fluorescent image (Amine-Fluorescein), and the righthand image shows the two superimposed (Merged).

[Fig. 18]

Fig. 18 shows vancomycin (Van) release behavior from a Van-loaded AL-ABA microcapsule (AL-ABA-Van capsule) prepared in Example I-9 and a Van-loaded AL microcapsule (AL-Van capsule) as a control. Fig. 18A shows the cumulative release rate (%) of Van on the vertical axis, while Fig. 18B shows the Van release rate (Vancomycin Release %) at each time point on the vertical axis. The Van release rate (Vancomycin Release %) is calculated from the absorbance in UV-vis spectrum measurement.

[Fig. 19]

Fig. 19 shows photographs illustrating the growth of Staphylococcus aureus around filter paper impregnated with sustained release solution from vancomycin-loaded capsules. The results using sustained release solution from a vancomycin-loaded AL capsule (AL-Van) are shown in (a), while the results using sustained release solution from a vancomycin-loaded AL-ABA capsule (AL-ABA-Van) are shown in (b). The top images show results immediately after addition of the sustained release solution, while the bottom images show results 24 hours after addition.

[Fig. 20]

Fig. 20 shows a comparison of Staphylococcus aureus growth inhibition areas using release liquids from vancomycin-loaded AL capsules (AL-Van) and vancomycin-loaded AL-ABA capsules (AL-ABA-Van) at different release times (n=3).

[Fig. 21]

Fig. 21 shows the UV-visible light absorption spectra (UV-vis) of a benzaldehyde-modified alginic acid-bacitracin conjugate (AL-ABA-Bac) synthesized in Example I-10, the benzaldehyde-modified alginic acid (AL-ABA) synthesized in Example 1, and bacitracin (Bac).

[Fig. 22]

Fig. 22 shows the FT-IR spectra of the benzaldehyde-modified alginic acid-bacitracin conjugate (AL-ABA-Bac) synthesized in Example I-10, bacitracin (Bac), the benzaldehyde-modified alginic acid (AL-ABA) synthesized in Example 1 and alginic acid (AL).

[Fig. 23]

Fig. 23 shows the $^1$H NMR spectrum of a benzaldehyde-modified alginic acid-dopamine conjugate (AL-ABA-DOPA) synthesized in Example I-11.

[Fig. 24]

Fig. 24 shows the FT-IR spectrum of the benzaldehyde-modified alginic acid-dopamine conjugate (AL-ABA-DOPA) synthesized in Example I-11.

[Fig. 25]

Fig. 25 shows the $^1$H NMR spectrum of a benzaldehyde-modified alginic acid-serotonin conjugate (AL-ABA-Serotonin) synthesized in Example I-13.

[Fig. 26]

Fig. 26 shows the FT-IR spectrum of the benzaldehyde-modified alginic acid-serotonin conjugate (AL-ABA-Serotonin) synthesized in Example I-13.

[Fig. 27]

Fig. 27 shows the $^1$H NMR spectrum of a benzaldehyde-modified alginic acid-celecoxib conjugate (AL-ABA-Celecoxib) synthesized in Example I-14.

[Fig. 28]

Fig. 28 shows methods for preparing AL-ABA-Apt in Example 1-15 and test procedures for release testing of the AL-ABA-Apt.

[Fig. 29]

Fig. 29 shows the release behavior of an HGF aptamer (Apt) from the AL-ABA-HGF aptamer (AL-ABA-Apt) synthesized in Example 1-15, a mixture of alginic acid and the HGF aptamer (ALG-Apt) and the HGF aptamer by itself (Apt) under physiological pH conditions (pH 7.4). The HGF aptamer release rate (cumulative release rate %) on the vertical axis is the cumulative release rate (%) of the HGF aptamer as calculated from the absorbance in UV-vis spectrum measurement.

[Fig. 30]

Fig. 31 shows optical photographs of benzaldehyde-modified alginic acid sponges prepared in Example I-16. Control indicates an AL sponge prepared as a control, and A, B and C indicate AL/AL-ABA mixed sponges in which the compounding ratios (weight ratios) of AL and AL-ABA are 75:25, 50:50 and 25:75, respectively.

[Fig. 31]

Fig. 31 shows SEM photographs of the benzaldehyde-modified alginic acid sponges prepared in Example I-16. Control indicates an AL sponge prepared as a control, and A, B and C indicate AL/AL-ABA mixed sponges in which the compounding ratios (weight ratios) of AL and AL-ABA are 75:25, 50:50 and 25:75, respectively.

[Fig. 32]

Fig. 32 shows the swelling and degradation profile (hydrogel weight change) of a Ca-crosslinked AL-ABA hydrogel.

[Fig. 33]

Fig. 33 is an SEM photograph (500x; 24 hours after start of swelling) of a sponge obtained by freeze drying a Ca-crosslinked AL-ABA hydrogel, confirming a porous structure of a dried hydrogel.

[Fig. 34]

Fig. 34 shows a schematic diagram of a crosslinked structure of AL-ABA with an amino group-containing polymer (DPI: dendritic polyethyleneimine), prepared in Example 1-18.

[Fig. 35]

Fig. 35 shows a photograph of a hydrogel consisting of a crosslinked structure of AL-ABA and DPI.

[Fig. 36A]

Fig. 36A shows the $^1$H NMR spectrum of PEG-COOH prepared in Example I-19.

[Fig. 36B]

Fig. 36B shows the $^1$H NMR spectrum of PEGDH prepared in Example I-19.

[Fig. 36C]

Fig. 36C shows a photograph of a hydrogel consisting of a crosslinked structure of AL-ABA and PEGDH prepared in Example I-19.

[Fig. 37]

Fig. 37 shows dynamic viscoelasticity measurement results for a hydrogel consisting of a crosslinked structure of AL-ABA and an amino group-containing polymer (PEGDH), prepared in Example I-19.

[Fig. 38]

Fig. 38 shows a photograph of a hydrogel of AL-ABA and polyallylamine prepared in Example I-21.

[Fig. 39]

Fig. 39 shows changes in appearance over time when calcium-crosslinked gels (AL-ABA gel, AL gel) of benzaldehyde-modified alginic acid (AL-ABA) and alginic acid (AL) were each administered to a pig esophagus and immersed in physiological saline in Example I-22. In Fig. 39, ALG (IL-6G) represents the AL gel, while ALG-ABA represents the AL-ABA gel.

[Fig. 40]

Fig. 40 shows changes over time in gel weight (adhesion rate, %) when calcium-crosslinked gels (AL-ABA gel, AL gel) of benzaldehyde-modified alginic acid (AL-ABA) and alginic acid (AL) were each administered to a pig esophagus and immersed in physiological saline in Example I-22. In Fig. 40, ALG (IL-6G) represents the AL gel, while ALG-ABA represents the AL-ABA gel.

[Fig. 41]

Fig. 41 is a schematic diagram illustrating binding of AL-ABA and AL bind to the esophageal mucosal layer (mucosa) and the submucosal layer (submucosa).

[Fig. 42]

Fig. 42 shows procedures for evaluating the adhesiveness of AL-ABA and AL to the mucosa and submucosa in Example I-23.

[Fig. 43]

Fig. 43 shows changes in appearance over time when gels of benzaldehyde-modified alginic acid (AL-ABA) and alginic acid (AL) calcium crosslinked at a concentration of 50 mM or 100 mM $Ca^{2+}$ (AL-ABA, AL) were each applied to pig esophageal mucosa (Control) and submucosa (ESD) and immersed in physiological saline in Example I-23. The circles (dotted lines) in the figure indicate areas where the material persists.

[Fig. 44]

Fig. 44 shows changes over time in gel weight (adhesion rate, %) when gels of benzaldehyde-modified alginic acid (AL-ABA) and alginic acid (AL) calcium crosslinked at a concentration of 50 mM or 100 mM $Ca^{2+}$ (AL-ABA, AL)

were each applied to pig esophageal mucosa (Con) and submucosa (ESD) and immersed in physiological saline in Example I-23.

[Fig. 45]

Fig. 45 is a diagram illustrating methods of tensile testing in Example I-23.

[Fig. 46]

Fig. 46 shows results of tensile testing of Ca-crosslinked hydrogels of AL-ABA and AL adhering to mucosa (Control) and submucosa (ESD) in Example I-23.

[Fig. 47]

Fig. 47(A) and Fig. 47(B) are diagrams illustrating test procedures for the lap shear method in Example I-24.

[Fig. 48]

Fig. 48 shows lap shear measurement results for adhesion strength (Pa) of conventional tissue adhesive materials and Ca-crosslinked hydrogels of AL-ABA and AL adhering to submucosa and skin in Example I-24.

[Fig. 49]

Fig. 49(A) is a diagram illustrating a device used for burst testing in Example I-25. Fig. 49(B) is a diagram illustrating the methods of burst testing.

[Fig. 50]

Fig. 50 shows the results of measurement of burst pressure (mmHg) in burst testing of a conventional tissue adhesive materials and Ca-crosslinked hydrogels of AL-ABA and AL adhering to submucosa and skin in Example I-25.

[Fig. 51]

Fig. 51 shows the $^1H$ NMR spectrum of a benzaldehyde-modified hyaluronic acid (HA-ABA) synthesized in Example II-1.

[Fig. 52]

Fig. 52 shows the UV-visible light absorption spectra (UV-vis) of the benzaldehyde-modified hyaluronic acid (HA-ABA) synthesized in Example II-1, hyaluronic acid (HA) and 4-aminobenzaldehyde (ABA).

[Fig. 53]

Fig. 53 shows the FT-IR spectra of the benzaldehyde-modified hyaluronic acid (HA-ABA) synthesized in Example II-1, hyaluronic acid (HA) and 4-aminobenzaldehyde (ABA).

[Fig. 54]

Fig. 54 shows cytotoxicity testing results (WST assay results) for hyaluronic acid (HA) and the benzaldehyde-modified hyaluronic acid (HA-ABA) synthesized in Example II-1 at different concentrations (0.01 mg/mL, 0.1 mg/mL and 1 mg/mL) with respect to MeT-5A cells (human mesothelial cell line), HUVEC cells (human umbilical vein endothelial cells), RAW264.7 cells (mouse macrophage-like cell line), NIH/3T3 cells (mouse embryo fibroblasts) and AB22 cells (mouse mesothelioma cells).

[Fig. 55]

Fig. 55 shows the $^1H$ NMR spectra of a benzaldehyde-modified hyaluronic acid-pemetrexed conjugate (HA-ABA-PMX) synthesized in Example II-2, benzaldehyde-modified hyaluronic acid (HA-ABA), hyaluronic acid (HA) and pemetrexed (PMX).

[Fig. 56]

Fig. 56 shows the UV-visible light absorption spectra (UV-vis) of the benzaldehyde-modified hyaluronic acid-pemetrexed conjugate (HA-ABA-PMX) synthesized in Example II-2 and benzaldehyde-modified hyaluronic acid (HA-ABA).

[Fig. 57]

Fig. 57 shows the release behavior of pemetrexed (PMX) from a benzaldehyde-modified hyaluronic acid-pemetrexed conjugate solution (HA-ABA-PMX) under different pH conditions (pH = 5.0, 6.0, 7.4), the release behavior of PMX from a PMX solution (Free PMX), and the release behavior of PMX from a mixed solution of HA and PMX (Free PMX mixed with HA). The vertical axis shows the cumulative drug release rate (%) of PMX, which is calculated from the absorbance in UV-vis spectrum measurement.

[Fig. 58]

Fig. 58A and Fig. 58B show the cell growth inhibition effects of the benzaldehyde-modified hyaluronic acid-pemetrexed conjugate (HA-ABA-PMX) synthesized in Example II-2 with different PMX concentrations ($10^{-4}$ $\mu$g/mL, $10^{-3}$ $\mu$g/mL, $10^{-2}$ $\mu$g/mL, $10^{-1}$ $\mu$g/mL, 1 $\mu$g/mL, 10 $\mu$g/mL), free pemetrexed (PMX), and HA-ADH-PMX comprising PMX bound to HA by irreversible amide bonds with respect to AB22 cells (mouse mesothelioma cells) and MeT-5A (human mesothelial cell line), respectively. Cell viability (%) is shown on the vertical axis.

[Fig. 59]

Fig. 59 shows the UV-visible light absorption spectra (UV-vis) of a benzaldehyde-modified hyaluronic acid-doxorubicin conjugate synthesized in Example II-3 (HA-ABA-DOX), the benzaldehyde-modified hyaluronic acid synthesized in Example II-1 (HA-ABA), and doxorubicin (DOX).

[Fig. 60]

Fig. 60 shows the release behavior of doxorubicin (DOX) from a benzaldehyde-modified hyaluronic acid-doxorubicin

conjugate (HA-ABA-DOX) under different pH conditions (pH = 5.0, 6.0, 7.4). The vertical axis shows the cumulative drug release rate (%) of DOX, which is calculated from the absorbance in UV-vis spectrum measurement.

[Fig. 61]

Fig. 61 shows the cell growth inhibition effects of the benzaldehyde-modified hyaluronic acid-doxorubicin conjugate (HA-ABA-DOX) synthesized in Example II-3 at different DOX concentrations ($10^{-3}$ μg/mL, $10^{-2}$ μg/mL, $10^{-1}$ μg/mL, 1 μg/mL, 10 μg/mL, 100 μg/mL) and free doxorubicin (DOX) with respect to AB22 cells (mouse mesothelioma cells). Cell viability (%) is shown on the vertical axis.

[Fig. 62]

Fig. 62 shows the UV-visible light spectra (UV-vis) of a benzaldehyde-modified hyaluronic acid-gemcitabine conjugate synthesized in Example II-4 (HA-ABA-GEM), the benzaldehyde-modified hyaluronic acid (HA-ABA) synthesized in Example II-1 and gemcitabine (GEM).

[Fig. 63]

Fig. 63 shows the $^{1}$H NMR spectrum of a benzaldehyde-modified hyaluronic acid-adenine conjugate (HA-ABA-adenine) synthesized in Example II-5.

[Fig. 64]

Fig. 64 shows the $^{1}$H NMR spectrum of a benzaldehyde-modified hyaluronic acid-cytosine conjugate (HA-ABA-cytosine) synthesized in Example II-5.

[Fig. 65]

Fig. 65 shows the $^{1}$H NMR spectrum of a benzaldehyde-modified hyaluronic acid-guanine conjugate (HA-ABA-guanine) synthesized in Example II-5.

[Fig. 66]

Fig. 66 shows the FT-IR spectra of the benzaldehyde-modified hyaluronic acid- DNA nucleotide conjugates (HA-ABA-adenine, HA-ABA-cytosine, HA-ABA-guanine) synthesized in Example II-5 and the benzaldehyde-modified hyaluronic acid (HA-ABA) synthesized in Example II-1.

[Fig. 67]

Fig. 67 shows the $^{1}$H NMR spectrum of the benzaldehyde-modified carboxymethyl cellulose (CMC-ABA) synthesized in Example III-1.

[Fig. 68]

Fig. 68 shows the FT-IR spectra of the benzaldehyde-modified carboxymethyl cellulose (CMC-ABA) synthesized in Example III-1 and carboxymethyl cellulose (CMC).

[Fig. 69]

Fig. 69 shows the $^{1}$H NMR spectrum of benzaldehyde-modified carboxymethyl dextran (CMDX-ABA) synthesized in Example IV-1.

[Fig. 70]

Fig. 70 shows the FT-IR spectra of benzaldehyde-modified carboxymethyl dextran (CMD-ABA) synthesized in Example IV-1 and carboxymethyl dextran (CMD).

[Fig. 71]

Fig. 71 shows the FT-IR spectra of benzaldehyde-modified carboxymethyl dextran-DNA nucleotide conjugates synthesized in Example IV-2 (CMD-ABA-adenine, CMD-ABA-cytosine, CMD-ABA-guanine), the benzaldehyde-modified carboxymethyl dextran (CMD-ABA) synthesized in Example IV-1 and carboxymethyl dextran (CMD).

[Fig. 72]

Fig. 72 shows the $^{1}$H NMR spectrum of benzaldehyde-modified chitosan (Chitosan-CBA) synthesized in Example V-1.

[Fig. 73]

Fig. 73 shows the FT-IR spectrum of benzaldehyde-modified chitosan (Chitosan-CBA) synthesized in Example V-1.

[Description of Embodiments]

In this Description, a "halogen atom" is a fluorine atom (F), chlorine atom (Cl), bromine atom (Br) or iodine atom (I).

In this Description, a "$C_{1-k}$ alkyl" is a linear or branched alkyl group with 1 to k carbon atoms.

In this Description, a "$C_{1-k}$ alkylene" is a linear or branched alkylene group with 1 to k carbon atoms.

In this Description, a "polysaccharide" means a compound consisting of two or more monosaccharides linked by glycoside bonds, a derivative thereof (for example a polysaccharide modified by esterification, maleimide modification, thiol modification, acrylate modification, aldehyde modification, disulfide (such as pyridyl disulfide) modification, alkyne (including cyclic alkyne) modification, tetrazine modification, furan ring modification or the like), or a salt or crosslinked body thereof. One consisting of 2 to 10 linked monosaccharides may be called an oligosaccharide, but in this Description the term "polysaccharide" encompasses oligosaccharides in addition to the narrow definition of "polysaccharide" meaning more than 10 linked monosaccharides.

In this Description, a "polysaccharide derivative-drug conjugate" is a complex formed by linking a drug to a polysaccharide derivative via bonds.

1. Polysaccharide derivative

One aspect of the present invention relates to a polysaccharide derivative (hereunder also called simply "the polysaccharide derivative") comprising a group represented by formula (A) below (hereunder sometimes called simply "modifying group (A)") introduced into a polysaccharide that is acidic, basic or both:

[C11]

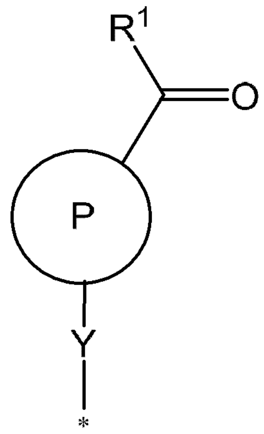

(A)

The polysaccharide derivative can form a Schiff base by reacting with a primary amino group under neutral to basic pH conditions. While the Schiff base formed between the polysaccharide derivative and the primary amino group is stable under neutral to basic pH conditions, it can dissociate under low pH conditions. This property makes it useful as a drug delivery carrier, bioabsorbable material, medical device or separation material device.

The polysaccharide derivative of this embodiment has at least one of the following advantages.

(1) The polysaccharide derivative can form a stable conjugate under neutral to basic pH conditions with various drugs having primary amino groups, and can also release drugs having primary amino groups. In particular, because of its aromatic structure the modifying group (A) is capable of forming stable Schiff bases with amine groups contained in various drugs even in water.

(2) Because the polysaccharide derivative is made from an acidic, basic or amphoteric polysaccharide, the modifying group (A) can be introduced at a good modification rate even if the modifying group (A) has a hydrophobic benzene ring or pyridine ring. Furthermore, even if the modifying group (A) has been introduced at a high modification rate, the resulting polysaccharide derivative has good water solubility due to the presence of unmodified acidic or basic functional groups (anionic groups or cationic functional groups). In particular, a polysaccharide having anionic functional groups (such as carboxyl groups) can yield a polysaccharide derivative with especially good water solubility even if the modification rate by the modifying group (A) is high.

(3) Because the polysaccharide has functional groups that are acidic, basic or both (amphoteric) (that is, functional groups having charge), it can be reacted with a highly hydrophobic phenyl aldehyde or pyridyl aldehyde compound in one stage under mild and safe conditions to easily introduce the modifying group (A) into the polysaccharide.

(4) In certain embodiments, a stable polysaccharide derivative-drug conjugate can be obtained easily and in one pot under one-stage mild and highly safe reaction conditions. Because there is no need to modify the drug before conjugate formation, and since complex experimental equipment and catalysts are also unnecessary for conjugate formation, in-situ preparation of the polysaccharide derivative-drug conjugate from the polysaccharide derivative and the drug is also possible.

(5) In certain embodiments, the polysaccharide derivative and the conjugate of the polysaccharide derivative and the drug can be subjected to a crosslinking reaction with a crosslinking agent to form crosslinked structures in a variety of forms (such as tube structures, fiber structures, fibers, beads, gels, semispherical gels, capsules, sponges and sheets). Furthermore, in certain embodiments the polysaccharide derivative and the conjugate of the polysaccharide derivative and the drug can form crosslinked structures of various forms with amino group-containing polymers.

(Modifying group (A): group represented by formula (A))

In the formula (A), $R^1$ represents a hydrogen atom or $C_{1-4}$ alkyl. Preferably $R^1$ is a hydrogen atom or a methyl, ethyl, n-propyl, isopropyl, tert-butyl, sec-butyl, iso-butyl or n-butyl group, or more preferably a hydrogen atom or a methyl group. In one embodiment, $R^1$ is a hydrogen atom.

In the formula (A), * is a linkage with the polysaccharide.

In the formula (A), ring P is a phenyl ring or pyridine ring. This phenyl ring or pyridine ring is optionally substituted with one or more substituents independently selected from the halogen atoms (F, Cl, Br and/or I) and $-CF_3$, $-NO_2$, carboxyl and $-SO_3H$ groups.

In one embodiment, ring P is a phenyl ring, and this phenyl ring is optionally substituted with 1 to 4 substituents selected from the halogen atoms (F, Cl, Br and/or I) and - $CF_3$, $-NO_2$, carboxyl and $-SO_3H$ groups. In one embodiment, ring P is an unsubstituted phenyl ring.

In one embodiment, ring P is a pyridine ring, and the pyridine ring is optionally substituted with 1 to 3 substituents independently selected from the halogen atoms (F, Cl, Br and/or I) and $-CF_3$, $-NO_2$, carboxyl and $-SO_3H$ groups. In one embodiment, ring P is an unsubstituted pyridine ring.

In the formula (A), Y represents a divalent group selected from the group consisting of -NH-, -C(=O)-, -S-, -O-, the alkylenes and $-(CH_2CH_2O)_n-$ and any combinations of these, with n being an integer from 1 to 9 (preferably from 1 to 4, or more preferably from 1 to 2). From the standpoint of hydrophilicity, the carbon number of the alkylene group of Y is 1 to 6, or preferably 1 to 4, or more preferably 1 or 2, or still more preferably 1.

In certain embodiments, Y is selected from $-L^1-NH-$ or $-L^2-C(=O)$, in which case $L^1$ and $L^2$ bind to ring P. That is, the modifying group (A) is selected from the following formulae (A-1) and (A-2):

[C12]

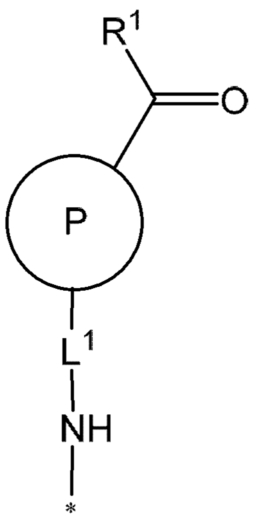

(A-1)

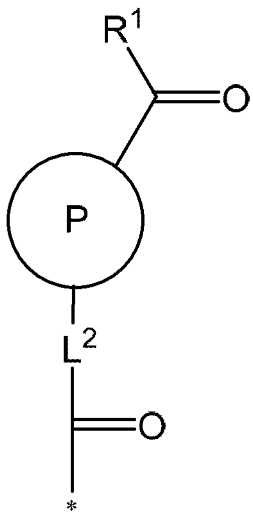

(A-2)

In the formulae (A-1) and (A-2), * represents a linkage with the polysaccharide. The definitions and preferred embodiments of $R^1$ and ring P in the formulae (A-1) and (A-2) are the same as the definitions and preferred embodiments of $R^1$ and ring P in the formula (A) above.

In formula (A-1), $L^1$ is a single bond or represents a divalent group selected from the group consisting of -C(=O)-, -S-, -O-, the alkylenes and $-(CH_2CH_2O)_n-$ and any combinations of these, and n is an integer from 1 to 9 (preferably from 1 to 4, or more preferably from 1 to 2). From the standpoint of hydrophilicity, the carbon number of the alkylene group of $L^1$ is preferably 1 to 6, or more preferably 1 or 4, or still more preferably 1 or 2, or yet more preferably 1.

In particular, $L^1$ is selected from a single bond, a $C_{1-6}$ alkylene, $-(CH_2CH_2O)_n-$, - $(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^1$ is preferably a $C_{1-4}$ alkylene, or more preferably a $C_{1-2}$ alkylene, or still more preferably methylene.

In one embodiment, $L^1$ is a single bond or a $C_{1-2}$ alkylene. In one embodiment, $L^1$ is a single bond.

In formula (A-2), $L^2$ is a single bond or represents a divalent group selected from the group consisting of -NH-,

-S-, -O-, the alkylenes and $-(CH_2CH_2O)_n-$ and any combinations of these, with n being an integer from 1 to 9 (preferably from 1 to 4, or more preferably from 1 to 2). From the standpoint of hydrophilicity, the carbon number of the alkylene group in $L^2$ is 1 to 6, or preferably 1 to 4, or more preferably 1 or 2, or still more preferably 1.

In particular, $L^2$ is selected from a single bond, a $C_{1-6}$ alkylene, $-(CH_2CH_2O)_n-$, $-(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^2$ is preferably a $C_{1-4}$ alkylene, or more preferably a $C_{1-2}$ alkylene, or still more preferably methylene.

In one embodiment, $L^2$ is a single bond or a $C_{1-2}$ alkylene. In one embodiment, $L^2$ is a single bond.

In certain embodiments, the modifying group (A) is a group represented by formula (A-1) above, ring P is a phenyl ring, and this phenyl ring is optionally substituted with 1 to 4 substituents independently selected from the halogen atoms (F, Cl, Br and/or I) and $-CF_3$, $-NO_2$, carboxyl and $-SO_3H$ groups.

For example, modifying group (A) is selected from the following formulae (1), (2) and (3) (sometimes called "formulae (1) to (3)" below):

[C13]

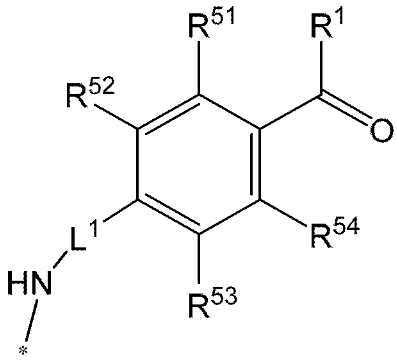

(1)

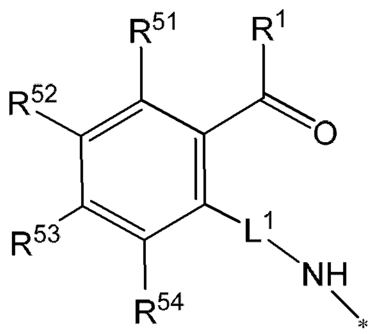

(2)

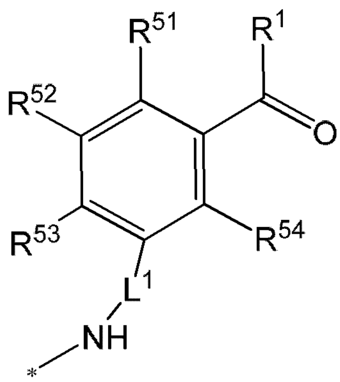

(3)

In formulae (1) to (3), * is a linkage with the polysaccharide. Furthermore, in formulae (1) to (3) the definitions and preferred embodiments of $R^1$ are the same as the definitions and preferred embodiments of $R^1$ in the formula (A), and the definitions and preferred embodiments of $L^1$ are the same as the definitions and preferred embodiments of $L^1$ in the formula (A-1).

In formulae (1) to (3) above, each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^{51}$, $R^{52}$, $R^{53}$ and $R^{54}$ are all hydrogen atoms. In one embodiment, each of $R^{51}$, $R^{52}$, $R^{53}$ and $R^{54}$ is independently selected from a hydrogen atom and a halogen atom (F, Cl, Br, or I; preferably F).

In specific embodiments, in formulae (1) to (3) above $R^1$ is a hydrogen atom, $L^1$ is a single bond, and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) above is a hydrogen atom, $L^1$ is a single bond, and $R^{51}$, $R^{52}$, $R^{53}$ and $R^{54}$ are all hydrogen atoms. In one embodiment, $R^1$ in formulae (1) to (3) above is a hydrogen atom, $L^1$ is a single bond, and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) $R^1$ is a methyl group, $L^1$ is a single bond, and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a methyl group, $L^1$ is a single bond, and $R^{51}$, $R^{52}$, $R^{53}$ and $R^{54}$ are all hydrogen atoms. In one embodiment, $R^1$ in formulae (1) to (3) is a methyl group, $L^1$ is a single bond, and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) above $R^1$ is a hydrogen atom, $L^1$ is a methylene ($-CH_2$) or ethylene ($-C_2H_4$), and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a hydrogen atom, $L^1$ is a methylene ($-CH_2$) or ethylene ($-C_2H_4$), and $R^{51}$, $R^{52}$, $R^{53}$ and $R^{54}$ are all hydrogen atoms. In one embodiment, $R^1$ in formulae (1) to (3) is a hydrogen atom, $L^1$ is a methylene ($-CH_2$) or ethylene ($-C_2H_4$), and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) $R^1$ is a methyl, $L^1$ is a methylene ($-CH_2$) or ethylene ($-C_2H_4$),

and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a methyl, $L^1$ is a methylene ($-CH_2$) or ethylene ($-C_2H_4$), and $R^{51}$, $R^{52}$, $R^{53}$ and $R^{54}$ are all hydrogen atoms. In one embodiment, $R^1$ in formulae (1) to (3) is a methyl, $L^1$ is a methylene ($-CH_2$) or ethylene ($-C_2H_4$), and each of $R^{51}$, $R^{52}$, $R^{53}$ and $R^{54}$ is independently a hydrogen atom or a halogen atom (F, Cl, Br, or I; preferably F).

In one embodiment, the modifying group (A) is selected from the groups represented by the following formulae:

[C14]

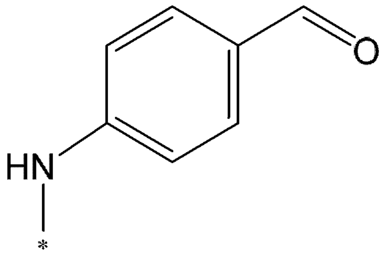

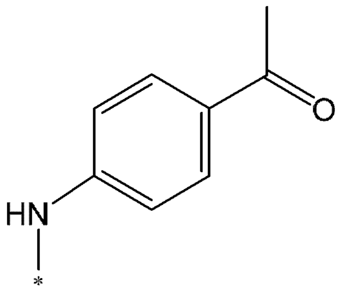

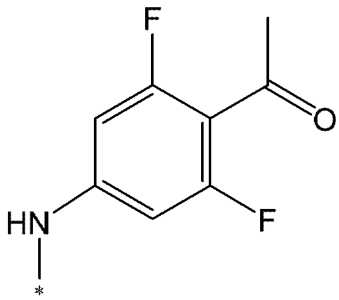

(in the formulae, * represents a linkage with the polysaccharide).

In one embodiment, the modifying group (A) is a group represented by the following formula:

[C15]

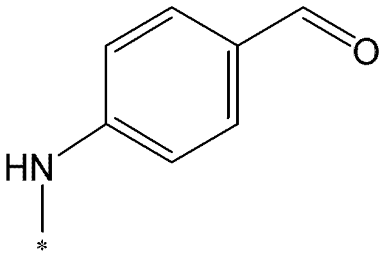

(in the formula, * represents a linkage with the polysaccharide).

In certain embodiments, the modifying group (A) is a group represented by formula (A-1) above, ring P is a pyridine ring, and the pyridine ring is optionally substituted with 1 to 3 substituents independently selected from the halogen atoms (F, Cl, Br and/or I) and $-CF_3$, $-NO_2$, carboxyl and $-SO_3H$ groups.

For example, the modifying group (A) is selected from the following formulae (4), (5), (6), (7), (8), (9), (10), (11), (12) and (13) (hereunder sometimes called "formulae (4) to (13)"):

[C16]

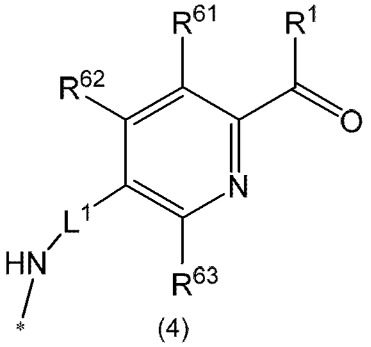
(4)

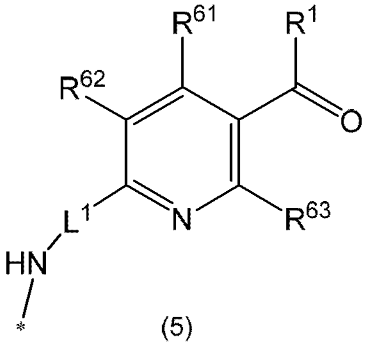
(5)

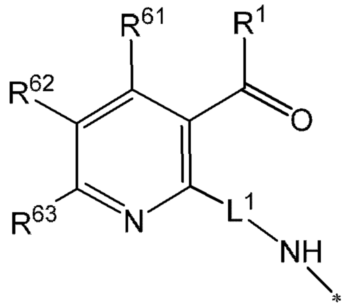
(6)

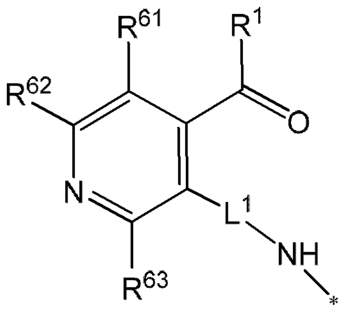
(7)

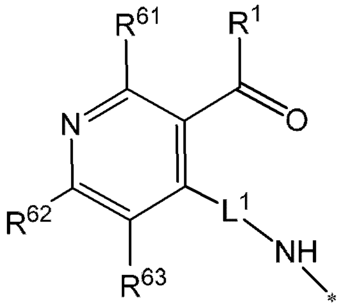
(8)

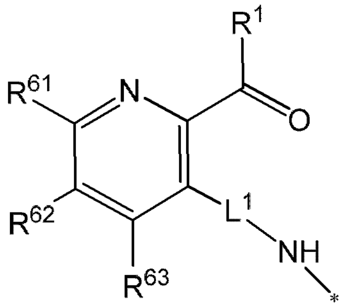
(9)

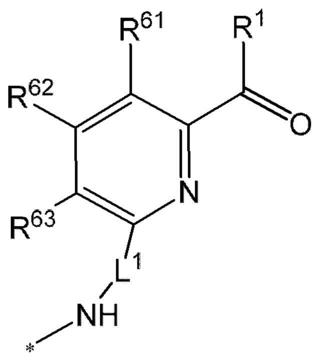
(10)

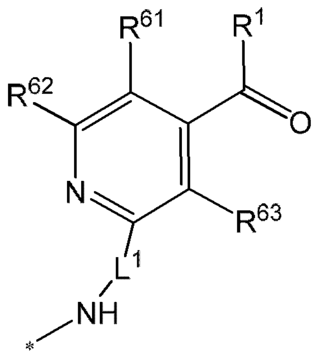
(11)

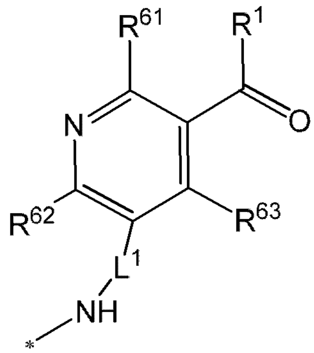
(12)

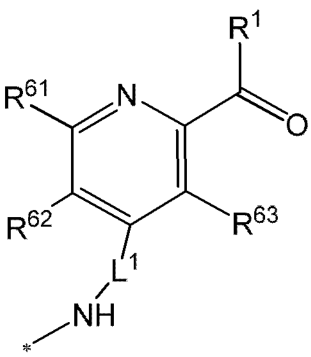
(13)

In formulae (4) to (13) above, * represents a linkage with the polysaccharide. The definition and preferred embodiments of $R^1$ in formulae (4) to (13) above are the same as the definition and preferred embodiments of $R^1$ in the formula (A), and the definition and preferred embodiments of $L^1$ are the same as the definition and preferred embodiments of $L^1$ in the formula (A-1).

In the formulae (4) to (13) above, each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^{61}$, $R^{62}$ and $R^{63}$ are all

hydrogen atoms. In one embodiment, each of $R^{61}$, $R^{62}$ and $R^{63}$ is independently selected from a hydrogen atom and a halogen atom (F, Cl, Br, I).

In specific embodiments, in the formulae (4) to (13) $R^1$ is a hydrogen atom, $L^1$ is a single bond, and each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^1$ in the formulae (4) to (13) is a hydrogen atom, $L^1$ is a single bond, and $R^{61}$, $R^{62}$ and $R^{63}$ are all hydrogen atoms. In one embodiment, $R^1$ in the formulae (4) to (13) is a hydrogen atom, $L^1$ is a single bond, and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a methyl group, $L^1$ is a single bond, and each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^1$ in the formulae (4) to (13) is a methyl group, $L^1$ is a single bond, and $R^{61}$, $R^{62}$ and $R^{63}$ are all hydrogen atoms. In one embodiment, $R^1$ in the formulae (4) to (13) is a methyl group, $L^1$ is a single bond, and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a hydrogen atom, $L^1$ is a methylene ($-CH_2$) or ethylene ($-C_2H_4$), and each of $R^{61}$, $R^{62}$ and $R^{63}$ is independently selected from a hydrogen atom, a halogen atom (F, Cl, Br, I), $-CF_3$, $-NO_2$, a carboxyl group and - $SO_3H$. In one embodiment, $R^1$ in the formulae (4) to (13) is a hydrogen atom, $L^1$ is a methylene ($-CH_2$) or ethylene ($-C_2H_4$), and $R^{61}$, $R^{62}$ and $R^{63}$ are all hydrogen atoms. In one embodiment, $R^1$ in the formulae (4) to (13) is a hydrogen atom, $L^1$ is a methylene ($-CH_2$) or ethylene ($-C_2H_4$), and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a methyl, $L^1$ is a methylene ($-CH_2$) or ethylene ($-C_2H_4$), and each of $R^{61}$, $R^{62}$ and $R^{63}$ is independently selected from a hydrogen atom, a halogen atom (F, Cl, Br, I), $-CF_3$, $-NO_2$, a carboxyl group and - $SO_3H$. In one embodiment, $R^1$ in the formulae (1) to (3) is a methyl, $L^1$ is a methylene ($-CH_2$) or ethylene ($-C_2H_4$), and $R^{61}$, $R^{62}$ and $R^{63}$ are all hydrogen atoms. In one embodiment, $R^1$ in the formulae (1) to (3) is a methyl, $L^1$ is a methylene ($-CH_2$) or ethylene ($-C_2H_4$), and each of $R^{61}$, $R^{62}$ and $R^{63}$ is independently a hydrogen atom or a halogen atom (F, Cl, Br, or I; preferably F).

In one embodiment, the modifying group (A) is selected from the groups represented by the following formulae:

[C17]

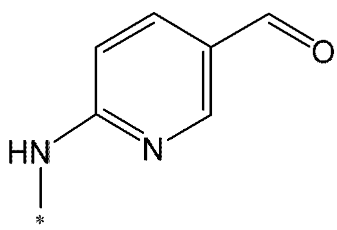

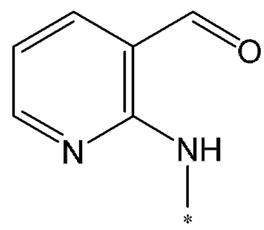

(in the formulae, * represents a linkage with the polysaccharide).

In certain embodiments, the modifying group (A) is a group represented by formula (A-2) above, ring P is a phenyl ring, and the phenyl ring is optionally substituted with 1 to 4 substituents independently selected from the halogen atoms (F, Cl, Br and/or I) and the $-CF_3$, $-NO_2$, carboxyl and $-SO_3H$ groups.

For example, the modifying group (A) is selected from the following formulae (14), (15) and (16) (hereunder sometimes called "formulae (14) to (16)"):

[C18]

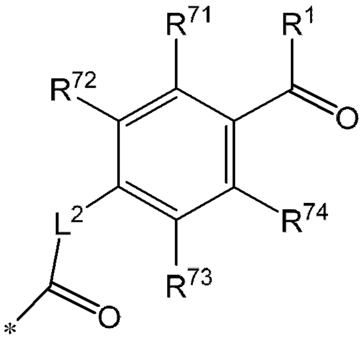

(14)

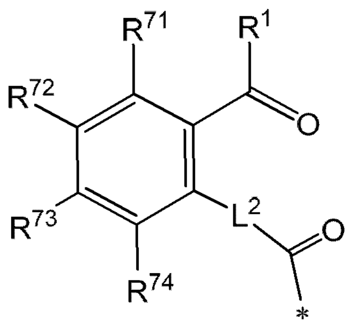

(15)

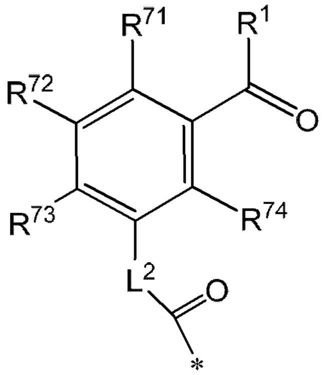

(16)

In formulae (14) to (16) above, * represents a linkage with the polysaccharide. The definition and preferred embodiments of $R^1$ in formulae (14) to (16) above are the same as the definition and preferred embodiments of $R^1$ in the formula (A), and the definition and preferred embodiments of $L^2$ are the same as the definition and preferred embodiments of $L^2$ in the formula (A-2).

In the formulae (1) to (3) above, each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^{71}$, $R^{72}$, $R^{73}$ and $R^{74}$ are all hydrogen atoms. In one embodiment, each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a hydrogen atom, $L^2$ is a single bond, and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a hydrogen atom, $L^2$ is a single bond, and $R^{71}$, $R^{72}$, $R^{73}$ and $R^{744}$ are all hydrogen atoms. In one embodiment, $R^1$ in the formulae (14) to (16) is a hydrogen atom, $L^2$ is a single bond, and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a methyl, $L^2$ is a single bond, and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a methyl, $L^2$ is a single bond, and $R^{71}$, $R^{72}$, $R^{73}$ and $R^{74}$ are all hydrogen atoms. In one embodiment, $R^1$ in the formulae (14) to (16) is a methyl, $L^2$ is a single bond, and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a hydrogen atom, $L^2$ is a methylene ($-CH_2$) or ethylene ($-C_2H_4$), and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a hydrogen atom, $L^2$ is a methylene ($-CH_2$) or ethylene ($-C_2H_4$), and $R^{71}$, $R^{72}$, $R^{73}$ and $R^{74}$ are all hydrogen atoms. In one embodiment, $R^1$ in the formulae (14) to (16) is a hydrogen atom, $L^2$ is a methylene ($-CH_2$) or ethylene ($-C_2H_4$), and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a methyl, $L^2$ is a methylene ($-CH_2$) or ethylene ($-C_2H_4$), and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a methyl, $L^2$ is a methylene ($-CH_2$) or ethylene ($-C_2H_4$), and $R^{71}$, $R^{72}$, $R^{73}$ and $R^{74}$ are all hydrogen atoms. In one embodiment, $R^1$ in the formulae (14) to (16) is a methyl, $L^2$ is a methylene ($-CH_2$) or ethylene ($-C_2H_4$), and each of $R^{71}$, $R^{72}$, $R^{73}$ and $R^{74}$ is independently selected from a hydrogen atom and a halogen atom (F, Cl, Br, or I; preferably F).

In one embodiment, the modifying group (A) is a group represented by the following formula:

[C19]

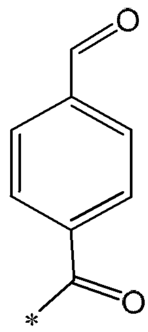

(in the formula * represents a linkage with the polysaccharide).

In certain embodiments, Y is selected from $-L^3-S-$ or $-L^4-$, in which case $L^3$ and $L^4$ are bound to ring P. That is, the modifying group (A) is selected from the following formulae (A-3) and (A-4):

[C20]

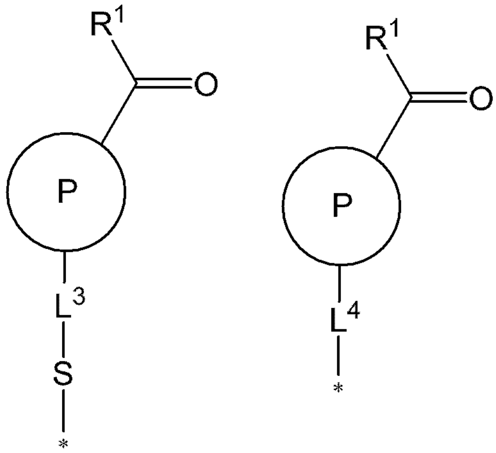

(A-3) (A-4)

In the formulae (A-3) and (A-4), * represents a linkage with the polysaccharide. Furthermore, the definitions and preferred embodiments of $R^1$ and ring P in the formulae (A-3) and (A-4) are the same as the definitions and preferred embodiments of $R^1$ and ring P in the formula (A) above.

In the formula (A-3), $L^3$ is a single bond or represents divalent group selected from the group consisting of -NH-, -C(=O)-, -O-, the alkylenes and $-(CH_2CH_2O)_n-$ and any combinations of these, with n being an integer from 1 to 9 (preferably 1 to 4, more preferably 1-2). From the standpoint of hydrophilicity, the carbon number of the alkylene group of $L^3$ is 1 to 6, or preferably 1 to 4, or more preferably 1 to 2, or still more preferably 1.

In particular, $L^3$ is selected from a single bond, the $C_{1-6}$ alkylenes, $-(CH_2CH_2O)_n-$, $-(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^3$ is preferably a $C_{1-4}$ alkylene, or more preferably a $C_{1-2}$ alkylene, or still more preferably methylene.

In one embodiment, $L^3$ is a single bond or $C_{1-2}$ alkylene. In one embodiment, $L^3$ is a single bond.

In the formula (A-4), $L^4$ is a single bond, or represents divalent group selected from the group consisting of -NH-, -C(=O)-, -S-, -O-, the alkylenes and $-(CH_2CH_2O)_n-$ and any combinations of these, with n being an integer from 1 to 9 (preferably 1 to 4, more preferably 1-2). From the standpoint of hydrophilicity, the carbon number of the alkylene group of $L^4$ is 1 to 6, or preferably 1 to 4, or more preferably 1 to 2, or still more preferably 1.

In particular, $L^4$ is selected from a single bond, the $C_{1-6}$ alkylenes, $-(CH_2CH_2O)_n-$, $-(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene of $L^4$ is preferably a $C_{1-4}$ alkylene, or more preferably a $C_{1-2}$ alkylene, or still more preferably methylene.

In one embodiment, $L^4$ is a $C_{1-2}$ alkylene.
The modifying group (A) has -C(=O)$R^1$ (aldehyde group or ketone group) in the head part, and this -C(=O)$R^1$ can form a Schiff base by reacting with a primary amino group. In the present invention, -C(=O)$R^1$ may also form a hyperconjugation structure with an adjacent phenyl ring or pyridine ring, which can stabilize the formed Schiff base and increase binding stability with the amino group.

(Polysaccharide)

The polysaccharide is not particularly limited as long as it is acidic, basic or amphoteric and permits introduction of the modifying group (A), and may be a polysaccharide that has been extracted and isolated from natural plant or animal sources, or a polysaccharide that has been genetically modified and produced in a microorganism, or a chemically synthesized polysaccharide.
An acidic polysaccharide is a polysaccharide having anionic functional groups (such as carboxyl groups, sulfate groups, phosphate groups or the like) in its structure. Examples of acidic polysaccharides include polysaccharides HAVING uronic acids (such as guluronic acid, mannuronic acid, glucuronic acid, iduronic acid, galacturonic acid, etc.), polysaccharides having sulfuric acid groups or phosphoric acid groups in part of their structures, and polysaccharides having both these kinds of structures. In addition to polysaccharides that intrinsically have anionic functional groups in their structures, acidic polysaccharides include polysaccharides (for example, neutral polysaccharides) that intrinsically have no anionic functional groups but have had anionic functional groups introduced by substituting anionic functional groups such as carboxyalkyl groups for the hydrogen atoms of some or all of the hydroxyl groups.
Specific examples of acidic polysaccharides include alginic acid, hyaluronic acid, carboxymethyl cellulose, carboxymethyl dextran, carboxymethyl starch, heparin, heparan sulfate, chondroitin sulfate, dermatan sulfate, regenerated oxidized cellulose, pectic acid, gellan gum, gum arabic, xanthan gum, agar, agaropectin, carrageenan, and derivatives thereof or salts of these.
A basic polysaccharide is a polysaccharide having cationic functional groups (such as amino groups) in its structure. In addition to polysaccharides that intrinsically have cationic functional groups in their structures, basic polysaccharides include polysaccharides obtained by introducing cationic functional groups into polysaccharides (for example, neutral polysaccharides) that intrinsically have no cationic functional groups.
Specific examples of basic polysaccharides include chitosan and its derivatives and salts of these.
An amphoteric polysaccharide is a polysaccharide having both anionic functional groups (such as carboxyl groups, sulfate groups and phosphate groups) and cationic functional groups (such as amino groups) in its structure. Specific examples of amphoteric polysaccharides include anion-modified basic polysaccharides such as succinate-modified chitosan; deacetylated polysaccharides such as deacetylated hyaluronic acid; amine denatured acidic polysaccharides such as cation-modified alginic acid and cation-modified hyaluronic acid; phosphocholine-modified polysaccharides such as phosphocholine-modified hyaluronic acid; carbobetaine-modified polysaccharides; and sulfobetaine-modified polysaccharides and the like.
Using an acidic, basic or amphoteric polysaccharide, it is possible to introduce the modifying group (A) at a good modification rate even when the modifying group (A) has a hydrophobic benzene ring or pyridyl ring. It is also possible to obtain a polysaccharide derivative with excellent water solubility even when a hydrophobic modifying group (A) is introduced at a high modification rate due to the presence of unmodified acidic or basic functional groups (anionic groups or cationic functional groups).
In certain embodiments, the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, chitosan, derivatives thereof or salts of these, regenerated oxidized cellulose, derivatives thereof or salts of these, and pectic acid, derivatives thereof or salts of these.
In the present invention, a polysaccharide derivative may be a polysaccharide which has been modified in any way. Examples include polysaccharides that have been modified by esterification, maleimide modification, thiol modification, acrylate modification, aldehyde modification, disulfide (such as pyridyl disulfide) modification, alkyne (including cyclic alkyne) modification, tetrazine modification, furan ring modification and the like. Methods of such modification are known to those skilled in the art, and the polysaccharide may be modified by a conventionally known method such as a carbodiimide reaction. For example, methods of maleimide modification are described in WO 2019/189330. The methods of Akira Takahashi et al., Biomacromolecules, 2013, (14)10, 3581-3588 for example may be used for alkyne (including cyclic alkyne) modification. For tetrazine modification, the methods described in Vianney Delplace et al., Nonswelling, Ultralow Content Inverse Electron-Demand Diels-Alder Hyaluronan Hydrogels with Tunable Gelation Time: Synthesis and In Vitro Evaluation, Advanced Functional Materials, 2020, 30(14) may be used for example. Methods of

furan ring modification include those described in Nimmo, Chelsea M. et al., Biomacromolecules, 2011, 12(3) 824-830 and RSC Adv., 2018, 8, 11036-11042 for example. By applying these modifications in combination with a copper free click reaction (such as a reaction between a cyclic alkyne and an azide), an inverse electron demand type Diels-Alder reaction (for example, a reaction between tetrazine and a cyclic alkene) or a Diels-Alder reaction (for example, a reaction between furan and maleimide), it is possible to introduce a second drug (an additional drug of a different kind) into the polysaccharide derivative or perform crosslinking.

In certain embodiments, the polysaccharide is an acidic polysaccharide. A polysaccharide having anionic functional groups can yield a polysaccharide derivative with particularly excellent water solubility even if the modification rate by the modifying group (A) is high.

In certain embodiments, the polysaccharide is a polysaccharide containing a carboxyl group. A polysaccharide containing a carboxyl group can form an amide bond by a reaction between the carboxyl group and an amino group, allowing the modifying group (A) to be introduced into the polysaccharide.

In specific embodiments, the polysaccharide is a polysaccharide having a carboxyl group, the group represented by the formula (A) is a group represented by the formula (A-1), and the group represented by the formula (A-1) is introduced into the polysaccharide by substitution for the -OH of the carboxyl group of the polysaccharide, forming an amide bond. A polysaccharide derivative with even greater stability can be obtained by forming an amide bond.

The polysaccharide having a carboxyl group may be any having at least one or more unmodified carboxyl groups.

The polysaccharide having a carboxyl group may be a polysaccharide having a carboxyl group in its structure, or a derivative thereof or a salt of these (for example, alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, pectic acid, derivatives thereof or salts of these, regenerated oxidized cellulose, derivatives thereof or salts of these, gellan gum, derivatives thereof or salts of these, gum arabic, derivatives thereof or salts of these, xanthan gum, derivatives thereof or salts of these, agarose, derivatives thereof or salts of these, and agaropectin, derivatives thereof or salts of these; or preferably alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, pectic acid, derivatives thereof or salts of these, and regenerated oxidized cellulose, derivatives thereof or salts of these) or else a polysaccharide having no carboxyl group (for example, chitosan, derivatives thereof or salts of these, curdlan, derivatives thereof or salts of these, agar, derivatives thereof or salts of these, carrageenan, derivatives thereof or salts of these, guar gum, derivatives thereof or salts of these, locust bean gum, derivatives thereof or salts of these, and tamarind seed gum, derivatives thereof or salts of these; or preferably chitosan, derivatives thereof or salts of these) into which a carboxyl group has been introduced by substitution of a carboxyalkyl group for the hydrogen atoms of some or all of the hydroxyl groups.

In specific embodiments, the polysaccharide having a carboxyl group is selected from alginic acid or its salts, hyaluronic acid or its salts, carboxymethyl cellulose or its salts, carboxymethyl dextran or its salts, carboxymethyl starch or its salts, heparin or its salts, heparan sulfate or its salts, chondroitin sulfate or its salts, dermatan sulfate or its salts, pectin or its salts, and regenerated oxidized cellulose or its salts.

In specific embodiments, the polysaccharide is selected from alginic acid, derivatives thereof or salts of these, hyaluronic acid, derivatives thereof or salts of these, carboxymethyl cellulose, derivatives thereof or salts of these, and carboxymethyl dextran, derivatives thereof or salts of these.

In specific embodiments, the polysaccharide is selected from alginic acid or its salts, hyaluronic acid or its salts, carboxymethyl cellulose or its salts, and carboxymethyl dextran or its salts.

In specific embodiments, the polysaccharide is alginic acid or a derivative thereof or a salt of these, or hyaluronic acid or a derivative thereof or a salt of these.

In specific embodiments, the polysaccharide is alginic acid or a salt thereof or hyaluronic acid or a salt of these.

(Alginate)

In one embodiment, the polysaccharide is alginic acid or a derivative thereof or a salt of these (hereunder also called an "alginate").

Alginic acid is a linear heteropolymer of two kinds of uronic acid, D-mannuronic acid (M) and L-guluronic acid (G). Specifically, it is a block copolymer comprising a homopolymer fraction of D-mannuronic acid (MM fraction), a homopolymer fraction of L-guluronic (GG fraction) and a fraction of randomly arranged D-mannuronic acid and L-guluronic acid (M/G fraction) in arbitrary combination. The ratio of D-mannuronic acid to L-guluronic acid in alginic acid (M/G ratio)

differs principally according to the type of seaweed or other organism from which it is derived, and may also be affected by the organism's habitat and season, with a wide range from high-G alginic acid (M/G ratio about 0.2) to high-M alginic (M/G ratio about 5)

A derivative of alginic acid is alginic acid with any modification, or a salt thereof, with no particular limitations. Any modifications to the alginic acid may include esterification, sulfation, maleimide modification, thiol modification, acrylate modification, aldehyde modification, disulfide (for example, pyridyl disulfide) modification and cation modification (for example, heparin-bound alginate) and the like. In one embodiment, the alginic acid derivative is an alginic acid ester, propylene glycol alginate, sulfated alginic acid in which the hydroxyl groups are sulfated, maleimide modified alginic acid, thiol modified alginic acid, acrylate modified alginic acid or cation modified alginic acid (such as the heparin-bound alginate described in Japanese Patent Application Publication No. 2001-233786). Such modification of alginic acid may be accomplished by known methods or analogous methods.

Salts of alginic acid or its derivatives include metal salts of alginic acid or its derivatives for example. For example, a hydrogen atom (hydrogen ion) of carboxylic acid at position 6 of alginic acid or its derivative may be ion substituted with a monovalent metal ion (for example, and alkali metal ion such as $Na^+$ or $K^+$) to create a water-soluble salt. Specific examples include sodium alginate, potassium alginate, sodium salts of alginic acid derivatives and potassium salts of alginic acid derivatives. In specific embodiments, the salt of alginic acid or its derivative is sodium alginate or a sodium salt of an alginic acid derivative. A solution of a monovalent metal salt of alginic acid or its derivative forms a gel when mixed with a crosslinking agent. Alternatively, a metal salt of alginic acid or its derivative may be a salt (crosslinked body) formed by ion substitution of a divalent metal ion (for example, an alkali earth metal ion such as $Mg^{2+}$ or $Ca^{2+}$) for a hydrogen atom (hydrogen ion) of carboxylic acid at position 6 of alginic acid or its derivative.

When originally extracted from brown algae, alginic acid has a high molecular weight and high viscosity, but the molecular weight falls and viscosity is reduced by processes such as heat drying, freeze drying and purification. Consequently, alginates with different molecular weights can be manufactured by appropriately controlling temperature at each stage of manufacture. An alginate with a high molecular weight can be obtained by controlling temperature at a low level at each stage of manufacture, while an alginate with a low molecular weight can be obtained by increasing the temperature. Alginates with different molecular weights can also be manufactured by methods such as appropriately selecting the type of brown algae used as a raw material or fractioning molecular weights during the manufacturing process. Furthermore, the molecular weights or viscosities of alginates produced by various methods can be measured, and separate lots of alginate with different molecular weights or viscosities can be mixed to obtain an alginate with the target molecular weight.

The viscosity of the alginate used in not particularly limited, but when measured in a 1 w/w% aqueous solution of the alginate, it is preferably from 10 mPa s to 1,000 mPa s, or more preferably from 50 mPa·s to 800 mPa·s.

The viscosity of the alginate in an aqueous solution can be measured by conventional methods. For example, it can be measured using by rotation viscometry using a coaxial double-cylinder rotational viscometer, a single-cylinder rotational viscometer (Brookfield viscometer), or a cone plate rotational viscometer. Preferably it is measured according to the viscosity measurement methods of the Japanese Pharmacopoeia (16th Edition). More preferably, a cone plate viscometer is used.

The position where the modifying group (A) is introduced into the alginate is not particularly limited, but preferably it is introduced at the position of the 6-position carboxyl groups of the D-mannuronic acid (M) and L-guluronic acid (G) (uronic acid) constituting the alginate.

In specific embodiments, the group represented by the formula (A-1) is introduced into the polysaccharide by substitution for the -OH of a carboxyl group of alginic acid.

In one embodiment, the polysaccharide derivative contains constituent units represent by formula (c11) and/or formula (c12) below:

[C21]

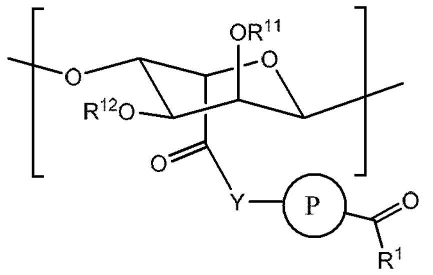

(c11)

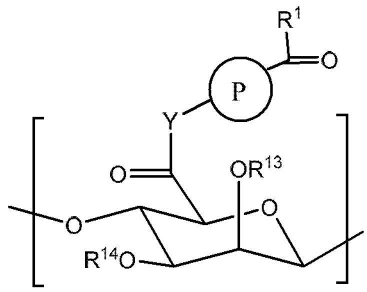

(c12)

In the formulae (c11) and (c12), the definitions and preferred embodiments of $R^1$ and ring P are the same as the definitions and preferred embodiments of $R^1$ and ring P in the formula (A).

In the formulae (c11) and (c12), Y represents -$L^1$-NH-, in which case $L^1$ is bound to the ring P. The definitions and preferred embodiments of $L^1$ are the same as the definitions and preferred embodiments of $L^1$ in the formula (A-1).

In the formulae (c11) and (c12), each of $R^{11}$, $R^{12}$, $R^{13}$ and $R^{14}$ is independently selected from a hydrogen atom, a $C_{1-6}$ alkyl and a -C(=O)-$C_{1-6}$ alkyl. In one embodiment, $R^{11}$, $R^{12}$, $R^{13}$ and $R^{14}$ are all hydrogen atoms.

(Hyaluronate)

In one embodiment, the polysaccharide is hyaluronic acid or a derivative thereof or a salt of these (hereunder also called a "hyaluronate").

Hyaluronic acid is a biodegradable and biocompatible polymer having a repeating structure consisting of linear chains of two sugars, D-glucuronic acid and N-acetyl-D-glucosamine.

A derivative of hyaluronic acid is hyaluronic acid with any modification, or a salt thereof, with no particular limitations. Any modifications to the alginic acid may include esterification, sulfation, maleimide modification, thiol modification, acrylate modification, aldehyde modification and disulfide (for example, pyridyl disulfide) modification. In one embodiment, the hyaluronic acid derivative is a hyaluronic acid ester, propylene glycol hyaluronate, sulfated hyaluronic acid in which the hydroxyl groups are sulfated, maleimide modified hyaluronic acid, thiol modified hyaluronic acid or acrylate modified hyaluronic acid. Such modification of hyaluronic acid may be accomplished by known methods or analogous methods.

Salts of hyaluronic acid or its derivatives include metal salts of hyaluronic acid or its derivatives for example. For example, a hydrogen atom (hydrogen ion) of carboxylic acid at position 6 of hyaluronic acid or its derivative may be ion substituted with a monovalent metal ion (for example, and alkali metal ion such as $Na^+$ or $K^+$) to create a water-soluble salt. Specific examples include sodium hyaluronate, potassium hyaluronate, sodium salts of hyaluronic acid derivatives and potassium salts of hyaluronic acid derivatives and the like. In specific embodiments, the salt of hyaluronic acid or its derivative is sodium hyaluronate or a sodium salt of a hyaluronic acid derivative. A solution of a monovalent metal salt of hyaluronic acid or its derivative forms a gel when mixed with a crosslinking agent. Alternatively, a metal salt of hyaluronic acid or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid at position 6 of hyaluronic acid or its derivative.

The position where the modifying group (A) is introduced into the hyaluronate is not particularly limited, but preferably it is introduced at the position of a carboxyl group of the D-glucuronic acid constituting the hyaluronic acid.

In specific embodiments, the group represented by the formula (A-1) is introduced into the polysaccharide by substitution for the -OH of a carboxyl group of hyaluronic acid.

In one embodiment, the polysaccharide derivative contains constituent units represent by formula (c13) below:

[C22]

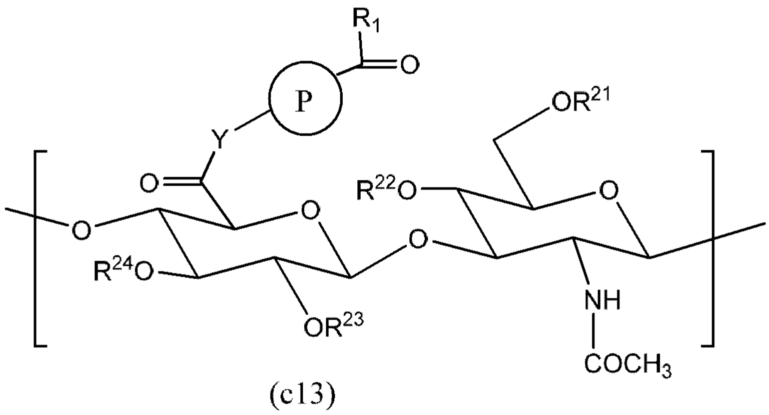

(c13)

In formula (c13), the definitions and preferred embodiments of $R^1$ and ring P are the same as the definitions and preferred embodiments of $R^1$ and ring P in the formula (A).
In formula (c13), Y represents $-L^1-NH-$, in which case $L^1$ is bound to the ring P. The definitions and preferred embodiments of $L^1$ are the same as the definitions and preferred embodiments of $L^1$ in formula (A-1).
In formula (c13), each of $R^{21}$, $R^{22}$, $R^{23}$ and $R^{24}$ is independently selected from a hydrogen atom, a $C_{1-6}$ alkyl and a $-C(=O)-C_{1-6}$ alkyl. In one embodiment, $R^{21}$, $R^{22}$, $R^{23}$ and $R^{24}$ are all hydrogen atoms.

(Carboxymethyl cellulose)

In one embodiment, the polysaccharide is carboxymethyl cellulose or a derivative thereof or a salt of these (hereunder called "the carboxymethyl cellulose").
Carboxymethyl cellulose is a derivative of cellulose that has been solubilized by introducing carboxymethyl groups into cellulose, and has excellent thickening, water absorption and water retention properties.
A derivative of carboxymethyl cellulose is carboxymethyl cellulose with any modification, or a salt thereof, with no particular limitations. Any modifications to the carboxymethyl cellulose may include esterification, sulfation, maleimide modification, thiol modification, acrylate modification, aldehyde modification and disulfide (for example, pyridyl disulfide) modification. Such modification of carboxymethyl cellulose may be accomplished by known methods or analogous methods.
Salts of carboxymethyl cellulose or its derivatives include metal salts of carboxymethyl cellulose or its derivatives for example. For example, a hydrogen atom (hydrogen ion) of carboxylic acid contained in carboxymethyl cellulose or its derivative may be ion substituted with a monovalent metal ion (for example, and alkali metal ion such as $Na^+$ or $K^+$) to create a water-soluble salt. Specific examples include sodium carboxymethyl cellulose, potassium carboxymethyl cellulose, sodium salts of carboxymethyl cellulose derivatives and potassium salts of carboxymethyl cellulose derivatives. In specific embodiments, the salt of carboxymethyl cellulose or its derivative is sodium carboxymethyl cellulose or a sodium salt of a carboxymethyl cellulose derivative. A solution of a monovalent metal salt of carboxymethyl cellulose or its derivative forms a gel when mixed with a crosslinking agent. Alternatively, a metal salt of carboxymethyl cellulose or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid contained in the carboxymethyl cellulose or its derivative.
The position where the modifying group (A) is introduced into the carboxymethyl cellulose is not particularly limited, but preferably it is introduced at the position of a carboxyl group contained in the carboxymethyl cellulose.
In specific embodiments, the group represented by the formula (A-1) is introduced into the polysaccharide by substitution for the -OH of a carboxyl group of carboxymethyl cellulose.
In one embodiment, the polysaccharide derivative contains constituent units represent by formula (c14) below:

[C23]

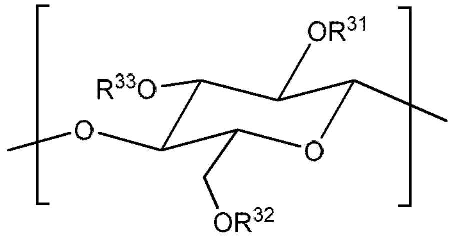

(c14)

In formula (c14), 1 to 3 of $R^{31}$, $R^{32}$ and $R^{33}$ are groups represented by formula (i) below:

[C24]

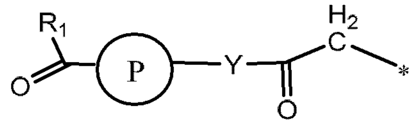

(i)

In the formula (i), the definitions and preferred embodiments of $R^1$ and ring P are the same as the definitions and preferred embodiments of $R^1$ and ring P in the formula (A).

In the formula (i), Y represents $-L^1-NH-$, in which case $L^1$ is bound to the ring P. The definition and preferred embodiments of $L^1$ are the same as the definition and preferred embodiments of $L^1$ in the formula (A-1).

The rest of $R^{31}$, $R^{32}$ and $R^{33}$ (substituents not represented by formula (i)) are each independently selected from a hydrogen atom, a $C_{1-6}$ alkyl, a $-C(=O)-C_{1-6}$ alkyl and - $CH_2COOH$, and preferably are selected from a hydrogen atom and $-CH_2COOH$, or more preferably are hydrogen atoms.

In one embodiment, $R^{31}$ is a group represented by formula (i) above, and $R^{32}$ and $R^{33}$ are hydrogen atoms.

(Carboxymethyl dextran)

In one embodiment, the polysaccharide is carboxymethyl dextran or a derivative thereof or a salt of these (hereunder called "the carboxymethyl dextran").

Carboxymethyl dextran is a carboxymethyl ether of dextran.

A derivative of carboxymethyl dextran is carboxymethyl dextran with any modification, or a salt thereof, with no particular limitations. Any modifications to the carboxymethyl dextran may include esterification, sulfation, maleimide modification, thiol modification, acrylate modification, aldehyde modification and disulfide (for example, pyridyl disulfide) modification. Such modification of carboxymethyl dextran may be accomplished by known methods or analogous methods.

Salts of carboxymethyl dextran or its derivatives include metal salts of carboxymethyl dextran or its derivatives for example. For example, a hydrogen atom (hydrogen ion) of carboxylic acid contained in carboxymethyl dextran or its derivative may be ion substituted with a monovalent metal ion (for example, and alkali metal ion such as $Na^+$ or $K^+$) to create a water-soluble salt. Specific examples include sodium carboxymethyl dextran, potassium carboxymethyl dextran, and sodium salts of carboxymethyl dextran derivatives and potassium salts of carboxymethyl dextran derivatives. In specific embodiments, the salt of carboxymethyl dextran or its derivative is a sodium salt of carboxymethyl dextran or a carboxymethyl dextran derivative. A solution of a monovalent metal salt of carboxymethyl dextran or its derivative forms a gel when mixed with a crosslinking agent. Alternatively, a metal salt of carboxymethyl dextran or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid contained in the carboxymethyl dextran or its derivative.

The position where the modifying group (A) is introduced into the carboxymethyl dextran is not particularly limited, but preferably it is introduced at the position of a carboxyl group contained in the carboxymethyl dextran.

In specific embodiments, the group represented by the formula (A-1) is introduced into the polysaccharide by substitution for the -OH of a carboxyl group of the carboxymethyl dextran.

In one embodiment, the polysaccharide derivative contains constituent units represent by formula (c15) below:

[C25]

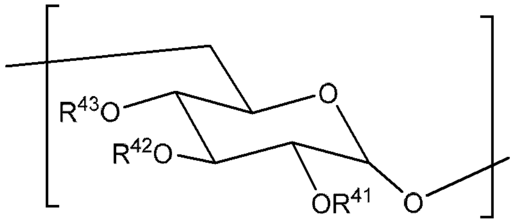

(c15)

In formula (c15), 1 to 3 of $R^{41}$, $R^{42}$ and $R^{43}$ are groups represented by formula (i) below:

[C26]

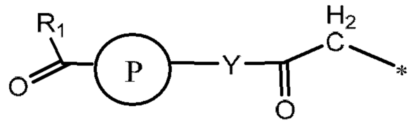

(i)

In formula (i), the definitions and preferred embodiments of $R^1$ and ring P are the same as the definitions and preferred embodiments of $R^1$ and ring P in the formula (A).

In formula (i), Y represents $-L^1$-NH-, in which case $L^1$ is bound to the ring P. The definitions and preferred embodiments of $L^1$ are the same as the definitions and preferred embodiments of $L^1$ in the formula (A-1).

The rest of $R^{41}$, $R^{42}$ and $R^{43}$ (substituents not represented by formula (i)) are each independently selected from a hydrogen atom, a $C_{1-6}$ alkyl, a -C(=O)-$C_{1-6}$ alkyl and - $CH_2COOH$, and preferably are selected from a hydrogen atom and -$CH_2COOH$, or more preferably are hydrogen atoms.

In one embodiment, $R^{41}$ is a group represented by formula (i) above, and $R^{42}$ and $R^{43}$ are hydrogen atoms.

The polysaccharide may also be heparin or a derivative thereof or a salt of these, heparan sulfate or a derivative thereof or a salt of these, chondroitin sulfate or a derivative thereof or a salt of these, dermatan sulfate or a derivative thereof or a salt of these, regenerated oxidized cellulose or a derivative thereof or a salt of these, or pectic acid or a derivative thereof or a salt of these. In one embodiment of the polysaccharide derivative, the polysaccharide is heparin or a derivative thereof or a salt of these, heparan sulfate or a derivative thereof or a salt of these, chondroitin sulfate or a derivative thereof or a salt of these, dermatan sulfate or a derivative thereof or a salt of these, regenerated oxidized cellulose or a derivative thereof or a salt of these or pectic acid or a derivative thereof or a salt of these, and the group represented by the formula (A-1) is introduced by substitution for the -OH of a carboxyl group contained in the polysaccharide.

In certain embodiments, the polysaccharide is a polysaccharide containing an amino group. A polysaccharide containing an amino group can form an amide bond by a reaction between the amino group and a carboxyl group, allowing the modifying group (A) to be introduced into the polysaccharide.

In specific embodiments, the polysaccharide is a polysaccharide containing an amino group, the group represented by the formula (A) is a group represented by the formula (A-2), and the group represented by the formula (A-2) is introduced into the polysaccharide by substitution for a hydrogen atom of the amino groups of the polysaccharide, forming an amide bond. A polysaccharide derivative with even greater stability can be obtained by forming an amide bond.

The polysaccharide having an amino group may be a polysaccharide that intrinsically has an amino group in its structure, or a derivative thereof or a salt of these (for example, chitosan or a derivative thereof or a salt of these), or a polysaccharide obtained by introducing an amino group into part of a polysaccharide having no amino groups.

The following is an example of a polysaccharide derivative obtained by introducing a group represented by the formula (A-2) into a polysaccharide having an amino group:

[C27]

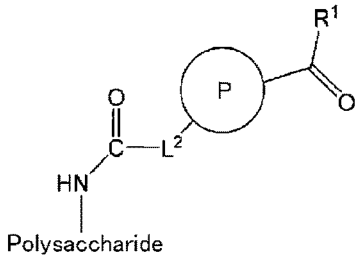

In the formula above, the definitions and preferred embodiments of $R^1$ and ring P are the same as the definitions and preferred embodiments of $R^1$ and ring P in the formula (A).

In the formula above, moreover, the definition and preferred embodiments of $L^2$ are the same as the definition and preferred embodiments of $L^2$ in the formula (A-2).

(Chitosan)

The polysaccharide may also be chitosan or a derivative thereof or a salt of these (hereunder also called "the chitosan"). Chitosan is a polysaccharide composed of D-glucosamine units and N-acetyl-D-glucosamine units bound by beta-(1-4) glycoside bonds. Chitosan can be obtained by partial deacetylation of the polysaccharide chitin. The degree of deacetylation is not particularly limited. In certain embodiments, the chitosan is deacetylated to a degree exceeding about 50% (typically exceeding about 75%).

The chitosan derivative is not particularly limited, and may be chitosan with any modification, or a salt thereof. Any modifications to the chitosan may include esterification, sulfation, maleimide modification, thiol modification, acrylate modification, aldehyde modification and disulfide (for example, pyridyl disulfide) modification. Examples of chitosan derivatives include carboxymethyl chitosan, hydroxybutyl chitin, N-acyl chitosan, O-acyl chitosan, N-alkyl chitosan, O-alkyl chitosan, N-alkylidene chitosan, O-sulfonyl chitosan, sulfated chitosan, phosphorylated chitosan, nitrated chitosan, alkali chitosan, and metal chelates of chitosan and the like. Such modification of chitosan may be accomplished by known methods or analogous methods. A commercial product may also be used.

Chitosan has a primary amino group. In one embodiment of the polysaccharide derivative, the polysaccharide is chitosan or its derivative or a salt of these, and the group represented by the formula (A-2) is introduced by substitution for a hydrogen atom of the amino group of the polysaccharide.

The position where the modifying group (A) is introduced into the chitosan is not particularly limited, but preferably it is introduced at the position of an amino group of the D-glucosamine units constituting the chitosan. In specific embodiments, a group represented by the formula (A-2) is introduced into the polysaccharide by substitution for a hydrogen atom of the amino group of chitosan.

In one embodiment, the polysaccharide derivative contains constituent units represented by the following formula (c16):

[C28]

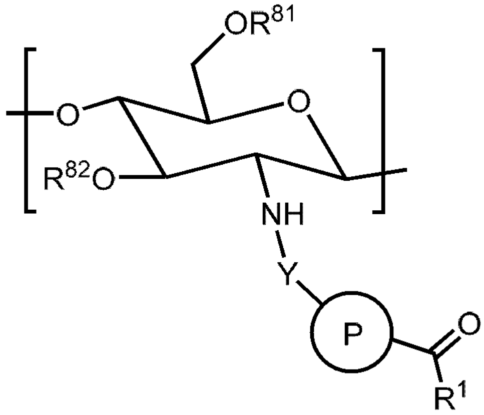

(c16)

In the formula (c16), the definitions and preferred embodiments of $R^1$ and ring P are the same as the definitions and preferred embodiments of $R^1$ and ring P in the formula (A).

In the formula (c16), Y represents $-L^2-C(=O)-$, in which case $L^2$ binds to the ring P. The definition and preferred embodiments of $L^2$ are the same as the definition and preferred embodiments of $L^2$ in the formula (A-2).

In formula (c16), each of $R^{81}$ and $R^{82}$ is independently selected from a hydrogen atom, a $C_{1-6}$ alkyl and a $-C(=O)-C_{1-6}$ alkyl. In one embodiment, $R^{81}$ and $R^{82}$ are both hydrogen atoms.

In specific embodiments, the polysaccharide is a polysaccharide having an ethylenic double bond group. A polysaccharide having an ethylenic double bond group can form a sulfide bond (-S-) by a reaction between the ethylenic double bond group and a thiol group (-SH), thereby allowing the modifying group (A) to be introduced into the polysaccharide. In specific embodiments, the polysaccharide is a polysaccharide containing an ethylenic double bond group, the group represented by the formula (A) is a group represented by the formula (A-3), and the group represented by the formula (A) is introduced into the polysaccharide by reacting with the ethylenic double bond group of the polysaccharide.

The polysaccharide having an ethylenic double bond is not particularly limited, but examples include polysaccharides (such as alginic acid, hyaluronic acid, carboxymethyl cellulose, carboxymethyl dextran, carboxymethyl starch, pectin, regenerated oxidized cellulose and chitosan) that have been modified by maleimide modification or acrylate modification, or salts of these.

For example, the following is an example of a polysaccharide derivative obtained by introducing a group represented by the formula (A-3) into a maleimide modified polysaccharide:

[C29]

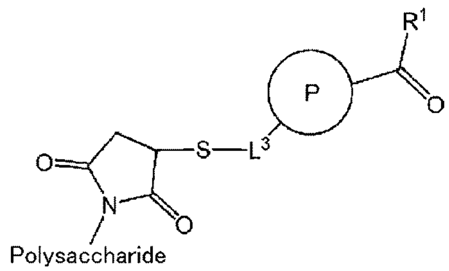

In the above formula, the definitions and preferred embodiments of $R^1$ and ring P are the same as the definitions and preferred embodiments of $R^1$ and ring P in the formula (A).

In the above formula, furthermore, the definition and preferred embodiments of $L^3$ are the same as the definition and preferred embodiments of $L^3$ in the formula (A-3).

The polysaccharide derivative may also have a structure in which the modifying group (A) is introduced at the position of a hydroxyl group contained in the polysaccharide.

In specific embodiments, the group represented by the formula (A-4) is introduced by substitution for the

hydrogen atom of the hydroxyl group contained in the polysaccharide. In this embodiment, as shown below, an ether bonds is formed in the polysaccharide derivative by an oxygen atom derived from a hydroxyl group contained in the polysaccharide.

[C30]

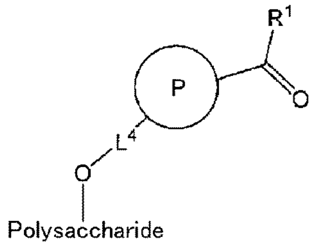

(Molecular weight and viscosity of polysaccharide derivative)

High-molecular-weight polysaccharides generally have a weight-average molecular weight in the range of 1,000 to 10,000,000, although the weight-average molecular weight is often difficult to determine exactly.

For example, for the molecular weight of a polysaccharide derivative, the weight-average molecular weight (Mw) as measured by gel filtration chromatography (GFC) is preferably from 1,000 to 5,000,000, or more preferably from 1,000 to 3,000,000.

The preferred range of the molecular weight of a polysaccharide derivative differs depending on the type of polysaccharide making up the polysaccharide derivative (presence or absence of degradation enzymes, inflammatory response or the like) and the use of the polysaccharide derivative.

For example, for DDS applications this is partly affected by the charge of the polysaccharide derivative, but considering that the kidney excretion limit is said to be 40,000 to 50,000, in general the weight-average molecular weight (Mw) of a polysaccharide derivative composed of a (non-biodegradable) polysaccharide having no in vivo degradation enzyme (such as dextran, cellulose derivatives, alginic acid and salts thereof and derivatives of these) is preferably in the range of 1,000 to 50,000, or more preferably in the range of 1,000 to 40,000.

However, since alginic acid has no in vivo degradation enzyme but also very little immunogenicity, a higher weight-average molecular weight (such as Mw 1,000 to 5,000,000, or 1,000 to 2,000,000) is possible in this case.

Because degradation enzymes for hyaluronic acid are abundant in the body, high weight-average molecular weights (such as up to 2,000,000, or up to 5,000,000 Mw) are possible, and at least 500,000 Mw is desirable because low-molecular-weight hyaluronic acid (around 100,000 Mw) is known to cause inflammation.

A measurement error of 10 to 20% or more is normal when the molecular weight of a high-molecular-weight polysaccharide is calculated by gel filtration chromatography. For example, the value may vary in the range of 320,000 to 480,000 in the case of a molecular weight of 400,000, or in the range of 400,000 to 600,000 in the case of 500,000, or in the range of 800,000 to 1,200,000 in the case of 1,000,000. Consequently, in the case of a polysaccharide the preferred weight-average molecular weight range is at least 1,000. Because manufacture is more difficult and the viscosity of an aqueous solution increases if the weight is too high, making it difficult to maintain the properties during long-term storage among other problems, the weight-average molecular weight is preferably not more than 5,000,000, or preferably not more than 3,000,000.

The weight-average molecular weight of the polysaccharide derivative with the introduced modifying group (A) is higher than the molecular weight of the polysaccharide before introduction of the modifying group (A) due to introduction of the modifying group (A). A polysaccharide derivative with the desired molecular weight can be obtained by selecting a polysaccharide with an appropriate molecular weight as the raw material.

Because in general naturally derived polymeric substances do not have a single molecular weight, but are aggregates of molecules with various molecular weights, the molecular weight is measured as a molecular weight distribution with a certain width. Gel filtration chromatography is a typical measurement method. Typical information about molecular weight distribution obtained from gel filtration chromatography includes the weight-average molecular weight (Mw), number-average molecular weight (Mn) and variance ratio (Mw/Mn).

The weight-average molecular weight is emphasized as a contributor to the average molecular weight of molecules with high molecular weights, and is represented by the following formula.

$$Mw = \Sigma(WiMi)/W = \Sigma(HiMi)/ \Sigma(Hi)$$

The number-average molecular weight is calculated by dividing the total weight of the macromolecules by the number of macromolecules.

$$Mn = W/ \Sigma Ni = \Sigma(MiNi)/ \Sigma Ni = \Sigma(Hi)/ \Sigma(Hi/Mi)$$

In the formulae, W is the total weight of the macromolecules, Wi is the weight of the i-th macromolecule, Mi is the molecular weight at the i-th elution time, Ni is the number of molecular weights Mi, and Hi is the height at the i-th elution time.

In measuring the molecular weights of naturally derived high-molecular-weight substances, the values are known to vary depending on the measurement method (for hyaluronic acid: Chikako Yomota et al., Bull. Natl. Health Sci., Vol. 117, pp 135-139 (1999), Chikako Yomota et al., Bull. Natl. Inst. Health Sci., Vol. 121, pp 30-33 (2003)). Published methods for measuring the molecular weights of alginate salts include methods of calculation from intrinsic viscosity and methods of calculation by SEC-MALLS (Size Exclusion Chromatography with Multiple Angle Laser Light Scattering Detection) as described in ASTM F2064-00 (2006), ASTM International Pub.. This publication recommends using a Multi Angle Light Scattering (MALS) detection device in combination with a calibration curve using pullulan as a standard substance (SEC-MALS measurement) when measuring molecular weight by size exclusion chromatography (gel filtration chromatography). SEC-MALS is described in ASTM F2065-16. Molecular weights obtained by SEC-MALS are also sometimes used as standard values in catalogues of alginate salts (FMC Biopolymer Co., PRONOVA™ sodium alginates catalogue).

In the present invention, unless otherwise specified, the molecular weight of a polysaccharide derivative is the weight-average molecular weight as calculated by gel filtration chromatography.

Typical conditions for gel filtration chromatography include the use of a calibration curve based on pullulan as a standard substance. The molecular weights of the pullulan used as a standard substance preferably include at least 1,600,000, 788,000, 404,000, 212,000 and 112,000. In addition, the eluent (200 mM sodium nitrate solution), column conditions and the like may also be specified. For the column conditions, it is desirable to use a polymethacrylate resin filler with at least 1 to 3 columns with exclusion limit molecular weights of at least 10,000,000. Typical columns include TSKgel GMPW$_{x1}$ (diameter 7.8 mm × 300 mm) and G2500 PW$_{XL}$ (diameter 7.8 mm × 300 mm) (manufactured by Tosoh Corp.).

(Modification rate)

The modification rate indicates the number of modifying groups (A) relative to constituent monosaccharide units contained in the polysaccharide derivative.

[Math. 1]

Modification rate = Number of modifying groups (A) contained in polysaccharide derivative/number of monosaccharide units contained in polysaccharide derivative

The modification rate by the modifying group (A) in the polysaccharide derivative is not particularly limited, and may be adjusted according to the purpose. For example, the modification rate by the modifying group (A) in the polysaccharide derivative can be adjusted to the desired range by adjusting the type of modifying group (A), the number of functional groups reacting with the modifying group (A) in the polysaccharide, and the reaction conditions when introducing the modifying group (A) and the like.

The modification rate by the modifying group (A) in the polysaccharide derivative may be for example in the range of from 0.01 to 1, from 0.02 to 0.6, from 0.05 to 0.2 or from 0.1 to 0.2. Such a range of modification rates may be useful for example when the polysaccharide derivative is used as a drug delivery carrier.

In another embodiment, the modification rate by the modifying group (A) in the polysaccharide derivative may be in the range of from 0.1 to 1, or from 0.1 to 0.8, or from 0.15 to 0.6 for example. Such a range of modification rates may be useful when the polysaccharide derivative is used in hydrogel form or the like.

In another embodiment, the modification rate by the modifying group (A) in the polysaccharide derivative may

be in the range of from 0.00001 to 0.01 for example.

When the polysaccharide is alginic acid for example, because it has one carboxyl group per monosaccharide unit, the modification rate by the modifying group (A) in the polysaccharide derivative when the modifying group (A) is introduced into the carboxyl groups is a maximum of 1, and may be in the range of from 0.01 to 1, or from 0.01 to 0.6, or from 0.05 to 0.2, or from 0.00001 to 0.01 for example.

When the polysaccharide is hyaluronic acid for example, because hyaluronic acid is composed of a monosaccharide unit (D-glucuronic acid) having one carboxyl group and a monosaccharide unit (N-acetyl-D-glucosamine) having no carboxyl group, the modification rate by the modifying group (A) in the polysaccharide derivative when the modifying group (A) is introduced into the carboxyl groups is a maximum of 0.5, and may be in the range of from 0.01 to 0.5, or from 0.05 to 0.2, or from 0.00001 to 0.01 for example.

When the polysaccharide is carboxymethyl cellulose for example, because the monosaccharide units have from 1 to 3 carboxyl groups, the modification rate by the modifying group (A) in the polysaccharide derivative when the modifying group (A) is introduced into the carboxyl groups is a maximum of 3 (carboxyl substitution at all 3 positions), and may be in the range of from 0.01 to 3, or from 0.05 to 1.5, or from 0.05 to 1, or from 0.00001 to 0.01 for example.

When the polysaccharide is carboxymethyl dextran for example, because the monosaccharide units have from 1 to 3 carboxyl groups, the modification rate by the modifying group (A) in the polysaccharide derivative when the modifying group (A) is introduced into the carboxyl groups is a maximum of 3 (carboxyl substitution at all 3 positions), and may be in the range of from 0.01 to 3, or from 0.05 to 1.5, or from 0.05 to 1, or from 0.00001 to 0.01 for example.

The modification rate can be calculated by $^1H$ NMR measurement ($D_2O$).

The modification rate can also be calculated by a colorimetric aldehyde assay.

In some cases, the modification rates calculated by $^1H$ NMR measurement and colorimetric aldehyde assay will not match exactly and there may be a difference between the two, but in the present invention it is sufficient that the modification rate as calculated by either $^1H$ NMR measurement or colorimetric aldehyde assay be within the above range.

A spacer may also be used when introducing the modifying group (A). The spacer may be one commonly used in the field (such as those described in Greg T. Hermanson, Bioconjugate Techniques, Third Edition (2013)), and specific examples include polyethylene glycol, polyamides and other peptides, and hydrocarbons.

2. Method for manufacturing polysaccharide derivative

The method for manufacturing the polysaccharide derivative is not particularly limited, but the polysaccharide derivative can be manufactured by a method that includes reacting a compound (a) represented by the following formula (a) with a polysaccharide having a functional group capable of reacting with the -X group of the compound (a). This yields a polysaccharide derivative (C) comprising a modifying group (A) corresponding to the compound (a) (a group represented by the formula (A) as explained above) introduced into a polysaccharide.

[C31]

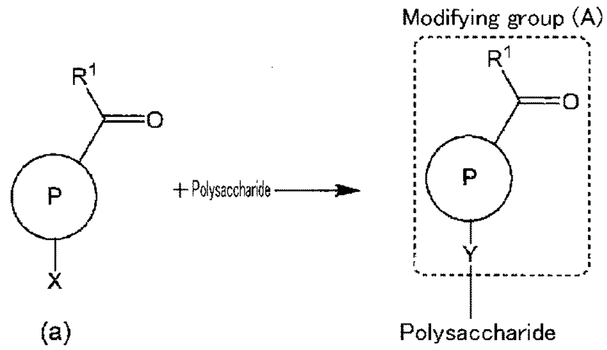


(a) (C)

In formula (a) above, X is selected according to the Y of the formula (C).

When Y is $-L^1-NH-$ (and $L^1$ binds to ring P), X is $-L^1-NH_2$.

When Y is $-L^2-C(=O)-$ (and $L^2$ binds to ring P), X is $-L^2-C(=O)OH$.

When Y is $-L^3-S-$ (and $L^3$ binds to ring P), X is $-L^3-SH$.

When Y is $L^4-$ (and $L^4$ binds to ring P), X is $-L^4-R^L$, in which $R^L$ is selected from the halogen atoms (F, Cl, Br or I).

The definitions and preferred embodiments of the ring P and $R^1$ in the formulae (a) and (C) above and Y in the formula (C) are the same as the definitions and preferred embodiments in the formula (A) and the formulae (A-1), (A-2), (A-3) and (A-4).

In the manufacturing method of this embodiment, because the polysaccharide has a functional group that is acidic, basic or both (amphoteric) (that is, a functional group having charge), the modifying group (A) can be introduced into the polysaccharide by a one-stage reaction performed under mild and highly safe conditions with the compound (a) represented by formula (a), which has a highly hydrophobic phenyl or pyridyl ring.

In specific embodiments, the method for manufacturing the polysaccharide derivative represented by formula (C) includes reacting an acidic, basic or amphoteric polysaccharide with a compound (a) represented by the formula (a) by adding the compound (a) in an aqueous solvent, a polar solvent or a higher alcohol to a solution containing the polysaccharide.

The modifying group (A) can be introduced into the polysaccharide at a high modification rate by means of such a reaction.

The method for manufacturing the polysaccharide derivative is explained below using an example in which Y is $-L^1-NH-$ (and $L^1$ binds to ring P) and X is $-L^1-NH_2$. Even if Y is not $-L^1-NH-$, a polysaccharide derivative represented by the formula (C) can still be manufactured by methods known in the literature.

One aspect of the present invention relates to a method for manufacturing a polysaccharide derivative represented by formula (C1) below.

The polysaccharide derivative represented by formula (C1) can be obtained by a condensation reaction between the carboxyl groups of the polysaccharide and the amino groups of the compound (a1) represented by the formula (a1) below. This reaction forms amide bonds between the amino groups of the compound (a1) and the carboxyl groups of the polysaccharide, yielding a polysaccharide derivative represented by formula (C1) below comprising a modifying group (A) corresponding to the compound (a1) (group represented by formula (A-1) as explained above) introduced into the polysaccharide.

[C32]

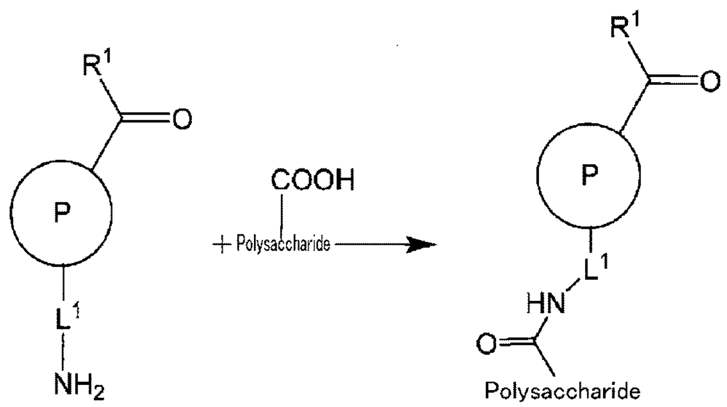


(a1) (C1)

The definitions and preferred embodiments of the ring P and $R^1$ in the formulae (a1) and (C1) above and $L^1$ in the formula (C1) are the same as the definitions and preferred embodiments in the formula (A) and formula (A-1).

The method of the condensation reaction is not particularly limited, and it can be accomplished by methods known in the literature, such as for example the methods described in "Experimental Chemistry Course 5th Edition 16, Synthesis of Organic Compounds IV, Carboxylic Acids and Derivatives, Esters, pp 35-70, Acid Amides and Acid Imides, pp 118-154, Amino Acids and Peptides, pp 258-283, 2007, Maruzen" and the like. The reaction is normally performed in a solvent.

In specific embodiments, the method for manufacturing the polysaccharide derivative represented by formula (C 1) includes performing a condensation reaction in a solvent (such as an aqueous solvent) between a polysaccharide having carboxyl groups and a compound (a1) represented by the formula (a1) below.

In one embodiment, the compound (a1) is dissolved or dispersed in an aqueous solvent, a polar solvent or a higher alcohol solvent and then added to a solution containing the polysaccharide and reacted with the polysaccharide. The modifying group (A) can thus be introduced into the polysaccharide at a high modification rate.

Because the compound (a1) represented by the formula (a1) generally has low solubility in aqueous solvents, a reaction can be accomplished with the compound (a1) in a suspended state by adding the compound (a1) to an aqueous solvent containing the polysaccharide and reacting it with the polysaccharide under stirring.

In one embodiment, the compound (a1) is dissolved in a polar solvent (preferably DMSO) or a higher alcohol solvent, and the resulting solution of the compound (a1) is added to an aqueous solvent containing the polysaccharide. The modification rate by the compound (a1) can be increased in this way.

It is surprising that the modification rate could be dramatically improved by the simple method of dissolving or dispersing the compound (a1) in a specific solvent and then adding it to the polysaccharide. The exact reason why the modification rate was improved is unknown, but the inventors hypothesize that because there is an excess of carboxyl groups relative to the compound (a1), the poorly soluble (hydrophobic) compound (a1) is consumed in the reaction in such an environment, promoting dissolution of subsequent compound (a1) and leading to an improved modification rate. In addition, it is thought that the modification rate is also improved because the reaction between the carboxyl groups of the polysaccharide and the amino groups of the compound (a) is more efficient than the reaction (Schiff base forming reaction) between the amino groups of the compound (a) and the aldehyde groups of the compound (a). However, the manufacturing method of this embodiment is not dependent on this mechanism.

Examples of polar solvents include N,N-dimethylformamide, dimethylsulfoxide (DMSO), dioxane, N-methyl-2-pyrrolidone (NMP) and the like.

Examples of aqueous solvents include water and mixed solvents of water with solvents selected from ether solvents such as tetrahydrofuran (THF) and 1,4-dioxane, alcohol solvents such as methanol, ethanol and 2-propanol, and polar solvents such as N,N-dimethylformamide, dimethylsulfoxide (DMSO), dioxane, N-methyl-2-pyrrolidone (NMP) and the like to the extent that the polysaccharide does not precipitate.

The condensation reaction between the compound (a1) and the polysaccharide is preferably performed under conditions of pH 5 to 10 (more preferably pH 5.5 to 8.5, or still more preferably pH 7.5 to 8.0). The modification rate by the modifying group (A) can be further improved by promoting a reaction while adjusting the pH within the above range. pH adjustment is preferably performed using a sodium hydroxide aqueous solution or a hydrochloric acid aqueous solution. A buffer may also be used in the reaction.

The condensation reaction between the compound (a1) and the polysaccharide is preferably performed in the presence of a condensing agent selected from 1,3-dicyclohexyl carbodiimide (DCC), 1-ethyl-3-(3-dimethylaminopropyl) carbodiimide hydrochloride (WSC·HCl), benzotriazole-1-yloxytris(dimethylamino) phosphonium hexafluorophosphate (BOP reagent), bis(2-oxo-3-oxazolidinyl) phosphinic chloride (BOP-Cl), 2-chloro-1,3-dimethylimidazolinium hexafluorophosphate (CIP), 4-(4,6-dimethoxy-1,3,5-triazine-2-yl)-4-methylmorpholinium chloride (DMT-MM) or the like, with or without an inorganic base such as sodium hydrogen carbonate or sodium carbonate or an organic base such as triethylamine or pyridine.

Such a condensation reaction may be performed at from 0°C to 50°C for example. A preferred temperature range is from 15°C to 40°C. By performing the reaction at a temperature close to room temperature, it is possible to shorten the reaction time and prevent a decline in the molecular weight of the polysaccharide. Side reactions (Schiff base formation reactions) between the amino groups of the compound (a) and the aldehyde groups of the compound (a) can also be suppressed by using such a temperature range.

An additive such as 1-hydroxy-1H-benzotriazole (HOBt) or 1-hydroxy-7-azabenzotriazole (HOAt) may also be added to suppress production of by-products in the condensation reaction.

Following the reaction, purification treatment may be performed by filtration and/or dialysis.

One embodiment of the present invention relates to a method for manufacturing the polysaccharide derivative represented by the following formula (C2). In certain embodiments, the method for manufacturing the polysaccharide derivative represented by formula (C2) includes performing a condensation reaction between a polysaccharide having amino groups and a compound (a2) represented by the following formula (a2).

The polysaccharide derivative represented by the formula (C2) can be obtained by performing a condensation reaction between the amino groups of the polysaccharide and the carboxyl groups of the compound (a2) represented by the formula (a2). This reaction forms amide bonds between the carboxyl groups of the compound (a2) and the amino groups of the polysaccharide, thereby yielding a polysaccharide derivative represented by the formula (C2) comprising a modifying group (A) corresponding to the compound (a2) (group represented by formula (A-2) as explained above) introduced into the polysaccharide.

[C33]

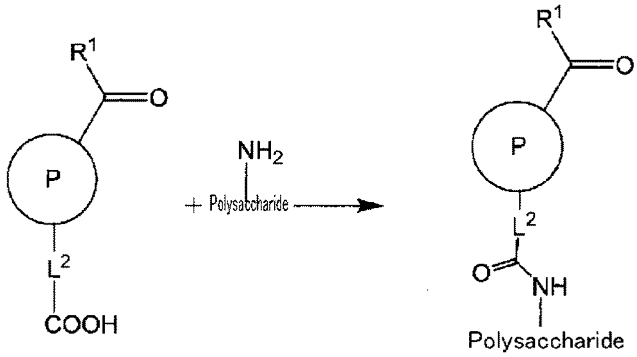

(a2) (C2)

The definitions and preferred embodiments of the ring P and $R^1$ in the formulae (a2) and (C2) above and $L^2$ in the formula (C2) are the same as the definitions and preferred embodiments in the formula (A) and formula (A-2).

The method of the condensation reaction is not particularly limited, and it can be accomplished by a conventional known method such as a method using a carbodiimide condensation agent.

The condensation reaction between the compound (a2) and the polysaccharide is preferably performed under conditions of pH 4 to 7 (more preferably pH 5 to 6). By performing a reaction with the pH adjusted within this range, it is possible to suppress precipitation of the reaction product and efficiently promote the reaction. pH adjustment is preferably performed using a sodium hydroxide aqueous solution or a hydrochloric acid aqueous solution. A buffer may also be used in the reaction.

Moreover, the ratio (molar ratio) of the carboxyl groups of the compound (a2) to the amino groups of the polysaccharide is preferably from 0.8:1 to 1.2:1 in the reaction between the compound (a2) and the polysaccharide. In such cases, it is possible to suppress precipitation of the reaction product and efficiently promote the reaction.

3. Polysaccharide derivative-drug conjugate

One embodiment of the present invention relates to a polysaccharide derivative-drug conjugate between a drug containing a primary amino group and a polysaccharide derivative of the above aspect. The polysaccharide derivative is capable of a conjugate by forming a Schiff base with a drug having a primary amino group. Specifically, an aldehyde group or ketone group ($-CR^1(=O)$) of the polysaccharide derivative reacts with the primary amino group of the drug to form a Schiff base.

Consequently, in the polysaccharide derivative-drug conjugate of this embodiment the drug and the group represented by formula (A) in the polysaccharide (modifying group (A)) are covalently bonded via a Schiff base, forming the structure represented by formula (D) below.

[C34]

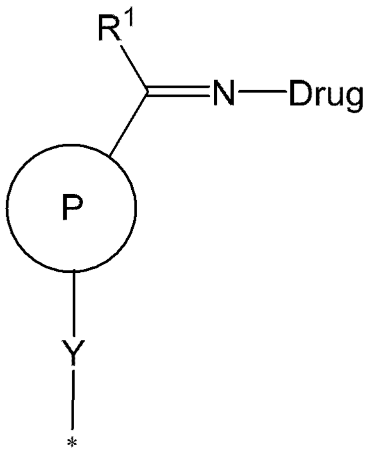

(D)

In the formula (D), "Drug" represents the part of the drug excluding the primary amino group.

In the formula (D), the definitions and preferred embodiments of $R^1$, ring P and Y are the same as the definitions

and preferred embodiments of $R^1$ and ring P in the formula (A).

(Drug having primary amino group)

The drug is not particularly limited as long as it has one or more primary amino groups in the molecule. The drug may be either a synthetic product or a natural product. Examples include low-molecular-weight compounds, middle-molecular-weight compounds, peptides, nucleic acids, nucleic acid derivatives, aptamers, vitamins, monoamines, amino acids, polyamines, antibodies, fluorescent dyes, contrast agents and the like.
The primary amino group may also be a hydrazide group ($-C(=O)-NH-NH_2$). A hydrazide group can react with an aldehyde group or ketone group ($-CR^1(=O)$) of the polysaccharide derivative to form a hydrazone bond (-C(=O)-NH-N=CH-). A hydrazone bond is normally more stable than a Schiff base, so slower sustained release can be expected.
Examples of low-molecular-weight compounds (molecular weight up to 500 for example) and middle-molecular-weight compounds (molecular weight 500 to 2,000 for example) having primary amino groups include doxorubicin, gemcitabine, aminosalicylic acid, pemetrexed, methotrexate, alendronate, eribulin, memantine, remdesivir, ampicillin, amoxicillin, aztreonam, tigemonam, vancomycin, cephalosporin C, gentamycin, trimethoprim, sulfamethoxazole, oseltamivir, mexiletine, midodrine, levodopa, tenofovir, darunavir, procaine, ethyl aminobenzoate, procainamide, fluvoxamine, milnacipran, baclofen, benserazide, carbidopa, droxidopa, gusperimus, ubenimex, fingolimod, amfenac, sulfamine, triamterene, mexiletine, amlodipine, azelnidipine, methyldopa, hydralazine, mosapride, sitagliptin phosphate hydrate, valacyclovir, acyclovir, celecoxib and the like.
The peptide is not particularly limited as long as it has a primary amino group at the N-end or the like. Examples of peptides include methionine enkephalin, leucine enkephalin, dynorphin A, beta-endorphin, bacitracin, daptomycin, colistin, elcatonin, oxytocin and the like.
The nucleic acid bases adenine, thymine, guanine and cytosine have primary amino groups, so nucleic acids and nucleic acid derivatives containing these nucleic acid bases have primary amino groups and can be used in the present invention. Examples of nucleic acid derivatives include cytarabine, cladribine, fludarabine and the like.
Nucleic acid (DNA, RNA) aptamers and peptide aptamers can be used as aptamers.
Examples of vitamins having primary amino groups include folic acid, vitamin B 1 (thiamine), vitamin B6 (pyridoxamine), vitamin B12 (cyanocobalamin), nicotinamide and the like.
Examples of monoamines having primary amino groups include dopamine, noradrenaline, serotonin, histamine, thiamine, octopamine and the like.
Examples of amino acids include various amino acids having primary amino groups in the molecule. The amino acid may be a natural amino acid or an artificial amino acid such as tranexamic acid.
Examples of polyamines include molecules existing in vivo that have multiple primary amines in the molecule, such as spermine, spermidine and putrescine.
Examples of fluorescent dyes include fluorescein-5-thiosemicarbazide (FTSC) and the like.
One drug alone or a mixture of two or more may be used.
The drug may be water soluble or water insoluble.
As long as it has a primary amino group, the drug may be in the form of a salt or in the form of a hydrate or solvate.

(Drug introduction rate)

The drug introduction rate of the modifying group (A) in the polysaccharide derivative-drug conjugate is not particularly limited, but is for example the ratio of the modifying groups (A) introduced into the polysaccharide derivative which have formed bonds (Schiff bases) with the drug, represented as a percentage. The drug introduction rate is not particularly limited, but may be from 1% to 100% for example.

(Sustained release properties)

The Schiff bases formed between the aldehyde or ketone groups ($-CR^1(=O)$) of the polysaccharide derivative and the primary amino groups of the drug exist stably under neutral to basic pH conditions, but are dissociated under low pH conditions, releasing the drug.
In this Description, "low pH conditions" mean conditions of less than pH 7, or typically pH 3.5 to 7.0.
Although some drug release does occur in neutral to alkaline (high pH) environments (such as pH 7.4 or more), this release is gradual. Under low pH conditions, on the other hand, drug release occurs rapidly, and a large quantity of the drug is released.
While the physiological pH in blood and the like is about pH 7.4, inflamed tissue and tumor tissue (pH 6.5 to 7.2) and the interior of lysosomes and endosomes (pH 4.5 to 5.5) are known as low pH environments. Since the polysaccharide derivative-drug conjugate of this embodiment can release drugs in response to low pH, it is capable for

example of suppressing drug release and maintaining a drug stably at pH 7.4 (corresponding to blood pH), and efficiently releasing the drug at target sites with low pH environments.

[C35]

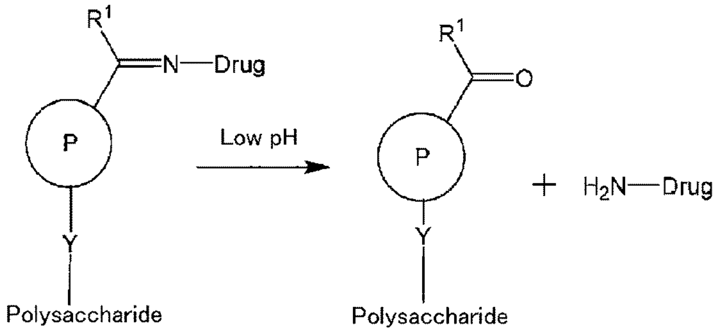


In the formula above, "Drug" represents the part of the drug apart from the primary amino group.

The definitions and preferred embodiments of $R^1$, ring P and Y in the formula above are the same as the definitions and preferred embodiments of $R^1$ and ring P in the formula (A).

(Manufacturing method)

Another aspect of the present invention relates to a method for manufacturing a polysaccharide derivative-drug conjugate. The manufacturing method of this embodiment includes mixing the polysaccharide derivative described above with a drug containing a primary amino group in a solvent. Mixing yields a polysaccharide derivative-drug conjugate by forming Schiff bases by a reaction between the aldehyde or ketone groups ($-CR^1(=O)$) of the polysaccharide derivative and the amino groups of the drug.

The manufacturing method of this embodiment can easily yield a stable polysaccharide derivative drug-conjugate in one pot under one-stage mild and highly stable reaction conditions. Because there is no need to modify the drug before conjugate formation, and since complex experimental equipment and catalysts are also unnecessary for conjugate formation in the manufacturing method of this embodiment, in-situ preparation of the polysaccharide derivative-drug conjugate from the polysaccharide derivative and the drug is also possible.

The solvent is not particularly limited, and one capable of dissolving polysaccharides may be selected appropriately. For example, an aqueous solvent or a polar solvent such as dimethylsulfoxide (DMSO) is preferred for its excellent ability to dissolve polysaccharides.

Examples of aqueous solvents include water and mixed solvents of water with solvents selected from ether solvents such as tetrahydrofuran (THF) and 1,4-dioxane, alcohol solvents such as methanol, ethanol and 2-propanol, and polar solvents such as N,N-dimethylformamide, dimethylsulfoxide (DMSO), and the like to the extent that the polysaccharide does not precipitate.

The temperature during mixing is also not particularly limited, and may be in the range of from 0°C to 50°C for example. A temperature range of from 15°C to 40°C is preferred. It is possible to both achieve an appropriate reaction speed and prevent reduced portion loss by performing the reaction at a temperature near room temperature.

The pH in the reaction between the polysaccharide derivative and the drug may be any at which the drug dissolves but does not denature, and from the standpoint of the reaction speed, conditions of pH 4.0 to 11.0 (more preferably pH 6.0 to 9.0, or still more preferably pH 7.0 to 8.0) are preferred. By appropriately adjusting the pH while allowing the reaction to proceed, it is possible to further improve the introduction efficiency of the drug into the modifying group (A). pH adjustment is preferably performed used hydrochloric acid or sodium hydroxide.

The reaction between the polysaccharide derivative and the drug is also preferably performed under shaded conditions if the drug has poor photostability.

The reaction time is not particularly limited, but is about 1 minute to 24 hours, or preferably 10 minutes to two hours.

The mixing ratio of the polysaccharide derivative and the drug is set appropriately according to the number of modifying groups (A) introduced into the polysaccharide derivative, the desired drug introduction rate, and the solubility of the resulting polysaccharide derivative drug-conjugate. For example, the drug may be mixed at a rate of 0.001 to 10 moles, or preferably 0.05 to 3 moles, or more preferably 0.1 to 1 mole per 1 mole of the introduced modifying groups (A) in the polysaccharide derivative.

The method for mixing the polysaccharide derivative and the drug is not particularly limited as long as both components can be mixed uniformly.

The polysaccharide derivative-drug conjugate (reaction liquid) obtained by mixing may be used as is without purification. After conjugate formation, it may be subjected to purification treatment such as dialysis, salting out, gel filtration, ion exchange chromatography or electrophoresis, and may also be freeze dried.

4. Composition

Another aspect of the present invention relates to a composition containing a polysaccharide derivative of the above aspect or a polysaccharide derivative-drug conjugate of the above aspect.

Certain embodiments provide a composition containing a polysaccharide derivative of the above aspect, or a polysaccharide derivative-drug conjugate of the above aspect, together with an aqueous solvent. The weight ratio of the polysaccharide derivative or conjugate and the aqueous solvent in the composition is from 0.0001:1 to 0.3:1 for example, or preferably from 0.001:1 to 0.1:1.

A composition containing a polysaccharide derivative of the above aspect together with a drug having a primary amino group is provided in certain embodiments. The molar ratio of the polysaccharide derivative and the drug in this composition is for example a ratio (A:drug) of from 1:0.001 to 1:10, or preferably from 1:0.05 to 1:3, or more preferably from 1:0.1 to 1:1.

Certain embodiments provide a composition containing a polysaccharide derivative of the above aspect, a drug having a primary amino group, and an aqueous solvent. The ratio of the total weight of the polysaccharide derivative and the drug to the weight of the aqueous solvent in this composition is for example from 0.0001:1 to 0.3:1, or preferably from 0.001:1 to 0.1:1. The molar ratio of the polysaccharide derivative and the drug is for example a ratio (A:drug) of 1:0.001 to 1:10, or preferably from 1:0.05 to 1:3, or more preferably from 1:0.1 to 1:1.

Certain embodiments provide a composition containing the crosslinked structure of the polysaccharide derivative described below together with an aqueous solvent. The weight ratio of the crosslinked structure to the aqueous solvent in this composition is for example from 0.0001:1 to 0.3:1, or preferably from 0.001:1 to 0.1:1.

Certain embodiments provide a composition containing the crosslinked structure of the polysaccharide derivative-drug conjugate described below (crosslinked structure-drug conjugate) together with an aqueous solvent. The weight ratio of the crosslinked structure to the aqueous solvent in this composition is for example from 0.0001:1 to 0.3:1, or preferably from 0.001:1 to 0.1:1.

The aqueous solvent may be similar to those mentioned in the context of the polysaccharide derivative manufacturing method and conjugate manufacturing method.

The polysaccharide derivative or polysaccharide derivative-drug conjugate of the present invention may be made into a medical composition together with generally known medical carriers and diluents and other additives, and administered either orally or non-orally to non-mammals or mammals including humans.

Examples of mammals are not limited but include humans, chimpanzees, apes, monkeys, cows, horses, sheep, goats, pigs, rabbits, dogs, cats, rats, mice, guinea pigs and the like. Examples of non-mammals are not limited but include birds, fish and insects.

One embodiment provides a medical composition containing a polysaccharide derivative of the above aspect, a drug, and pharmaceutically acceptable additives.

One embodiment provides a medical composition containing a polysaccharide derivative-drug conjugate of the above aspect together with pharmaceutically acceptable additives.

Examples of the pharmaceutically acceptable additives include excipients, fillers, bulking agents, binders, humectants, disintegrants, lubricants, surfactants, dispersants, buffers, preservatives, solubilizers, antiseptics, flavoring agents, analgesics, stabilizers and isotonic agents and the like commonly used in drug manufacture. These additives may be selected appropriately and used to prepare the drug composition by ordinary methods.

The polysaccharide derivative or polysaccharide derivative-drug conjugate of the present invention may be compounded into a food composition together with additives commonly used in the food sector (for example, at least one ingredient selected from the various nutritional supplements (amino acids, vitamins, minerals, etc.), sugars, milk products, sweeteners, flavorings, fragrances, antioxidants, preservatives, colorings, emulsifiers, pH adjusters, organic acids, buffers, fruit juices and the like).

5. Crosslinked structure

One aspect of the present invention relates to a crosslinked structure containing a polysaccharide derivative.

(1) Crosslinked structure of polysaccharide derivative crosslinked by crosslinking agent

In certain aspects, the crosslinked structure is a crosslinked structure of a polysaccharide derivative or a crosslinked structure of a polysaccharide derivative-drug conjugate (also called a "crosslinked structure-drug conjugate"). The crosslinked structure of a polysaccharide derivative is one in which the polysaccharide derivative is crosslinked via crosslinking groups to form a three-dimensional network structure. The crosslinked structure of a polysaccharide derivative-drug conjugate (crosslinked structure-drug conjugate) is one in which the polysaccharide derivative-drug conjugate is crosslinked via crosslinking groups to form a three-dimensional network structure. The crosslinked structure of the polysaccharide derivative can be obtained by performing a crosslinking reaction using a crosslinking agent on a polysaccharide derivative having crosslinking groups. The crosslinked structure of a polysaccharide derivative-drug conjugate (crosslinked structure-drug conjugate) can typically be obtained by a method of performing a crosslinking reaction with a crosslinking agent on a polysaccharide derivative-drug conjugate having crosslinking groups, or by a method of first manufacturing a crosslinked structure of a polysaccharide derivative, and then binding the crosslinked structure to a drug via Schiff bases.

For example, unmodified carboxyl groups may function as crosslinking groups to form a crosslinked structure via a crosslinking agent such as a divalent metal ion. A composition containing a polysaccharide derivative or polysaccharide derivative-drug conjugate containing unmodified carboxyl groups can be made into a crosslinked structure by reacting it in a solution containing a crosslinking agent.

The forms of these crosslinked structures are not particularly limited, but examples include tube structures, fibrous structures, fibers, beads, gels, semispherical gels, capsules, sponges, sheets, films and the like.

Specific embodiments provide a gel, capsule, sponge, bead, fiber, tube, sheet or film containing a polysaccharide derivative, polysaccharide derivative-drug conjugate, crosslinked structure or crosslinked structure-drug conjugate of the above aspects.

Specifically, a solution containing a polysaccharide derivative having unmodified carboxyl groups can be added dropwise or the like to a solution containing a divalent metal ion as a crosslinking agent, and partially crosslinked to obtain a crosslinked structure. Similarly, a solution containing a polysaccharide derivative-drug conjugate having unmodified carboxyl groups can be added dropwise or the like to a solution containing a divalent metal ion as a crosslinking agent, and partially crosslinked to obtain a crosslinked structure.

A crosslinked structure manufactured by such methods may be in the form of a capsule, bead, fiber, tube or film for example.

Alternatively, a solution containing a polysaccharide derivative having unmodified carboxyl groups and a solution containing a divalent metal ion as a crosslinking agent can each be applied to a base material to partially crosslink the polysaccharide derivative and obtain a crosslinked structure. A crosslinked structure manufactured by such methods may be in the form of a gel (hydrogel).

A crosslinked structure in the form of a sponge can then be obtained by freeze drying the gel.

After the crosslinked structure is formed, the drug can be bound to the crosslinked structure via a Schiff base by a method similar to the method of manufacturing the polysaccharide derivative-drug conjugate, such as for example by a method that includes first forming a crosslinked structure and then mixing the crosslinked structure in a solvent with a drug containing a primary amino group. Mixing causes the aldehyde or ketone groups ($-CR^1(=O)$) of the crosslinked structure to form Schiff bases by reacting with the amino groups of the drug, yielding a crosslinked structure-drug conjugate.

The drug can also be attached physically or by chemical bonds to the crosslinked structure of the polysaccharide derivative to thereby obtain a drug carried on a crosslinked structure of a polysaccharide derivative.

For example, a solution containing the drug can be applied to the crosslinked structure of the polysaccharide derivative, or the crosslinked structure of the polysaccharide derivative can be immersed in this solution, or the drug can be printed on the crosslinked structure of the polysaccharide derivative to thereby obtain a drug carried on a crosslinked structure of a polysaccharide derivative.

Specific examples of the divalent metal ion used as the crosslinking agent in the crosslinking reaction include calcium ions, magnesium ions, barium ions, strontium ions, zinc ions and the like, and a calcium ion is preferred. More specifically, $CaCl_2$, $MgCl_2$, $CaSO_4$, $ZnCl_2$, $BaCl_2$ or $SrCl_2$ or the like (preferably $CaCl_2$, $CaSO_4$, $ZnCl_2$, $SrCl_2$ or $BaCl_2$) may be used as a divalent metal ion compound.

In addition to this divalent metal ion (divalent metal ion compound), a trivalent metal ion compound ($FeCl_3$) such as $Fe^{3+}$ or a crosslinking reagent having 2 to 4 amino groups in the molecule or the like may also be used as a crosslinking agent. Examples of crosslinking reagents having 2 to 4 amino groups in the molecule include diaminoalkanes which may have lysyl groups ($-COCH(NH_2)-(CH_2)_4-NH_2$) on nitrogen atoms, or in other words diaminoalkanes and derivatives thereof having lysylamino groups formed by substitution of amino groups with lysyl groups, and specific examples include diaminoethane, diaminopropane, N-(lysyl)-diaminoethane and the like.

In addition to these, when the drug has multiple primary amino groups the drug itself may function as a crosslinking agent when introduced into the polysaccharide. For example, the polysaccharide derivative may form a three-dimensional mesh structure when crosslinked by an intermediate-molecular-weight compound (peptide) (for example, bacitracin) having multiple primary amino groups.

The amount of the crosslinking agent used is preferably adjusted appropriately according to the amount and molecular weight of the polysaccharide derivative and the types of polysaccharides constituting the polysaccharide derivative. For example, when a calcium ion is used as the crosslinking agent, the calcium ion concentration of a solution containing the crosslinking agent is not particularly limited, but may be from 1 mM to 1 M, or preferably from 5 mM to 500 mM, or more preferably from 10 mM to 300 mM.

The degree of crosslinking may also be adjusted by adjusting the amount of the crosslinking agent used.

(2) Crosslinked structure of polysaccharide derivative and amino group-containing polymer

In certain embodiments, the crosslinked structure contains a polysaccharide derivative and at least one of an amino group-containing polymer and an amino group-containing low-molecular-weight compound containing two or more primary amino groups, hydrazide groups or aminooxy groups, and is crosslinked by covalent bonds formed via Schiff bases between the primary amino groups, hydrazide groups or aminooxy groups of the amino group-containing polymer and amino group-containing low-molecular-weight compound and a group represented by the formula (A) (modifying group (A)) in the polysaccharide derivative. Specifically, an aldehyde group or ketone group ($-CR^1(=O)$) of the modifying group (A) of the polysaccharide derivative reacts with the primary amino groups, hydrazide groups or aminooxy groups of the amino group-containing polymer and amino group-containing low-molecular-weight compound to form Schiff bases ($-CR^1=N-$). The hydrazide groups are represented by $-C(=O)-NH-NH_2$, and form hydrazone bonds by reacting with the aldehyde groups or ketone groups of the modifying group (A). The aminooxy groups are represented by $-O-NH_2$, and form oxime bonds by a reaction between the terminal $-NH_2$ groups and the aldehyde groups or ketone groups of the modifying groups (A).

The amino group-containing polymer is a substance with a molecular weight of at least 1,000 containing two or more primary amino groups, hydrazide groups or aminooxy groups. In this Description, an "amino group-containing polymer" also includes having a molecular weight of about 1,000 to 10,000, commonly called oligomers.

The amino group-containing low-molecular-weight compound is a substance with a molecular weight of less than 1,000 containing two or more primary amino groups, hydrazide groups or aminooxy groups.

The amino group-containing polymer and amino group-containing low-molecular-weight compound may have been manufactured by synthesis, or may be naturally occurring substances.

The amino group-containing polymer containing primary amino groups, hydrazide groups or aminooxy groups (hereunder also called simply the "amino group-containing polymer") may be any polymer containing a total of two or more of at least kind selected from a primary amino group, a hydrazide group and an aminooxy group, without any particular limitations. The primary amino groups, hydrazide groups or aminooxy groups may be present at the end of the polymer, in the side chains of the polymer, or in pendant groups in the polymer.

The amino group-containing polymer may be of at least one kind selected from the polyamines, polyalkylene glycols substituted with amino groups, aminooxy groups or hydrazide groups, polyallylamines, polyvinylamines, polyacrylamines, amino group-containing polysaccharides, amino group-containing proteins and polyamino acids. Commercial products may be used for these, or they may be synthesized by conventional known methods.

A polyamine may be either linear, branched or dendritic. "Dendritic" means that the polyamine has a dendritic hyperbranched morphology, and is a polymer having multiple arms of equal or unequal length.

The polyamine is not particularly limited, but examples include linear, branched or dendritic polyalkyleneimines and branched or dendritic polyetheramines.

A polyalkyleneimine is a polymer having an alkylene imine structures as a repeating unit, and having a terminal primary amino group. The alkylene part and imine part of the alkyleneimine structure may each be substituted. Linear polyalkyleneimines contain alkyleneimine structures having secondary amino groups. Branched polyalkyleneimines and dendritic polyalkyleneimines contain alkyleneimine structures having primary amino groups, secondary amino groups or tertiary amino groups. The polyalkyleneimine preferably has a lower (such as C1 to C6, or C1 to C3) alkyleneimine structure, and specific examples include, but are not limited, to polyethylenimine, polypropylenimine and the like.

A branched on dendritic polyetheramine is a polymer having an alkylene oxide structure as a repeating unit, and having a terminal primary amino group. Examples include, but are not limited to, amino-terminated star-shaped polyethylene oxide, amino-terminated dendritic polyethylene oxide, amino-terminated comb-shaped polyethylene oxide, amino-terminated star-shaped polypropylene oxide, amino-terminated dendritic polypropylene oxide, amino-terminated comb-shaped polypropylene oxide, amino-terminated star-shaped polyethylene oxide-polypropylene oxide copolymer, amino-terminated dendritic polyethylene oxide-polypropylene oxide copolymer, amino-terminated comb-shaped polyethylene oxide-polypropylene oxide copolymer and the like. An amino-terminated star-shaped polymer may be a polymer having 3, 4, 6 or 8 arms terminated with primary amines. For example, amino-terminated star-shaped polyethylene glycol

is star-shaped polyethylene glycol having 3, 4, 6 or 8 arms terminated with primary amines (3-, 4-, 6- or 8-arm star PEG amine). However, examples are not limited to these. A commercial product such as polyoxyalkylene triamine sold under the trade name of Jeffamine® triamine by Huntsman LLC (Houston, TX) may be used as a branched or dendritic polyetheramine.

Examples of polyalkylene glycols substituted with amino, hydrazide or aminooxy groups include polyethylene glycol (PEG), polypropylene glycol or polyethylene oxide-polypropylene oxide copolymers substituted with multiple (two or more) amino groups, hydrazide groups or aminooxy groups.

A polyallylamine is a polymer having an allylamine structure as a repeating unit. The allyl part in the allylamine structure may also be substituted. In addition to the repeating units of the amine structure, the polyallylamine may also have another repeating unit as a copolymerization component.

A polyvinylamine is a polymer having a vinylamine structure as a repeating unit, and the vinyl part in the vinylamine structure may be substituted. In addition to the repeating units of the vinylamine structure, the polyvinylamine may also have another repeating unit as a copolymerization component.

A polyacrylamine is a polymer containing an acrylic structure with side chains containing primary amino groups, and the vinyl part in the acrylic structure may be substituted. For example, this may be an acrylic polymer containing an amine structure with a polyalkyelenimine grafted to the side chains. In addition to the repeating units of the acrylic structure having side chains containing primary amino groups, the polyacrylamine may also another repeating unit as a copolymerization component. A commercial product such as an aminoethylated acrylic polymer sold under the trade name Polyment® (Nippon Shokubai) may be used.

Examples of amino group-containing polysaccharides include polysaccharides such as chitosan containing amino groups. An amino group-containing polysaccharide may also be obtained by modifying a polysaccharide lacking amino groups to introduce amino groups into the polysaccharide (aminated polysaccharide). Examples of aminated polysaccharides include aminodextran or the like with introduced amino groups.

Examples of amino group-containing proteins include fibrinogen, albumin, gelatins, collagens and the like.

Examples of polyamino acids (polypeptides) include polylysine, polyarginine, polyglutamic acid, polyaspartic acid and the like.

The weight-average molecular weight of an amino group-containing polymer is not particularly limited, but is generally at least 1,00. There is no particular upper limit to the weight-average molecular weight of an amino group-containing polymer, but generally it is not more than 1,000,000.

An amino group-containing low-molecular-weight compound may be any low-molecular-weight compound containing a total of at least two of at least one kind of group selected from a primary amino group, a hydrazide group and an aminooxy group, without any particular limitations. Examples include hydrazide crosslinking agents such as adipic dihydrazide, sebacic dihydrazide, dodecanedioic dihydrazide and isophthalic dihydrazide.

A salt may also be formed by the amino group in an amino group-containing polymer or amino group-containing low-molecular weight compound. Salts of amino group-containing polymers or amino group-containing low-molecular-weight compounds include halide salts (such as hydrochlorides), phosphate salts, phosphite salts, carbonate salts, bicarbonate salts, sulfate salts, hydrogen sulfate salts, hydroxides, nitrate salts, persulfate salts, sulfite salts, acetates, ascorbate salts, citrate salts, oxalate salts, succinate salts, tartrate salts, and taurocholate or cholate salts.

Fig. 34 shows a schematic diagram of a crosslinked structure prepared in Example I-17 below, which is formed from a polysaccharide derivative (AL-ABA) and an amino group-containing polymer (DPI: polyethyleneimine). As shown in Fig. 34, the crosslinked structure has a structure in which the primary amino groups contained in the amino group-containing polymer (polyethyleneimine) are crosslinked by covalent binding via Schiff bases (-C=N-) with the benzaldehyde groups contained in the polysaccharide derivative.

The crosslinked structure of the polysaccharide derivative with the amino group-containing polymer or amino group-containing low-molecular-weight compound is typically manufactured by mixing the polysaccharide derivative with the amino group-containing polymer or amino group-containing low-molecular-weight compound in a solvent. From the standpoint of biocompatibility, an aqueous solvent (such as water or physiological saline) is preferred as the solvent.

Once a crosslinked structure of the polysaccharide derivative and the amino group-containing polymer or amino group-containing low-molecular-weight compound has been obtained, a crosslinking agent (such as a divalent metal ion) different from the amino group-containing low-molecular weight compound may be used to obtain a structure crosslinked via crosslinking groups (for example, unmodified carboxyl groups) contained in the crosslinked structure. Specifically, crosslinked structures can be obtained by first mixing the polysaccharide derivative and the amino group-containing polymer or amino group-containing low-molecular-weight compound in a solvent, and then dripping a solution containing a divalent metal ion as a crosslinking agent into the solution of the crosslinked structure; or by first mixing the polysaccharide derivative and the amino group-containing polymer or amino group-containing low-molecular-weight compound in a solvent, and then dripping the solution containing the crosslinked structure into a solution containing a divalent metal ion as a crosslinking agent to perform partial crosslinking.

The type and amount of the crosslinking agent are not particularly limited, and may be similar to the type and

amount of the crosslinking agent in the crosslinking reaction of the polysaccharide derivative using a crosslinking agent above.

The form of the crosslinked structure of the polysaccharide derivative and the amino group-containing polymer or amino group-containing low-molecular-weight compound is not particularly limited, and examples include tube structures, fibrous structures, fibers, beads, gels, semispherical gels, capsules, sponges, sheets, films and the like.

In one embodiment, the crosslinked structure of the polysaccharide derivative and the amino group-containing polymer or amino group-containing low-molecular-weight compound may be in the form of a gel (hydrogel). The gel may also be freeze dried to form a crosslinked structure in sponge form.

6. Uses of polysaccharide derivative, polysaccharide derivative-drug conjugate, crosslinked structure, crosslinked structure-drug conjugate and composition

The polysaccharide derivative, polysaccharide derivative-drug conjugate, crosslinked structure, crosslinked structure-drug conjugate or composition may be used in a wide range of fields including food, medicine, cosmetics, everyday goods, textiles and papermaking. The forms of the polysaccharide derivative, polysaccharide derivative-drug conjugate, crosslinked structure, crosslinked structure-drug conjugate and composition are not particularly limited, and may be selected according to the use. Examples include tube structures, fibrous structures, fibers, beads, gels, semispherical gels, capsules, sponges, sheets, films and the like. In one embodiment, the polysaccharide derivative, polysaccharide derivative-drug conjugate, crosslinked structure, crosslinked structure-drug conjugate or composition may be in the form of a gel, sponge, film or capsule.

The polysaccharide derivative and crosslinked structure may be used in place of conventional polysaccharide materials in a wide range of fields including food, medicine, cosmetics, household goods, textiles and papermaking.

Because the polysaccharide derivative, polysaccharide derivative-drug conjugate, crosslinked structure, crosslinked structure-drug conjugate or composition has excellent biodegradability and biocompatibility, it can be used favorably as a medical material.

When it is used as a medical material, the medical material may be a drug delivery device, suture, material for in vivo hemostasis, adhesion or anti-adhesion, tissue adhesive material, wound dressing, cell culture substrate, material for regeneration engineering of substrates for cell transplantation, antithrombic material, diagnostic drug, dialysis carrier or the like. The medical material may be used in mammals or non-mammals.

In particular, since the polysaccharide derivative binds to drugs having primarily amino groups under neutral pH and high pH conditions but can release such drugs under low pH conditions (pH-responsive drug release), it can be used favorably as a drug delivery carrier. One aspect of the present invention provides a drug delivery device containing the above polysaccharide derivative, polysaccharide derivative-drug conjugate, crosslinked structure or crosslinked structure-drug conjugate or a composition containing these. The drug delivery device can be used as a means for selectively and efficiently introducing the drug carried on the polysaccharide derivative into a target tissue. Another aspect provides a method for releasing a desired encapsulated drug into a target tissue, or a method for controlling such release.

The form of the crosslinked structure when used as a medical material may be a tubular, fibrous, fiber, bead, capsule, gel, semispherical gel, sponge, sheet or film form or the like, or preferably a bead, capsule, gel or semispherical gel or sponge form, or more preferably a capsule, hydrogel or sponge form.

A tissue adhesive material containing the above polysaccharide derivative, polysaccharide derivative-drug conjugate, crosslinked structure or crosslinked structure-drug conjugate is provided by certain embodiments. In specific embodiments, the tissue adhesive material is a biological tissue adhesive or a sealant for use in attaching biological tissue.

A tissue adhesive material containing the above polysaccharide derivative or crosslinked structure is provided by certain embodiments. The polysaccharide derivative or crosslinked structure can attach to biological tissue through interactions between the aldehyde groups or ketone groups ($-C(=O)R^1$) of the introduced modifying group (A) and functional groups in the biological tissue.

In specific embodiments, the polysaccharide derivative or crosslinked structure can attach to biological tissue by forming Schiff bases by a reaction between the aldehyde groups or ketone groups ($-C(=O)R^1$) of the introduced modifying group (A) and primary amino groups in the biological tissue.

In certain embodiments, the tissue adhesive material contains a polysaccharide derivative-drug conjugate or crosslinked structure-drug conjugate. Preferably, the polysaccharide derivative-drug conjugate or crosslinked structure-drug conjugate has a drug having a primary amino group bound to some of the modifying groups (A), and also has unreacted modifying groups (A) with no drug bound thereto. With this embodiment, the drug can be delivered to an adhesion site while at the same time the introduced aldehyde groups or ketone groups ($-C(=O)R^1$) of the unmodified modifying groups (A) react with the primary amino groups of the biological tissue to form Schiff bases and attach the material to the biological tissue.

The form of the tissue adhesive material is not particularly limited, and examples include tube structures, fibrous

structures, fibers, beads, capsules, gels, semispherical gels, sponges, sheets, films and the like, but a bead, capsule, gel, semispherical gel or sponge is preferred, a capsule, hydrogel or sponge is more preferred, and a hydrogel is especially preferred. A hydrogel is useful as a tissue adhesive or sealant for medical applications when rapid decomposition is required to prevent undesirable tissue-tissue adhesion due to injury, surgery or the like for example.

The method for using the tissue adhesive material is not particularly limited.

In certain embodiments, the tissue adhesive material is a tissue adhesive used for attaching two biological tissues together.

In certain embodiments, a tissue adhesive containing the above polysaccharide derivative or crosslinked structure is applied to at least one attachment site of biological tissue, and two or more sites are brought into contact to attach two biological tissues.

In certain embodiments, a tissue adhesive containing the above polysaccharide derivative or crosslinked structure is applied to at least one attachment site of biological tissue, a crosslinking agent (such as a solution containing a divalent metal ion) is then applied to the same attachment site, and two or more sites are brought into contact to attach two biological tissues. In certain embodiments, a crosslinking agent (such as a solution containing a divalent metal ion) is applied to at least one attachment site of biological tissue, a tissue adhesive containing the above polysaccharide derivative or crosslinked structure is then applied to the same attachment site, and two or more sites are brought into contact to attach two biological tissues. By applying a crosslinking agent, it is possible to form a hydrogel or increase the degree of hydrogel crosslinking.

In certain embodiments, the tissue adhesive material is a sealant used to seal air/fluid leaks in biological tissue or seal or pack small voids or defects in biological tissue.

In certain embodiments, a sealant containing the polysaccharide derivative or crosslinked structure is applied to biological tissue and left there to seal air/fluid leaks in the biological tissue, or to seal or pack small voids or defects in the biological tissue.

In certain embodiments, a sealant containing the polysaccharide derivative or crosslinked structure is applied to biological tissue, and a crosslinking agent (such as a solution containing a divalent metal ion) is then applied to the same application site and left there to seal air/fluid leaks in the biological tissue, or to seal or pack small voids or defects in the biological tissue. In certain embodiments, a crosslinking agent (such as a solution containing a divalent metal ion) is applied to biological tissue, and a sealant containing the polysaccharide derivative or crosslinked structure is then applied to the same application site and left there to seal air/fluid leaks in the biological tissue, or to seal or pack small voids or defects in the biological tissue. By applying a crosslinking agent, it is possible to form a hydrogel or increase the degree of hydrogel crosslinking.

In certain embodiments, the sealant may be combined with a tissue section derived from biological tissue, and skin flap, periosteum or the like may be used for the tissue section. A tissue section may be attached to tissue in combination with these sealants.

In certain embodiments, the sealant may also be combined with a biodegradable or non-biodegradable nonwoven fabric, sheet, film or the like, and used to attach the nonwoven fabric, sheet or film. The biodegradable nonwoven fabric, sheet or film may be made of a material such as polyglycolic acid, L-lactide/ε-caprolactone polymer, polylactic acid, glycolic acid/lactic acid polyester or sodium alginate, while the non-biodegradable nonwoven fabric, sheet or film may be made of a material such as polytetrafluoroethylene (PTFE). The sealants may be attached to tissue in combination with such a nonwoven fabric, sheet or film.

In specific embodiments, the tissue adhesive material (tissue adhesive, sealant) is attached to biological tissue in the form of an aqueous solution or dispersion.

In specific embodiments, the tissue adhesive material (tissue adhesive, sealant) is attached to biological tissue in the form of a gel, sponge, sheet or film.

The tissue adhesive material may also contain various additives according to the intended use. These various additives may include one or more selected from the pH adjusters, antibiotics, colorants and surfactants.

The biological tissue to which the tissue adhesive material is applied is not particularly limited, but may be skin, oral cavity, esophagus, stomach, small intestine, large intestine (colon), bone, nerve, exon, cartilage, blood vessel, cornea, muscle, fascia, brain, prostate, breast, endometrium, lung, spleen, liver, testis, ovary, neck, lymph node, bone marrow or kidney tissue or the like. Of these, skin, mucous membrane of the digestive tract (oral cavity, esophagus, stomach, small intestine, large intestine) or submucosa (mucous membrane injury site) of the digestive tract or the like is preferred.

In a preferred embodiment, the biological tissue to which the tissue adhesive material is applied is biological tissue having amino groups exposed on the surface. In this case, covalent bonds are formed via Schiff bases between the amino groups and the aldehyde groups or ketone groups ($-C(=O)R^1$) of the modifying groups (A) of the tissue adhesive material, which can improve the attachment strength. Examples of biological tissue having amino groups exposed on the surface include skin (especially collagen exposure sites as discussed below), submucosa of the digestive tract (mucous membrane injury sites) and the like.

The specific site of the biological tissue is also not particularly limited, and sites where tissue adhesive materials have been conventionally used may be adopted appropriately. Examples of such application sites include suture sites following surgery, bleeding sites, fracture fragment fixing sites, anastomosis sites of peripheral nerves and microvessels, sites affected by tendon adhesion and tendon sutures, and adhesion sites of organ injuries and the like.

Certain embodiments provide a kit containing a tissue adhesive material for application to a biological material to attach the biological material, together with instructions for use of the tissue adhesive material.

Certain embodiments provide a kit containing a precursor of a tissue adhesive material for application to a biological material to attach the biological material, together with instructions for use of the tissue adhesive material. In one embodiment, the precursor of the tissue adhesive material includes an un-crosslinked polysaccharide derivative or partially crosslinked polysaccharide derivative, or a conjugate of these with a drug, together with a crosslinking agent (such as a solution containing a divalent metal ion).

Certain embodiments provide an anti-adhesion material containing the polysaccharide derivative, polysaccharide derivative-drug conjugate, crosslinked structure or crosslinked structure-drug conjugate.

Because the polysaccharide derivative, polysaccharide derivative-drug conjugate, crosslinked structure or crosslinked structure-drug conjugate has excellent biocompatibility, it can be used as a bioabsorbable material or medical device.

Since the polysaccharide derivative and crosslinked structure bind with drugs having primary amino groups, they can be used as separation materials. Examples of separation materials include chromatography carriers, nonwoven fabrics, membrane materials and the like.

The polysaccharide derivative, polysaccharide derivative-drug conjugate, crosslinked structure and crosslinked structure-drug conjugate can be used as foods, supplements and food additives.

All publications cited in this Description, such as documents of prior art and patent documents including patent applications and patent publications, are incorporated by reference in this Description. This Description also encompasses the matter disclosed in the Claims, Description and Drawings of Japanese Patent Application No. 2020-137010 (filed on August 14, 2020), which is the basis for the prior claim of this application.

Furthermore, the objects, features, advantages and ideas of the present invention are clear to a person skilled in the art from the descriptions of this Description, and a person skilled in the art can easily implement the present invention based on the descriptions of this Description. The best mode and specific examples for implementing the present invention illustrate preferred embodiments of the present invention, and are presented in order to exemplify or explain the invention, not to limit its scope. Based on the description herein, it will be apparent to those skilled in the art that various modifications can be made within the spirit and scope of the invention disclosed herein.

## Examples

The present invention will be described in more detail below with reference to Examples and Comparative Examples, but the present invention is not limited to these Examples.

In this Description, "room temperature" normally means about 10°C to 35°C. "%" means percent by weight unless otherwise specified.

In this Description, the term "about" may mean $\pm$10%.

Measurement of the nuclear magnetic resonance spectrum ($^1$H NMR) was performed using a JEOL JNM-A500 Alpha FT-NMR spectrometer (500 MHz) (JEOL) with a heavy solvent ($D_2O$).

The abbreviations used in the examples are commonly used abbreviations known to those skilled in the art. Certain abbreviations are shown below.

AL: Alginic acid (sodium)
Alg: Alginic acid (sodium)
ABA: 4-aminobenzaldehyde
AL-ABA: Benzaldehyde-modified alginic acid
HOBt: 1-hydroxybenzotriazole
WSCD/HCl, EDC·HCl: 1-ethyl-3-(3-dimethylaminopropyl) carbodiimide hydrochloride
THF: Tetrahydrofuran
DMSO: Dimethylsulfoxide
NaCl: Sodium chloride
eq: Equivalent
Van: Vancomycin
PBS: Phosphate-buffered saline
FTSC: Fluorescein-5-thiosemicarbazide
FBS: Fetal bovine serum

DMEM: Dulbecco's modified Eagle Medium
Bac: Bacitracin
HA: Hyaluronic acid
PMX: Pemetrexed
CMC: Carboxymethyl cellulose
DCC: N,N'-dicyclohexyl carbodiimide
CMD, CMDX: Carboxymethyl dextran
Mw: Weight-average molecular weight

In the synthesis schemes and reaction formulae shown in the examples below, the polysaccharides (AL, HA, CMC, CMDX) are shown in free form (with carboxyl groups), but the carboxyl groups (-COOH) in the polysaccharides (AL, HA, CMC, CMDX) shown in the synthesis schemes and reaction formulae may also be in an ionized state ($-COO^-$) or salt state (-COOX). For example, in Example 1 below the AL is used in the form of a sodium salt (sodium alginate), so in the synthesis scheme 1 the carboxyl groups (-COOH) in the AL may be in an ionized state ($-COO^-$) or salt state (-COONa).

I. Alginic acid derivative

1. AL-ABA

[Example I-1] Benzaldehyde modified alginic acid (AL-ABA)

<Synthesis of AL-ABA (1)>

[C36]

Synthesis scheme 1

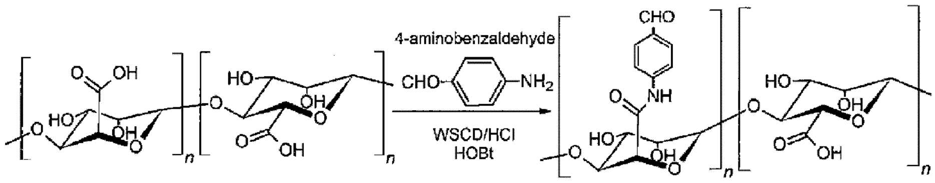


AL AL-ABA

For purposes of convenience, the synthesis scheme 1 above shows a reaction to introduce a modifying group derived from 4-aminobenzaldehyde (ABA) into a carboxyl group of the guluronic acid unit (lefthand monosaccharide unit), but the modifying group derived from ABA may also be introduced into a carboxyl group of the mannuronic acid unit (righthand monosaccharide unit). In the scheme 1, the AL-ABA has modifying groups derived from ABA introduced into the carboxyl groups of both the guluronic acid unit (lefthand monosaccharide unit) and the mannuronic acid unit (righthand monosaccharide unit).

AL-ABA (1) was synthesized according to the following steps (1) to (6) by an amidation reaction via carbodiimide according to the above synthesis scheme.

(Synthesis procedures)

(1) 200 mg (1 mmol) of sodium alginate (AL) (IL-6G, viscosity 50 to 80 mPa·s (1%), weight-average molecular weight Mw = 750,000 to 850,000, manufactured by Kimica Co., Ltd.) was dissolved in 50 mL of pure water, and stirred overnight to prepare an alginic acid solution.
(2) 242.9 mg (2 mmol, 1 eq) of ABA was dissolved in 20 mL of THF, and stirred overnight to prepare an ABA solution.
(3) 270.3 mg (2 mmol, 2 eq) of 1-hydroxybenzotriazole (HOBt) was dissolved in 10 mL of DMSO, and added dropwise one drop at a time to the alginic acid solution prepared in (1) above. Next, 383.7 mg (2 mmol, 2 eq) of 1-ethyl-3-(3-dimethylaminopropyl) carbodiimide hydrochloride (WSCD/HCl) was dissolved in 5 mL of pure water, and added dropwise one drop at a time to the alginic acid solution.

(4) The ABA solution prepared in (2) above was added dropwise one drop at a time to the alginic acid solution obtained in (3) above.

(5) The solution obtained in (4) above was adjusted to pH 5.5, stirred for 3 to 4 hours, and then diluted with 100 mL of pure water.

(6) The solution obtained in (5) above was filtered under reduced pressure, and dialyzed for 2 days with NaCl and for 2 days with pure water. This was then frozen with liquid nitrogen and freeze dried for 3 days to obtain AL-ABA (1).

<$^1$H NMR spectrum measurement, UV-visible light absorption spectrum (UV-vis) measurement and FT-IR spectrum measurement>

The AL-ABA (1) was subjected to $^1$H NMR spectrum measurement, UV-visible light absorption spectrum (UV-vis) measurement and Fourier transform infrared (FT-IR) spectrum measurement. The results are shown in Figs. 1 to 3.

In the $^1$H NMR spectrum (Fig. 1), peaks derived from ABA (peaks b and c derived from benzene rings and peak d derived from aldehyde) were observed in the AL-ABA after modification. Characteristic peaks derived from ABA (at about 235 nm and about 330 nm) were also observed in the UV-vis spectrum (Fig. 2) of the AL-ABA after modification, while in the FT-IR spectrum (Fig. 3) absorption (peak at about 1740 $cm^{-1}$) derived from the C=O of the amide, which is the attachment point of ABA, was observed in the AL-ABA after modification. These results confirmed synthesis of benzaldehyde modified alginic acid (AL-ABA) formed by binding between amino groups of 4-aminobenzaldehyde and carboxyl groups of alginic acid. Based on the $^1$H NMR spectrum, the ABA modification rate of the carboxyl groups (-COOH) of the alginic acid was calculated to be 0.069.

<Cell toxicity evaluation (WST assay) of benzaldehyde-modified alginic acid (AL-ABA)>

(Test procedures)

MeT-5A (human mesothelial cell line), NIH-3T3 (mouse embryo fibroblasts), HUVEC (human umbilical vein endothelial cells) and RAW264.7 cells (mouse macrophage-like cell line) were seeded on 24-well plates and incubated for 24 hours. The medium was then replaced with media having AL-ABA and AL dissolved at different concentrations (0.01 mg/mL, 0.1 mg/mL, 1 mg/mL). Cell viability (%) 48 hours after sample addition was then measured by WST-8 assay (Cell Counting Kit-8, Dojindo). The AL-ABA (1) synthesized in Example I-1 was used as the AL-ABA.

(Results)

The results are shown in Fig. 4. Cell viability was equivalent with the AL-ABA and the AL. This confirms that AL-ABA has low cell toxicity and high biocompatibility.

[Example 1-2] Benzaldehyde-modified alginic acids (AL-ABA)

AL-ABA (2) and AL-ABA (3) were synthesized by the different methods described below.

<Synthesis of AL-ABA (2)>

1 g (0.0046 mol) of AL-500 (sodium alginate, viscosity 400 to 600 mPa·s, manufactured by Mochida Pharmaceutical Co., Ltd.) was dissolved in 300 mL of distilled water, and stirred for 6 to 8 hours. 2.81 g (0.020 mol) of HOBt was dissolved in 10 mL of DMSO, and added dropwise to the AL-500 solution. Next, 3.97 g (0.020 mol) of WSCD/HCl was dissolved in 10 mL of distilled water, and added dropwise to the AL-500 solution. The AL-500 solution was stirred for 10 minutes to obtain an alginic acid solution. 0.84 g (0.0039 mol) of 4-aminobenzaldehyde (ABA) in 10 mL of DMSO was added dropwise to the alginic acid solution. The pH of the reaction mixture was maintained at pH 7.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ABA (2).

<Synthesis of AL-ABA (3)>

AL-ABA (3) was obtained by the same methods as the AL-ABA (2) except that the amount of HOBt was changed

to 1.04 g (0.007 mol), the amount of WSCD/HCl was changed to 1.98 g (0.010 mol), and the pH of the reaction mixture was changed to 5.5.

<$^1$H NMR spectrum measurement and FT-IR spectrum measurement>

The AL-ABA (2) and AL-ABA (3) were subjected to $^1$H NMR spectrum measurement and FT-IR spectrum measurement (not shown). These results confirmed synthesis of ABA-modified alginic acids (AL-ABA) formed by binding between amino groups of ABA and carboxyl groups of alginic acid.

Based on the $^1$H NMR spectrum, the ABA modification rate of the carboxyl groups (-COOH) of the alginic acid was calculated for the AL-ABA (2) and AL-ABA (3). The results are shown in Table 1 below together with the reaction conditions for the AL-ABA (2) and AL-ABA (3).

<Aldehyde measurement by colorimetric aldehyde assay>

Using a blue (MAK140) colorimetric aldehyde assay kit, the aldehyde amounts in AL-ABA (2) and AL-ABA (3) were measured and used to calculate the ABA modification rates of the carboxyl groups (-COOH) of the alginic acids.

(Assay procedures)

(1) 10 μL of 10 mM standard solution was diluted with 990 μL of assay buffer to prepare a 100 μM standard solution. The 100 μM standard solution was also subjected to 2-fold serial dilution with assay buffer. 50 μL of diluted standard solution was added to a 96-well plate to produce 0 (blank), 1.56, 3.125, 6.25, 12.5, 25, 50 and 100 μM standards.
(2) 50 μL of Master Reaction Mix was added to each well. This was mixed thoroughly with a horizontal shaker or by pipetting, and the reaction mixture was incubated for 20 to 30 minutes at room temperature. The plate was protected from light during incubation.
(3) 50 μL of Blue Enhancer was added to each well.
(4) The reaction mixture was protected from light while being incubated for a further 20 minutes at room temperature.
(5) After the incubation period, absorbance was measured at 620 to 660 nm.

(Results)

The ABA modification rate of the carboxyl groups (-COOH) of the alginic acid as calculated from the amount of aldehyde was 0.56 or 0.64 for the AL-ABA (2) and 0.33 for the AL-ABA (3). The results are shown in Table 1 below.

[Table 1]

Table 1 Reaction conditions and ABA modification rates for AL-ABA (2) and AL-ABA (3)

| | ABA | ABA Solution | WSCD | HOBt | Reaction pH | DS NMR | DS Colorimetric |
|---|---|---|---|---|---|---|---|
| AL-ABA(2) | 1.5 mol eq. | DMSO | 8 mol eq. | 8 mol eq. | 7.5 | 64%*1<br>78%*2 | 56%*1<br>64%*2 |
| AL-ABA(3) | 1.5 mol eq. | DMSO | 4.5 mol eq. | 4.5 mol eq. | 5.5 | 21% | 33% |
| *1: Low DS, *2: High DS | | | | | | | |

In Table 1, DS represents the degree of substitution, which is the ABA modification rate converted to a percentage (ABA modification rate × 100). DS NMR is the value calculated from the $^1$H NMR spectrum, while DS Colorimetric is the value calculated from the colorimetric aldehyde assay.

*1 and *2 of the AL-ABA (2) represent AL-ABA prepared in different batches. In the examples below, *1 may be called AL-ABA (2) (Low DS) and *2 may be called AL-ABA (2) (High DS).

2. AL-AAP, AL-ADFBA, AL-APCA, AL-ANA

Alginic acids modified with 4-aminoacetophenone (AAP), 4-amino-2,6-difluorobenzaldehyde (ADFBA), 2-amino-3-pyridinecarboxyaldehyde (APCA) or 6-aminonicotinealdehyde (ANA) were obtained in the same manner as Example I-1. In the schemes below, the modifying groups derived from AAP, ADFBA, APCA or ANA are shown introduced into the carboxyl groups of the guluronic acid units (lefthand monosaccharide units) for purposes of convenience, but these modifying groups may also be introduced into the carboxyl groups of the mannuronic acid units (righthand monosaccharide units). That is, the AL-AAP, AL-ADFBA, AL-APCA and AL-ANA may have modifying groups derived from AAP, ADFBA, APCA or ANA introduced into both the carboxyl groups of the guluronic acid units (lefthand monosaccharide units) and the carboxyl groups of the mannuronic acid units (righthand monosaccharide units).

[Example I-3] AAP-modified alginic acid (AL-AAP)

[C37]

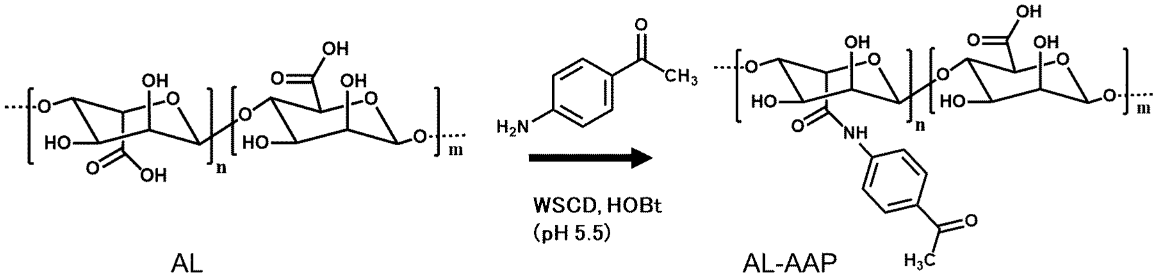


AL AL-AAP

0.5 g (0.0023 mol) of AL-500 (sodium alginate, viscosity 400 to 600 mPa s, manufactured by Mochida Pharmaceutical Co., Ltd.) was dissolved in 100 mL of distilled water, and stirred for 6 to 8 hours. 1.4 g (0.010 mol) of HOBt was dissolved in 10 mL of DMSO, and added dropwise to the AL-500 solution. Next, 1.97 g (0.010 mol) of WSCD/HCl was dissolved in 10 mL of distilled water, and added dropwise to the AL-500 solution. The AL-500 solution was stirred for 10 minutes to obtain an alginic acid solution. 0.310 g (0.0023 mol) of 4'-aminoacetophenone (AAP) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-AAP.

<$^1$H NMR spectrum measurement and FT-IR spectrum measurement>

The AL-AAP was subjected to $^1$H NMR spectrum measurement and FT-IR spectrum measurement, with the results shown in Fig. 5 and Fig. 6. These results confirmed synthesis of AAP-modified alginic acid (AL-AAP) formed by binding between amino groups of AAP and carboxyl groups of alginic acid.

Based on the $^1$H NMR spectrum, the AAP modification rate of the carboxyl groups (-COOH) of the alginic acid was calculated to be 0.31.

<Aldehyde measurement by colorimetric aldehyde assay>

The AAP modification rate of the carboxyl groups (-COOH) of the alginic acid was 0.34 when calculated based on the aldehyde amount as measured with a blue (MAK140) colorimetric aldehyde assay kit.

[Example I-4] ADFBA-modified alginic acid (AL-ADFBA)

[C38]

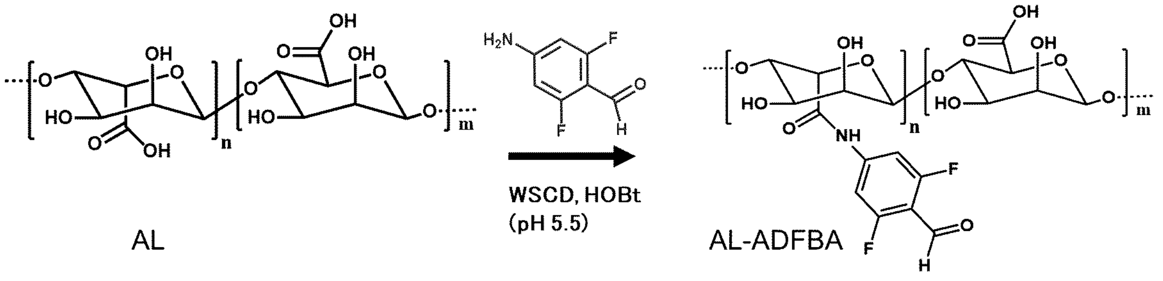


AL AL-ADFBA

An alginic acid solution was obtained as in the Example I-3. 0.361 g (0.0023 mol) of 4'-4-amino-2,6-difluorobenzaldehyde (ADFBA) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ADFBA.

<$^1$H NMR spectrum measurement and FT-IR spectrum measurement>

The AL-ADFBA was subjected to $^1$H NMR spectrum measurement and FT-IR spectrum measurement, with the results shown in Fig. 7 and Fig. 8. These results confirmed synthesis of ADFBA-modified alginic acid (AL-ADFBA) formed by binding between amino groups of ADFBA and carboxyl groups of alginic acid.

Based on the $^1$H NMR spectrum, the ADFBA modification rate of the carboxyl groups (-COOH) of the alginic acid was calculated to be 0.12.

<Aldehyde measurement by colorimetric aldehyde assay>

The ADFBA modification rate of the carboxyl groups (-COOH) of the alginic acid was 0.18 when calculated based on the aldehyde amount as measured with a blue (MAK140) colorimetric aldehyde assay kit.

[Example I-5] APCA-modified alginic acid (AL-APCA)

[C39]

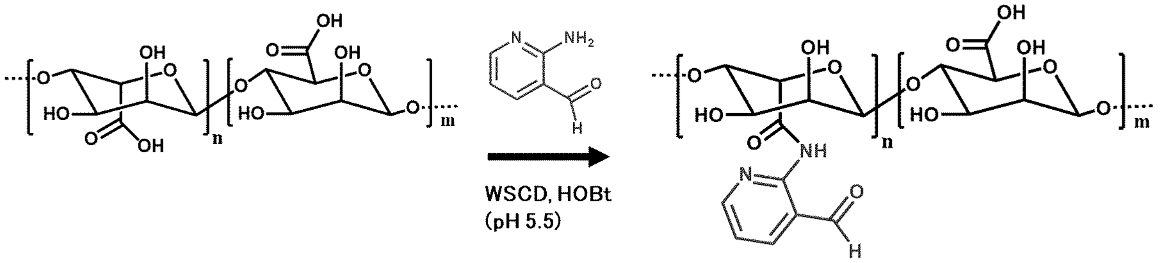


AL AL-APCA

An alginic acid solution was obtained as in the Example I-3. 0.28 g (0.0023 mol) of 2-amino-3-pyridinecarboxyaldehyde (APCA) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-APCA.

<$^1$H NMR spectrum measurement and FT-IR spectrum measurement>

The AL-APCA was subjected to $^1$H NMR spectrum measurement and FT-IR spectrum measurement, with the results shown in Fig. 9 and Fig. 10. These results confirmed synthesis of APCA-modified alginic acid (AL-APCA) formed by binding between amino groups of APCA and carboxyl groups of alginic acid.

Based on the $^1$H NMR spectrum, the APCA modification rate of the carboxyl groups (-COOH) of the alginic acid was calculated to be 0.41.

<Aldehyde measurement by colorimetric aldehyde assay>

The APCA modification rate of the carboxyl groups (-COOH) of the alginic acid was 0.33 when calculated based on the aldehyde amount as measured with a blue (MAK140) colorimetric aldehyde assay kit.

[Example I-6] ANA-modified alginic acid (AL-ANA)

[C40]

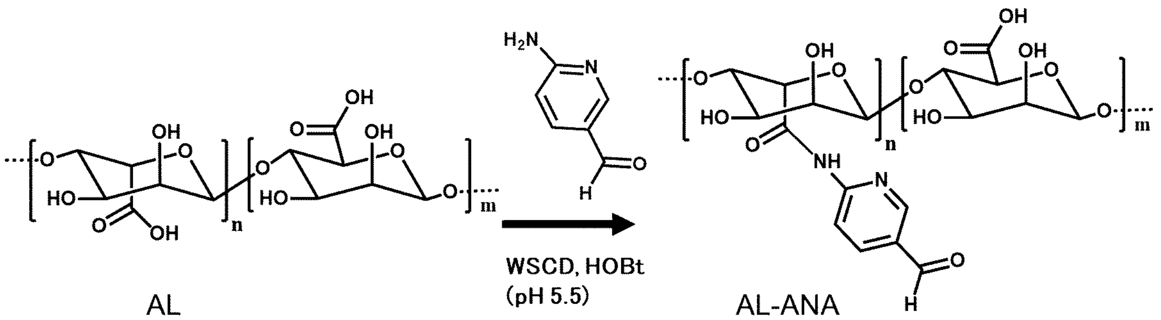


AL AL-ANA

An alginic acid solution was obtained as in the Example I-3. 0.280 g (0.0023 mol) of 6-aminonicotinealdehyde (ANA) in 10 mL of DMSO was added dropwise to the alginic acid solution. The pH of the final reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ANA.

<$^1$H NMR spectrum measurement and FT-IR spectrum measurement>

The AL-ANA was subjected to $^1$H NMR spectrum measurement and FT-IR spectrum measurement, with the results shown in Fig. 11 and Fig. 12. These results confirmed synthesis of ANA-modified alginic acid (AL-ANA) formed by binding between amino groups of ANA and carboxyl groups of alginic acid.

Based on the $^1$H NMR spectrum, the ANA modification rate of the carboxyl groups (-COOH) of the alginic acid was calculated to be 0.17.

<Aldehyde measurement by colorimetric aldehyde assay>

The ANA modification rate of the carboxyl groups (-COOH) of the alginic acid was 0.26 when calculated based on the aldehyde amount as measured with a blue (MAK140) colorimetric aldehyde assay kit.

The following table shows the modification rates by the modifying groups (A) as determined from both the $^1$H NMR spectrum (NMR) and a colorimetric aldehyde assay (Colorimetric).

[Table 2]

Table 2 Modification rates of alginic acid derivatives

| Example | Alginic acid derivative | Modifying group (A) | Modification rate by modifying group (A) | |
|---|---|---|---|---|
| | | | NMR | Colorimetric |
| I-1 | AL-ABA(1) | ABA | 0.069 | - |
| I-2 | AL-ABA(2)High DS | ABA | 0.78 | 0.64 |
| I-2 | Al-ABA(2) Low DS | ABA | 0.64 | 0.56 |
| I-2 | AL-ABA(3) | ABA | 0.21 | 0.33 |
| I-5 | AL-APCA | APCA | 0.41 | 0.33 |
| I-3 | AL-AAP | AAP | 0.31 | 0.34 |
| I-6 | AL-ANA | ANA | 0.17 | 0.26 |
| I-4 | AL-ADFBA | ADFBA | 0.12 | 0.18 |

3. AL-ABA drug conjugate

[Example I-7] Benzaldehyde-modified alginic acid-vancomycin conjugate (AL-ABA-Van)

<Synthesis of AL-ABA-Van>

[C41]

Synthesis scheme 2

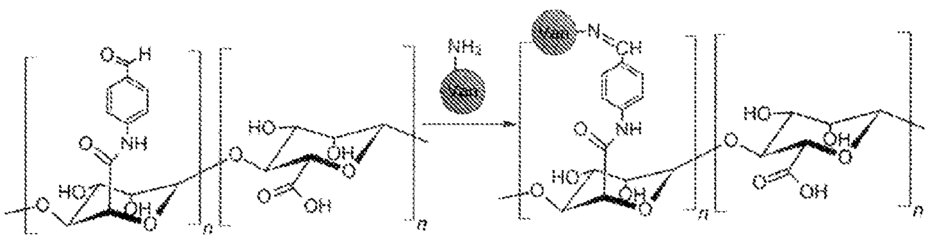

AL-ABA

AL-ABA-Van

Vancomycin (Van)

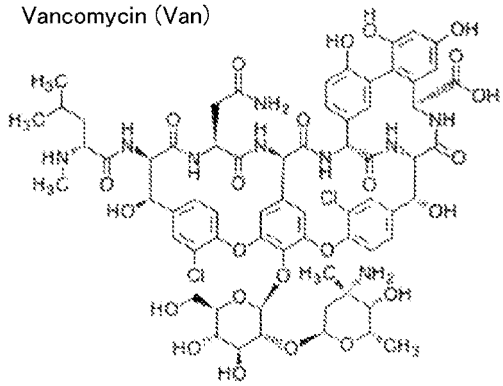

Vancomycin (Van) is an antibiotic having a primary amino group. A conjugate (AL-ABA-Van) of Van bound to AL-ABA was synthesized by the following steps according to the above synthesis scheme by performing a Schiff base reaction between the aldehyde groups of the AL-ABA and the primary amino groups of the vancomycin.

Specifically, 50 mg of the AL-ABA (1) obtained in Example I-1 was dissolved in 70 mL of pure water, and stirred

for at least 1 hour. 60 mg of vancomycin hydrochloride was dissolved in 60 mL of pure water, and added to the AL-ABA solution. 0.1 M NaOH was added dropwise to adjust the pH to near 7.0, and the mixture was reacted overnight under stirring at room temperature under shaded conditions. This was diluted with 150 mL of pure water, dialyzed for 2 days with pure water (equipment: Spectra/Pro® 1 Dialysis Membrane Standard RC Tubing, MWCO: 6-8 kD) and freeze-dried for 3 days, and 86.8 mg of white AL-ABA-Van was collected (yield: 78.9%).

<$^1$H NMR spectrum measurement, UV-visual light absorption spectrum (UV-vis) measurement and FT-IR spectrum measurement>

The resulting AL-ABA-Van was subjected to $^1$H NMR spectrum measurement, UV-visual light absorption spectrum (UV-vis) measurement and FT-IR spectrum measurement, with the results shown in Figs. 13 to 15.

A peak derived from ABA and a peak derived from vancomycin (Van) were observed in the $^1$H NMR spectrum of the AL-ABA-Van (Fig. 13). A peak derived from ABA and a peak derived from vancomycin (Van) (arrows in figure; a shift to lower wavelengths was observed) were also observed in the UV-vis spectrum of the AL-ABA-Van (Fig. 14). In the FT-IR spectrum (Fig. 15), the AL-ABA-Van exhibited a peak of absorption (at about 1740 $cm^{-1}$) derived from the C=O of the amide, which is the binding point between AL and ABA.

These results confirm formation of a conjugate between AL-ABA and Van. Based on the $^1$H NMR spectrum, the conjugate rate of the drug (Van) to the carboxyl groups (-COOH) of the alginic acid was 5.9%. This shows that 82.4% of the ABA in the AL-ABA reacted with the Van.

These results show that conjugation can be instantly completely simply by mixing AL-ABA and Van in an aqueous solution. Another advantage is that no further purification is necessary because the reaction produces only water. Since this reaction can be applied to a variety of drugs containing primary amines, AL-ABA is expected to provide a simple and general-purpose platform for developing alginic acid-drug conjugates.

<Release test of Van from benzaldehyde-modified alginic acid-vancomycin conjugate (AL-ABA-Van)>

(Test procedures)

The release behavior or Van from an AL-ABA-Van solution at different pH values was investigated using a dialysis membrane (pore size: 50 kDa). Specifically, 1 mL of an AL-ABA-Van solution of the AL-ABA-Van obtained in Example I-7 above dissolved in 3 mg/mL of pure water was injected into a dialysis membrane cut to 9 cm, and both ends of the dialysis membrane were tied with kite thread. This was placed in an 80 mL security container, 79 mL of PBS solution was added, and the mixture was stirred at the maximum rotational speed with a 1 cm stirrer tip in a 37°C thermostatic tank. 1.0 mL samples were taken from the solution in the security container at specific time points (1, 2, 3, 4, 6, 8, 12, 24, and 48 hours). The sample solutions were subjected to UV-vis spectrum measurement, the drug concentration in the solutions was assayed based on absorbance, and the release rate of the drug Van (vancomycin release %) was calculated. The above measurements were conducted with pH 5.0, pH 6.0 and pH 7.4 PBS solutions (n = 4 at each pH).

For purposes of comparison as control tests, the same release testing was also performed with the AL-ABA-Van solution replaced by a mixed solution (AL+Van) of sodium alginate and vancomycin obtained by dissolving 3 mg/mL of sodium alginate (AL) (IL-6G, viscosity 50 to 80 mPa·s (1%), manufactured by Kimica Co., Ltd.) and 8.6 mg/mL of vancomycin (Van) in pure water, and by a solution (Van only) obtained by dissolving 8.6 mg/mL of vancomycin (Van) in pure water.

(Results)

The results are shown in Fig. 16. Fig. 16A shows the release behavior of Van from the AL-ABA-Van solution (Van cumulative release rate, %), Fig. 16B shows the release behavior of Van from an AL+Van mixed solution (Van cumulative release rate, %), and Fig. 16C shows the release behavior of Van from a Van solution (Van cumulative release rate, %).

While over 80% of the Van was released in the AL-ABA-Van solution at pH 5.0 after 10 hours, almost 50% had not been released at pH 7.4. These results show that Van is released more rapidly the lower the pH. This is thought to be because that the imine bonds between AL-ABA and Van are more easily dissociated at a low pH.

In the AL+Van solution and Van solution (control tests), on the other hand, almost all of the Van was released in the first 10 hours, and there was no difference in release behavior depending on pH.

These results show that an AL-ABA-Van conjugate can release Van continuously in contrast to the control groups (AL+Van, Van only), and also that the drug is released selectively at low pH, allowing the Van release speed to be varied pH-dependently.

[C42]

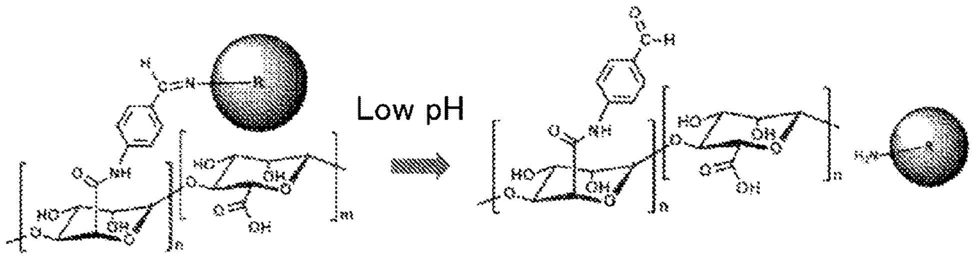


[Example I-8] FTSC-loaded AL-ABA microcapsule (AL-ABA-FTSC capsule)

(Capsule preparation)

[C43]

Fluorescein-5-thiosemicarbazide (FTSC)

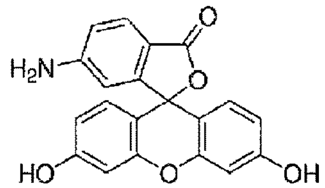

Fluorescein-5-thiosemicarbazide (FTSC) is a fluorescein dye having a primary amino group. An FTSC-loaded AL-ABA capsule was prepared.

Specifically, the AL-ABA (1) prepared in Example I-1 was dissolved in pure water to prepare a 2 wt% AL-ABA solution. This was added dropwise to a 50 mM CaCh aqueous solution to obtain a capsule (AL-ABA capsule). The FTSC was dissolved in DMEM (Dulbecco's modified Eagle medium) containing saline and 10% FBS (fetal bovine serum), and the prepared AL-ABA capsule was immersed in this to load the FTSC into the capsule by reactive dispersion.

For purposes of comparison as a control test, a capsule (AL capsule) was obtained as described above except that the AL-ABA was replaced with sodium alginate (AL) (IL-6G, viscosity 50 to 80 mPa s (1%), manufactured by Kimica Co., Ltd.). The resulting AL capsule was immersed similarly in physiological saline containing FTSC to verify whether or not the FTSC could be loaded.

(Results)

After being immersed in the FTSC solution, the AL and AL-ABA capsules were observed by confocal microscopy. Fig. 17 shows transmitted images (Transmitted), fluorescent images (Amine-Fluorescein), and merged images (Merged).

It can be seen from Fig. 17 that micro-scale capsules were obtained using AL-ABA as well as AL. While almost no fluorescence from the FTSC was observed with the AL capsule, obvious fluorescence from the FTSC was observed with the AL-ABA capsule, showing that the capsule was loaded with FTSC in situ. This confirms that benzaldehyde-modified alginic acid (AL-ABA) retains the same $Ca^{2+}$ crosslinking ability as alginic acid (AL), allowing it to easily form capsules, and can also be loaded with an amine compound by simple immersion.

[Example I-9] Van-loaded AL-ABA microcapsule (AL-ABA-Van capsule)

(Capsule preparation)

The AL-ABA (1) prepared in Example I-1 was dissolved in pure water to prepare a 2 wt% AL-ABA solution. 15

mg of vancomycin (Van) was added to obtain a mixed solution of AL-ABA and Van. The mixed solution of AL-ABA and Van was added dropwise to a 50 mM CaCh aqueous solution to obtain a capsule (AL-ABA-Van capsule).

For purposes of comparison as a control test, a capsule (AL-Van capsule) was obtained as described above except that the AL-ABA was replaced with sodium alginate (AL) (IL-6G, viscosity 50 to 80 mPa·s (1%), manufactured by Kimica Co., Ltd.).

(Results)

It was confirmed that even using a mixed solution of AL-ABA and Van, the benzaldehyde-modified alginic acid (AL-ABA) retained $Ca^{2+}$ crosslinking ability, and a capsule could be prepared as easily as using alginic acid (AL).

<Release test of Van from AL-ABA-Van capsule>

(Test procedures)

The AL-ABA-Van capsule prepared above was collected and added to a solution of 10 mM CaCh in 1 mL of saline. External solution (sustained-release liquid) was collected at specific time points (3, 24 and 48 hours), and the entire amount of the external solution was replaced with a solution of 10 mM CaCh in 1 mL of physiological saline.

The external solution (sustained-release liquid) collected at each time point was subjected to UV-vis spectrum measurement, the drug concentration in the solution was assayed based on absorbance, and the release rate of the drug Van (vancomycin release %) was calculated).

For purposes of comparison as a control test, a release test was performed using an AL-Van capsule instead of the AL-ABA-Van capsule.

(Results)

The results are shown in Fig. 18. Fig. 18A shows the cumulative release rate (%) of Van from each capsule, and Fig. 18B shows the change in the release rate (%) of Van from each capsule at each point in time.

Fig. 18A and Fig. 18B confirm that both the AL-Van capsule and AL-ABA-Van capsule gradually release vancomycin, with 60% of the Van being released after 48 hours. There was no great difference between the two in the cumulative released amount (Fig. 18A), but this may be because the effect of loading by the Schiff bases was obscured by the fact that the speed of release of Van from the microcapsules by subsequent dispersion was slower than the speed of release of Van by dissociation of the Schiff bases with the ABA.

<Bacterial growth inhibition test>

To examine the antibacterial effects of a vancomycin-loaded AL-ABA microcapsule, sustained release solution collected at each time point from the above test of Van release from the AL-ABA-Van capsule was used to evaluate the growth inhibition effects of each sustained release solution by a halo test using Staphylococcus aureus. The specific test procedures are as follows.

(Test procedures)

(1) Inoculation of Staphylococcus aureus on agar medium

1.1 15 g of agar was added to 1 liter of Mueller Hinton medium (BD Co.) and dissolved and sterilized with an autoclave, after which 15 mL was added per 100 mm dish and cooled to room temperature to prepare agar medium.

1.2 700 μL of Mueller Hinton medium was added to a 1.5 mL microtube (Asnol sterilization tube), inoculated with a toothpick with Staphylococcus aureus stock, and cultured overnight in a 37°C incubator.

1.3 The bacterial liquid was diluted to $1.0 \times 10^7$ cells/mL, and 100 μL was added to the agar medium and spread evenly with a spreading stick.

(2) Impregnation of filter paper with sustained-release liquid and administration on agar medium

2.11 mL of each sustained-release liquid collected in the release test was filter sterilized with a syringe filter

(0.22 μm).

2.2 Filter paper (As One: MFWG 4780) was cut into a circle 16 mm in diameter.

2.3 The cut-out filter paper was placed in the center of agar medium inoculated with Staphylococcus aureus.

2.4 40 μL of sustained-release liquid was evenly dripped onto the filter paper to impregnate the paper.

2.5 The dish was transferred to an incubator and cultured for 24 hours at 37°C.

2.6 The dish was removed from the incubator and the appearance of the proliferated bacteria was photographed with a camera and image analyzed with Image J (provided by NIH) to measure the area of the part where proliferation was inhibited.

(Results)

Fig. 19 shows the results of observation of Staphylococcus aureus growth on agar medium under each condition. A circular region without bacterial proliferation was observed on the edge of the filter paper with both the Van-loaded AL capsule (AL-Van) and the Van-loaded AL-ABA capsule (AL-ABA-Van), confirming a growth inhibition effect.

The area where growth was inhibited was also calculated by image analysis, and compared under each condition with the results shown in Fig. 20. While the growth inhibition area decreased over time with the AL capsule (AL-Van) from 3 hours to 24 hours to 48 hours after the start of sustained release, this decrease was suppressed with the AL-ABA capsule (AL-ABA-Van), and the difference in area tended to be less after 3 hours and 48 hours. These results suggest that with the AL-Van capsule most of the vancomycin was released during the initial period, but the released amount decreased, and the antibacterial effect was reduced over time, while with the AL-ABA-Van initial release of the vancomycin was controlled, so that an effective concentration could be maintained for a longer period of time.

[Example I-10] Benzaldehyde-modified alginic acid-bacitracin conjugate (AL-ABA-Bac)

<Synthesis of AL-ABA-Bac>

[C44]

Bacitracin (Bac)

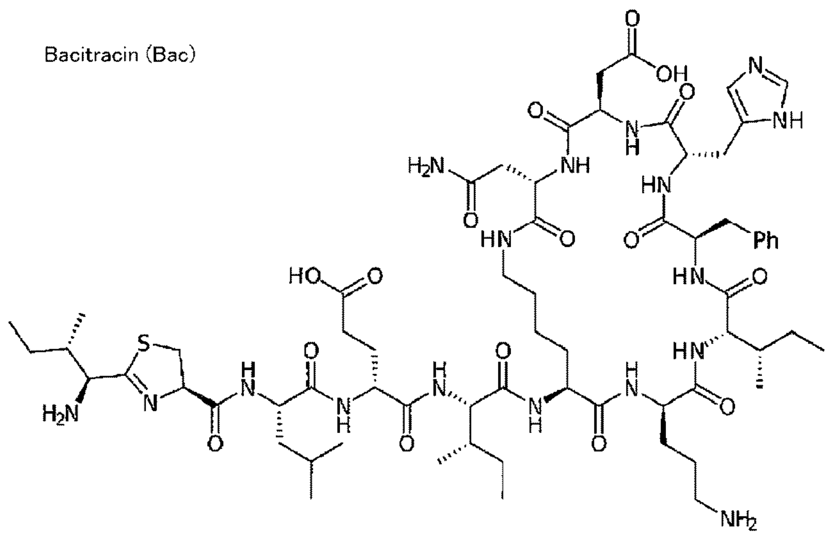

Bacitracin (Bac) is a polypeptide antibiotic of intermediate molecular weight having two primary amino groups. A benzaldehyde-modified alginic acid-bacitracin conjugate (AL-ABA-Bac) was synthesized by the same methods used in the Example I-7.

Specifically, 50 mg of the AL-ABA (1) obtained in Example I-1 was dissolved in 70 mL of pure water, and stirred for at least 1 hour. 15 mg of bacitracin (Bac) was dissolved in 15 mL of pure water, and added to the AL-ABA solution. 0.1 M NaOH was dripped in to adjust the pH to near 7.0, and the mixture was reacted overnight under stirring at room temperature under shaded conditions. This was diluted with 150 mL of pure water, dialyzed for 2 days with pure water (equipment: Spectra/Pro® 1 Dialysis Membrane Standard RC Tubing, MWCO: 6-8 kD) and freeze-dried for 3 days, and

47.6 mg of AL-ABA-Bac in gel form was collected (yield: 73.2%).

<UV-visual light absorption spectrum (UV-vis) measurement and FT-IR spectrum measurement>

The resulting AL-ABA-Bac was subjected to UV-visual light absorption spectrum (UV-vis) measurement and FT-IR spectrum measurement, with the results shown in Fig. 21 and Fig. 22.

In the UV-vis spectrum (Fig. 21), the AL-ABA-Bac exhibited peaks derived from bacitracin (Bac) and ABA (arrows in figure; a shift to lower wavelengths was observed). In the FT-IR spectrum (Fig. 16), the AL-ABA-Bac exhibited a peak of absorption (at about 1740 $cm^{-1}$) derived from the C=O of the amide, which is the binding point between AL and ABA.

These results confirm formation of a conjugate between AL-ABA and Bac.

The collected AL-ABA-Bac had formed a hydrogel. It is thought that because the two amino groups in the Bac formed bonds with the aldehyde groups of the ABA, the Bac itself functioned as a crosslinking agent (gelling agent) to form a gel. Long-term sustained release can be expected due to this gel structure.

Apart from bacitracin (Bac), the polypeptide antibiotics include many other drugs (such as daptomycin, colistin and elcatonin) having two or more amino groups, and it is expected that with these drugs also the drug itself will be able to function as a crosslinking agent to form a gel structure.

[Example I-11] Benzaldehyde-modified alginic acid-dopamine conjugate (AL-ABA-DOPA)

<Synthesis of AL-ABA-DOPA>

[C45]

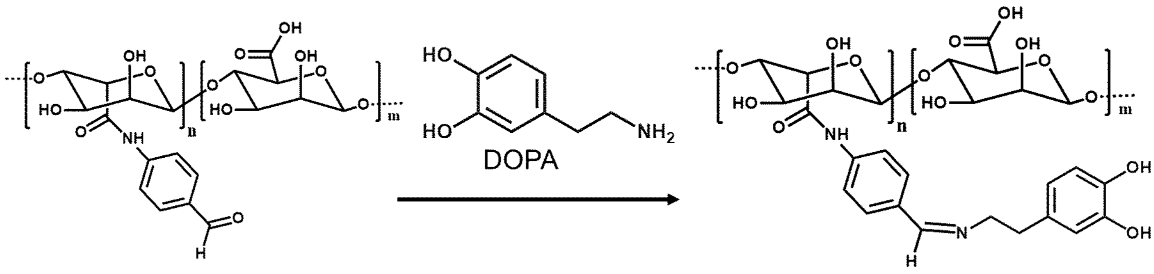

AL-ABA

DOPA

AL-ABA-DOPA

Dopamine (DOPA) is a neurotransmitter that plays an important role in the brain and body, and is an organic chemical in the catecholamine and phenethylamine families. DOPA has a primary amine group. A benzaldehyde-modified alginic acid-dopamine conjugate (AL-ABA-DOPA) was synthesized according to the following procedures.

Dopamine (DOPA) is a neurotransmitter that plays an important role in the brain and body, and has a primary amino group. A benzaldehyde-modified alginic acid-dopamine conjugate (AL-ABA-DOPA) was synthesized according to the following procedures.

0.25 g (0.00115 moles) of the AL-ABA (2) prepared in Example I-2 (Low DS; modification rate (colorimetric): 0.56) was dissolved in 100 mL of PBS (pH 7.4), and stirred for 6 to 8 hours. 0.43 g (0.0023 moles) of dopamine hydrochloride (Sigma Aldrich) was dissolved in 10 mL of PBS (pH 7.4), and added dropwise to the AL-ABA solution. The solution was dialyzed for 72 hours with deionized water using a dialysis tube (Spectra/Pro®, MWCO: 6-8 kDa) to obtain an AL-ABA-DOPA solution, which was then freeze dried.

<$^1$H NMR spectrum measurement and FT-IR spectrum measurement>

The AL-ABA-DOPA was subjected to $^1$H NMR spectrum measurement and FT-IR spectrum measurement to confirm that a conjugate of AL-ABA and dopamine had formed. The results are shown in Fig. 23 and Fig. 24.

Based on the $^1$H NMR spectrum, the conjugate rate of the DOPA drug to the ABA in the AL-ABA was 47% (ABA modification rate by DOPA: 0.47). Because the modification rate (colorimetric) calculated by colorimetric aldehyde assay is more accurate than the modification rate (NMR) calculated from the $^1$H NMR spectrum as the ABA modification rate in the AL-ABA, the DOPA conjugate rate was calculated based on the modification rate (colorimetric) from the

colorimetric aldehyde assay. In the examples below, when the modification rate (colorimetric) has been measured, the conjugate rate of the drug with ABA is calculated based on the modification rate (colorimetric) value.

[Example I-12] Sponge loaded with AL-ABA-DOPA (AL-ABA-DOPA sponge)

(Preparation procedures)

AL-ABA (modification rate (NMR): 0.60) was synthesized under the same conditions as the AL-ABA (2) of Example I-2. The AL-ABA was dissolved in PBS (pH 7.4) to prepare a 0.5% AL-ABA solution. Dopamine (DOPA) was added in the amount of 2 molar equivalents of the ABA, and the solution was stirred for 16 to 20 minutes at room temperature. The solution was then dialyzed for 72 hours with pure water, and then freeze dried to obtain AL-ABA-DOPA.
The AL-ABA-DOPA was dissolved in PBS to obtain a 1.0 wt% AL-ABA-DOPA solution. The AL-ABA-DOPA solution was mixed with 1.0 mL of a 10 mM CaCh aqueous solution in a 35 mm petri dish. After hydrogel formation, this was frozen overnight at -20°C and then freeze dried. Freeze drying yielded an AL-ABA-DOPA sponge.
A hydrogel and sponge of the polysaccharide derivative-drug conjugate AL-ABA-DOPA could be formed by crosslinking with a calcium ion.

[Example I-13] Benzaldehyde-modified alginic acid-serotonin conjugate (AL-ABA-Serotonin)

<Synthesis of AL-ABA-Serotonin>

[C46]

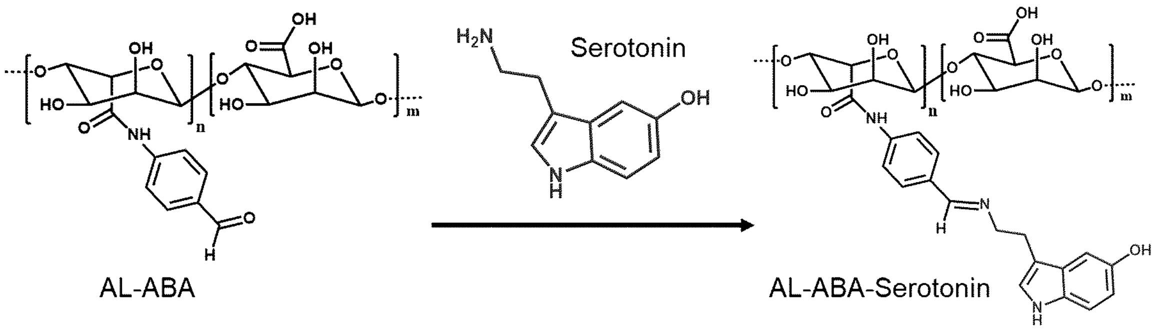


Serotonin (5-hydroxytryptamine) is a monoamine neurotransmitter having a primary amino group. A benzaldehyde-modified alginic acid-serotonin conjugate (AL-ABA-Serotonin) was synthesized by the following procedures.
0.25 g (0.00115 moles) of the AL-ABA (2) prepared in Example I-2 (Low DS; modification rate (colorimetric): 0.56) prepared in Example I-2 was dissolved in 100 mL of PBS (pH 7.4), and stirred for 6 to 8 hours. 0.489 g (0.0023 moles) of serotonin hydrochloride (Sigma Aldrich) was dissolved in 10 mL of PBS (pH 7.4), and added dropwise to the AL-ABA solution. The solution was dialyzed for 72 hours with deionized water using a dialysis tube (Spectra/Pro®, MWCO: 6-8 kDa), and then freeze dried.

<$^1$H NMR spectrum measurement and FT-IR spectrum measurement>

The AL-ABA-Serotonin was subjected to $^1$H NMR spectrum measurement and FT-IR spectrum measurement to confirm that a conjugate of AL-ABA and serotonin had formed. The results are shown in Fig. 25 and Fig. 26.
Based on the $^1$H NMR spectrum, the conjugate rate of the serotonin drug to the ABA in the AL-ABA was 46% (ABA modification rate by serotonin: 0.46).

[Example I-14] Benzaldehyde-modified alginic acid-celecoxib conjugate (AL-ABA-Celecoxib)

<Synthesis of AL-ABA-Celecoxib>

[C47]

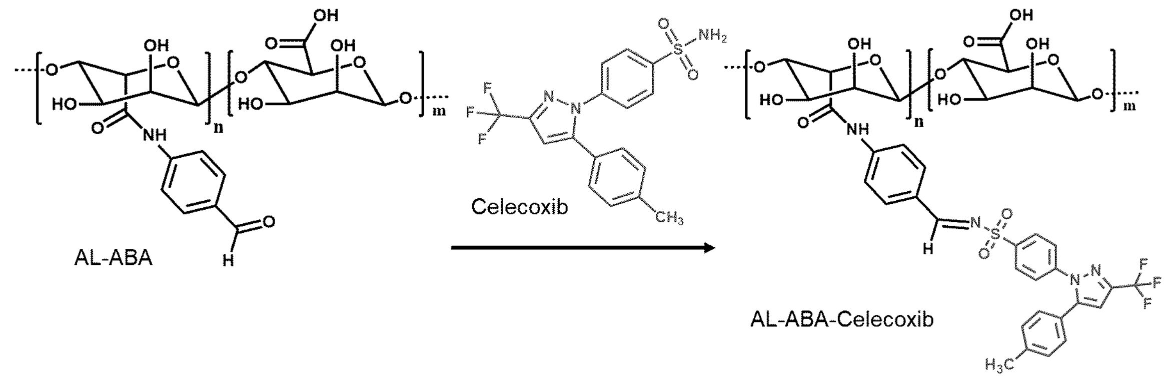


Celecoxib is a non-steroidal anti-inflammatory/analgesic having a primary amino group. A benzaldehyde-modified alginic acid-celecoxib conjugate (AL-ABA-Celecoxib) was synthesized by the following procedures.

0.25 g (0.00115 moles) of the AL-ABA (2) prepared in Example I-2 (Low DS; modification rate (colorimetric): 0.56) was dissolved in 100 mL of PBS (pH 7.4), and stirred for 6 to 8 hours. 0.877 g (0.0023 mol) of celecoxib hydrochloride (TCI) was dissolved in 10 mL of DMSO, and added dropwise to the AL-ABA solution. The solution was dialyzed for 72 hours with deionized water using a dialysis tube (Spectra/Pro®, MWCO: 6-8 kDa), and then freeze dried.

<$^1$H NMR spectrum measurement and FT-IR spectrum measurement>

The AL-ABA-Celecoxib was subjected to $^1$H NMR spectrum measurement and FT-IR spectrum measurement (not shown) to confirm that a conjugate of AL-ABA and celecoxib had formed. The $^1$H NMR spectrum measurement results are shown in Fig. 27.

Based on the $^1$H NMR spectrum, the conjugate rate of the celecoxib drug to the ABA in the AL-ABA was 11% (ABA modification rate by celecoxib: 0.11).

[Example I-15] Benzaldehyde-modified alginic acid-HGF aptamer conjugate (AL-ABA-Apt)

With the aim of developing a novel anti-adhesion hydrogel using AL-ABA having a sustained release function with an HGF aptamer having a mesothelial cell growth promoting effect (manufactured by Eurofins Genomics Co., Ltd., Lot: 1649399-3), a benzaldehyde-modified alginic acid-HGF aptamer conjugate (AL-ABA-Apt) was synthesized and sustained release of the HGF aptamer from the AL-ABA-Apt was tested. The test procedures are shown in Fig. 28.

(Test)

<Synthesis of AL-ABA-Apt>

AL-ABA (modification rate (NMR) 4.0%) was synthesized under the same conditions as the AL-ABA (1) of Example I-1. The AL-ABA was dissolved in phosphate-buffered saline (PBS, pH = 7.4) to obtain a 0.4 wt% AL-ABA solution. HGF aptamer containing amino groups in the amount of 1.0 equivalents of the aldehyde groups of the AL-ABA was added to the AL-ABA solution, and stirred for 1 hour. This was then dialyzed for 3 days with pure water to remove unreacted HGF aptamer, and freeze dried.

The AL-ABA-HGF aptamer conjugate (AL-ABA-Apt) and the HGF aptamer were each subjected to UV-visual light absorption spectrum (UV-vis) measurement, and loading of the HGF aptamer (Apt) by the AL-ABA was evaluated based on the peak value at 256 nm in the UV-vis spectrum.

<Release test of Apt from AL-ABA-Apt>

1.0 wt% of AL-ABA was dissolved in PBS (pH = 7.4). HGF aptamer containing amino groups in the amount of 1.0 equivalents of the aldehyde groups in the AL-ABA was then added to the AL-ABA aqueous solution. This was then stirred for 1 hour to synthesize an AL-ABA-HGF aptamer conjugate (AL-ABA-Apt). The AL-ABA-HGF aptamer was then placed in a dialysis membrane (MWCO = 50 kDa), and stirred in PBS (pH = 7.4). 1 mL samples of the external solution were taken at specific time points after the start of stirring, and the cumulative release rate (%) of the HGF aptamer was

determined by UV-vis measurement. As a control test, a mixture of ordinary alginic acid and HGF aptamer (ALG-Apt) and the HGF aptamer by itself (Apt) were each dissolved in PBS, placed in a dialysis membrane and subjected to the same sustained release testing, and the sustained release speeds were compared.

<Preparation of AL-ABA-HGF aptamer/$Ca^{2+}$ gel>

The AL-ABA-HGF aptamer (AL-ABA-APt) prepared above was dissolved in pure water to a concentration of 1.0 wt% to obtain an AL-ABA-HGF aptamer aqueous solution. The AL-ABA-HGF aptamer aqueous solution and a 100 mM CaCh aqueous solution were mixed 300 μL each in a microtube, and the presence or absence of gelling was examined.

(Results)

<Synthesis of AL-ABA-HGF aptamer>

A peak derived from the HGF aptamer was confirmed near 256 nm in the UV-vis spectrum (not shown) of the synthesized AL-ABA-HGF aptamer, indicating that synthesis of the AL-ABA-HGF aptamer (AL-ABA-Apt) was successful.

<Sustained release of HGF aptamer from AL-ABA-HGF aptamer>

Fig. 29 shows the results of sustained release testing of the HGF aptamer (Apt) from the AL-ABA-HGF aptamer (AL-ABA-Apt), the mixture of alginic acid and HGF aptamer (ALG-Apt), and the HGF aptamer by itself (Apt). In the AL-ABA-Apt group, release of the HGF aptamer (Apt) was more gradual than in the ALG-Apt and Apt groups. This shows that sustained release of the HGF aptamer can be achieved under physiological pH conditions by forming a conjugate of the HGF aptamer with AL-ABA.

<Preparation of AL-ABA-HGF aptamer/$Ca^{2+}$ gel>

The AL-ABA-HGF aptamer was gelled immediately by $Ca^{2+}$, and gel preparation was successful. Instantaneous gelling of AL-ABA by $Ca^{2+}$ crosslinking has been confirmed as shown in Example I-16 and Example I-17 below. This suggest that conjugation of the HGF aptamer with AL-ABA has almost no effect on the gelling performance of AL-ABA.

Synthesis of an AL-ABA-HGF aptamer loaded with the HGF aptamer having a mesothelial cell growth promotion effect was successful, and sustained release of the HGF aptamer from the AL-ABA-HGF aptamer was shown to be possible. Preparation of a gel of the AL-ABA-HGF aptamer (AL-ABA-Apt) by $Ca^{2+}$ crosslinking was also successful, suggesting that this could be used as an anti-adhesion material.

4. Sponge and hydrogel of AL-ABA

[Example I-16] Sponge of calcium-crosslinked benzaldehyde-modified alginic acid (AL-ABA sponge)

(Preparation procedures)

A total of 20 mg of sodium alginate (AL) (IL-6G, viscosity 50 to 80 mPa·s (1%), manufactured by Kimica Co., Ltd.) and the AL-ABA (1) obtained in Example I-1 were dissolved in 2 mL of pure water at weight ratios of 75:25, 50:50 and 25:75 to prepare AL-ABA aqueous solutions. Each of these was mixed in a dish with 2 mL of 10 mM CaCh aqueous solution to obtain an AL-ABA hydrogel. This was then frozen overnight in a -20°C refrigerator and freeze dried for 3 days in a freeze drier to obtain an AL-ABA sponge.

For purposes of comparison as a control test, a sponge (AL sponge) was obtained in the same way except that the AL-ABA was replaced with AL alone (AL 100 wt%).

(Results)

Optical photographs of the AL-ABA sponges obtained in the above example and the Control AL sponge are shown in Fig. 30, and SEM photographs in Fig. 31.

It was confirmed that benzaldehyde-modified alginic acid sponges (AL-ABA sponges) could be prepared by the same simple methods used for AL. Fig. 31 shows that the AL-ABA sponges had porous structures similar to that of the AL sponge. It is believed that the unmodified carboxyl groups of the AL and AL-ABA were crosslinked via calcium (Ca) ions, forming crosslinked structures. The content and crosslinking density of the benzaldehyde (ABA), which is the

modifying group contained in the resulting sponges, can be controlled by mixing AL with AL-ABA.

[Example I-17] Calcium-crosslinked benzaldehyde-modified alginic acid hydrogel

(Ca crosslinked AL-ABA hydrogel)

The AL-ABA (2) prepared in Example I-2 (High DS, modification rate (colorimetric): 0.64) was crosslinked with calcium to prepare a hydrogel (calcium-crosslinked AL-ABA hydrogel). Specifically, 20 mg of the AL-ABA (2) prepared in Example I-2 (High DS, modification rate (colorimetric): 0.64) was dissolved in 1 mL of pure water to prepare an AL-ABA aqueous solution. This was mixed with 1 mL of 100 mM CaCh aqueous solution in a dish to obtain a calcium-crosslinked AL-ABA hydrogel.

<In vitro swelling and degradation test>

The Ca-crosslinked AL-ABA hydrogel obtained above was swelled in pH 7.4 phosphate-buffered saline (PBS). Specifically, 4 new hydrogel samples were prepared, and each sample was weighed, added to 25 mL of PBS solution at 37°C, and swelled for 72 hours. Degradation of each sample after 72 hours of swelling was observed. The weights of the hydrogel samples were measured every 24 hours for 2 weeks. The PBS was exchanged every week for new PBS that had been equilibrated at 37°C. A completely swollen Ca-crosslinked AL-ABA hydrogel was freeze dried, and its morphology was observed with an SEM unit.

(Results)

Fig. 32 shows the swelling and degradation profile (hydrogel weight changes) of the Ca-crosslinked AL-ABA hydrogel. The weight change value is an average of 4 samples.

Fig. 33 shows an SEM photograph of a cross-section cut with a knife from a sponge obtained by freeze drying a Ca-crosslinked AL-ABA hydrogel (500x; 24 hours after start of swelling), confirming the porous structure of the dried hydrogel.

5. Hydrogel containing AL-ABA and amino group-containing polymer

[Example I-18] Hydrogel of AL-ABA and DPI (AL-ABA-DPI)

[C48]

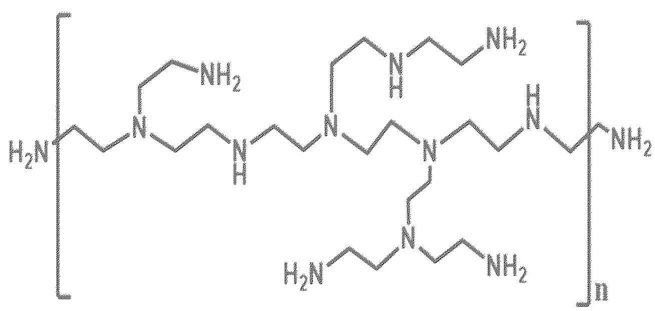

DPI

The AL-ABA (2) prepared in Example I-2 (Low DS, modification rate (colorimetric): 0.56) was dissolved in saline as a solvent to prepare a 2% w/v AL-ABA solution. Dendritic polyethyleneimine (DPI) (BASF Co., product name PS, weight-average molecular weight M = 750,000) was dissolved in water to prepare a 10% w/v DPI solution. The two solutions were mixed so that the aldehyde groups of the AL-ABA solution and the primary amino groups ($-NH_2$) of the DPI solution were in equal molar amounts (1:1). Specifically, 250 $\mu$L of the 10% w/v DPI solution (amino groups: 2.59 $\times$ $10^{-4}$ mol) was mixed mechanically with a pipette into 5 mL of the 2% w/v AL-ABA solution (aldehyde groups: 2.59 $\times$ $10^{-4}$ mol). A hydrogel formed within 20 to 30 seconds (Fig. 35). The hydrogel was stable for 2 weeks in water.

[Example I-19] Hydrogel of AL-ABA and PEG(4k)-dihydrazide (AL-ABA-PEGDH)

<Synthesis of PEGDH>

[C49]

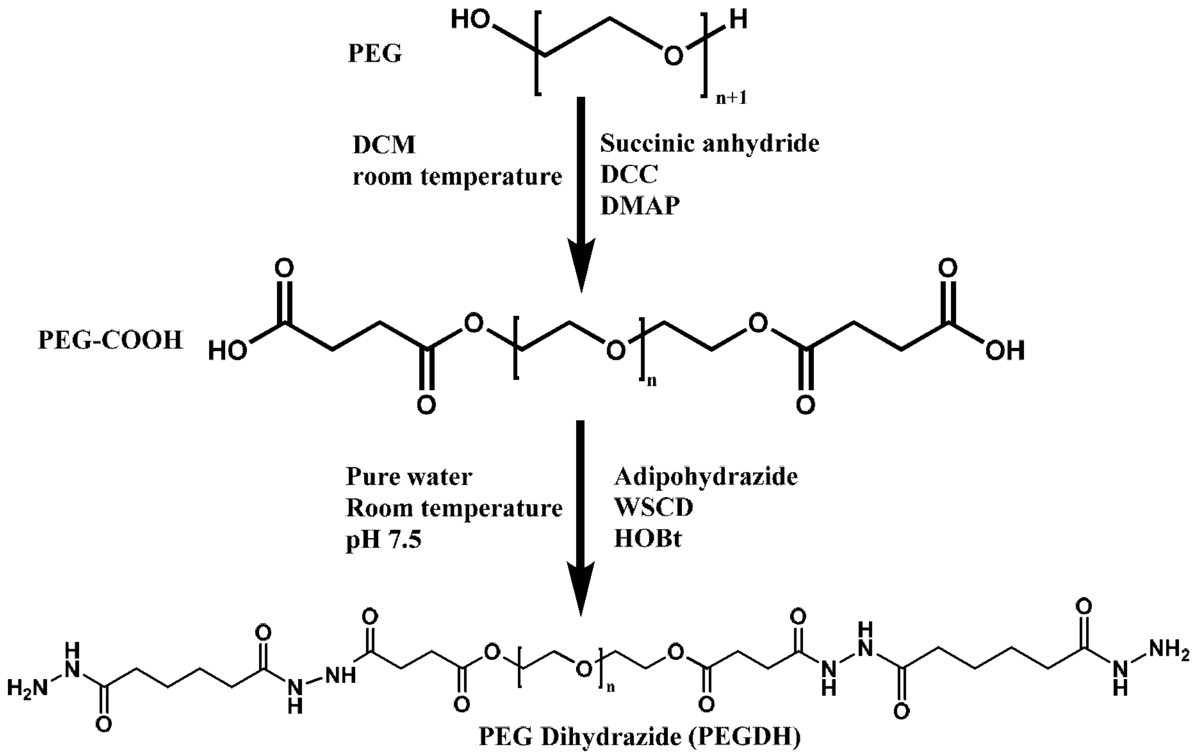


The PEGDH was prepared by a two-stage reaction.

First, 10 g (0.0025 mol) of PEG (Mw 4,000, 162-09115, Wako) was dissolved at room temperature in a 300 mL round-bottom flask in 100 mL of dichloromethane (DCM) solvent. 2.0 g (0.02 mol) of succinic anhydride was added to the PEG solution, followed by 2.57 g (0.0125 mol) of N,N'-dicyclohexyl carbodiimide and 1.83 g (0.015 mol) of 4-dimethylaminopyridine, and the flask was closed with a rubber stopper and stirred for 24 hours at room temperature. The reaction mixture was then filtered with filter paper, and the filtrate was evaporated at 50°C with a rotary evaporator. Once the DCM had completely evaporated, the viscous reaction mixture was left in the flask, 100 mL of distilled water was added, this was stirred for a further 1 hour, and the solution was dialyzed for 48 hours with pure water using a dialysis tube (MWCO: 1 kDa, Spectra/Pro®). After dialysis, the solution was freeze dried and characterized by $^1$H NMR ($D_2O$ solvent). The yield was about 73%, and the conversion rate of PEG to PEG-COOH was about 100%.

The second stage is a carbodiimide reaction. 4 g (0.00097 mol) of newly prepared PEG-COOH was taken in a 200 mL triangular flask, and dissolved for 2 to 4 hours in 50 mL of distilled water. 1.05 g (0.0070 mol) of HOBt was dissolved in 10 mL of DMSO (Wako), after which 1.49 g (0.0.0078 mol) of WSCD HCl (Peptide Institute, Inc.) was dissolved in 10 mL of distilled water and added dropwise to the PEG-COOH solution. The solution was stirred for a further 10 minutes. 3.399 g (0.019 mol) of adipohydrazide was dissolved in 15 mL of pure water, and added to the PEG-COOH solution. The pH of the final reaction mixture was maintained around 7.5 pH with 1 N NaOH as the mixture was stirred for 16 to 20 hours at room temperature. The solution was then dialyzed extensively for 48 hours with deionized water using a dialysis tube (MWCO 1 kDa, Spectra/Pro®), and then freeze dried. Binding of PEGDH was confirmed by $^1$H NMR. The degree of substitution was about 91%.

<Synthesis of AL-ABA-PEGDH>

The AL-ABA (2) prepared in Example I-2 (High DS, modification rate (colorimetric): 0.64) was dissolved in physiological saline as a solvent to prepare a 1.5% w/v AL-ABA (2) solution.

The PEGDH prepared above was dissolved in water to prepare a 10% w/v PEGDH solution. The two solutions were mixed so that the aldehyde groups of the AL-ABA (2) solution and the hydrazide groups of the PEGDH solution were in equal molar amounts (1:1). Specifically, 0.5 mL of 10% w/v PEGDH solution (hydrazide groups: $2.27 \times 10^{-5}$ mol) was mixed mechanically with a pipette into 1 mL of 1.5% w/v AL-ABA solution (aldehyde groups: $6.9 \times 10^{-5}$ mol). A hydrogel formed within 30 seconds (Fig. 36).

<Dynamic viscoelasticity measurement>

The storage modulus G' and loss modulus G" were measured with a rheometer.

The results are shown in Fig. 37. Within the measurement frequency range, the G' of the AL-ABA-PEGDH was confirmed to be greater than the G", confirming actual formation of a hydrogel.

[Example I-20] Benzaldehyde-modified alginic acid films (non-crosslinked and crosslinked AL-ABA films)

<Film preparation>

(1) Non-crosslinked AL-ABA film

A non-crosslinked AL-ABA film was prepared by solution casting and a vacuum heat drying process. Specifically, the AL-ABA (2) prepared in Example I-2 (High DS, modification rate (colorimetric): 0.64) was dissolved in distilled water to prepare a 1.5% w/v AL-ABA solution. After this had completely dissolved, bubbles were removed for 15 minutes by ultrasound treatment. The solution was then poured into a 60 mm petri dish, and the petri dish was kept for 40 to 48 hours in a vacuum drier at 50°C, after which the film was detached and stored at 4°C. The film was a transparent, non-porous film with a thickness of about 63 μm.

(2) Crosslinked AL-ABA film

The non-crosslinked AL-ABA film obtained in (1) above was crosslinked with PEGDH to obtain a crosslinked AL-ABA film. Specifically, the PEGDH synthesized in Example I-19 above was dissolved in an aqueous butanol solution (butanol:water = 9: 1), and the film obtained in (1) above was immersed in this solution and maintained for 6 to 8 hours at room temperature. The film was removed from the PEGDH solution, dried in atmosphere, and stored at 4°C. The resulting crosslinked AL-ABA film was a transparent non-porous film.

<Swelling and dissolution test of films>

(Test procedures)

The non-crosslinked AL-ABA film obtained in (1) above, the crosslinked AL-ABA film obtained in (2) above and an AL film were each immersed for 2 to 4 weeks in PBS (pH = 7.4), and swelling and dissolution of the films were observed. The AL film was prepared by the methods of (1) above except that AL-500 (sodium alginate, viscosity 400 to 600 mPa·s, manufactured by Mochida Pharmaceutical Co., Ltd.) was used in place of the AL-ABA (2) prepared in Example I-2.

(Results)

The results are shown in the following Table.

[Table 3]

Table 3 Film swelling and dissolution test results

| | Swelling after 6 days | Dissolution |
|---|---|---|
| AL Film | - | Dissolved |
| Non-crosslinked AL-ABA film | Large swelling | Dissolved after 1 week |

(continued)

| | Swelling after 6 days | Dissolution |
|---|---|---|
| Crosslinked AL-ABA film | Swelling somewhat less than with non-crosslinked AL-ABA film | No dissolution after 20 days |

[Example I-21] Hydrogels of AL-ABA with polyallylamines (AL-ABA-PAA1, AL-ABA-PAA2, AL-ABA-PAA3, AL-ABA-PAA4)

[C50]

(i)

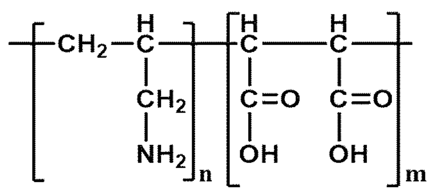

PAA1

(ii)

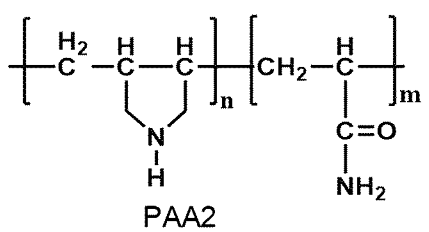

PAA2

(iii)

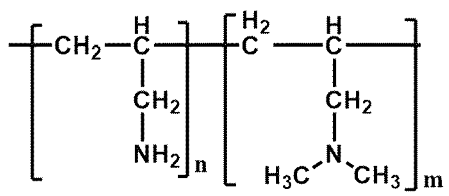

PAA3

(iv)

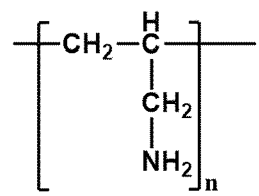

PAA4

(i) PAA1: Product name allylamine maleic acid copolymer (Product No. PAA-1151, Nittobo Medical Co., Ltd., 20 wt% viscosity 20 cp at 20°C)
(ii) PAA2: Diallyl dimethyl ammonium chloride acrylamide copolymer (Product No. PAS-J-81, Nittobo Medical Co., Ltd., 25 wt% viscosity 900 cp at 20°C), weight-average molecular weight Mw: 180,000
(iii) PAA3: Allylamine hydrochloride dimethylallylamine salt copolymer (Product No. PAA-1112CL, Nittobo Medical Co., Ltd., 15 wt% viscosity 5 cp at 20°C)
(iv) PAA4: Allylamine hydrochloride polymer (Product No. PAA-HCl-10L, Nittobo Medical Co., Ltd., 40 wt% viscosity 1500 cp at 20°C, weight-average molecular weight Mw: 150,000

The polyallylamines (PAA1 to PAA4) of (i) to (iv) above were each dissolved in water to prepare 10% w/v PAA solutions (PAA1 solution, PAA2 solution, PAA3 solution, PAA4 solution).

The AL-ABA (2) prepared in Example I-2 (Low DS; modification rate (colorimetric): 0.56) was dissolved in physiological saline as a solvent to prepare a 2% w/v AL-ABA solution. The two solutions were mixed so that the aldehyde groups of the AL-ABA solution and the primary amino groups ($-NH_2$) of the PAA solution were in equal amounts (1:1). Specifically, each 10% w/v PAA solution (PAA1 solution, PAA2 solution, PAA3 solution, PAA4 solution) (amino groups: $2.59 \times 10^{-4}$ mol in each) was mixed mechanically with a pipette into 5 mL of the 2% w/v AL-ABA solution (aldehyde groups: $2.59 \times 10^{-4}$ mol). A hydrogel formed within 20 to 30 seconds in all cases (Fig. 38).

6. Tissue adhesive materials using AL-ABA

[Example I-22] Benzaldehyde-modified alginic acid (AL-ABA) as tissue adhesive material (adhesion to submucosa)

<Evaluation of adhesion behavior to mucosa>

The adhesiveness of AL-ABA on submucosa was studied. Because submucosa is rich in collagen, it contains many amino groups. The following test confirmed that crosslinked structures are formed by Schiff bases between the amino groups of the submucosa and the aldehyde groups of the AL-ABA, potentially improving adhesiveness. The specific test procedures are as follows.

(Test procedures)

1. Pig esophagus was cut open lengthwise, and then cut into 2 cm × 2 cm sections. The mucosal layer lining the esophagus was cut away to expose the submucosa. 80 mL security containers were filled with 25 mL each of physiological saline with 0.1% of added sodium benzoate. The esophagus sections with the exposed submucosa were immersed one by one in these, placed in the shaker of a 37°C incubator, and shaken overnight. After being shaken the esophagus sections were weighed (n = 4).
2. AL-ABA and unmodified AL (IL-6G) for purposes of comparison were each dissolved to 2 w/v% in pure water. The AL-ABA (1) prepared in Example I-1 was used as the AL-ABA.
3. The alginic acid solution (AL-ABA aqueous solution or AL aqueous solution) prepared in 2 above was placed in one side of a double syringe, and 50 mM CaCh aqueous solution in the other side.
4. The sections prepared in 1 above were removed from the security containers and placed on petri dishes. 0.5 mL of the alginic acid solution prepared in 3 and 0.5 mL of 50 mM CaCh aqueous solution were sprayed simultaneously from above the sections using the double syringe with nitrogen gas. The nitrogen gas flow rate was 2 L/min. This was left standing for 10 minutes at room temperature (25°C) to gel. This treatment caused the benzaldehyde-modified alginic acid (AL-ABA) or alginic acid (AL) to be crosslinked by the calcium ($Ca^{2+}$) and form a hydrogel (AL-ABA gel, AL gel).
5. The gel on the petri dish was rinsed with pure water, moisture on the esophagus section was wiped off, and the esophagus section was weighed. The weight of the gel on the esophagus section was calculated by subtracting the weight of the esophagus section itself (measured in 1 above) from this weight. This was then given as the gel weight after 0 hours.
6. The physiological saline with the 0.1% added sodium benzoate in the security container was replaced with fresh physiological saline. The gel surface of the esophagus section was then immersed in this upside-down. This was placed on a shaker and shaken in a 37°C incubator.
7. The esophagus section was weighed after specified times (after 1, 2, 3, 4, 6, 8, 12, 24 and 48 hours), and the remaining gel weight was calculated by subtracting the weight of the esophagus section itself (measured in 1 above) from this weight. The percentage of residual gel at each time point was determined from the gel weight after 0 hours and the remaining gel weight [(remaining gel weight)/(gel weight after 0 hours) × 100]. The external appearance of the gel was also observed at each time point.

These steps were applied to 4 samples each of AL and AL-ABA, and the average value of the percentage of residual gel at each time point was given as the adhesion rate (%) of the gel.

(Results)

The results are shown in Fig. 39 and Fig. 40. Fig. 39 shows the external appearance of the AL gel and AL-ABA gel at each time point. Fig. 40 shows the adhesion rate (%) of the AL gel and AL-ABA gel at each time point.

As shown in Fig. 39, the gel had peeled off in 3 out of 4 samples in the AL spray group (ALG (IL-6G), AL gel) after 1 hour. In the remaining sample, the gel had peeled off after 2 hours. In the AL-ABA spray group (ALG-ABA, AL-ABA gel), the gel began to peel off gradually after 2 hours, and some residual gel was observed even after 12 hours. Fig. 40 also shows that while the gel adhesion rate (%) reached zero after 3 hours in the AL spray group (ALG (IL-6G), AL gel), in the AL-ABA spray group (ALG-ABA, AL-ABA gel) the gel adhesion rate was still about 40%. These results suggest that adhesiveness can be improved by modifying AL with ABA.

[Example I-23] AL-ABA as tissue adhesive material (evaluating adhesiveness on esophageal mucosa and submucosa and effects of $Ca^{2+}$ concentration) (37°C)

<Adhesion test on mucosa and submucosa>

The adhesiveness of AL-ABA and AL on esophageal mucosa and submucosa was investigated in a 37°C environment. The samples with mucosa were called Controls, while the samples in which the mucosa was peeled off to expose the submucosa were called ESD samples. Two $Ca^{2+}$ concentrations of 50 M and 100 mM were also tested to investigate changes in adhesiveness at different $Ca^{2+}$ concentrations. The test procedures is shown in Fig. 42. The specific procedures are as follows.

(Materials)

AL-ABA: AL-ABA (modification rate (NMR): 0.052) prepared under the same conditions as the AL-ABA (1) of Example I-1, dissolved to 2 w/v% in pure water
AL: IL-6G (IL-6G, viscosity 50 to 80 mPa s (1%), manufactured by Kimica Co., Ltd.), dissolved to 2 w/v% in pure water
CaCh aqueous solution (for crosslinking): 50 mM or 100 mM
Esophageal sections (Control: with mucosa; ESD: mucosa peeled off to expose submucosa)

(Test procedures)

1. Pig esophagus was cut open lengthwise, and then cut into 2 cm × 2 cm sections. In the submucosa group (ESD), the mucosal layer was removed with scissors.
2. The mass of the cut pig esophageal sections (Control) and the sections with exposed submucosa (ESD) was measured.
3. AL or AL-ABA was dissolved in 2 w/v% of pure water.
4. 2.5 mL syringes were filled with the solution prepared in 3 and a CaCh solution, and set in a double syringe (0.5 mL alginic acid solution + 0.5 mL CaCh solution per sample).
5. The double syringe was fitted with a spray tip, and each esophageal section was sprayed using nitrogen gas at a rate of 4 L/minute (n = 4 per sample).
6. This was left for 10 minutes to gel. This treatment caused the benzaldehyde-modified alginic acid (AL-ABA) or alginic acid (AL) to be crosslinked by the calcium ($Ca^{2+}$) and form hydrogels (AL-ABA gel, AL gel). The mass of the esophageal section at this point was measured.
7. 25 mL of physiological saline with 0.1% methyl benzoate and 1.25 mM CaCh added thereto was placed in a glass petri dish 6 cm in diameter, and each esophageal section was immersed in this and shaken in a shaker. The 1.25 mM $Ca^{2+}$ mimics the $Ca^{2+}$ found in saliva.
8. The esophagus sections were weighed after specified times (after 1, 2, 3, 4, 6, 8, 12, 24, 48 and 72 hours), and the mass of the remaining gel was calculated. The percentage of residual gel at each time point was determined from the gel weight after 0 hours and the remaining gel weight [(remaining gel weight)/(gel weight after 0 hours) × 100]. The external appearance of the gel was also observed at each time point.

These steps were applied to 4 samples each of AL and AL-ABA with $Ca^{2+}$ concentrations of 50 mM and 100 mM using the esophageal sections (Control) and sections with exposed mucosa (ESD), and the average value of the percentage of residual gel at each time point was given as the adhesion rate (%) of the gel.

(Results)

Fig. 43 shows the external appearance of the gels at each time point (after 2, 4, 12, 24, 48 and 72 hours). Fig. 44 shows the percentage of remaining gel (gel adhesion rate, %) at each time point.

The samples with AL-ABA sprayed on submucosa (AL-ABA ESD) and the samples with AL-ABA sprayed on mucosa (AL-ABA Control; AL-ABA Con) maintained gel adhesion for a longer time in comparison with the AL spray groups (AL ESD and AL Control (Con)). This confirms that adhesiveness can be improved by modifying AL with ABA.

In particular, the samples with AL-ABA sprayed on submucosa (AL-ABA ESD) exhibited a greater improvement in adhesive strength than the samples with AL-ABA sprayed on mucosa (AL-ABA Control; AL-ABA Con). It is presumed

that strong adhesion was achieved by binding of the ABA to the amino groups of the submucosa.

Moreover, gel adhesion was also maintained for a longer time at the higher CaCh concentration (100 mM). It is believed that the crosslinking density was improved by increasing the amount of Ca ions, thereby increasing the mechanical strength of the gel and prolonging the degradation time. Because cohesive failure occurred at the maximum breaking strength in the tensile test, it appears that adhesive strength increased due to the increased mechanical strength of the gel.

<Adhesiveness evaluation by tensile testing>

Gel peeling and gel dissolution both occurred simultaneously in the above adhesive test. A tensile test was performed to investigate only the adhesiveness of AL-ABA. Fig. 45 shows the procedures in the tensile test.

(Materials)

AL-ABA: AL-ABA (modification rate (NMR): 0.052) prepared by the same methods as in Example I-1, dissolved to 2 w/v% in pure water
AL: IL-6G (IL-6G, viscosity 50 to 80 mPa·s (1%), manufactured by Kimica Co., Ltd.), dissolved to 2 w/v% in pure water
CaCh aqueous solution (for crosslinking): 50 mM or 100 mM
Esophageal sections (Control: with mucosa; ESD: mucosa peeled off to expose submucosa)

(Equipment)

Physical property measurement rheometer (Shiro Sangyo, M993R-3000S)

(Test Procedures)

1. Pig esophagus was cut open lengthwise, and then cut into 1 cm × 3 cm sections. In the mucosa group (Control), the cut sections were used as is. In the submucosa (ESD) group, the mucosal layer of each section was peeled off (n = 3).
2. Alginic acid solution (AL-ABA or AL) was added to a 1 cm × 1 cm area of each section.
3. CaCh solution was added from above to gel the alginic acid solution.
4. A weight (30 mL of water in 80 mL container) was lowered from above to remove air.
5. This was set for 1 hour in a 37°C thermostatic tank.
6. Breaking strength (N) was measured with the rheometer.
7. The breaking pressure (Pressure, Pa) was calculated from the breaking strength (N) of 6 and the area ($m^2$) of the gel (region where alginic acid solution was added).

(Results)

The results are shown in Fig. 46. In comparison with the AL hydrogel, the AL-ABA hydrogel to have significantly greater adhesive strength on the mucosa and submucosa. Changes in adhesive strength due to the concentration of the crosslinking agent ($Ca^{2+}$ ion) were also confirmed.

[Example I-24] AL-ABA as tissue adhesive material (evaluating adhesiveness on submucosa and skin tissue by lap shear method)

The adhesive strengths of AL-ABA, AL and a conventional tissue adhesive on living tissue were evaluated by a lap shear test. Specifically, the adhesive strength of each material on living tissue was evaluated by the following procedures using the pregel solution below with pig submucosa and pig skin tissue. Fig. 47 shows the test procedures for the lap shear method.

(Pregel solution)

- AL-ABA: 4 w/v% aqueous solution of AL-ABA (2) prepared in Example I-2 (High DS; modification rate (colorimetric): 0.64)
- AL500: 4 w/v% AL-500 (sodium alginate, viscosity 400 to 600 mPa s, manufactured by Mochida Pharmaceutical Co., Ltd.)
- Fibrin Glue: fibrin glue adhesive (Veriplast P, manufactured by CSL Behring Co., Ltd.)
- Hydrofit® (urethane hemostatic; Terumo Japan)
- Dermabond® (cyanoacrylate skin surface adhesive; Johnson and Johnson)

The pregel solutions were gelled as follows.
For the AL-ABA and AL500, the pregel solution (AL-ABA or AL500) was first applied and the biological tissues overlapped, after which 200 μL of a 50 mM or 100 mM CaCh aqueous solution was applied to both sides of the biological tissue (200 μL × 2).
The fibrin glue was gelled with a double syringe.
The Hydrofit® is a one-component sealant that was applied as is.
The Dermabond® is a one-component sealant that was applied as is.

(Biological tissue)

Submucosa: Pig esophagus was cut to a length of 40 mm and a width of 10 mm, and the mucosal layer lining the esophagus was cut away to expose the submucosa.
Skin tissue: Pig skin tissue was cut to a length of 40 mm and a width of 10 mm.

(Equipment)

CR3000-EX Dynamic Chemical Analyzer (DMA), manufactured by Sun Scientific Co., Ltd.

(Test procedures for lap shear test)

0.5 mL of pregel solution was coated on the overlapped areas (length 10 mm by width 10 mm) at the ends of two pieces of biological tissue (length 40 mm by width 10 mm), and gelled after overlapping. 20 minutes after gelling, a lap shear test was performed at a rate of 5 mm/minute with a Dynamic Chemical Analyzer (DMA). The adhesive energy was measured by a 180° peel test, and adhesive strength was calculated by the following formula.

$$\text{Adhesive strength} = \text{strength } (\text{N/m}^2)/\text{area of overlapping region } (\text{m}^2)$$

(Results)

The Results are shown in Fig. 48.
The AL-ABA hydrogel was shown to be useful as a tissue adhesive on biological tissue such as submucosa and skin tissue.

[Example I-25] AL-ABA as tissue adhesive material (tissue sealant) (evaluating effectiveness as sealant on submucosal tissue and skin tissue by burst test)

To investigate effectiveness as a tissue sealant, a burst test was performed using the device reported in Lei Zhou et al., ADVANCED FUNCTIONAL MATERIALS Vol. 31, Issue 14, 2021, 2007457, "Injectable Self-Healing Natural Biopolymer-Based Hydrogel Adhesive with Thermoresponsive Reversible Adhesion for Minimally Invasive Surgery" with slight modifications. Specifically, a burst test was performed by the following burst test procedures using the following pregel solutions with pig submucosa and pig skin tissue as the biological tissues. The equipment used in the burst test and the burst test procedures are shown in Fig. 49.

(Pregel solution)

- AL-ABA: 4 w/v% aqueous solution of AL-ABA (2) prepared in Example I-2 (High DS; modification rate (colorimetric): 0.64)

- AL500: 4 w/v% AL-500 (sodium alginate, viscosity 400 to 600 mPa s, manufactured by Mochida Pharmaceutical Co., Ltd.)
- Fibrin Glue: fibrin glue adhesive (Veriplast P, manufactured by CSL Behring Co., Ltd.)
- Hydrofit® (urethane hemostatic; Terumo Japan)
- Dermabond® (cyanoacrylate skin surface adhesive; Johnson and Johnson)

The pregel solutions were gelled as follows.

For the AL-ABA and AL500, the pregel solution (AL-ABA or AL500) was injected into a puncture site, 500 μL of 100 mM CaCh aqueous solution was dripped from above, and crosslinking was performed for 5 to 10 minutes.

The fibrin glue was gelled with a double syringe.

The Hydrofit® is a one-component sealant that was applied as is.

The Dermabond® is a one-component sealant that was applied as is.

(Biological tissue)

Submucosa: Pig esophagus was cut to a length of 50 mm and a width of 50 mm, and the mucosal layer lining the esophagus was cut away to expose the submucosa.

Skin tissue: Pig skin tissue was cut to a length of 50 mm and a width of 50 mm.

(Burst test procedures)

Biological tissue was set on the upper surface of a burst pressure apparatus, and punctured to prepare a hole 2 mm in diameter in the middle of the tissue. 2.5 to 3 mL of pregel solution was injected onto the prepared 2 mm hole (puncture site), and gelled.

The measurement apparatus was connected to a syringe pump filled with PBS solution. Finally, the PBS solution was injected into the apparatus, and the maximum burst pressure (mmHg) was recorded with a digital pressure gauge. The test was repeated 5 times.

(Results)

The results are shown in the following table and Fig. 50.

[Table 3]

Table 3 Burst test results

| Burst pressure (mmHg) | | | | | |
|---|---|---|---|---|---|
| Biological tissue | AL-ABA | AL500 | Fibrin Glue | Hydrofit | Dermabond |
| Submucosa | 79.5 (±23.59) | 10.49 (±0.97) | 61.9 (±19.59) | 127.4 (±9.67) | Not burst |
| Skin | 53.4 (±13.59) | 14.18 (±2.13) | 64.3 (±20.5) | 134.9 (±34.31) | Not burst |

As shown in Fig. 50, the AL-ABA hydrogen did not perform significantly differently from the fibrin glue, which indicates that it can be used as a tissue sealant.

II. Hyaluronic acid derivatives

[Example II-1] Benzaldehyde-modified hyaluronic acid (HA-ABA)

<Synthesis of HA-ABA>

[C51]

Synthesis scheme 3

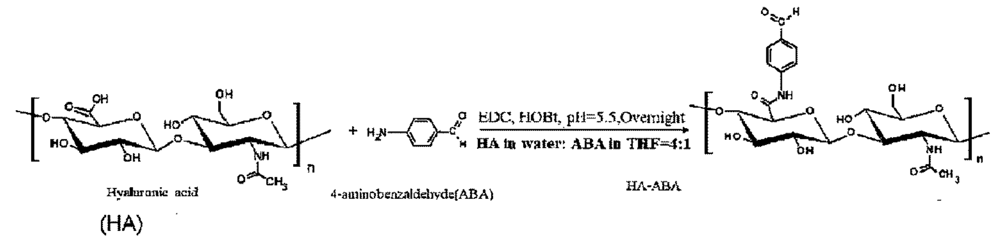


Following the above synthesis scheme, HA-ABA was synthesized according to the following procedures by an amidation reaction via carbodiimide.

(Synthesis procedures)

100 mL of HA (Mw: 890 kDa) aqueous solution (2 mg/mL) was dissolved in 25 mL of THF. This was mixed with 121 mg of 4-amino-benzaldehyde (ABA) (corresponding to 2 equivalents of ABA relative to the carboxyl groups of HA). 2 equivalents of 1-ethyl-3-(3-dimethylaminopropyl) carbodiimide hydrochloride (EDC·HCl) and 2 equivalents of 1-hydroxybenzotriazole (HOBt) were added, the pH was adjusted to 5.5, and the mixture was stirred overnight at room temperature. This was then purified by dialysis and freeze dried to obtain HA-ABA.

<Molecular weight measurement>

The weight-average molecular weight (Mw) of the HA was measured under the following conditions by gel filtration chromatography (GPC).

(Measurement procedures)

The molecular weight distribution of the polymer was measured by gel filtration chromatography (GPC). A liquid transport unit for high performance liquid chromatography (LC-10ADVP, Shimadzu Corp.) was used as the pump, and an intelligent refractive index detector (830-RI, JASCO) as the detector. Separation was performed at room temperature using TSK-GEL GMPWXL (molecular weight cutoff 104 to 106, Tosoh) and TSK-GEL-3000 (molecular weight cutoff 103 to 105, Tosoh) as the columns. The flow rate was set at 0.5 mL/min, and the above phosphoric acid buffer (pH 6.7) was used as the eluent. A calibration curve was prepared using dextran (8,000 Da, 15,000 Da, 40,000 Da, 70,000 Da, 500,000 Da, Extrasynthese Co.). The day before measurement, solvent substitution was performed overnight with phosphoric acid buffer at a flow rate of 0.2 mL/min. The calibration curve solutions and sample solutions were each filtered with a 0.22 μm filter. Stabilization of the RI value was confirmed before measurement, measurement was initiated, and after use solvent substitution was performed again overnight with pure water at a flow rate of 0.2 mL/min.

<$^1$H NMR spectrum measurement, UV-visible light absorption spectrum (UV-vis) measurement and FT-IR spectrum measurement>

The HA-ABA was subjected to $^1$H NMR spectrum measurement, UV-visible light absorption spectrum (UV-vis) measurement and FT-IR spectrum measurement. The results are shown in Fig. 51 to Fig. 53.

In the $^1$H NMR spectrum (Fig. 51), peaks derived from ABA (peak a derived from methyl groups of HA, peaks b and c derived from benzene rings and peak d derived from aldehyde) were observed in the HA-ABA. A characteristic peak (at about 250 nm) derived from the benzyl groups of ABA was observed in the UV-vis (Fig. 52) spectrum of the HA-ABA, and absorption (peak at about 2950 $cm^{-1}$) attributable to ABA was also observed in the FT-IR spectrum of the HA-ABA (Fig. 53). These results confirmed synthesis of benzaldehyde-modified hyaluronic acid (HA-ABA) formed by binding between amino groups of 4-aminobenzaldehyde and carboxyl groups of hyaluronic acid. Based on the peaks at 1.95 ppm (a: $-CH_3$ of acetamide part of N-acetyl-D-glucosamine), 6.75 ppm (b: -CH of benzyl rings), 7.65 ppm (c: -CH of benzyl rings) and 9.44 ppm (d: -CH of aldehyde) in the $^1$H NMR spectrum, the ABA modification rate of the carboxyl group (-COOH) of the hyaluronic acid was calculated as 0.16.

<Cell toxicity evaluation (WST assay) of benzaldehyde-modified hyaluronic acid (HA-ABA)>

(Test procedures)

MeT-5A (human mesothelial cell line), HUVEC (human umbilical vein endothelial cells), RAW264.7 cells (mouse macrophage-like cell line), NIH/3T3 (mouse embryo fibroblasts) and AB22 cells (mouse mesothelioma cells) were seeded on 24-well plates and incubated for 24 hours. The medium was then replaced with media having HA-ABA and HA dissolved at different concentrations (0.01 mg/mL, 0.1 mg/mL, 1 mg/mL). Cell viability (%) 48 hours after sample addition was then measured by WST-8 assay (Cell Counting Kit-8, Dojindo).

(Results)

The results are shown in Fig. 54. The HA-ABA exhibited high cell viability equivalent to HA in most cells. In the NIH/3T3 mouse embryo fibroblasts, cell viability reduced to 20% with 1 mg/mL of HA-ABA. This may be related to different expression of CD44 in different kinds of cells.

[Example II-2] Benzaldehyde-modified hyaluronic acid-pemetrexed conjugate (HA-ABA-PMX)

<Synthesis of HA-ABA-PMX>

[C52]

Synthesis scheme 4

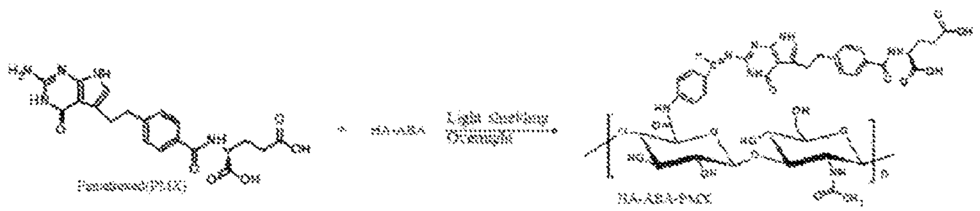

Pemetrexed (PMX) is an anticancer drug having a primary amino group. PMX was linked to HA-ABA according to the above synthesis scheme by a Schiff base reaction between the aldehyde groups of the HA-ABA and the amino groups of the pemetrexed.

Specifically, the HA-ABA obtained in Example II-1 was dissolved in pure water to a concentration of 1 mg/mL, and 1.5 equivalents of PMX dissolved in DMSO were added dropwise. This was reacted overnight at room temperature under shaded conditions. This was then dialyzed and freeze dried to obtain HA-ABA-PMX.

<$^1$H NMR spectrum measurement, and UV-visible light absorption spectrum (UV-vis) measurement>

The resulting HA-ABA-PMX was subjected to $^1$H NMR spectrum measurement, and UV-visible light absorption spectrum (UV-vis) measurement. The results are shown in Fig. 55 and Fig. 56.

In the $^1$H NMR spectrum (Fig. 55), peaks derived from HA-ABA and PMX were observed in the HA-ABA-PMX. In the UV-vis spectrum (Fig. 56), an increase in a characteristic peak of PMX (225 nm) was observed in the HA-ABA-PMX. These results confirm formation of a conjugate between HA-ABA and PMX. Based on the $^1$H NMR spectrum, the conjugate rate of the drug (PMX) to the carboxyl groups (-COOH) of the HA was 11.5%. This shows that 71.8% of the ABA in the HA-ABA reacted with the PMX.

These results show that conjugation of PMX to the HA-ABA was accomplished simply by mixing the HA-ABA and PMX in an aqueous solvent. Since this reaction can be applied to a variety of drugs containing primary amines, HA-ABA is expected to provide a simple and general-purpose platform for developing hyaluronic acid-drug conjugates.

<Release test of PMX from benzaldehyde-modified hyaluronic acid-pemetrexed conjugate (HA-ABA-PMX)>

(Test procedures)

The release behavior or PMX from HA-ABA-PMX at different pH values was investigated using a dialysis tube (MWCO: 1 kDa). 1 mL of an aqueous solution of the HA-ABA-PMX (1 mg/mL) obtained in Example II-2 above was placed in a dialysis tube, and shaken at 50 rpm while being incubated with 79 mL of PBS (37°C, pH = 5.0, 6.0 7.4). The external solution (sustained release solution) was collected at specific time points, and new PBS was substituted for the entire amount. The drug concentration in the collected external solution (sustained release solution) was measured by UV-vis, and the release rate of the drug PMX was calculated.

For purposes of comparison as control tests, the same release test was also performed with the HA-ABA-PMX solution replaced by an aqueous solution of PMX (0.12 mg/mL) (Free PMX), a mixed solution of HA and PMX obtained by dissolving hyaluronic acid (HA) (1 mg/mL) and PMX (0.12 mg/mL) in water (Free PMX mixed with HA), and an aqueous solution of HA-ADH-PMX having the HA bound irreversibly to the PMX by amide bonds.

The HA-ADH-PMX has the following structure. The HA-ADH-PMX was synthesized by the methods described in Amano Y., European Journal of Pharmaceutical Sciences, 2019, 138: 105008.

[C53]

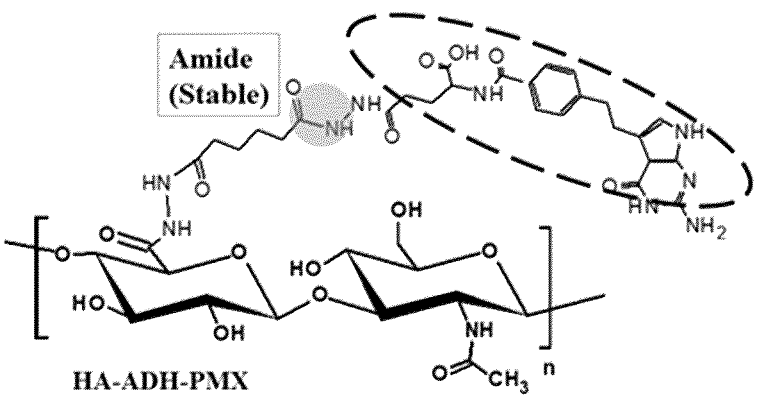


HA-ADH-PMX

(Results)

The results are shown in Fig. 57. Fig. 57 shows the release behavior of pemetrexed (PMX) from a benzaldehyde-modified hyaluronic acid-pemetrexed conjugate solution (HA-ABA-PMX) at different pH values (pH = 5.0, 6.0, 7.4), the release behavior of PMX from a PMX solution (Free PMX), and the release behavior of PMX from a mixed solution of HA and PMX (Free PMX mixed with HA). The vertical axis shows the cumulative release rate of PMX (cumulative drug release, %).

While more than 80% of the PMX was released at pH 5.0 after 24 hours with the HA-ABA-PMX, 40% had not been released at pH 7.4. These results show that PMX is released more rapidly at lower pH values. This is thought to be due to the fact that the imine bonds between HA-ABA and PMX are disassociated more easily at low pH.

In the case of the free PMX solution (Free PMX) and the mixed solution of free PMX and HA (Free PMX mixed with HA), on the other hand, there was no sustained release effect and almost all of the PMX was rapidly released within 2 hours. In the case of the solution of HA-ADH-PMX having irreversible amide bonds, almost no release of PMX from the HA-ADH-PMX was observed. The results suggest that HA-ABA-PMX allows pH dependent sustained drug release due to the formation of Schiff bases.

These results show that in comparison with a control group containing free PMX, the HA-ABA-PMX conjugate allows continuous sustained release of PMX, and also allows pH-dependent changes in the release rate of PMX, with the drug being released selectively at low pH.

[C54]

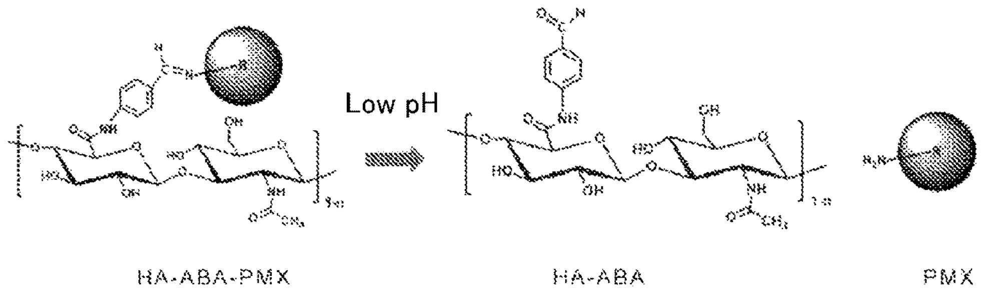


<Cell toxicity evaluation (WST assay) of benzaldehyde-modified hyaluronic acid-pemetrexed conjugate (HA-ABA-PMX)>

(Test procedures)

As with the HA-ABA, the cell toxicity of HA-ABA-PMX in MeT-5A (human mesothelial cell line) and AB22 cells (mouse mesothelioma cells) was evaluated by WST assay.

Specifically, MeT-5A (human mesothelial cell line) and AB22 cells (mouse mesothelioma cells) were seeded on 24-well plates and incubated for 24 hours. The medium was then replaced with medium having HA-ABA-PMX dissolved at different PMX concentrations ($10^{-4}$ μg/mL, $10^{-3}$ μg/mL, $10^{-2}$ μg/mL, $10^{-1}$ μg/mL, 1 μg/mL, 10 μg/mL). Cell viability (%) 48 hours after sample addition was then measured by WST-8 assay (Cell Counting Kit-8, Dojindo).

For purposes of comparison as a control test, cell toxicity was also evaluated using free PMX and HA-ADH-PMX in place of HA-ABA-PMX.

(Results)

The results are shown in Fig. 58. Fig. 58A and Fig. 58B show the cell growth inhibition effects (vertical axis: cell viability (%)) of HA-ABA-PMX, free pemetrexed (PMX) and HA-ADH-PMX having PMX irreversibly bound to HA by amide bonds at different PMX concentrations ($10^{-4}$ μg/mL, $10^{-3}$ μg/mL, $10^{-2}$ μg/mL, $10^{-1}$ μg/mL, 1 μg/mL, 10 μg/mL) in AB22 cells (mouse mesothelioma cells) and MeT-5A cells (human mesothelial cell line), respectively.

Both the HA-ABA-PMX and the free PMX exhibited dose-dependent cell growth inhibition effects in AB22 and MeT-5A. Moreover, the HA-ABA-PMX reduced cell viability at lower concentrations than the free PMX and the HA-ADH-PMX having PMX irreversibly bound to HA by amide bonds. These results suggest the strong cell growth inhibition effects of HA-ABA-PMX. This is thought to be due to enhanced cellular uptake via HA-CD44 interactions, along with irreversible dissociation of the conjugated PMX.

[Example II-3] Benzaldehyde-modified hyaluronic acid-doxorubicin conjugate (HA-ABA-DOX)

<Synthesis of HA-ABA-DOX>

[C55]

Doxorubicin (DOX)

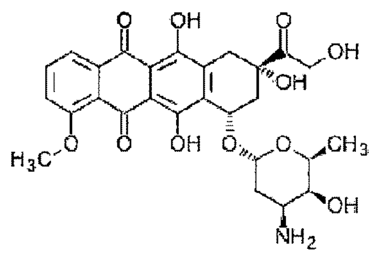

Doxorubicin (DOX) is an anticancer drug having a primary amino group. A benzaldehyde-modified hyaluronic acid-doxorubicin conjugate (HA-ABA-DOX) was synthesized by the same methods as Example II-2.

Specifically, the HA-ABA obtained in Example II-1 was dissolved in pure water at a concentration of 1 mg/mL, and 1.5 equivalents of DOX hydrochloride dissolved in pure water were added dropwise. This was reacted overnight at room temperature under shaded conditions. This was then dialyzed and freeze dried to obtain HA-ABA-DOX.

<UV-vis absorption spectrum (UV-vis) measurement>

The resulting HA-ABA-DOX was subjected to UV-vis absorption spectrum (UV-vis) measurement. The results are shown in Fig. 59.

In UV-vis (Fig. 59), the HA-ABA-DOX exhibited a peak attributable to doxorubicin (DOX), confirming formation of a conjugate of HA-ABA and DOX.

<Release test of DOX from benzaldehyde-modified hyaluronic acid-doxorubicin conjugate (HA-ABA-DOX)>

(Test procedures)

Release of DOX from HA-ABA-DOX under different pH conditions was investigated in the same manner as in Example II-2. Specifically, 1 mL of an aqueous solution of the HA-ABA-DOX (1 mg/mL) obtained in Example II-3 above was placed in a dialysis tube (MWCO: 1 kDa), and shaken at 50 rpm while being incubated with 79 mL of PBS (37°C, pH = 5.0, 6.0 7.4). The external solution (sustained release solution) was collected at specific time points, and new PBS was substituted for the entire amount. The drug concentration in the collected external solution (sustained release solution) was measured by UV-vis, and the release rate of the drug DOX was calculated.

(Results)

The results are shown in Fig. 60. Fig. 60 shows the release behavior of doxorubicin (DOX) from a benzaldehyde-modified hyaluronic acid-doxorubicin conjugate solution (HA-ABA-DOX) at different pH values (pH = 5.0, 6.0, 7.4). The vertical axis shows the cumulative release rate of DOX (cumulative drug release, %).

It was shown that the HA-ABA-DOX conjugate is capable of selective drug release at low pH, and that the DOX release rate changes in a pH-dependent manner.

<Cell toxicity evaluation (WST assay) of benzaldehyde-modified hyaluronic acid-doxorubicin conjugate (HA-ABA-DOX)>

(Test procedures)

As with the HA-ABA, the cell toxicity of HA-ABA-DOX in AB22 cells (mouse mesothelioma cells) was evaluated by WST assay.

Specifically, AB22 cells (mouse mesothelioma cells) were seeded on 24-well plates and incubated for 24 hours. The medium was then replaced with media having HA-ABA-PMX dissolved at different DOX concentrations ($10^{-3}$ $\mu$g/mL, $10^{-2}$ $\mu$g/mL, $10^{-1}$ $\mu$g/mL, 1 $\mu$g/mL, 10 $\mu$g/mL, 100 $\mu$g/mL). Cell viability (%) 48 hours after sample addition was then measured by WST-8 assay (Cell Counting Kit-8, Dojindo).

For purposes of comparison as a control test, cell toxicity was also evaluated using free DOX in place of HA-ABA-DOX.

(Results)

The results are shown in Fig. 61. Fig. 61 shows that HA-ABA-DOX and free DOX both had dose-dependent cell growth inhibition effects on AB22. In comparison with the free DOX, the HA-ABA-DOX depressed cell viability at lower concentrations. These results suggest the strong cell growth inhibition effects of HA-ABA-DOX.

[Example II-4] Benzaldehyde-modified hyaluronic acid-gemcitabine conjugate (HA-ABA-GEM)

<Synthesis of HA-ABA-GEM>

[C56]

Gemcitabine (GEM)

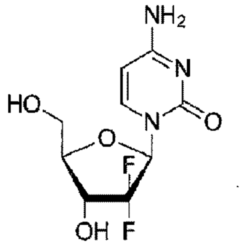

Gemcitabine (GEM) is an anticancer drug having a primary amino group. A benzaldehyde-modified hyaluronic acid-gemcitabine conjugate (HA-ABA-GEM) was synthesized in the same manner as in Example II-2.

Specifically, the HA-ABA obtained in Example II-1 was dissolved in pure water at a concentration of 1 mg/mL, and 1.5 equivalents of GEM dissolved in pure water were added dropwise. This was reacted overnight at room temperature under shaded conditions. This was then dialyzed and freeze dried to obtain HA-ABA-GEM.

<UV-vis absorption spectrum (UV-vis) measurement>

The resulting HA-ABA-GEM was subjected to UV-vis absorption spectrum (UV-vis) measurement. The results are shown in Fig. 62.

In UV-vis (Fig. 62), the AL-ABA-GEM exhibited a peak attributable to gemcitabine (GEM), confirming the formation of a conjugate of HA-ABA and GEM.

[Example II-5] Benzaldehyde-modified hyaluronic acid-DNA nucleotide conjugates (HA-ABA-adenine, HA-ABA-cytosine, HA-ABA-guanine)

<Synthesis of HA-ABA-adenine, HA-ABA-cytosine and HA-ABA-guanine>

[C57]

Adenine

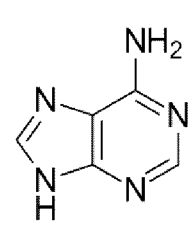

Cytosine

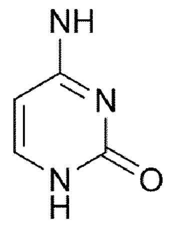

Guanine

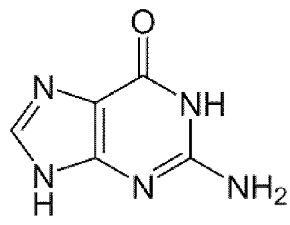

Conjugates of benzaldehyde-modified hyaluronic acid with the DNA nucleotides adenine, cytosine and guanine (HA-ABA-adenine, HA-ABA-cytosine, HA-ABA-guanine) were synthesized in the same manner as in Example II-2.

Specifically, the HA-ABA obtained in Example 8 was dissolved in pure water at a concentration of 1 mg/mL, and 2.5 equivalents of adenine, cytosine or guanine dissolved in pure water were added dropwise. This was reacted overnight at room temperature under shaded conditions, and then dialyzed and freeze dried to obtain HA-ABA-adenine, HA-ABA-cytosine and HA-ABA-guanine.

<$^{1}$H NMR spectrum measurement and FT-IR spectrum measurement>

The resulting HA-ABA-adenine, HA-ABA-cytosine and HA-ABA-guanine were subjected to $^{1}$H NMR spectrum

measurement and FT-IR spectrum measurement, with the results shown in Fig. 63 to Fig. 66.

A peak derived from HA (e) and peaks derived from adenine (a to d) were observed in the $^1$H NMR spectrum (Fig. 63) of the HA-ABA-adenine.

A peak derived from HA (e) and peaks derived from cytosine (a to d) were observed in the $^1$H NMR spectrum (Fig. 64) of the HA-ABA-cytosine.

A peak derived from HA (d) and peaks derived from guanine (a to c) were observed in the $^1$H NMR spectrum (Fig. 65) of the HA-ABA-guanine.

Moreover, absorption attributable to binding between HA and ABA (stretching and ring vibration of C=O, C=C, C=N) was observed in the FT-IR spectra (Fig. 66) of the HA-ABA-adenine, HA-ABA-cytosine and HA-ABA-guanine as well as HA-ABA.

These results confirm that conjugates were formed between HA-ABA and DNA nucleotides (adenine, cytosine or guanine). The modification rates (drug introduction rates) of the drugs (DNA nucleotides) were calculated from the $^1$H NMR spectra. The conjugate rate of adenine on the carboxyl groups (-COOH) of HA was 8%, indicating that 38% of the ABA in the HA-ABA had reacted with the adenine. The conjugate rate of the cytosine on the carboxyl groups (-COOH) of HA was 16%, indicating that 76.1% of the ABA in the HA-ABA had reacted with the cytosine. The conjugate rate of the guanine on the carboxyl groups (-COOH) of HA was 9%, indicating that 42.8% of the ABA in the HA-ABA had reacted with the guanine.

These results show that a DNA nucleotide can be made to bind (conjugate) with HA-ABA simply by mixing the HA-ABA with the DNA nucleotide in an aqueous medium. HA-ABA is therefore expected to provide a simple and general-purpose platform for developing hyaluronic acid-nucleic acid drug conjugates.

III. Carboxymethyl cellulose derivative

[Example III-1] Benzaldehyde-modified carboxymethyl cellulose (CMC-ABA)

<Synthesis of CMC-ABA>

[C58]

Synthesis scheme 5

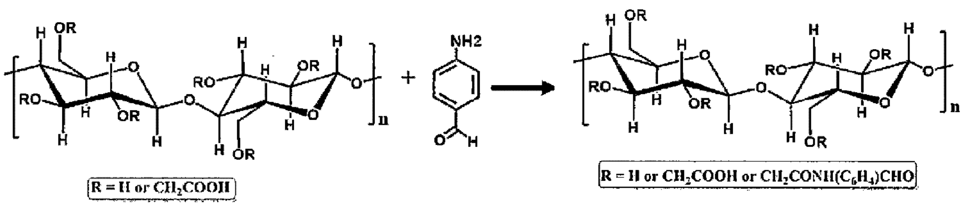

CMC ABA CMC-ABA

Following the above synthesis scheme, CMC-ABA was synthesized by the following procedures by an amidation reaction via carbodiimide.

(Synthesis procedures)

0.3 g of CMC (Sigma-Aldrich, Product No. 419338-100G, product name: Sodium carboxymethyl cellulose, weight-average molecular weight: about 700,000 (catalog value)) was dissolved in 100 mL of distilled water in a 100 mL round-bottom flask, and shaken overnight to prepare a CMC solution. 2.16 g (0.016 mol) of HOBt (1-hydroxybenzotriazole hydrate, Tokyo Chemical Industry Co., Ltd., Product No.: H0468, weight-average molecular weight: 135.13) was dissolved in 10 mL of DMSO and added dropwise to the CMC solution, after which 3.06 g (0.016 mol) of WSCD HCl (Peptide Institute, Inc., Product No. 1030, weight-average molecular weight: 191) was dissolved in 15 mL of water and added dropwise to the CMC solution. The solution was then further stirred for 10 minutes. 1.38 g (0.011 mol) of 4-aminobenzaldehyde (ABA) was dissolved in 12 mL of tetrahydrofuran (THF, Fujifilm Wako Pure Chemical, Product No. 206-08744, weight-average molecular weight: 72.11), and added dropwise to the CMC solution. 1N NaOH (sodium hydroxide, Fujifilm Wako Pure Chemical, Product No. 198-13765, weight-average molecular weight: 40) was added drop by drop to the mixture as the pH was adjusted to a range of 7.5 to 8. The mixture was then stirred for 16 to 20 hours

at room temperature. The reaction mixture was centrifuged for 10 minutes at 3,000 rpm. The supernatant was collected and dialyzed for 72 hours with pure water using a dialysis membrane (MWCO 6-8 kDa, Spectra/Pro®), and freeze dried to obtain CMC-ABA.

<$^1$H NMR spectrum measurement and FT-IR spectrum measurement>

The CMC-ABA was subjected to $^1$H NMR spectrum measurement and FT-IR spectrum measurement, with the results shown in Fig. 67 and Fig. 68.

A peak derived from CMC (e) and peaks derived from ABA (a to d) were observed in the $^1$H NMR spectrum (Fig. 67). Absorption attributable to binding between CMC and ABA (C=O stretching of amide bond, C-H stretching of aldehyde and N-H stretching) was also observed in the FT-IR spectrum (Fig. 68) of the CMC-ABA. Moreover, based on the $^1$H NMR spectrum the ABA modification rate of the carboxyl groups (-COOH) of the CMC was calculated to be 0.4866 (86.6 mol%).

IV. Carboxymethyl dextran derivatives

[Example IV-1] Benzaldehyde-modified carboxymethyl dextran (CMDX-ABA)

<Synthesis of CMDX-ABA>

[C59]

Synthesis scheme 6

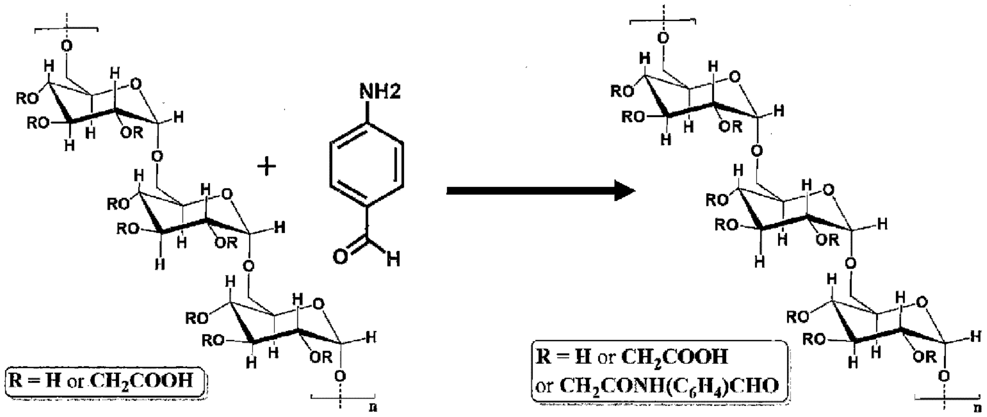

CMDX ABA CMDX-ABA

Following the above synthesis scheme, CMDX-ABA was synthesized by the following procedures by an amidation reaction via carbodiimide.

(Synthesis procedures)

0.5 g (0.0030 mol) of CMDX (Meito Industry Co., Ltd., Product name: carboxymethyl dextran, Product No. CMD-500-0613, weight-average molecular weight: 580,000 (catalog value)) was dissolved in 50 mL of pure water in a 100 mL round-bottom flask, and shaken overnight to prepare a CMDX solution. 2.83 g (0.021 mol) of HOBt was dissolved in 10 mL of DMSO and added dropwise to the CMDX solution, after which 4.01 g (0.021 mol) of WSCD HCl was dissolved in 20 mL of water and added dropwise to the CMDX solution. The solution was then further stirred for 10 minutes. 1.81 g (0.015 mol) of 4-amino-benzaldehyde (ABA) was dissolved in 12 mL of THF, and added dropwise to the CMDX solution. 1N NaOH was added drop by drop as the pH of the mixture was adjusted to a range of 7.5 to 8. The mixture was then stirred for 16 to 20 hours at room temperature. The reaction mixture was centrifuged for 10 minutes at 3,000 rpm. The supernatant was dialyzed for 72 hours with pure water using a dialysis membrane (MWCO 6-8 kDa, Spectra/Pro®), and freeze dried to obtain CMDX-ABA.

<$^1$H NMR spectrum measurement and FT-IR spectrum measurement>

The CMDX-ABA was subjected to $^1$H NMR spectrum measurement and FT-IR spectrum measurement, with the results shown in Figs. 69 and 70.

Peaks derived from CMDX (e, f) and peaks derived from ABA (a to d) were observed in the $^1$H NMR spectrum (Fig. 69). Absorption attributable to binding between CMDX and ABA (C=O stretching of amide bond, C-H stretching of aldehyde and N-H stretching) was also observed in the FT-IR spectrum (Fig. 70) of the CMDX-ABA. Moreover, based on the $^1$H NMR spectrum the ABA modification rate of the carboxyl groups (-COOH) of the CMDX was calculated to be 0.43 (43 mol%).

[Example IV-2] Benzaldehyde-modified carboxymethyl dextran-DNA nucleotide conjugates (CMD-ABA-adenine, CMD-ABA-cytosine, CMD-ABA-guanine)

<Synthesis of CMD-ABA-adenine, CMD-ABA-cytosine and CMD-ABA-guanine>

Conjugates of benzaldehyde-modified carboxymethyl dextran with the DNA nucleotides adenine, cytosine and guanine (CMD-ABA-adenine, CMD-ABA-cytosine, CMD-ABA-guanine) were synthesized.

Specifically, the CMDX-ABA obtained in Example IV-1 was dissolved in pure water at a concentration of 1 mg/mL, and 2.5 equivalents of adenine, cytosine or guanine dissolved in pure water were added dropwise. This was reacted overnight at room temperature under shaded conditions, and then dialyzed and freeze dried to obtain CMD-ABA-adenine, CMD-ABA-cytosine and CMD-ABA-guanine.

<FT-IR spectrum measurement>

The resulting CMD-ABA-adenine, CMD-ABA-cytosine and CMD-ABA-guanine were subjected to FT-IR spectrum measurement, with the results shown in Fig. 71.

Absorption from C=N stretching of imine bonds at 1740 cm$^{-1}$, from C=O stretching of amide bonds at 1640-1690 cm$^{-1}$, and from N-H stretching at 1550-1640 cm$^{-1}$ was observed in the FT-IR spectra (Fig. 71) of the CMD-ABA-adenine, CMD-ABA-cytosine and CMD-ABA-guanine. These results that conjugates were formed between the CMD-ABA and the DNA nucleotides (adenine, cytosine or guanine).

V. Chitosan derivative

[Example V-1] Benzaldehyde-modified chitosan (Chitosan-CBA)

<Synthesis of Chitosan-CBA>

[C60]

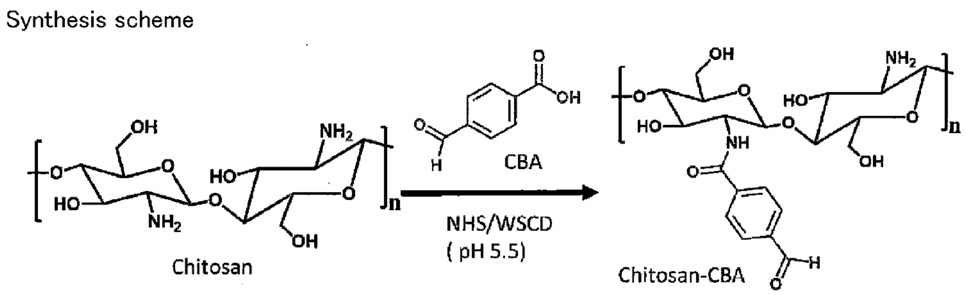


Following the above synthesis scheme, Chitosan-CBA was synthesized by the following procedures by an amidation reaction via carbodiimide.

1 g (0.0062 mol) of chitosan (Sigma Aldrich, Product No. 448877, CAS No. 9012-76-4, 75% to 85% deacetylated, weight-average molecular weight MW: 190,000 to 310,000 Da) was dissolved in 150 mL of 1.5% acetic acid solution to obtain a chitosan solution. 0.93 g (0.0062 mol) of 4-carboxybenzaldehyde (CBA) was then dissolved in 20 mL of water.

3.7 g (0.027 mol) of HOBt was dissolved in 10 mL of DMSO and added dropwise to the CBA solution, after which 5.32 g (0.027 mol) of WSCD/HCl was dissolved in 10 mL of distilled water and added dropwise to the CBA solution, which was then stirred for 15 to 20 minutes. The CBA solution was then added dropwise for 5 to 7 minutes to the chitosan solution, and the reaction mixture was stirred at room temperature for 20 to 24 hours with the pH maintained at about 5.5. The solution was then dialyzed with deionized water for 72 hours using a dialysis tube (MWCO: 6-8 kDa, Spectra/Pro®) and freeze dried to obtain Chitosan-CBA.

<$^1$H NMR spectrum measurement and FT-IR spectrum measurement>

The Chitosan-CBA was subjected to $^1$H NMR spectrum measurement and FT-IR spectrum measurement, with the results shown in Figs. 72 and 73. These results confirm synthesis of CBA-modified chitosan (Chitosan-CBA) by binding between the carboxyl groups of CBA and the amino groups of chitosan.

Based on the $^1$H NMR spectrum, the CBA modification rate of the chitosan amino groups ($-NH_2$) was calculated to be 0.20.

**Claims**

1. A polysaccharide derivative obtained by introducing a group represented by formula (A) below into a polysaccharide which is acidic, basic or amphoteric:

[C61]

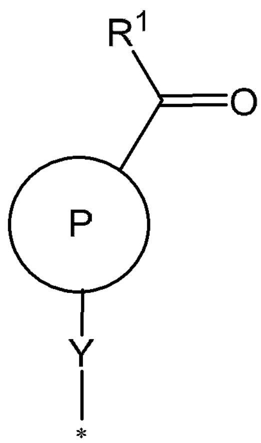

(A)

(in the formula, $R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
Y represents a divalent group selected from the group consisting of -NH-, -C(=O)-, - S-, -O-, the $C_{1-6}$ alkylenes and $-(CH_2CH_2O)_n-$ and any combinations of these, with n being an integer from 1 to 9, and
* represents a linkage with the polysaccharide).

2. The polysaccharide derivative according to claim 1, wherein the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, chitosan, derivatives thereof or salts of these, regenerated oxidized cellulose, derivatives thereof or salts of these, and pectic acid, derivatives thereof or salts of these.

3. The polysaccharide derivative according to claim 1 or 2, wherein the group represented by formula (A) above is a group selected from formula (A-1) or (A-2) below:

[C62]

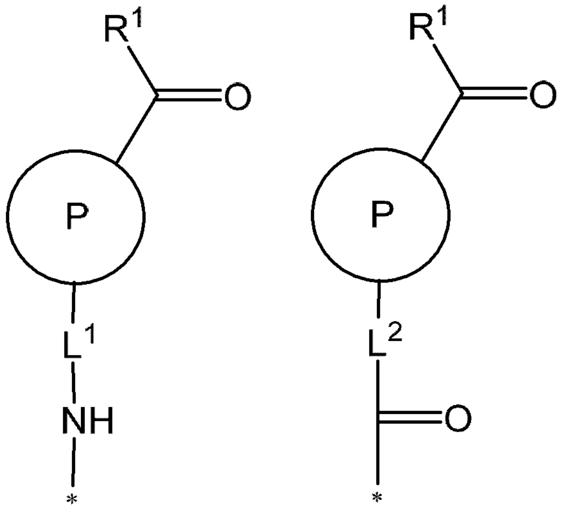

(A-1) (A-2)

(in formulae (A-1) and (A-2), $R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
$L^1$ is a single bond or represents a divalent group selected from the group consisting of -C(=O)-, -S-, -O-, the $C_{1-6}$ alkylenes and $-(CH_2CH_2O)_n-$ and any combinations of these,
$L^2$ is a single bond or represents a divalent group selected from the group consisting of -NH-, -S-, -O-, the $C_{1-6}$ alkylenes and $-(CH_2CH_2O)_n-$ and any combinations of these,
n is an integer from 1 to 9, and
* represents a linkage with the polysaccharide).

**4.** The polysaccharide derivative according to any one of claims 1 to 3, wherein

the polysaccharide is a polysaccharide containing a carboxyl group and/or amino group, and
the group represented by the formula (A) is introduced into the polysaccharide by forming an amide bond with a carboxyl group or amino group of the polysaccharide.

**5.** The polysaccharide derivative according to any of claims 1 to 4, wherein

the polysaccharide is a polysaccharide containing a carboxyl group,
the group represented by the formula (A) is a group represented by the formula (A-1), and
the group represented by the formula (A-1) is introduced into the polysaccharide by substitution for the -OH of the carboxyl group of the polysaccharide, forming an amide bond.

**6.** The polysaccharide derivative according to any of claims 1 to 5, containing at least one structural unit selected from formulae (c11), (c12), (c13), (c14) and (c15) below:

[C63]

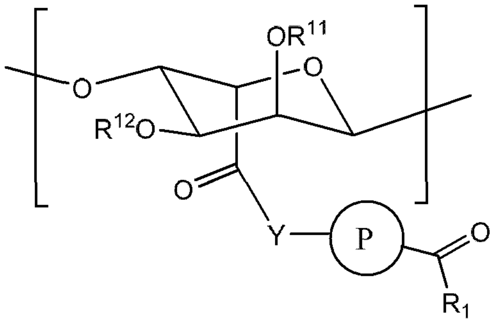

(c11)

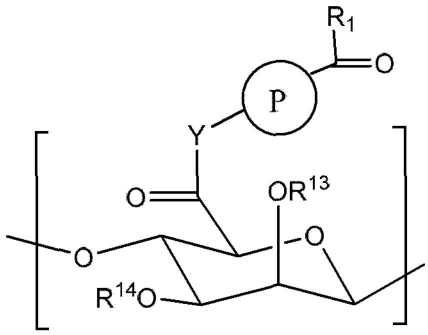

(c12)

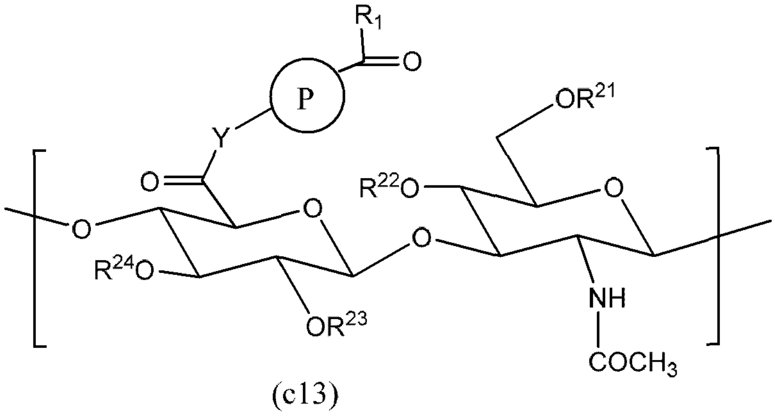

(c13)

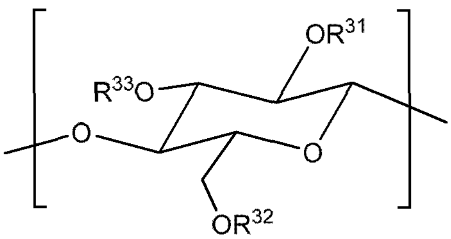

(c14)

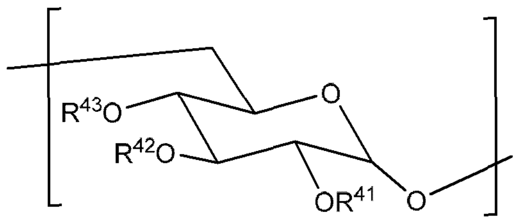

(c15)

(in the formulae, each of $R^{11}$, $R^{12}$, $R^{13}$ and $R^{14}$ is independently selected from a hydrogen atom, a $C_{1-6}$ alkyl and a $-C(=O)-C_{1-6}$ alkyl,

each of $R^{21}$, $R^{22}$, $R^{23}$ and $R^{24}$ independently represents a hydrogen atom, and 1 to 3 of $R^{31}$, $R^{32}$ and $R^{33}$ represent:

[C64]

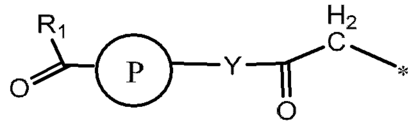

while each of the remainder of $R^{31}$, $R^{32}$ and $R^{33}$ is independently selected from a hydrogen atom, a $C_{1-6}$ alkyl, a $-C(=O)-C_{1-6}$ alkyl and $-CH_2COOH$, and 1 to 3 of $R^{41}$, $R^{42}$ and $R^{43}$ represent:

[C65]

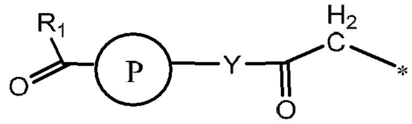

while each of the remainder of $R^{41}$, $R^{42}$ and $R^{43}$ is independently selected from a hydrogen atom, a $C_{1-6}$ alkyl, a -C(=O)-$C_{1-6}$ alkyl and -$CH_2COOH$,

$R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and -$CF_3$, -$NO_2$, carboxyl and -$SO_3H$ groups,

Y represents -$L^1$-NH-, in which $L^1$ is bound to ring P, and

$L^1$ is selected from a single bond, a $C_{1-6}$ alkylene and -$(CH_2CH_2O)_n$-, with n being an integer from 1 to 9).

**7.** The polysaccharide derivative according to any of claims 1 to 4, wherein

the polysaccharide is a polysaccharide containing an amino group,

the group represented by the formula (A) is a group represented by the formula (A-2), and

the group represented by the formula (A-2) is introduced into the polysaccharide by substitution for a hydrogen atom of the amino group of the polysaccharide, forming an amide bond.

**8.** The polysaccharide derivative according to any of claims 1 to 4 and claim 7, containing a structural unit represented by formula (c16) below:

[C66]

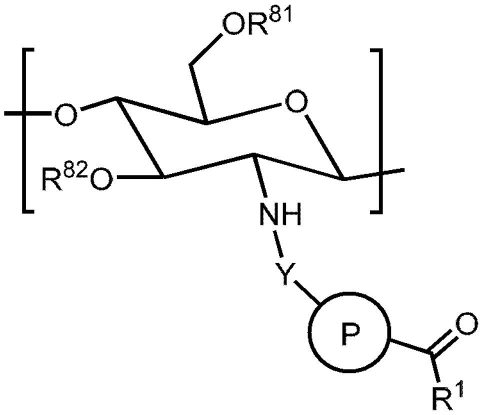

(c16)

(in the formula, each of $R^{81}$ and $R^{82}$ is independently selected from a hydrogen atom, a $C_{1-6}$ alkyl and a -C(=O)-$C_{1-6}$ alkyl,

$R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and -$CF_3$, -$NO_2$, carboxyl and -$SO_3H$ groups,

Y represents -$L^2$-C(=O)-, in which $L^2$ is bound to ring P,

$L^2$ is selected from a single bond, a $C_{1-6}$ alkylene, -$(CH_2CH_2O)_n$-, -$(CH_2)_{m1}$-$(CH_2CH_2O)_n$-$(CH_2)_{m2}$- and -$(CH_2)_{m1}$-O-$(CH_2CH_2O)_n$-$(CH_2)_{m2}$,

n is an integer from 1 to 9, and

each of $m_1$ and $m_2$ is independently an integer from 1 to 9).

**9.** The polysaccharide derivative according to any of claims 1 to 8, wherein the modification rate by the group represented

of the formula (A) in the polysaccharide derivative is 0.01 to 1.

**10.** A polysaccharide derivative-drug conjugate between a drug containing a primary amino group and the polysaccharide derivative according to any of claims 1 to 9, wherein a structure represented by formula (D) below is formed between the primary amino group in the drug and the group represented by formula (A) in the polysaccharide:

[C67]

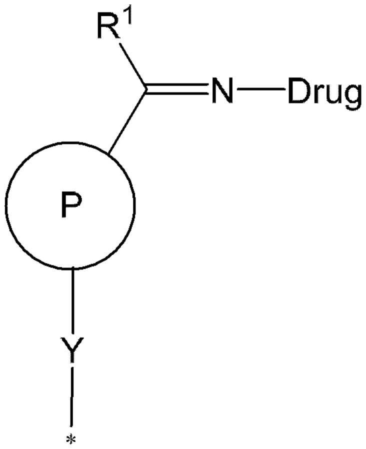

(D)

(in the formula, "Drug" represents the drug part excluding the primary amino group, and ring P, $R^1$ and * are defined as in claim 1).

**11.** The polysaccharide derivative-drug conjugate according to claim 10, wherein the drug is released from the polysaccharide derivative-drug conjugate under low pH conditions.

**12.** The polysaccharide derivative-drug conjugate according to claim 10 or 11, wherein the drug is at least one selected from the low-molecular-weight compounds, middle-molecular-weight compounds, peptides, nucleic acids, nucleic acid derivatives, aptamers, vitamins, monoamines, amino acids, polyamines, antibodies, fluorescent dyes and contrast agents.

**13.** A crosslinked structure containing the polysaccharide derivative according to any one of claims 1 to 9, wherein the polysaccharide derivative is crosslinked via a crosslinking group.

**14.** A crosslinked structure containing the polysaccharide derivative according to any one of claims 1 to 9 together with one or both of an amino group-containing polymer and an amino group-containing low-molecular-weight compound containing two or more primary amino groups, hydrazide groups or aminooxy groups, wherein the crosslinked structure is crosslinked by covalent bonds formed via Schiff bases between the primary amino groups, hydrazide groups or aminooxy groups contained in the amino group-containing polymer and amino group-containing low-molecular-weight compound and the group represented by the formula (A) in the polysaccharide derivative.

**15.** The crosslinked structure according to claim 14, wherein the amino group-containing polymer is at least one selected from the linear, branched or dendritic polyamines; the polyalkylene glycols substituted with amino groups, hydrazide groups or aminooxy groups; polyallylamine; polyvinylamine; polyacrylamine; the amino-group containing polysaccharides; the amino group-containing proteins; and the polyamino acids.

**16.** A crosslinked structure-drug conjugate between a drug containing a primary amino group and the crosslinked structure according to any one of claims 13 to 15, wherein the primary amino group contained in the drug and the group represented by formula (A) in the crosslinked structure are covalently bonded via a Schiff base.

**17.** A composition containing the polysaccharide derivative according to any one of claims 1 to 9, the polysaccharide derivative-drug conjugate according to any one of claims 10 to 12, the crosslinked structure according to any one of claims 13 to 15, or the crosslinked structure-drug conjugate according to claim 16.

**18.** A gel, sponge, film or capsule containing the polysaccharide derivative according to any one of claims 1 to 9, the polysaccharide derivative-drug conjugate according to any one of claims 10 to 12, the crosslinked structure according to any one of claims 13 to 15, the crosslinked structure-drug conjugate according to claim 16, or the composition according to claim 17.

**19.** A tissue adhesive material containing the polysaccharide derivative according to any one of claims 1 to 9, the polysaccharide derivative-drug conjugate according to any one of claims 10 to 12, the crosslinked structure according to any one of claims 13 to 15, or the crosslinked structure-drug conjugate according to claim 16.

**20.** A drug delivery carrier or separation material containing the polysaccharide derivative according to any one of claims 1 to 9 or the crosslinked structure according to any one of claims 13 to 15.

**21.** A method for manufacturing a polysaccharide derivative represented by formula (C1) below, wherein a polysaccharide containing a carboxyl group and a compound (a1) represented by formula (a1) below are subjected to a condensation reaction in a solvent:

[C68]

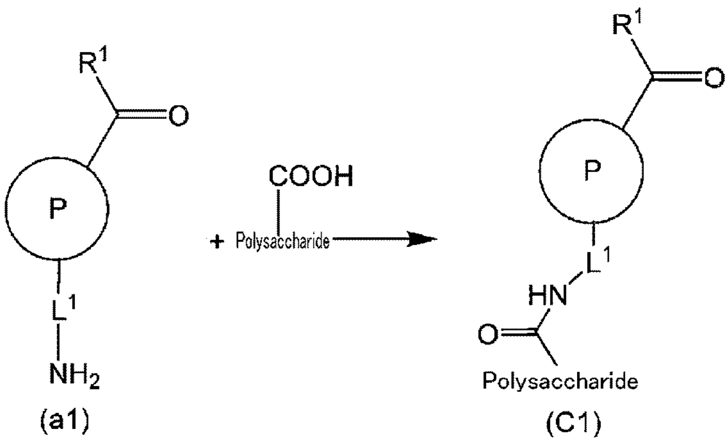

(in the formula, $L^1$ is selected from a single bond, a $C_{1-6}$ alkylene and $-(CH_2CH_2O)_n-$, n is an integer from 1 to 9, and ring P and $R^1$ are defined as in claim 1).

**22.** The method according to claim 21, wherein the reaction between the compound (a1) and the polysaccharide is performed under conditions of pH 5 to 10.

**23.** A method for manufacturing the polysaccharide derivative-drug conjugate according to any one of claims 10 to 12, wherein the polysaccharide derivative according to any one of claims 1 to 9 and a drug containing a primary amino group are mixed in a solvent.

Fig. 1

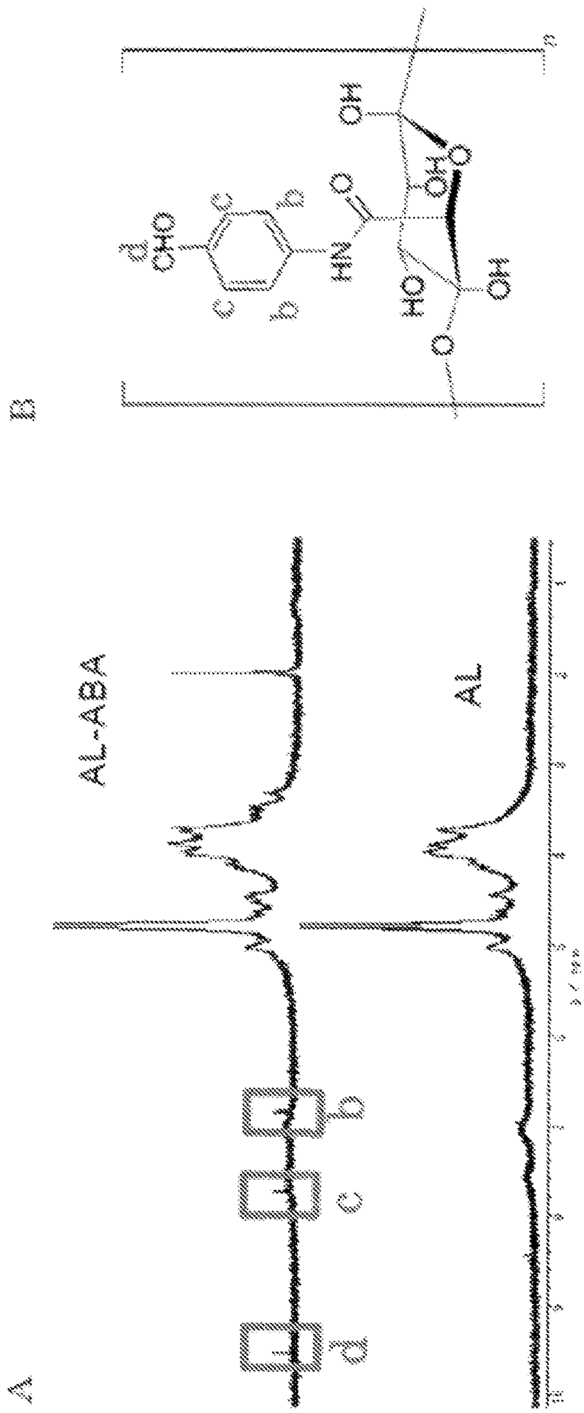

Fig. 2

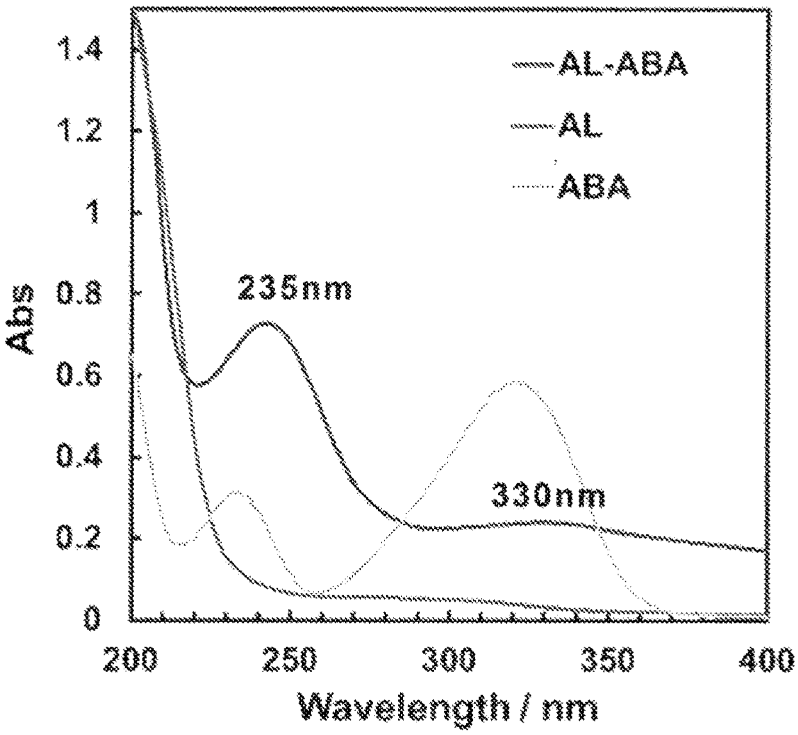
AL-ABA
AL
ABA
235nm
330nm
Abs
1.4
1.2
1
0.8
0.6
0.4
0.2
0
200
250
300
350
400
Wavelength / nm

Fig. 3

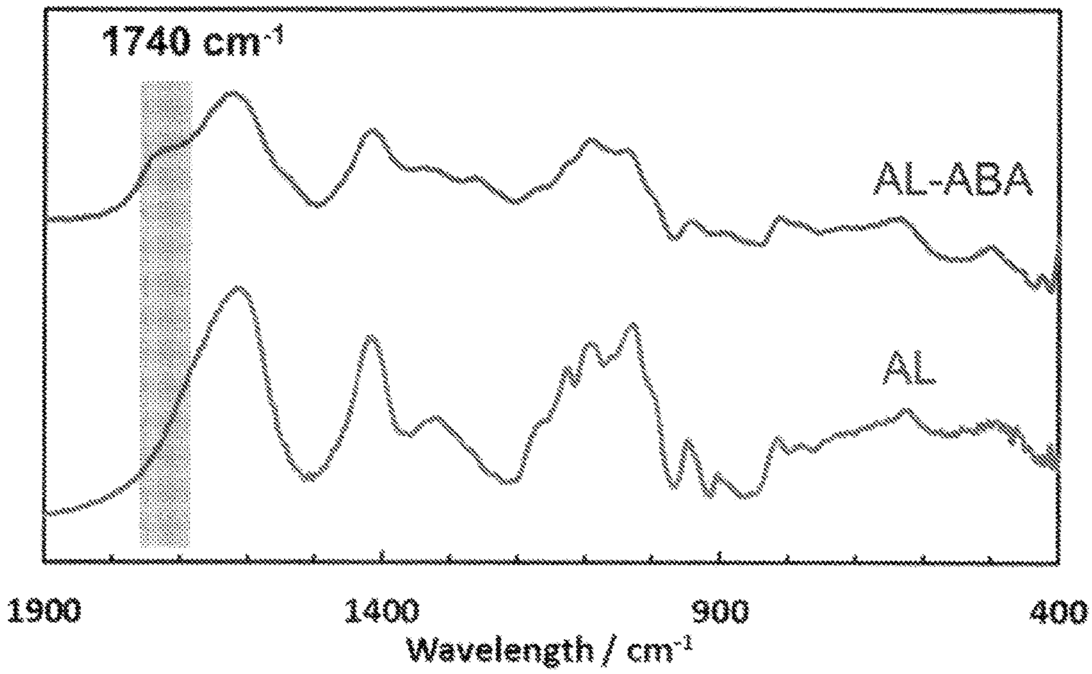
1740 cm-1
AL-ABA
AL
1900
1400
900
400
Wavelength / cm-1

Fig. 4

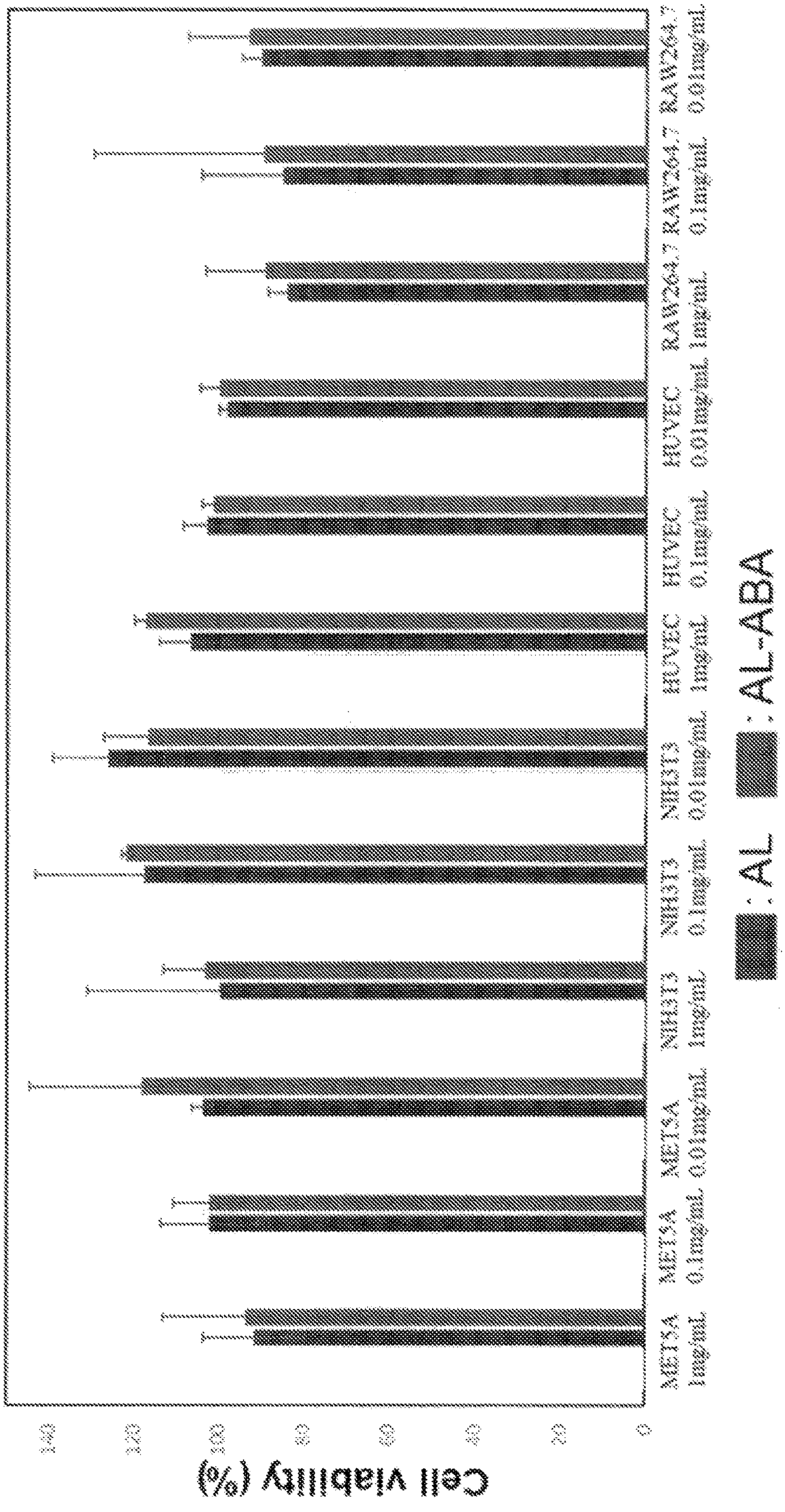
Cell viability (%)
0
20
40
60
80
100
120
140
MET5A 1mg/mL
MET5A 0.1mg/mL
MET5A 0.01mg/mL
NIH3T3 1mg/mL
NIH3T3 0.1mg/mL
NIH3T3 0.01mg/mL
HUVEC 1mg/mL
HUVEC 0.1mg/mL
HUVEC 0.01mg/mL
RAW264.7 1mg/mL
RAW264.7 0.1mg/mL
RAW264.7 0.01mg/mL
: AL
: AL-ABA

Fig. 5

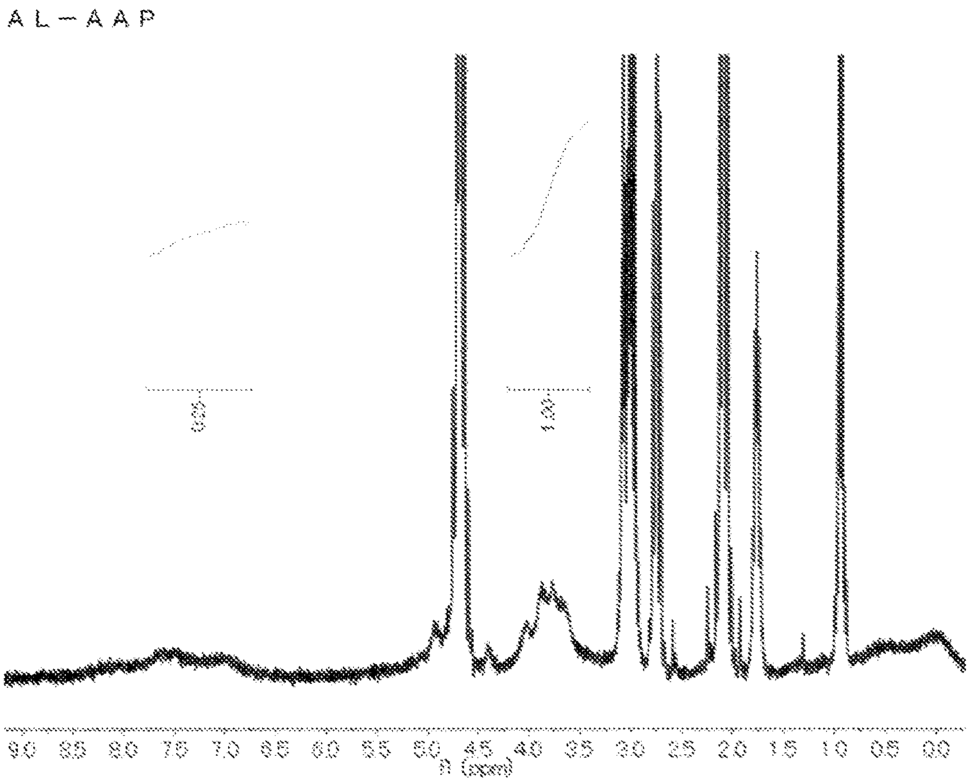
AL-AAP
0.03
1.00
9.0 8.5 8.0 7.5 7.0 6.5 6.0 5.5 5.0 4.5 4.0 3.5 3.0 2.5 2.0 1.5 1.0 0.5 0.0
f1 (ppm)

Fig. 6

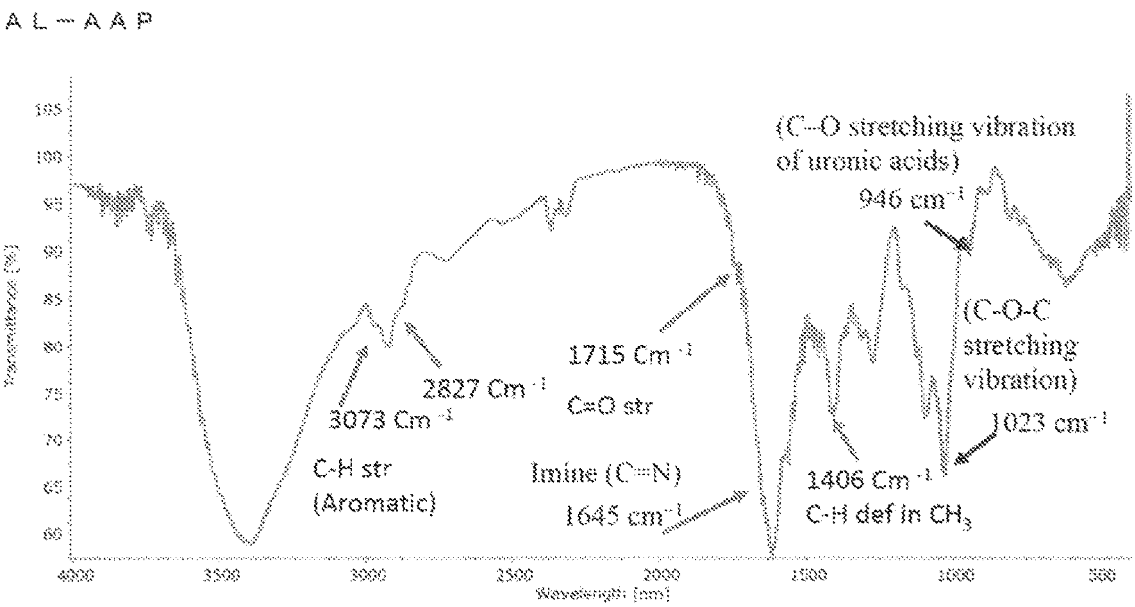
AL-AAP
Transmittance [%]
Wavelength [nm]
(C–O stretching vibration of uronic acids)
946 cm⁻¹
(C-O-C stretching vibration)
1023 cm⁻¹
1715 Cm⁻¹
C=O str
2827 Cm⁻¹
3073 Cm⁻¹
C-H str (Aromatic)
Imine (C=N)
1645 cm⁻¹
1406 Cm⁻¹
C-H def in CH₃

Fig. 7

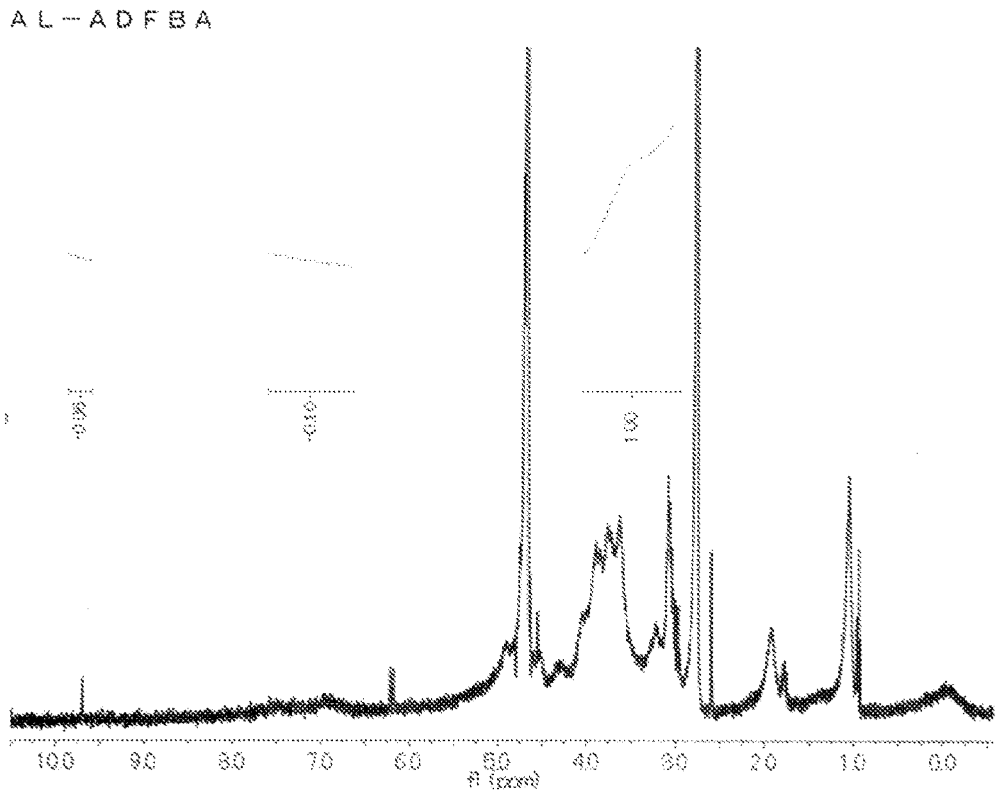
AL－ADFBA
10.0
9.0
8.0
7.0
6.0
5.0
4.0
3.0
2.0
1.0
0.0
f1 (ppm)

Fig. 8

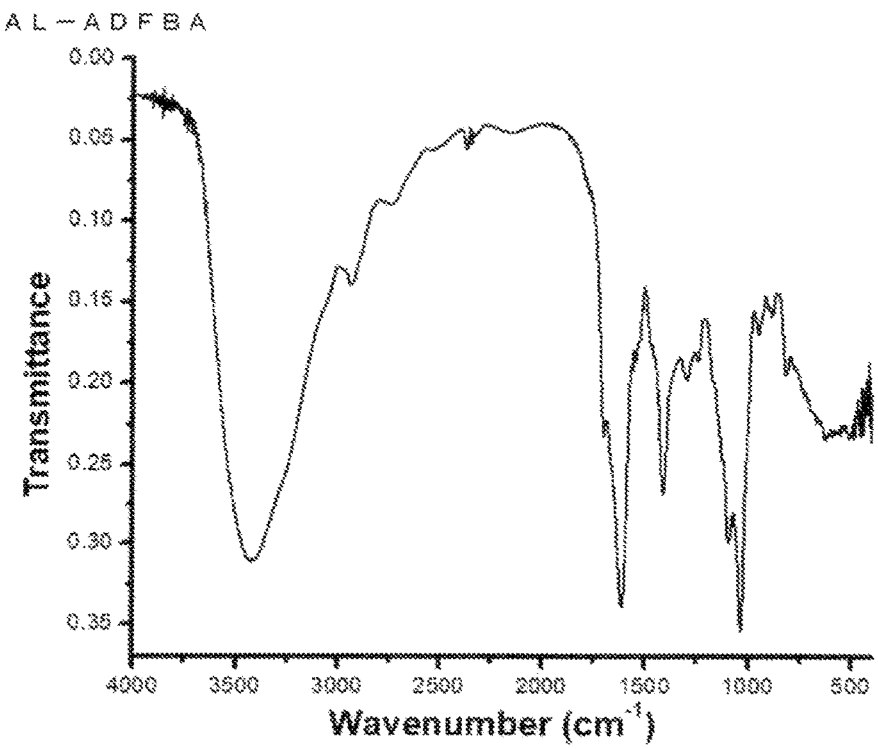
AL－ADFBA
Transmittance
0.00
0.05
0.10
0.15
0.20
0.25
0.30
0.35
4000
3500
3000
2500
2000
1500
1000
500
Wavenumber (cm-1)

Fig. 9

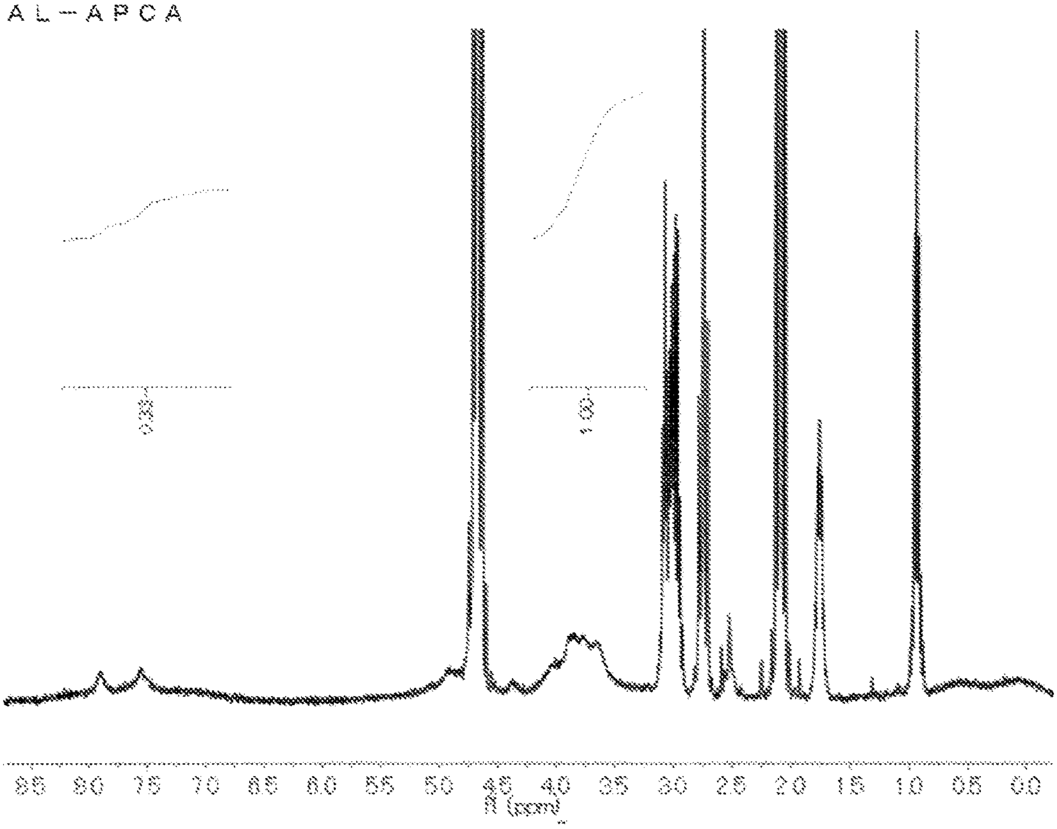
AL-APCA
0.33
1.00
8.5 8.0 7.5 7.0 6.5 6.0 5.5 5.0 4.5 4.0 3.5 3.0 2.5 2.0 1.5 1.0 0.5 0.0
f1 (ppm)

Fig. 10

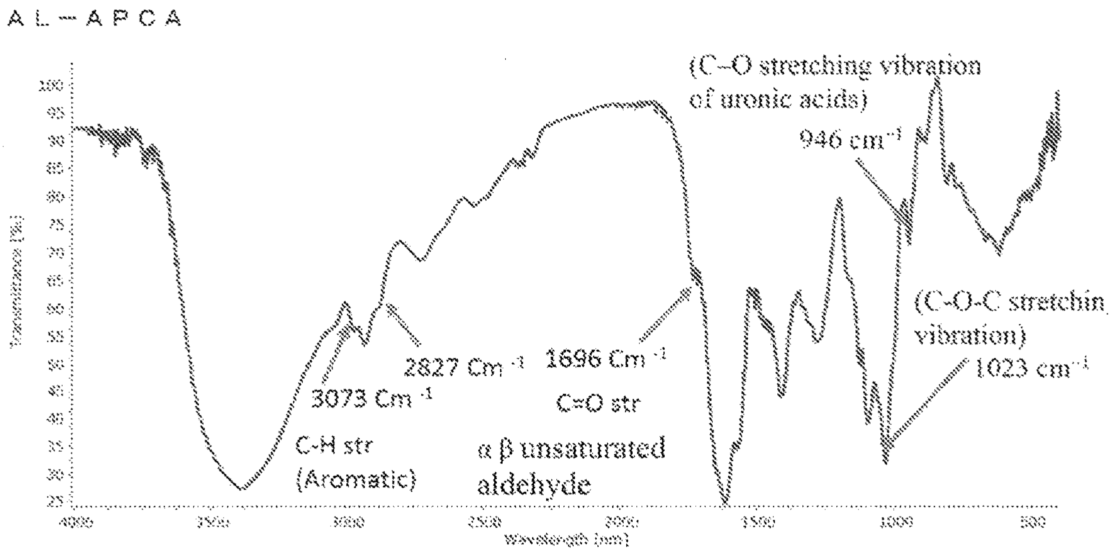
AL-APCA
(C-O stretching vibration
of uronic acids)
946 cm⁻¹
(C-O-C stretchin
vibration)
1023 cm⁻¹
2827 Cm⁻¹
3073 Cm⁻¹
C-H str
(Aromatic)
1696 Cm⁻¹
C=O str
α β unsaturated
aldehyde
Transmittance [%]
Wavelength [nm]

Fig. 11

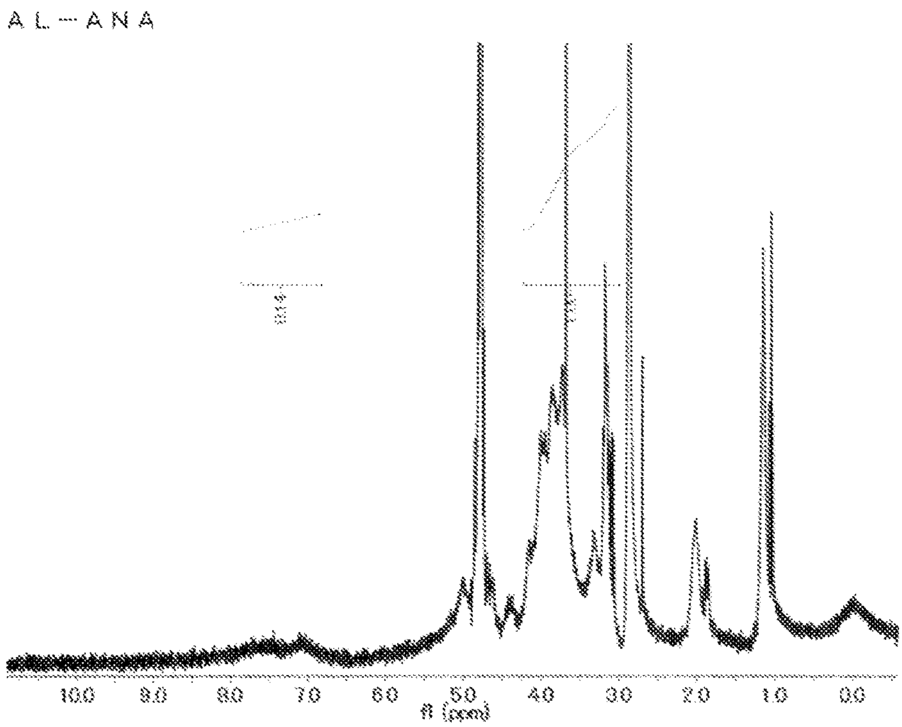
AL-ANA
10.0
9.0
8.0
7.0
6.0
5.0
4.0
3.0
2.0
1.0
0.0
f1 (ppm)

Fig. 12

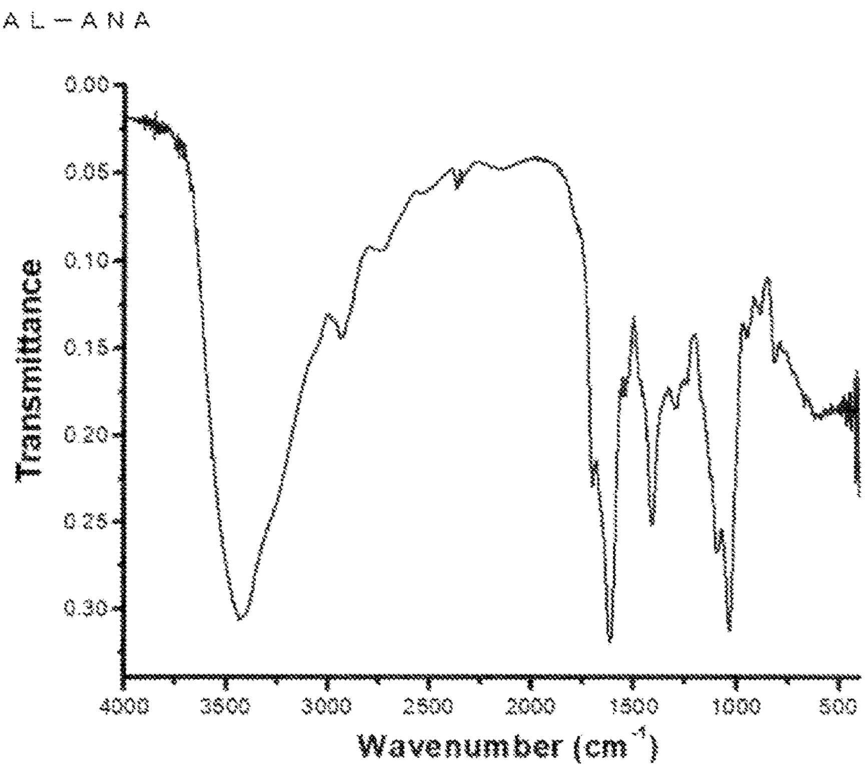
AL-ANA
Transmittance
0.00
0.05
0.10
0.15
0.20
0.25
0.30
4000
3500
3000
2500
2000
1500
1000
500
Wavenumber (cm-1)

Fig. 13

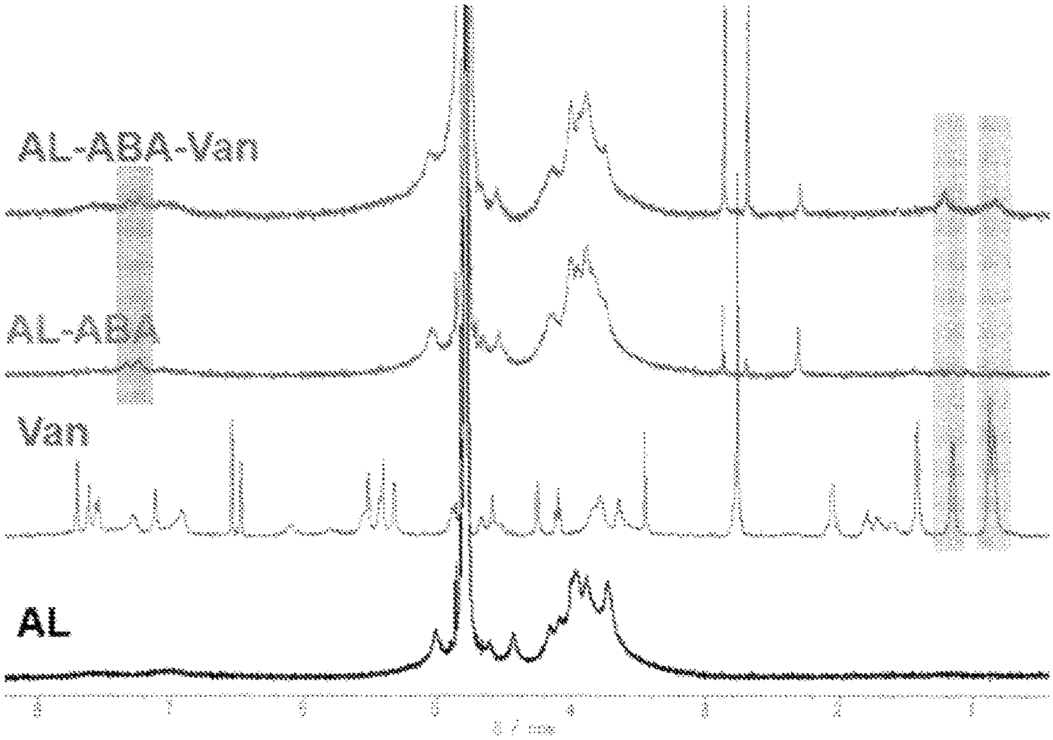
AL-ABA-Van
AL-ABA
Van
AL

Fig. 14

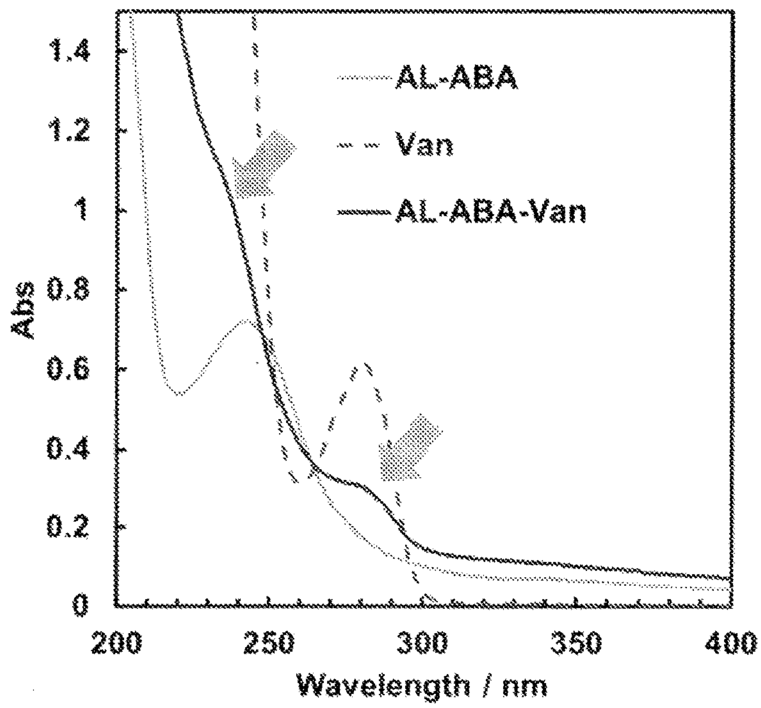
AL-ABA
Van
AL-ABA-Van
Abs
1.4
1.2
1
0.8
0.6
0.4
0.2
0
200
250
300
350
400
Wavelength / nm

Fig. 15

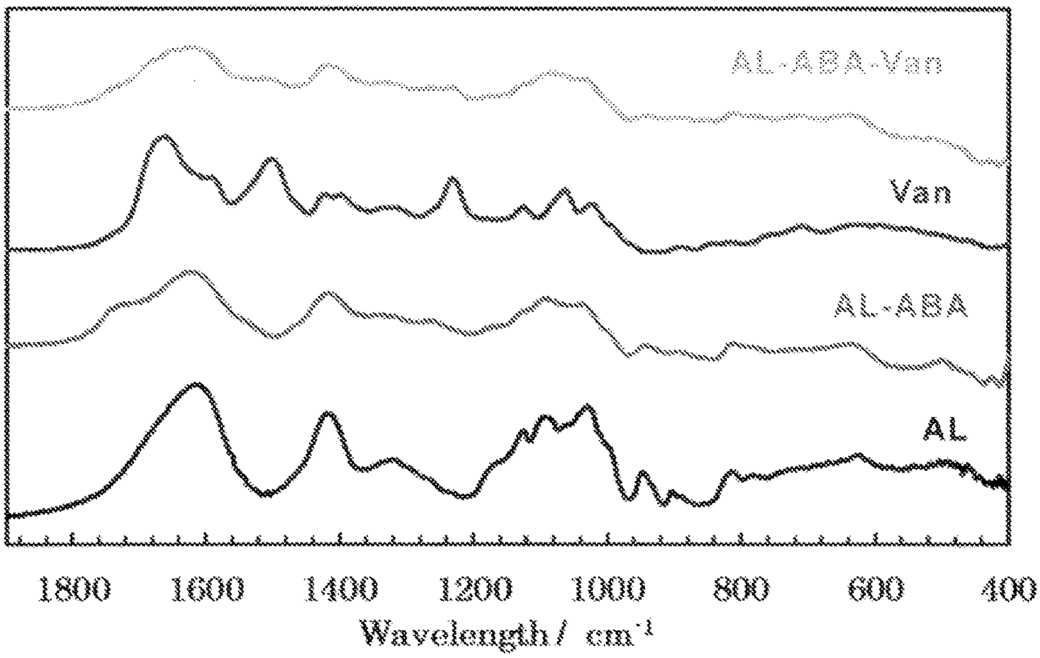


Fig. 16A

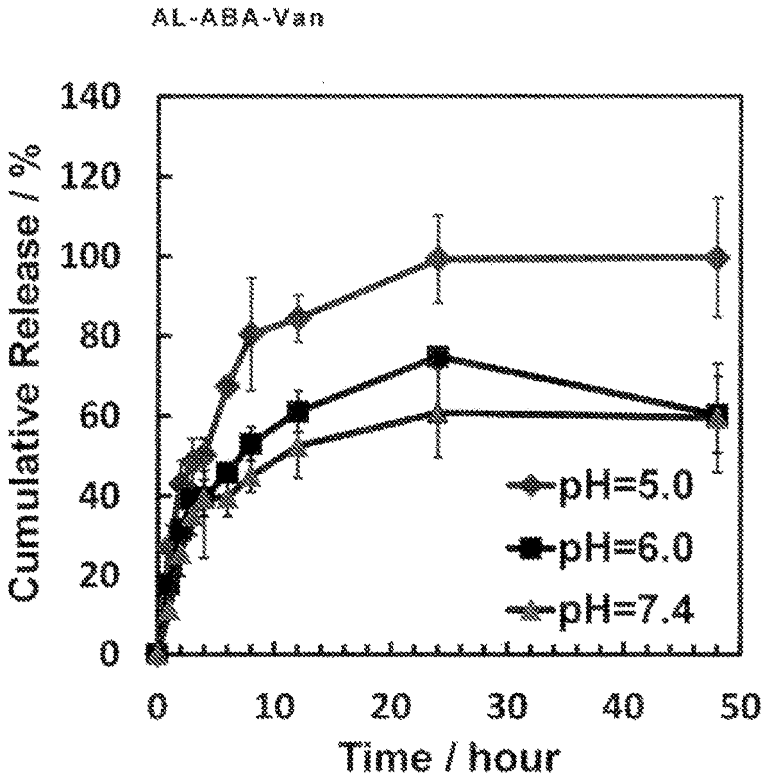

Fig. 16B

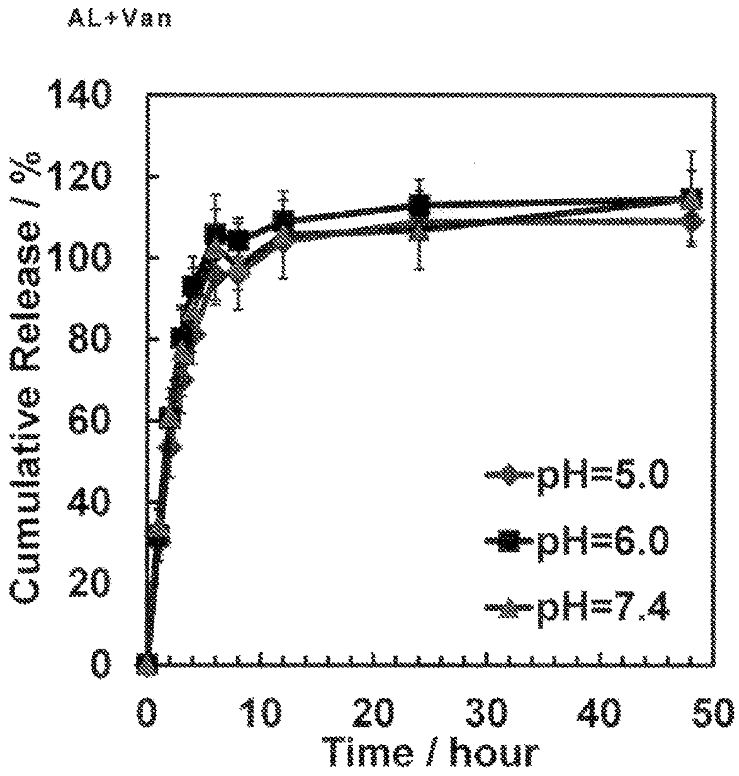


Fig. 16C

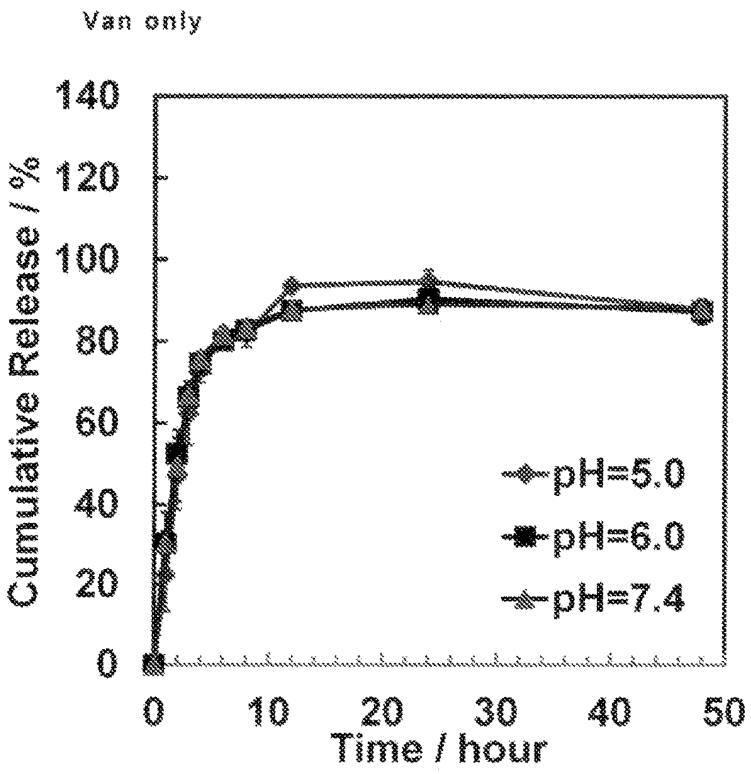

Fig. 17

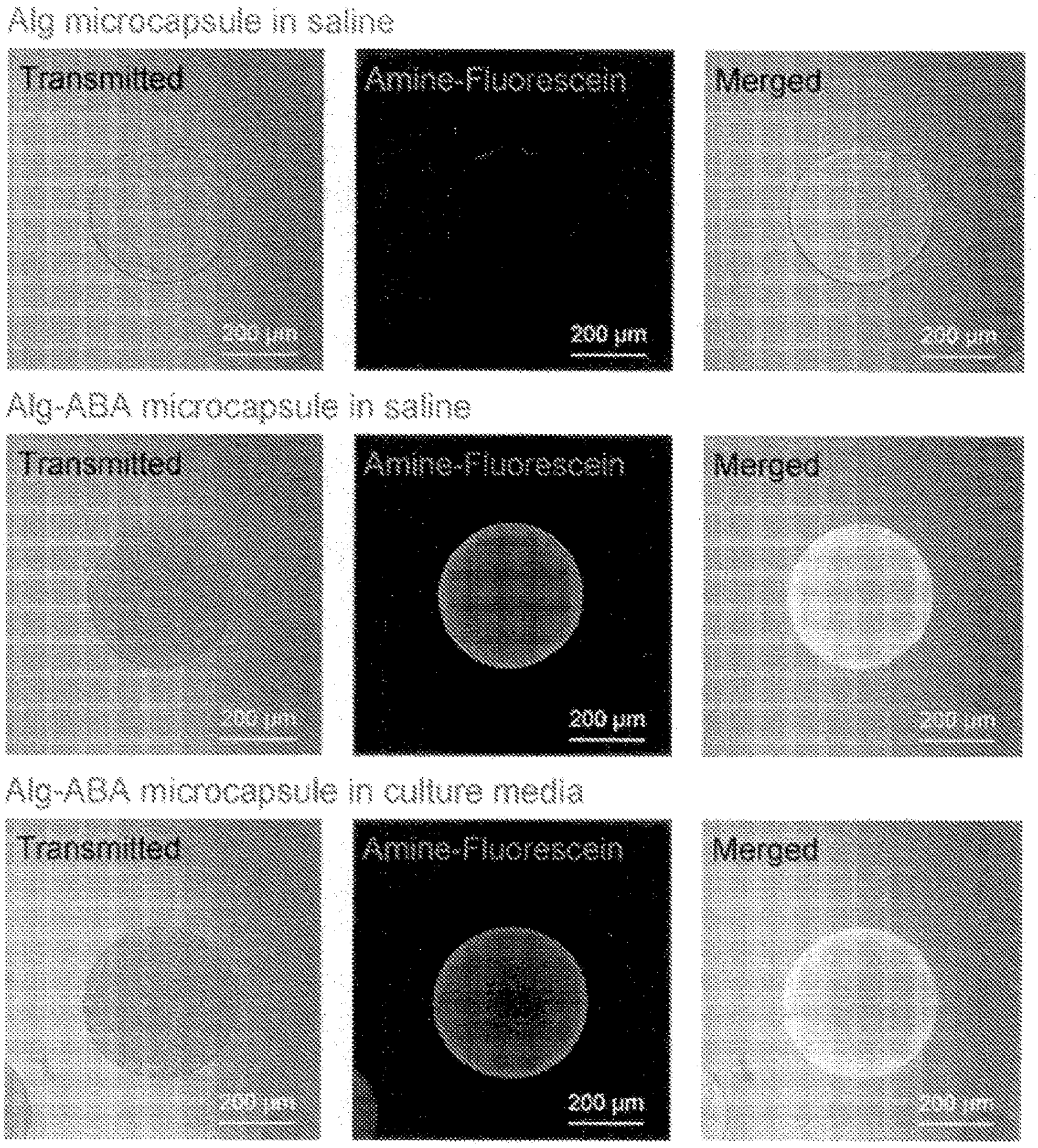
Alg microcapsule in saline
Transmitted
Amine-Fluorescein
Merged
200 μm
200 μm
200 μm
Alg-ABA microcapsule in saline
Transmitted
Amine-Fluorescein
Merged
200 μm
200 μm
200 μm
Alg-ABA microcapsule in culture media
Transmitted
Amine-Fluorescein
Merged
200 μm
200 μm
200 μm

Fig. 18

A

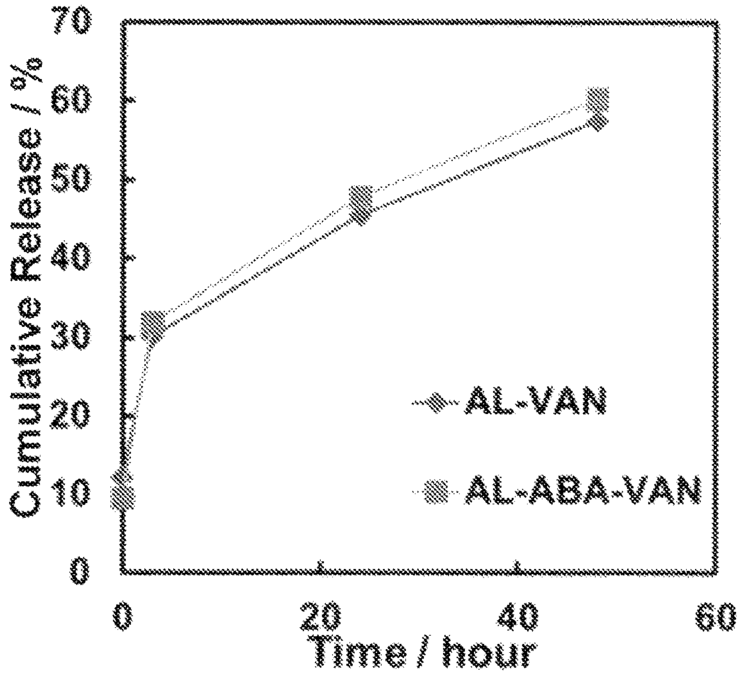

Cumulative Release / %
70
60
50
40
30
20
10
0
0
20
40
60
Time / hour
AL-VAN
AL-ABA-VAN


B

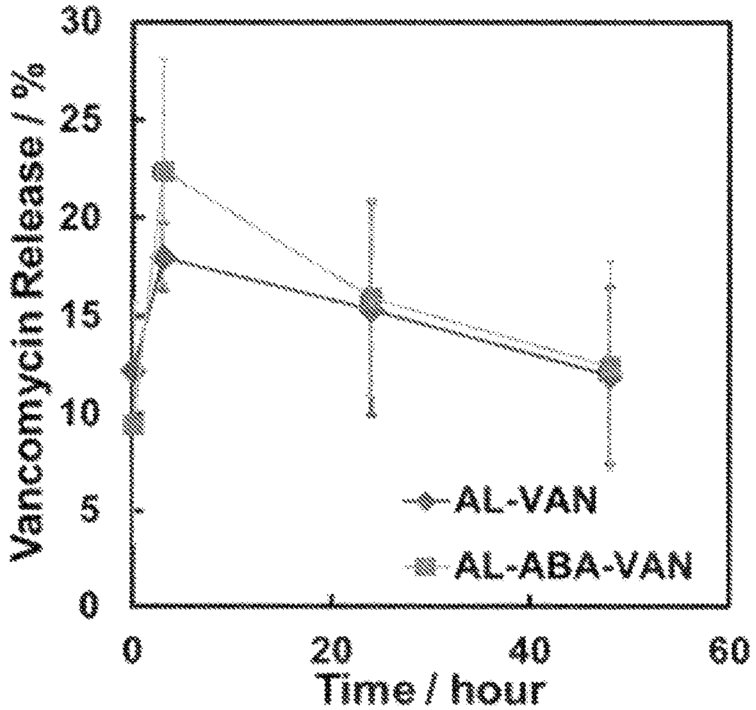

Vancomycin Release / %
30
25
20
15
10
5
0
0
20
40
60
Time / hour
AL-VAN
AL-ABA-VAN

Fig. 19

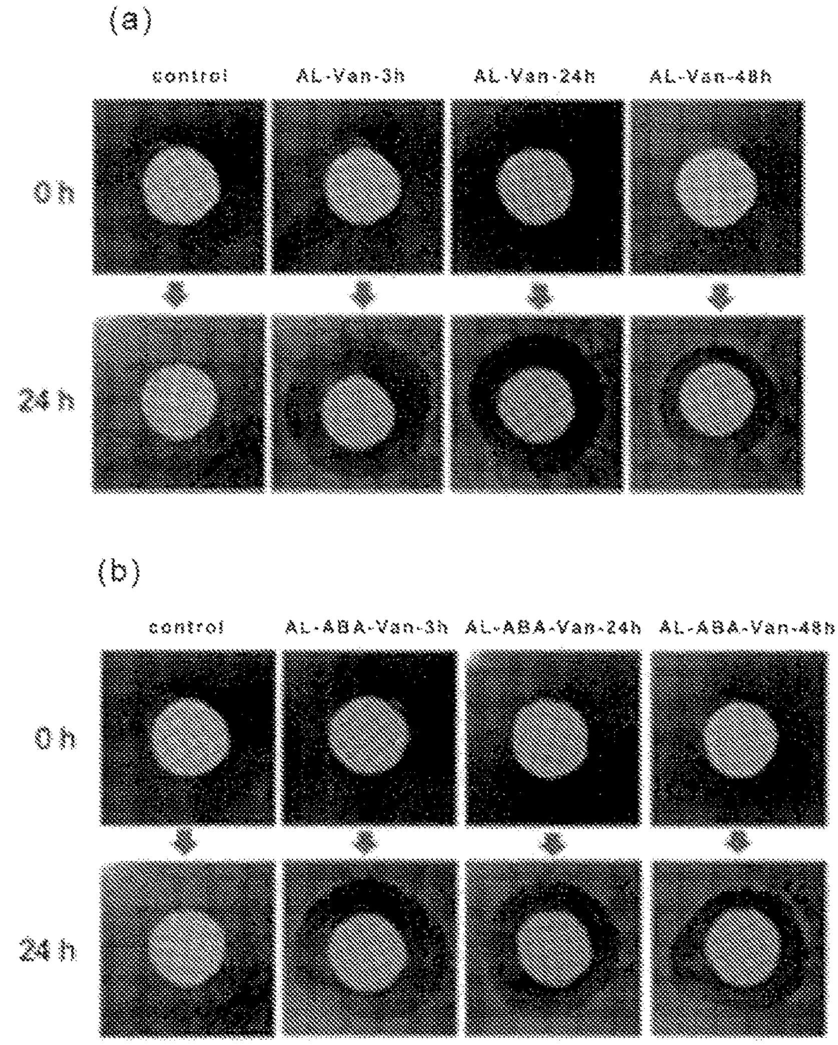
(a)
control
AL-Van-3h
AL-Van-24h
AL-Van-48h
0 h
24 h
(b)
control
AL-ABA-Van-3h
AL-ABA-Van-24h
AL-ABA-Van-48h
0 h
24 h

Fig. 20

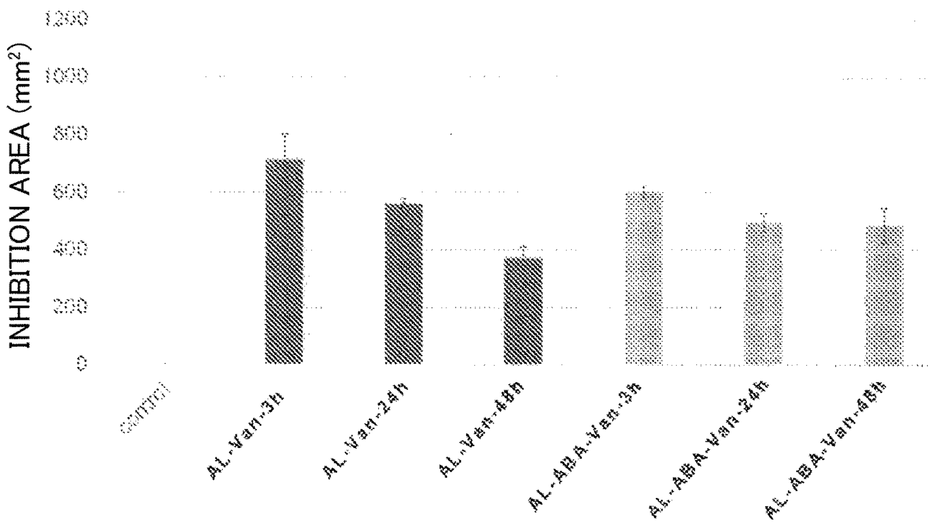
INHIBITION AREA (mm²)
1200
1000
800
600
400
200
0
control
AL-Van-3h
AL-Van-24h
AL-Van-48h
AL-ABA-Van-3h
AL-ABA-Van-24h
AL-ABA-Van-48h

Fig. 21

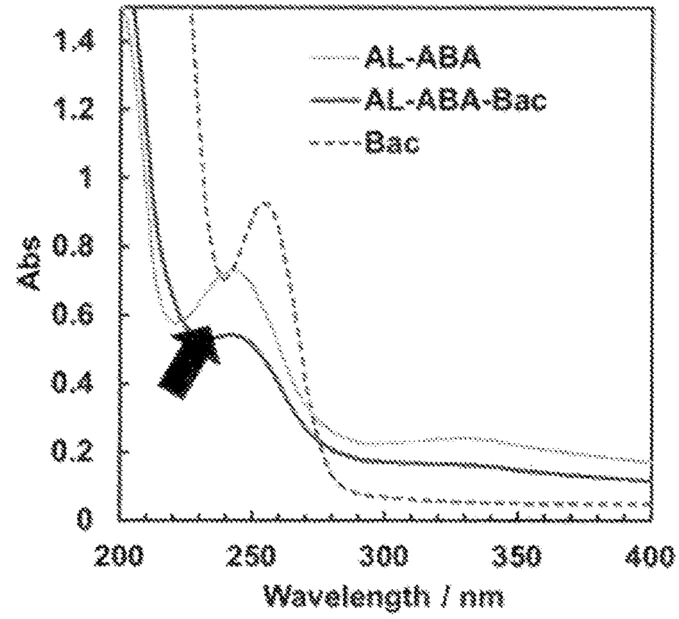
AL-ABA
AL-ABA-Bac
Bac
Abs
1.4
1.2
1
0.8
0.6
0.4
0.2
0
200
250
300
350
400
Wavelength / nm

Fig. 22

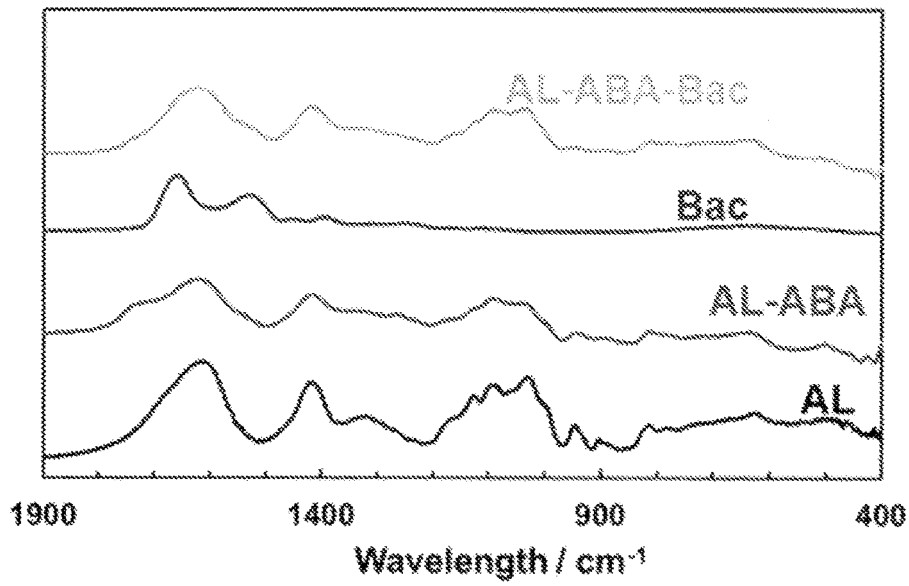
AL-ABA-Bac
Bac
AL-ABA
AL
1900
1400
900
400
Wavelength / cm-1

Fig. 23

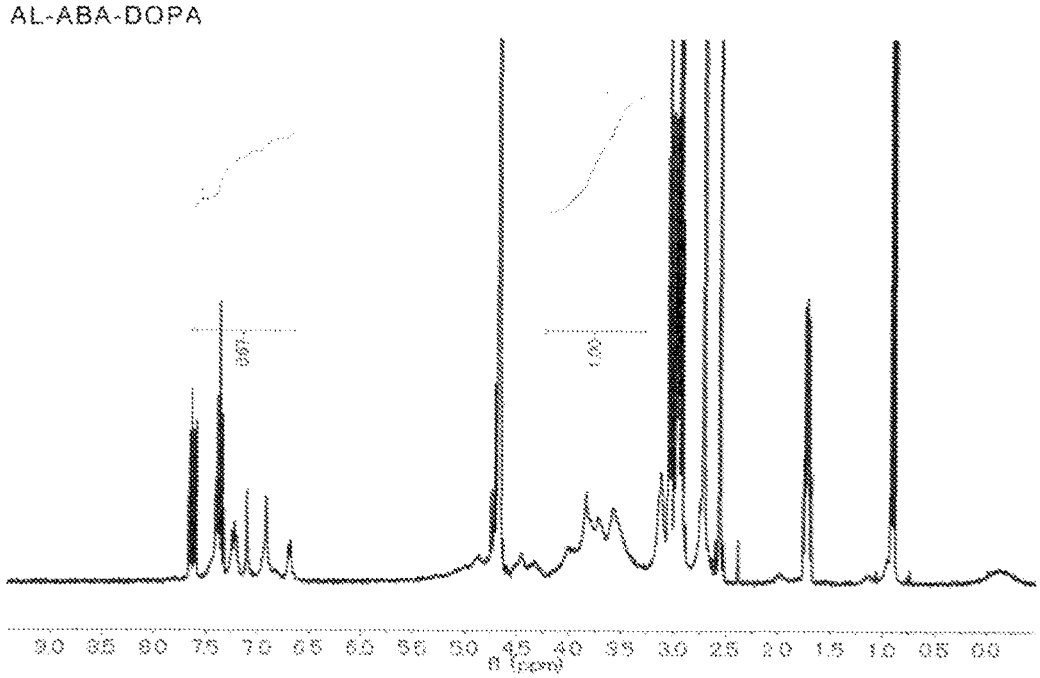
AL-ABA-DOPA
9.0 8.5 8.0 7.5 7.0 6.5 6.0 5.5 5.0 4.5 4.0 3.5 3.0 2.5 2.0 1.5 1.0 0.5 0.0
δ (ppm)

Fig. 24

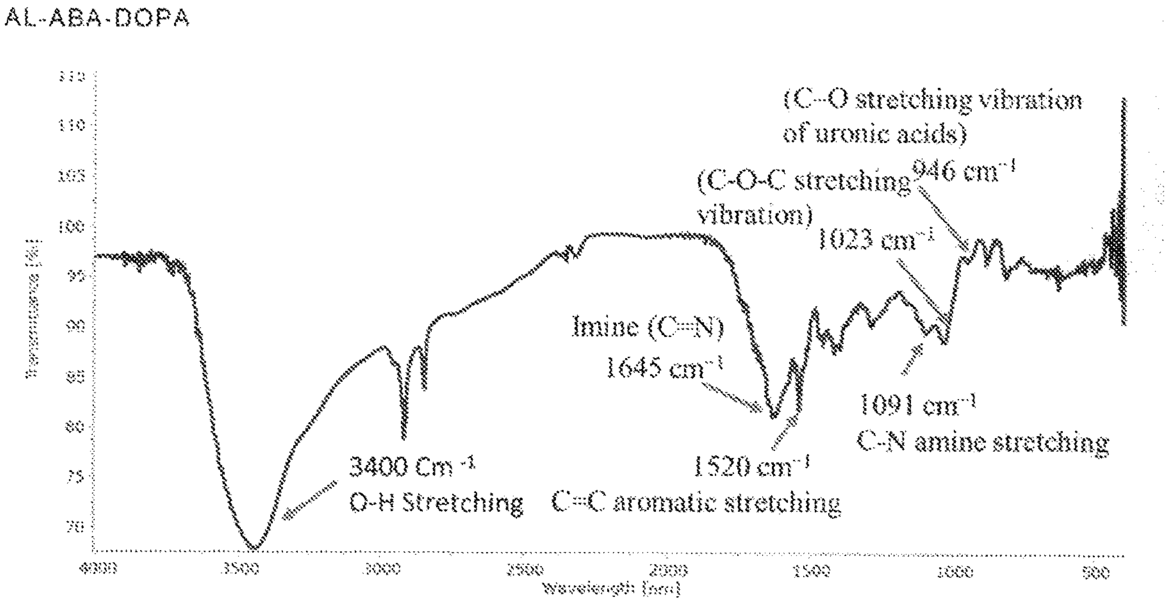
AL-ABA-DOPA
(C–O stretching vibration of uronic acids)
946 cm⁻¹
(C-O-C stretching vibration)
1023 cm⁻¹
Imine (C=N)
1645 cm⁻¹
1091 cm⁻¹
C-N amine stretching
3400 Cm⁻¹
O-H Stretching
1520 cm⁻¹
C=C aromatic stretching
Transmittance [%]
Wavelength [nm]

Fig. 25

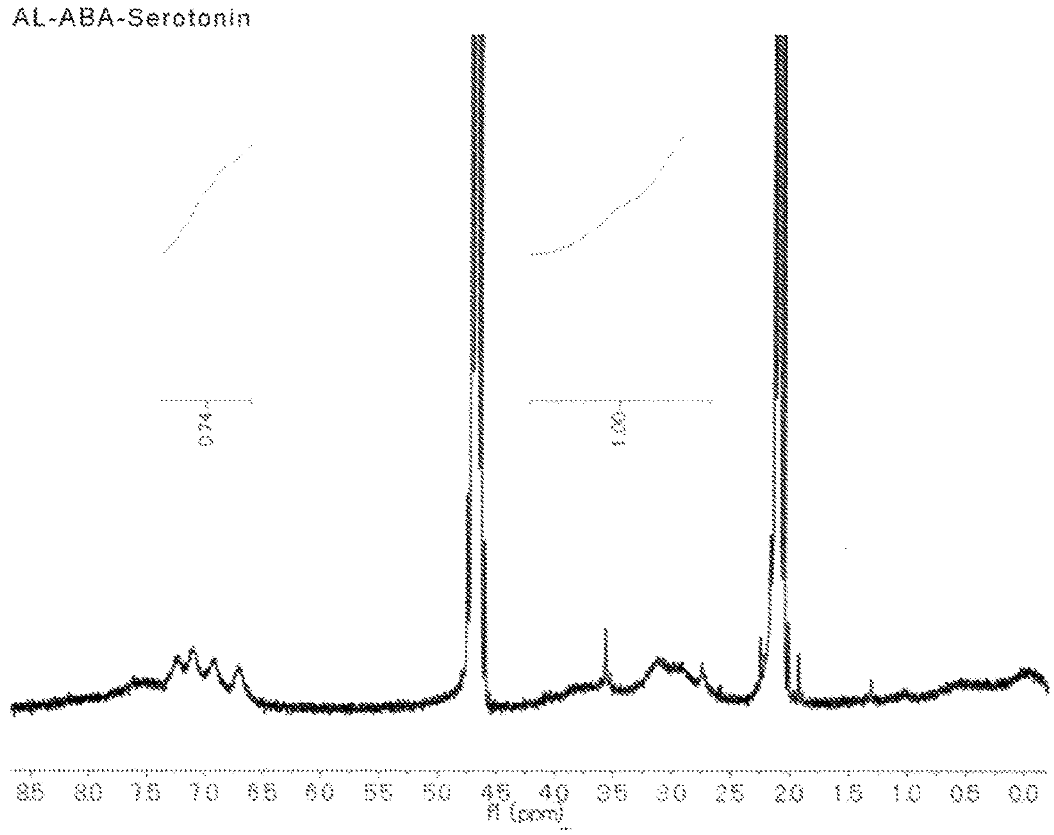
AL-ABA-Serotonin
0.74
1.00
8.5 8.0 7.5 7.0 6.5 6.0 5.5 5.0 4.5 4.0 3.5 3.0 2.5 2.0 1.5 1.0 0.5 0.0
f1 (ppm)

Fig. 26

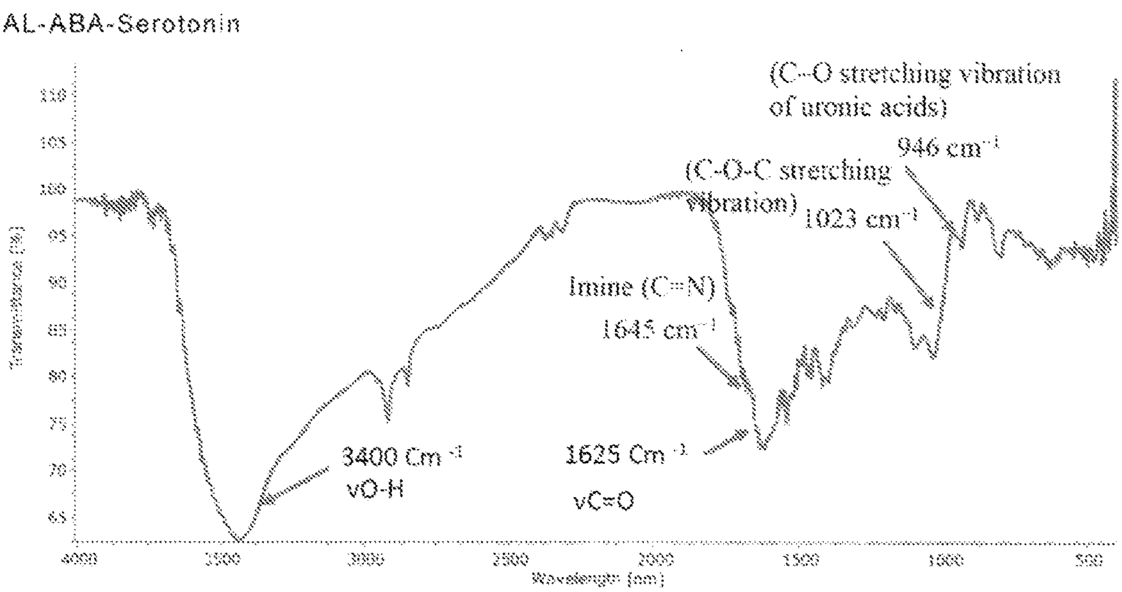
AL-ABA-Serotonin
(C–O stretching vibration
of uronic acids)
946 cm⁻¹
(C-O-C stretching
vibration)
1023 cm⁻¹
Imine (C=N)
1645 cm⁻¹
3400 Cm⁻¹
vO-H
1625 Cm⁻¹
vC=O
Wavelength (nm)

Fig. 27

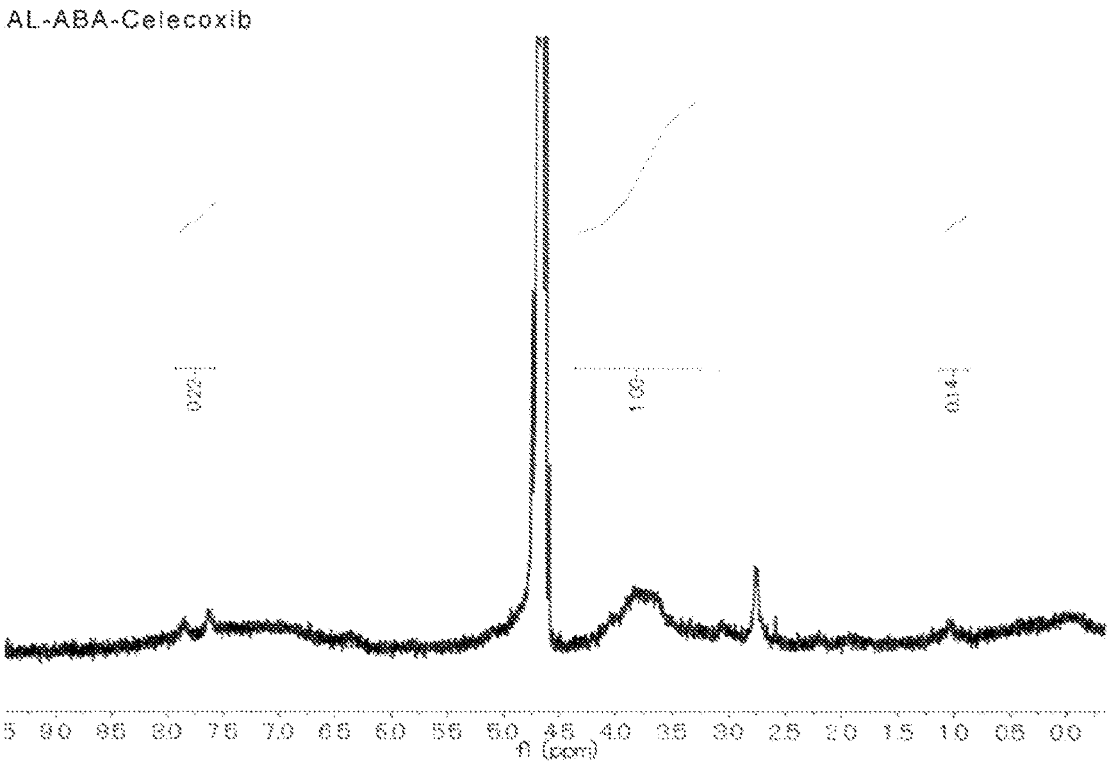
AL-ABA-Celecoxib
f1 (ppm)

Fig. 28

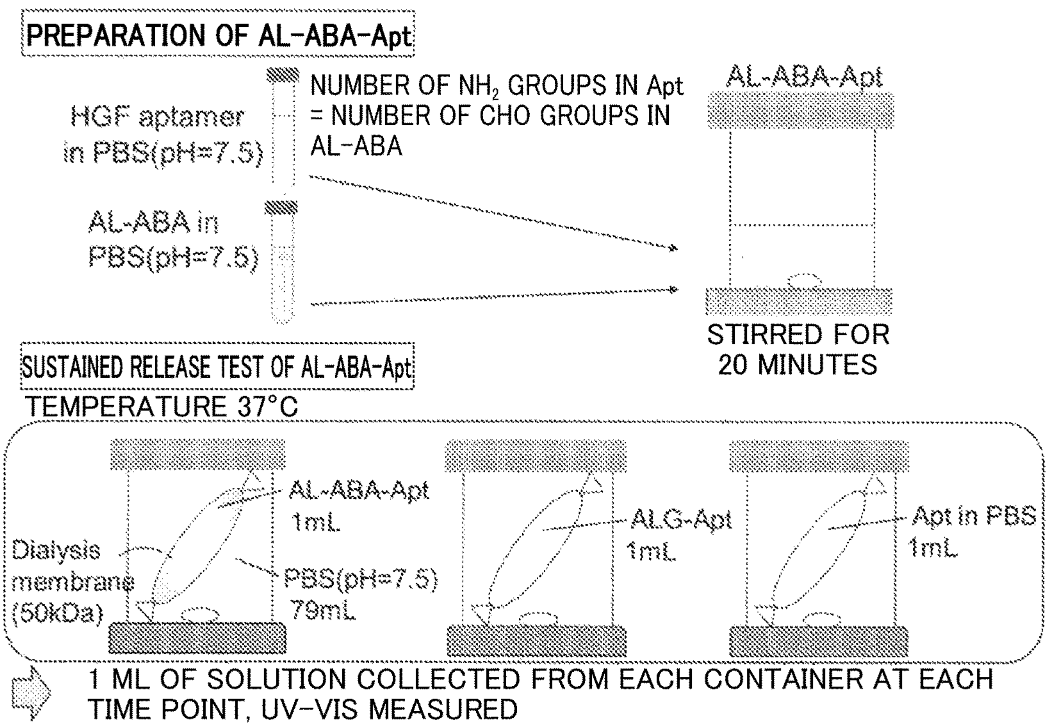
PREPARATION OF AL-ABA-Apt
HGF aptamer in PBS(pH=7.5)
NUMBER OF NH2 GROUPS IN Apt = NUMBER OF CHO GROUPS IN AL-ABA
AL-ABA in PBS(pH=7.5)
AL-ABA-Apt
STIRRED FOR 20 MINUTES
SUSTAINED RELEASE TEST OF AL-ABA-Apt
TEMPERATURE 37°C
AL-ABA-Apt 1mL
Dialysis membrane (50kDa)
PBS(pH=7.5) 79mL
ALG-Apt 1mL
Apt in PBS 1mL
1 ML OF SOLUTION COLLECTED FROM EACH CONTAINER AT EACH TIME POINT, UV-VIS MEASURED

Fig. 29

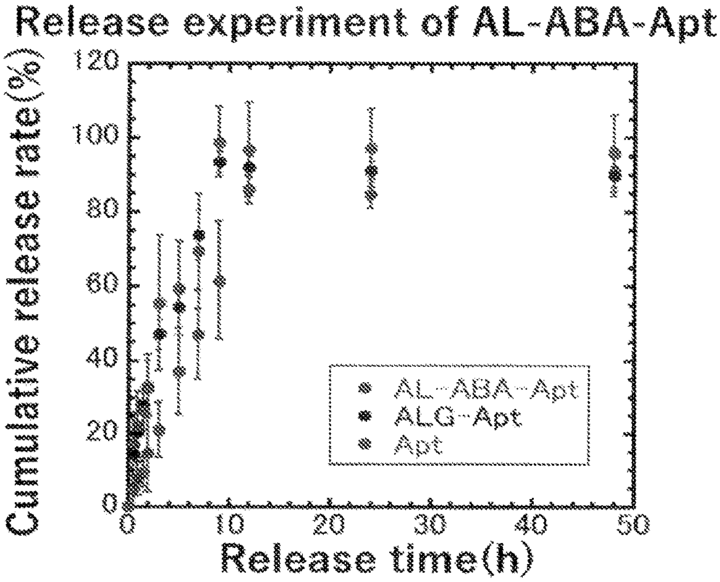


Fig. 30

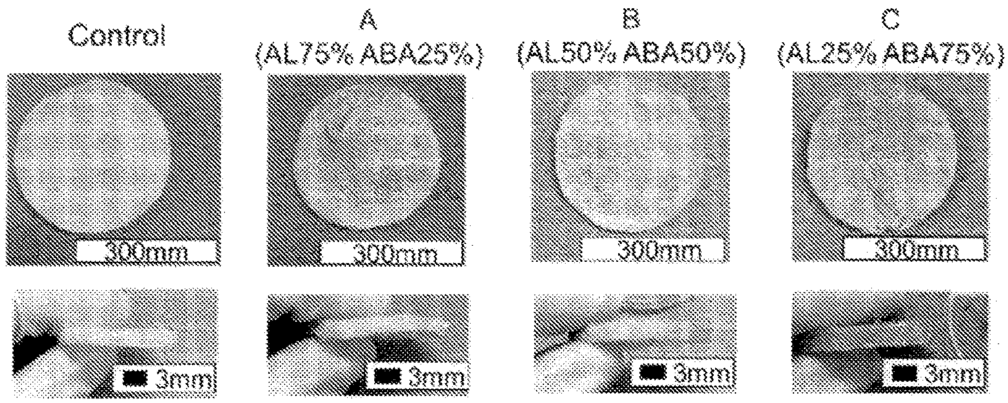


Fig. 31

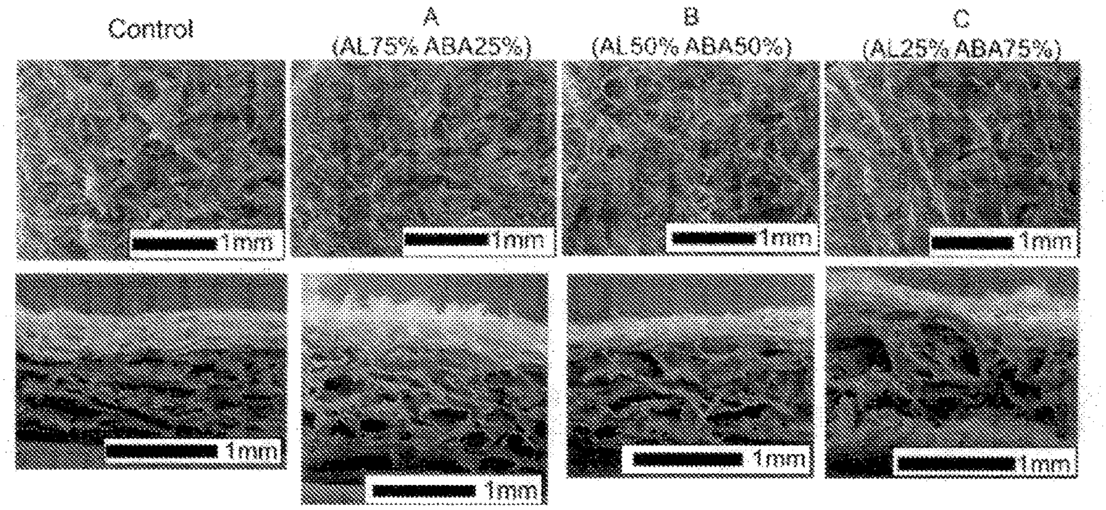

Fig. 32

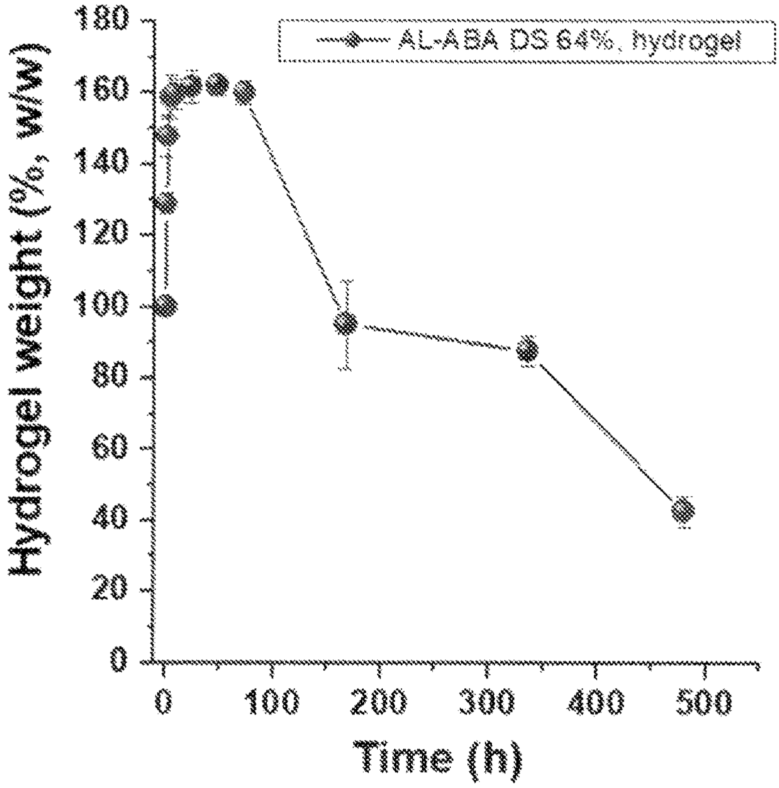
AL-ABA DS 64%, hydrogel
Hydrogel weight (%, w/w)
Time (h)
0
20
40
60
80
100
120
140
160
180
0
100
200
300
400
500

Fig. 33

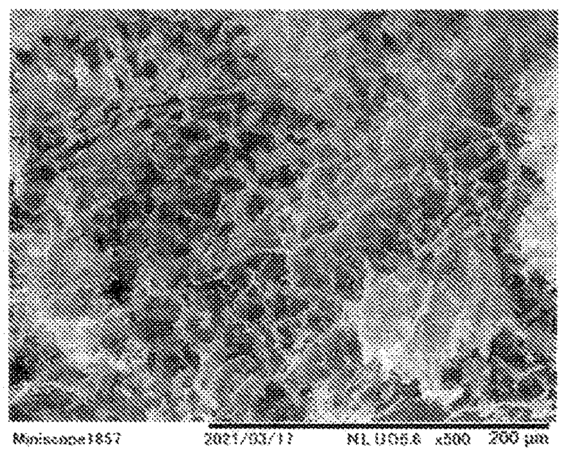
200 μm

Fig. 34

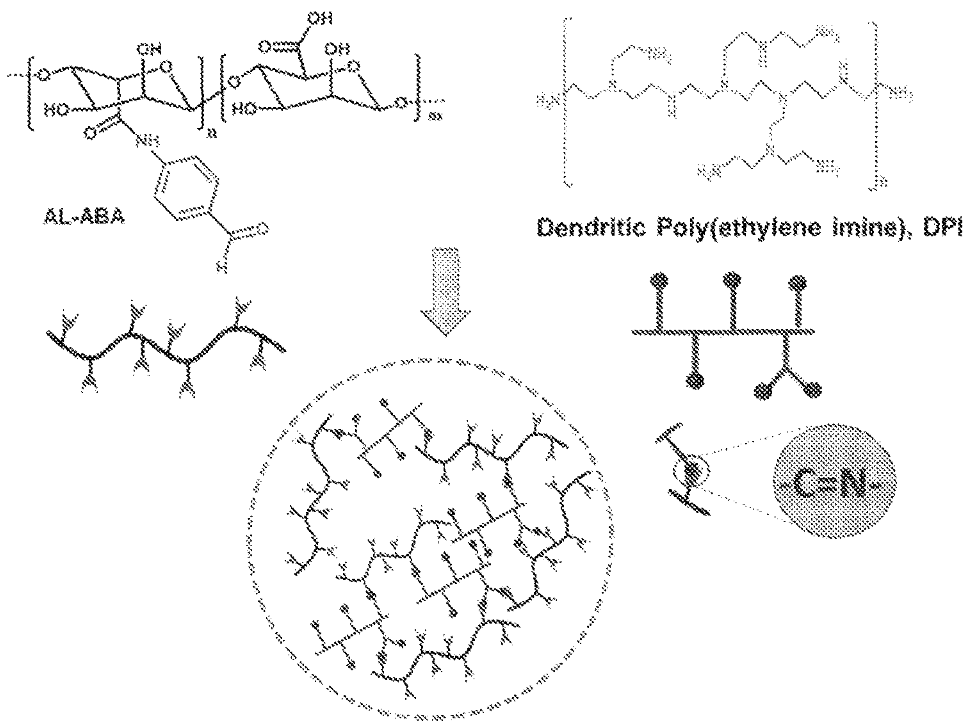


Fig. 35

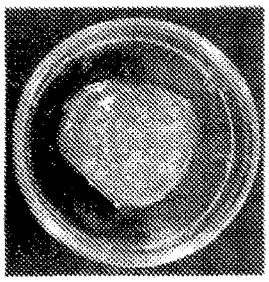

Fig. 36A

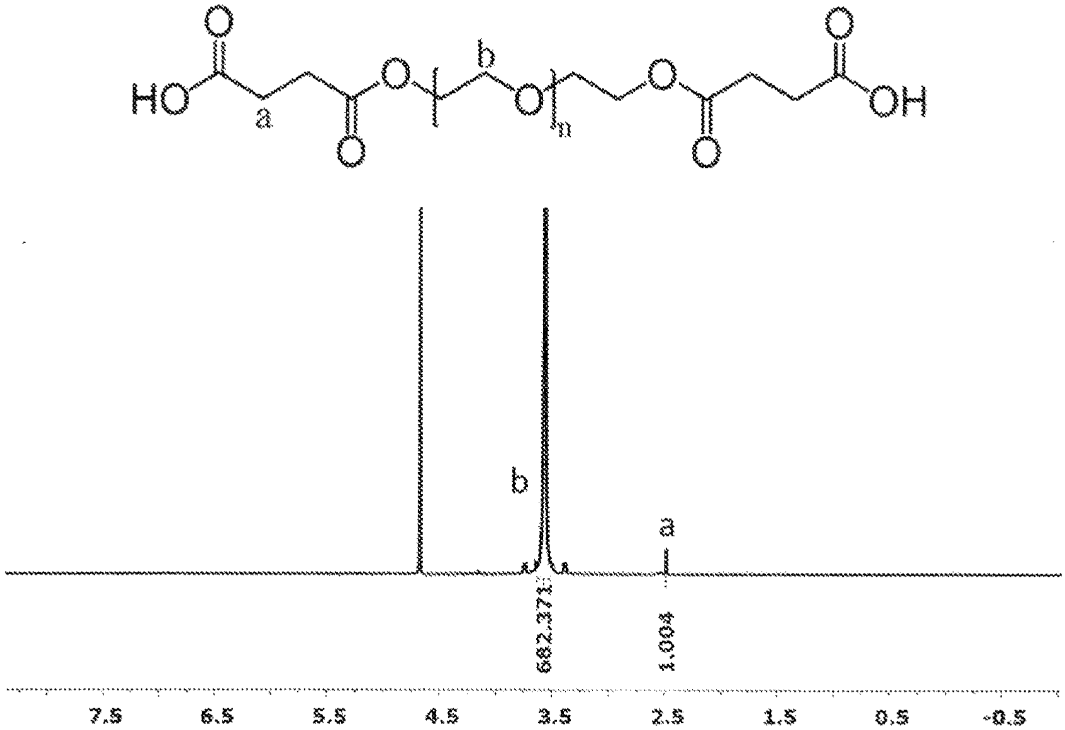


Fig. 36B

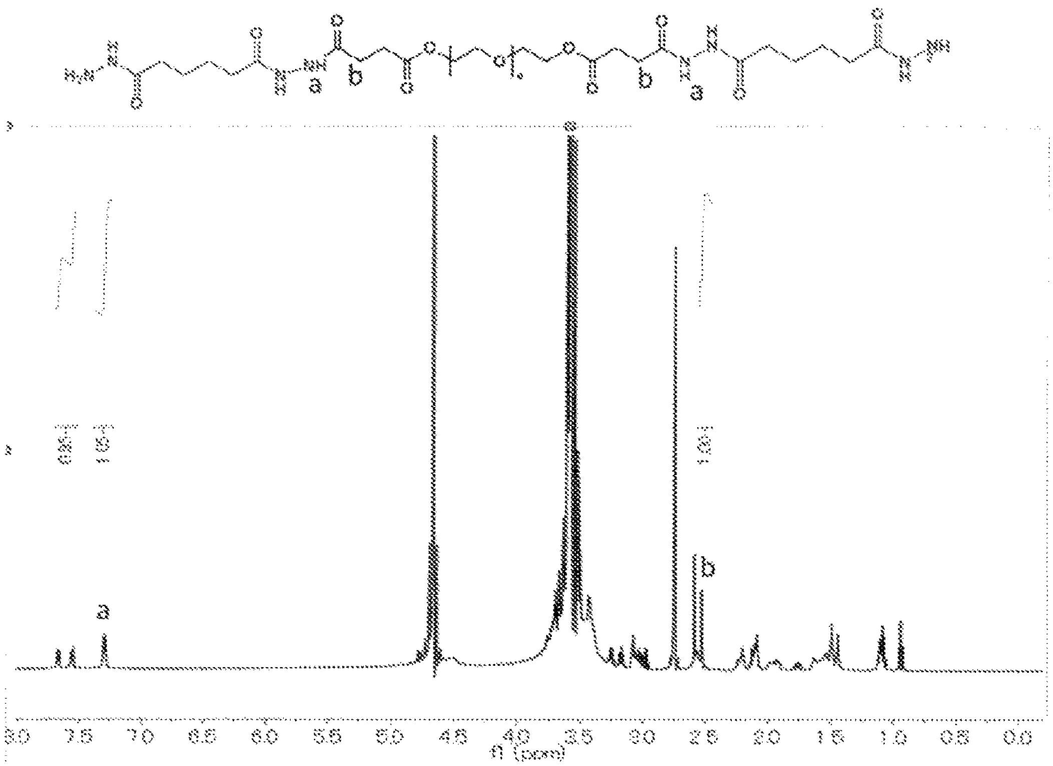

Fig. 36C

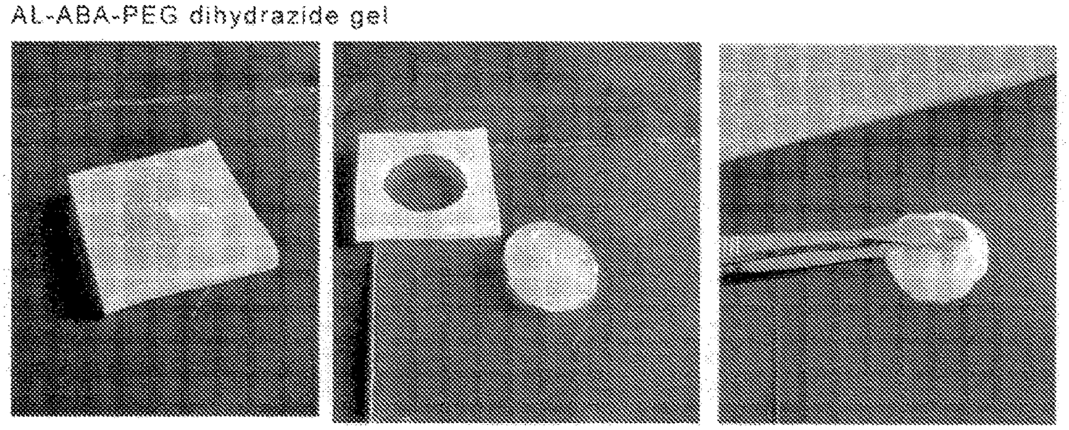


Fig. 37

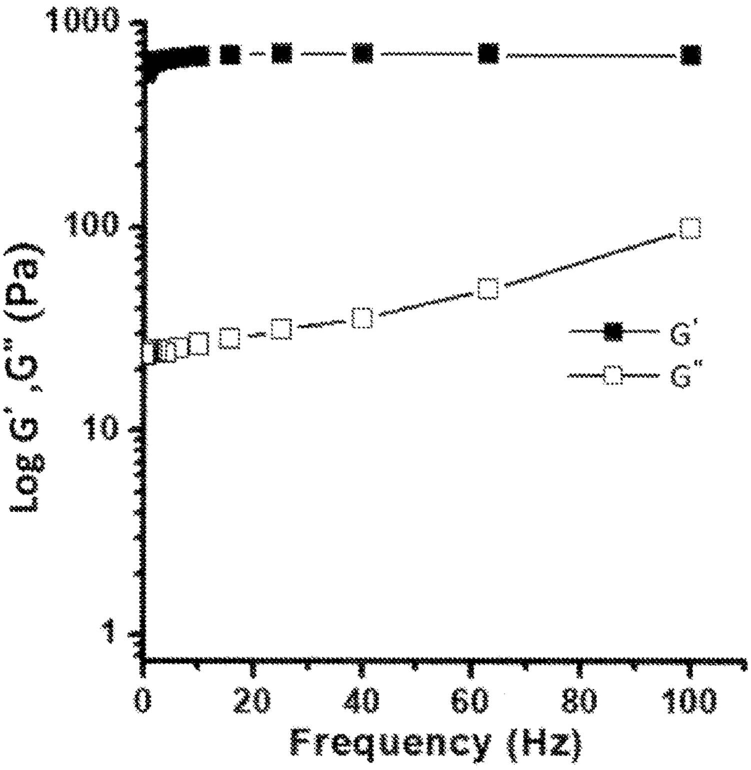


Fig. 38

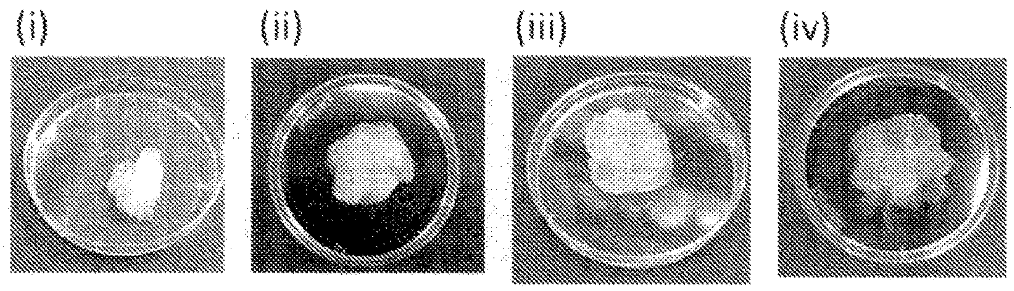

Fig. 39

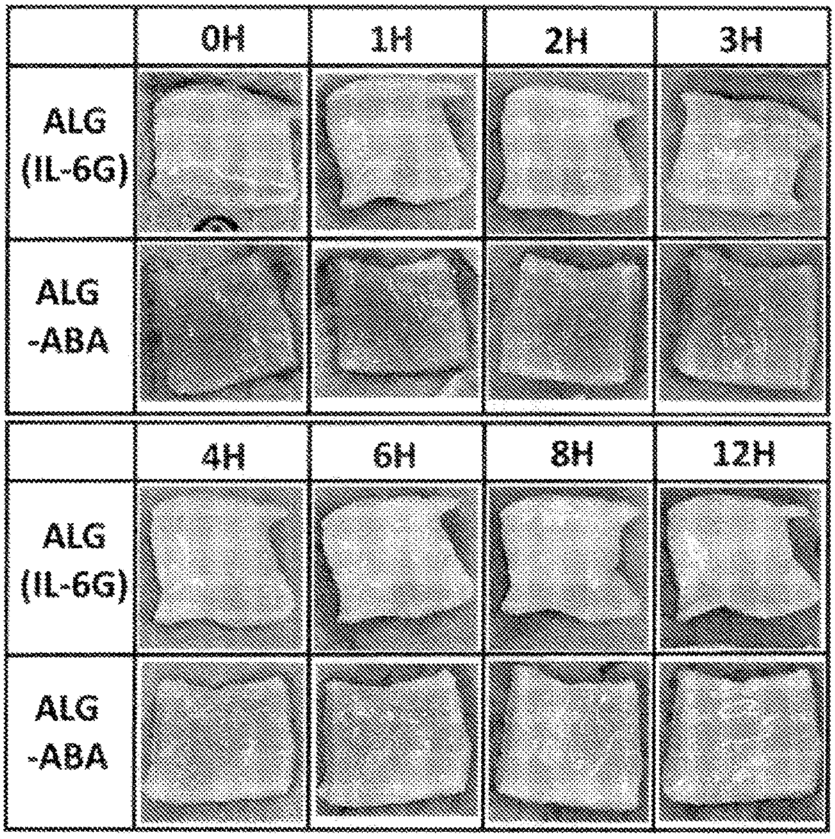
0H
1H
2H
3H
ALG (IL-6G)
ALG -ABA
4H
6H
8H
12H
ALG (IL-6G)
ALG -ABA

Fig. 40

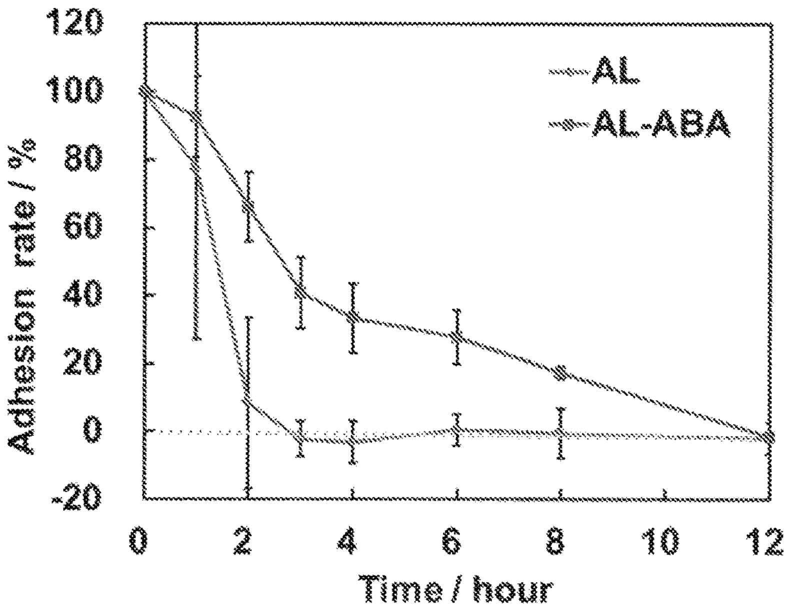
AL
AL-ABA
Adhesion rate / %
Time / hour
120
100
80
60
40
20
0
-20
0
2
4
6
8
10
12

Fig. 41

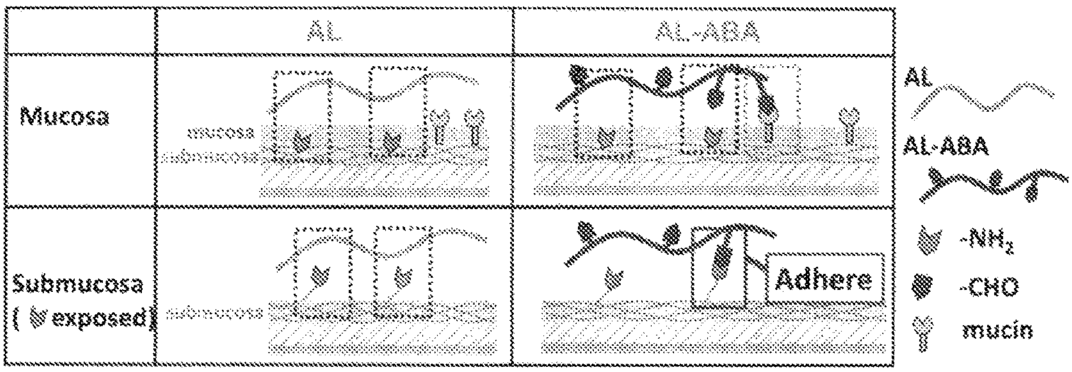
AL
AL-ABA
Mucosa
mucosa
submucosa
Submucosa
( exposed)
Adhere
AL
AL-ABA
-NH2
-CHO
mucin

Fig. 42

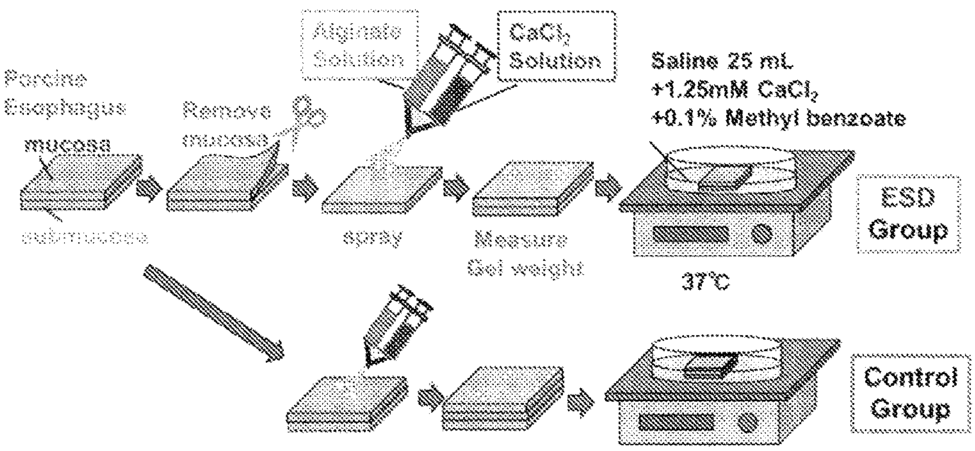
Porcine
Esophagus
mucosa
submucosa
Remove
mucosa
Alginate
Solution
CaCl2
Solution
spray
Measure
Gel weight
Saline 25 mL
+1.25mM CaCl2
+0.1% Methyl benzoate
37°C
ESD
Group
Control
Group

Fig. 43

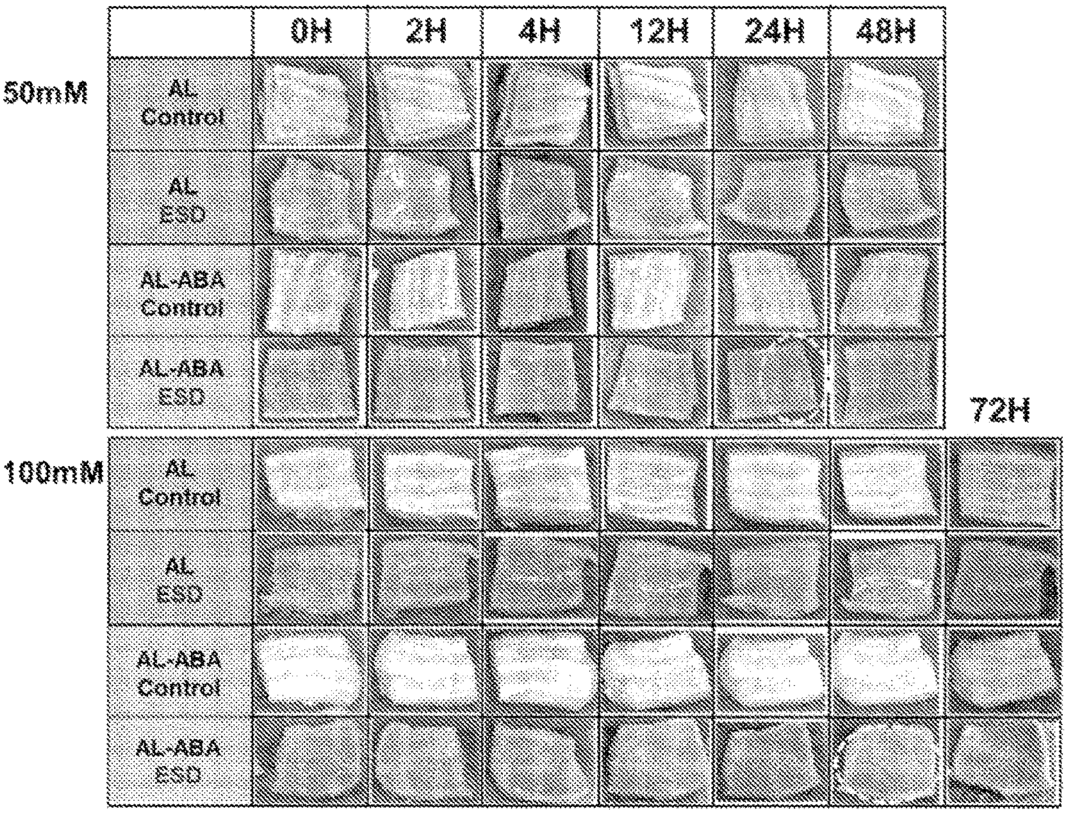
0H
2H
4H
12H
24H
48H
72H
50mM
100mM
AL Control
AL ESD
AL-ABA Control
AL-ABA ESD

Fig. 44

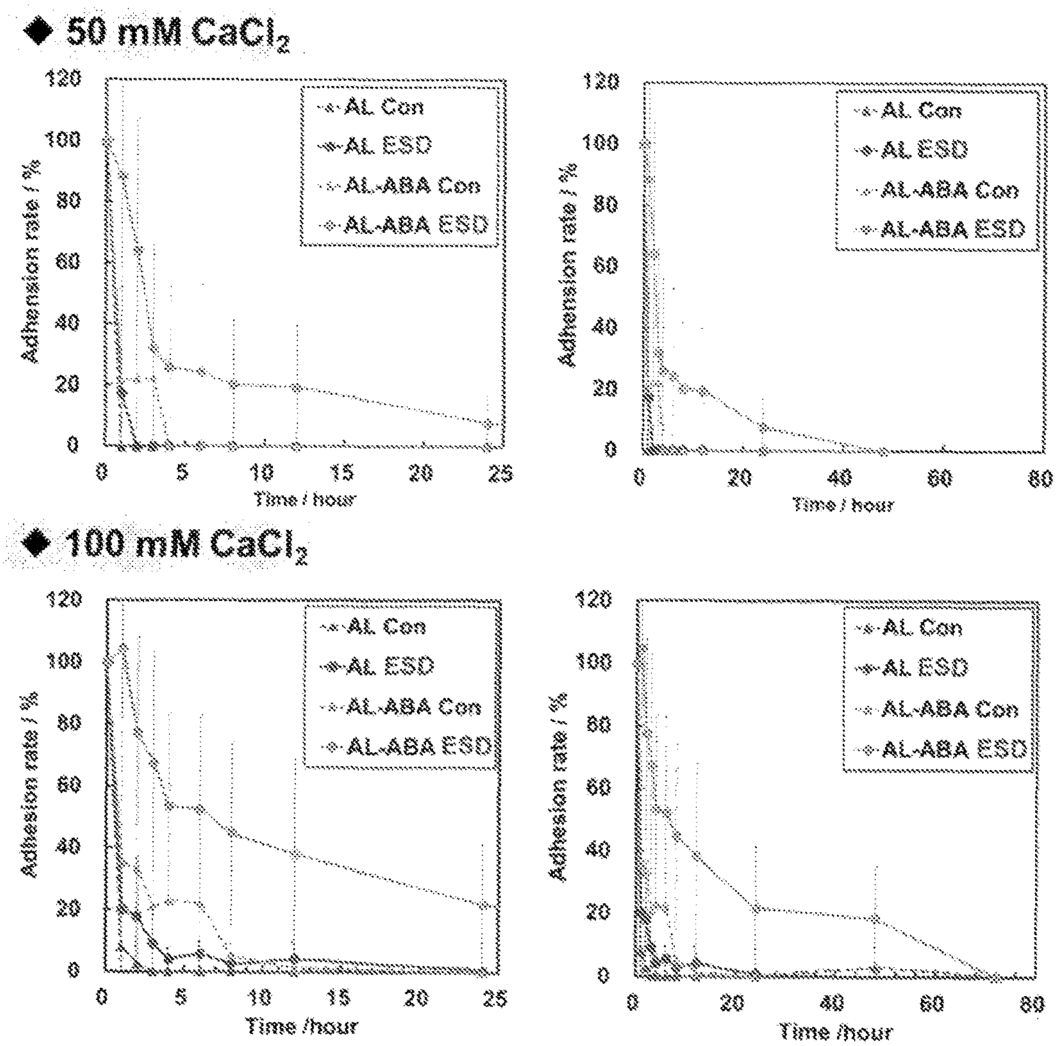
◆ 50 mM CaCl2
Adhesion rate / %
Time / hour
AL Con
AL ESD
AL-ABA Con
AL-ABA ESD
◆ 100 mM CaCl2
Time /hour

Fig. 45

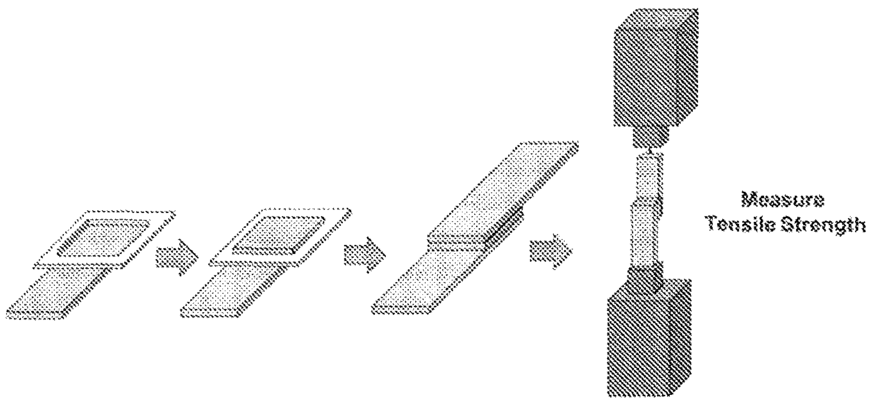
Measure
Tensile Strength

Fig. 46

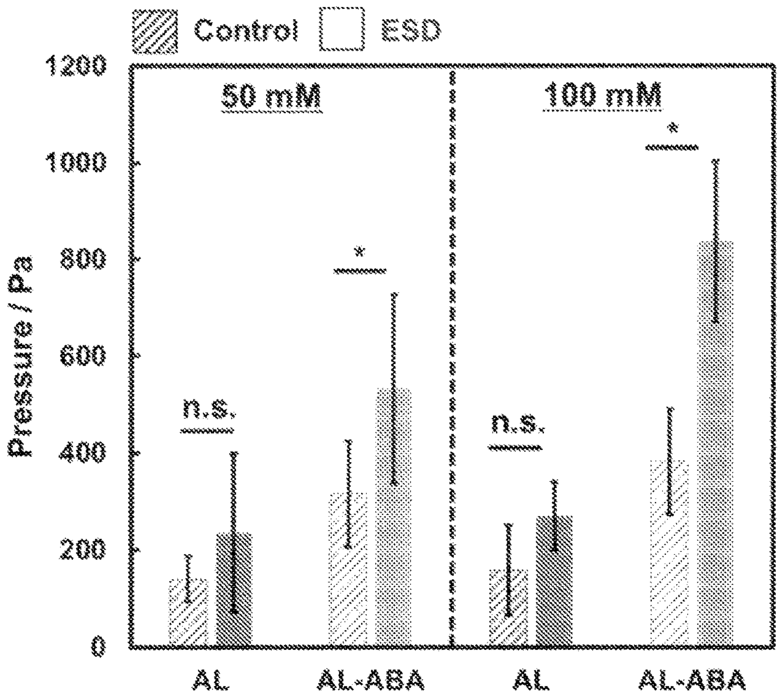
Control
ESD
50 mM
100 mM
Pressure / Pa
0
200
400
600
800
1000
1200
n.s.
*
n.s.
*
AL
AL-ABA
AL
AL-ABA

Fig. 47

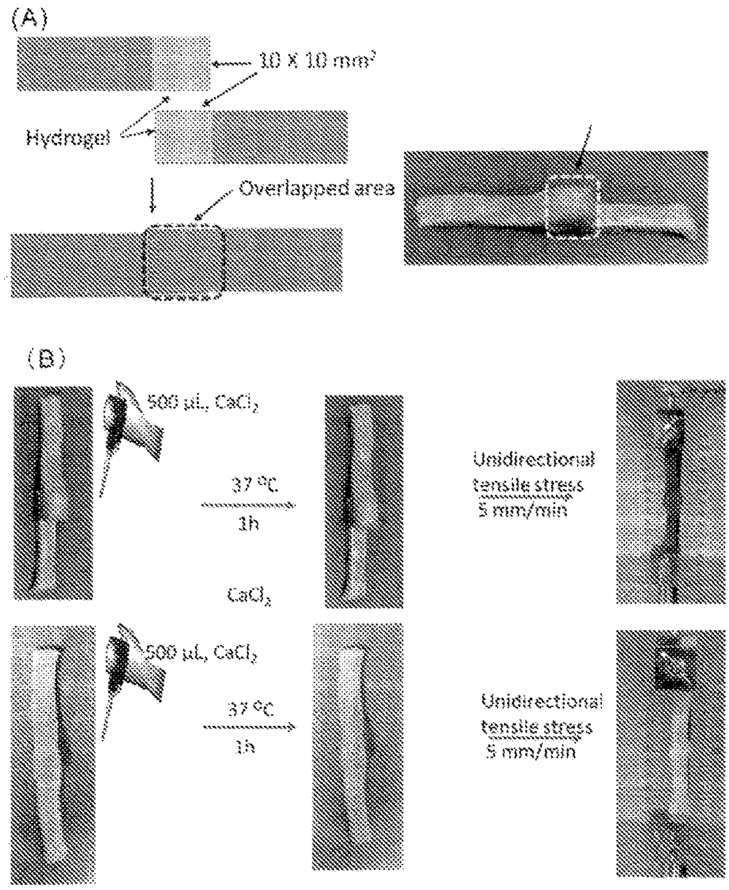
(A)
10 X 10 mm²
Hydrogel
Overlapped area
(B)
500 μL, CaCl₂
37 ºC
1h
Unidirectional
tensile stress
5 mm/min
CaCl₂
500 μL, CaCl₂
37 ºC
1h
Unidirectional
tensile stress
5 mm/min

Fig. 48

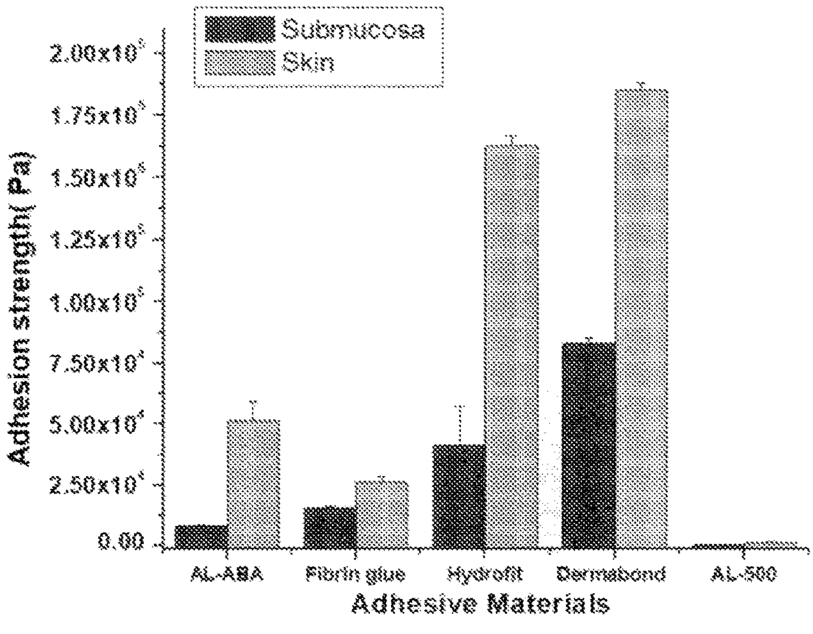
Submucosa
Skin
Adhesion strength( Pa)
2.00x10^5
1.75x10^5
1.50x10^5
1.25x10^5
1.00x10^5
7.50x10^4
5.00x10^4
2.50x10^4
0.00
AL-ABA
Fibrin glue
Hydrofit
Dermabond
AL-500
Adhesive Materials

Fig. 49

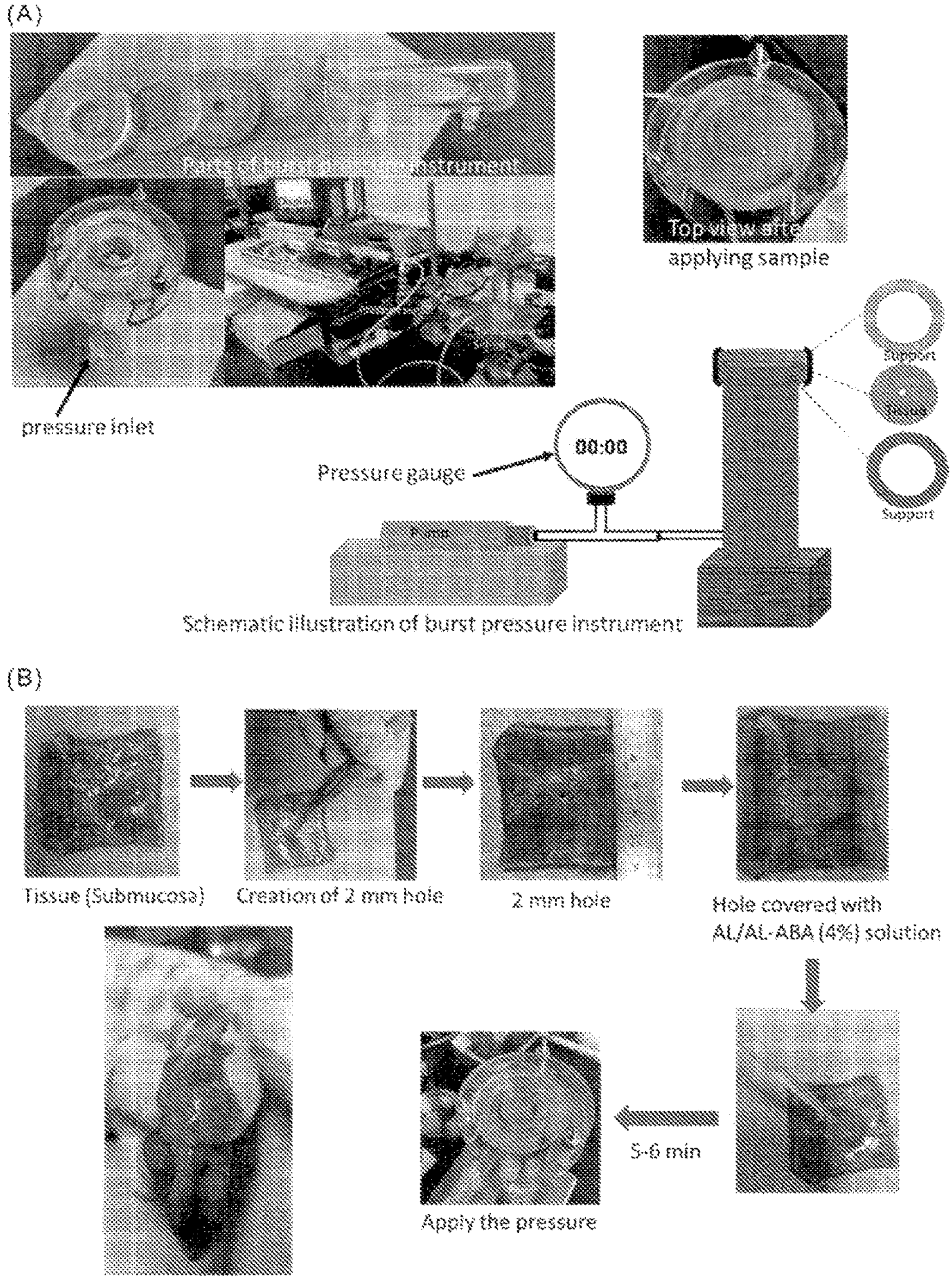
(A)
applying sample
pressure inlet
Pressure gauge
00:00
Support
Tissue
Support
Schematic illustration of burst pressure instrument
(B)
Tissue (Submucosa)
Creation of 2 mm hole
2 mm hole
Hole covered with
AL/AL-ABA (4%) solution
5-6 min
Apply the pressure

Fig. 50

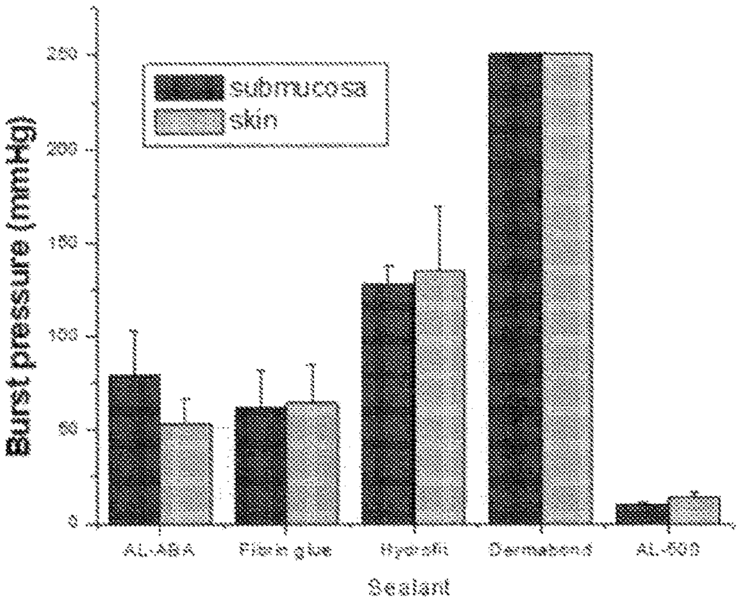
Burst pressure (mmHg)
0
50
100
150
200
250
submucosa
skin
AL-ASA
Fibrin glue
Dermabond
Sealant

Fig. 51

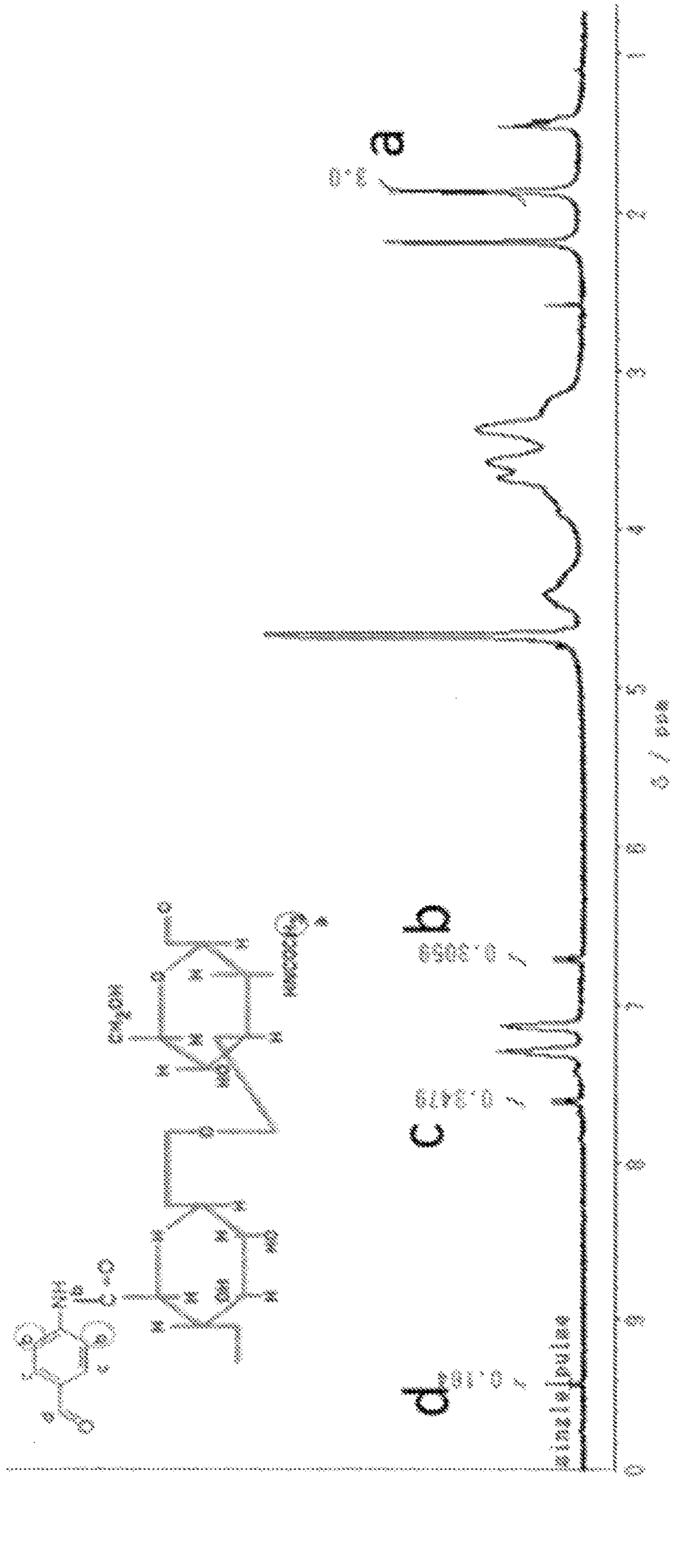

Fig. 52

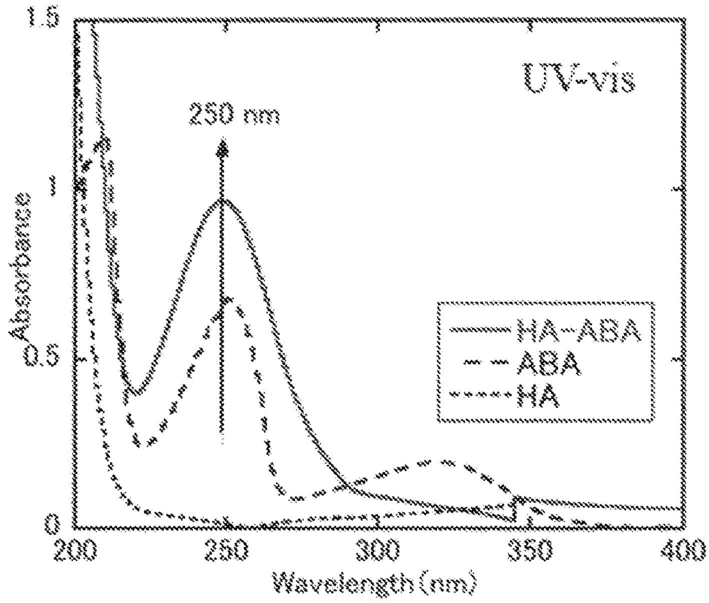
UV-vis
250 nm
HA-ABA
ABA
HA
Absorbance
Wavelength (nm)
0
0.5
1
1.5
200
250
300
350
400

Fig. 53

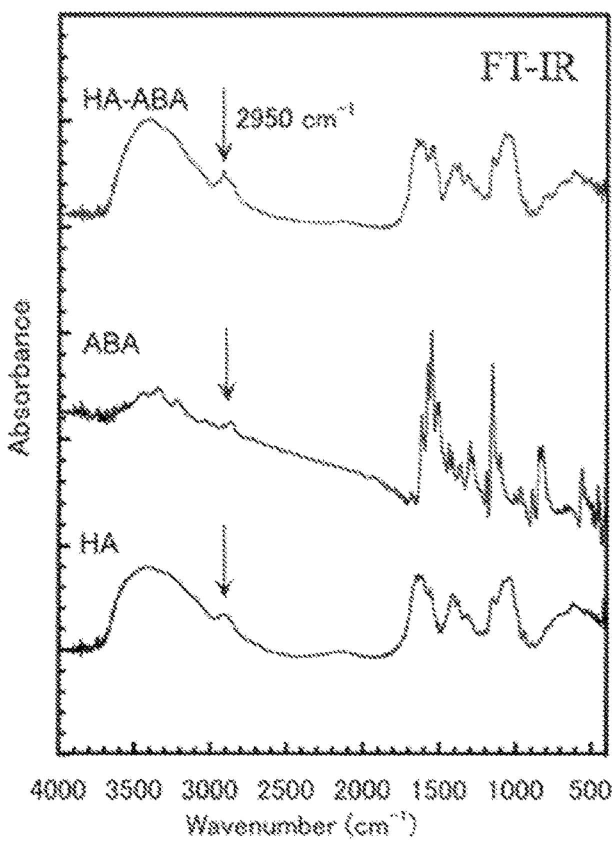
FT-IR
HA-ABA
2950 cm⁻¹
ABA
HA
Absorbance
4000 3500 3000 2500 2000 1500 1000 500
Wavenumber (cm⁻¹)

Fig. 54

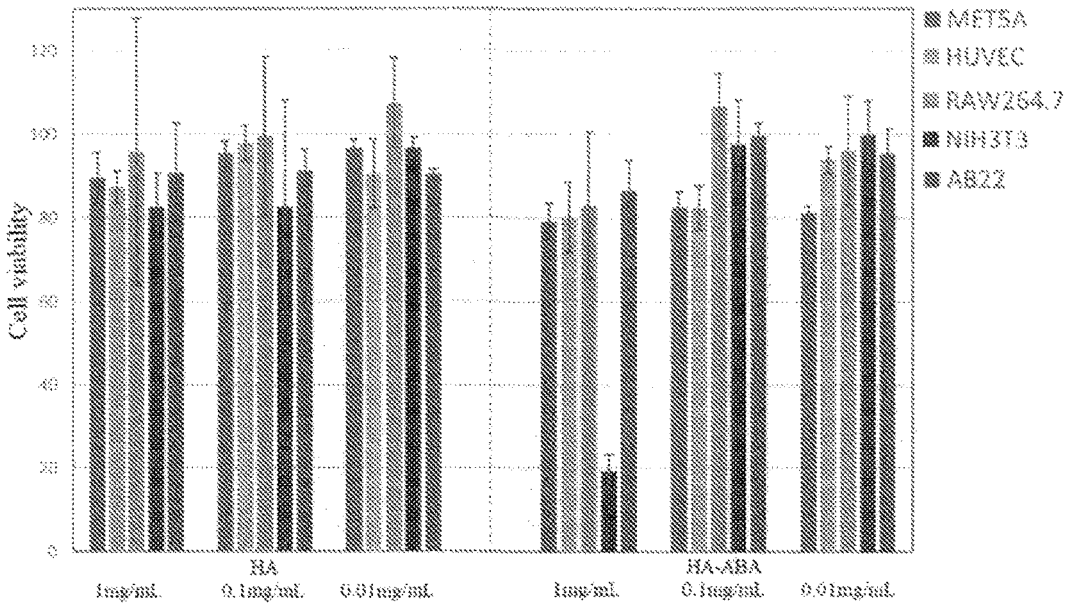
METSA
HUVEC
RAW264.7
NIH3T3
AB22
Cell viability
HA
1mg/mL
0.1mg/mL
0.01mg/mL
HA-ABA
1mg/mL
0.1mg/mL
0.01mg/mL

Fig. 55

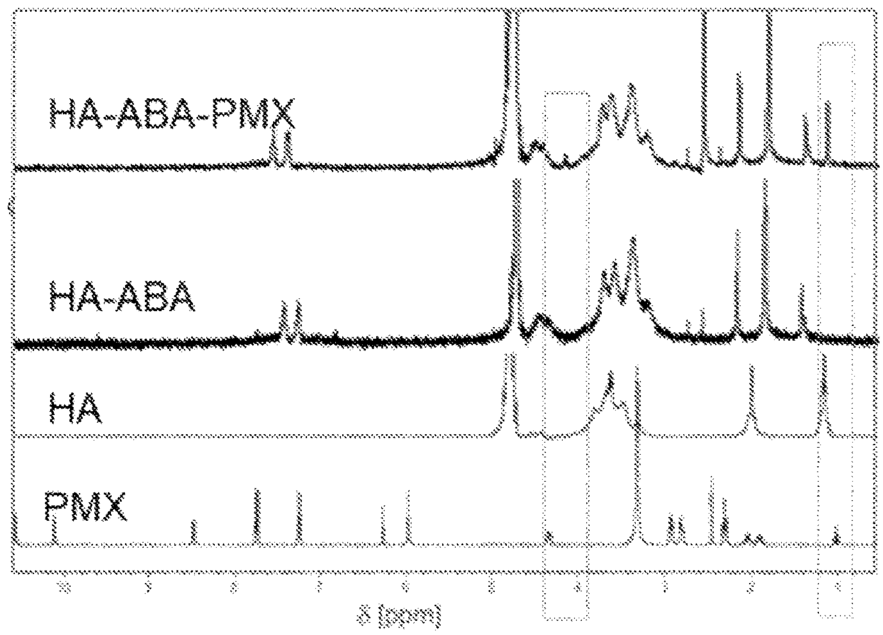
HA-ABA-PMX
HA-ABA
HA
PMX
δ [ppm]

Fig. 56

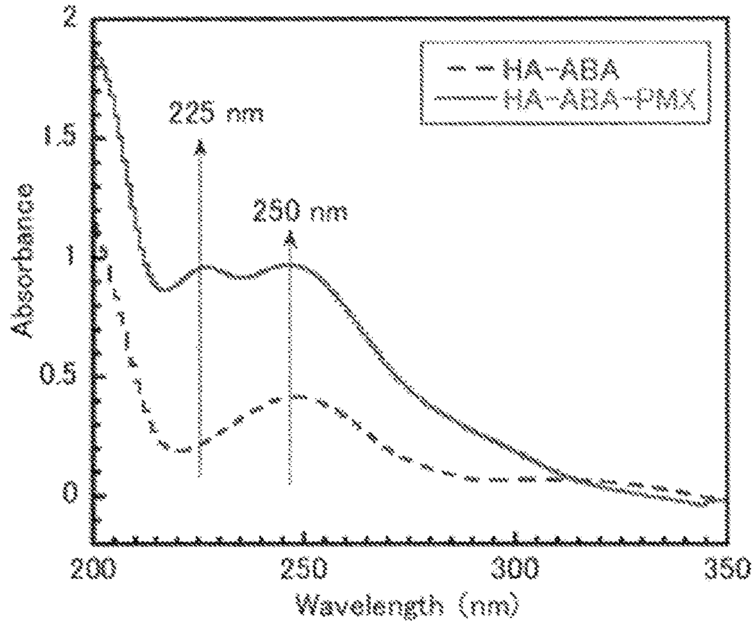
HA-ABA
HA-ABA-PMX
225 nm
250 nm
Absorbance
Wavelength (nm)
0
0.5
1
1.5
2
200
250
300
350

Fig. 57

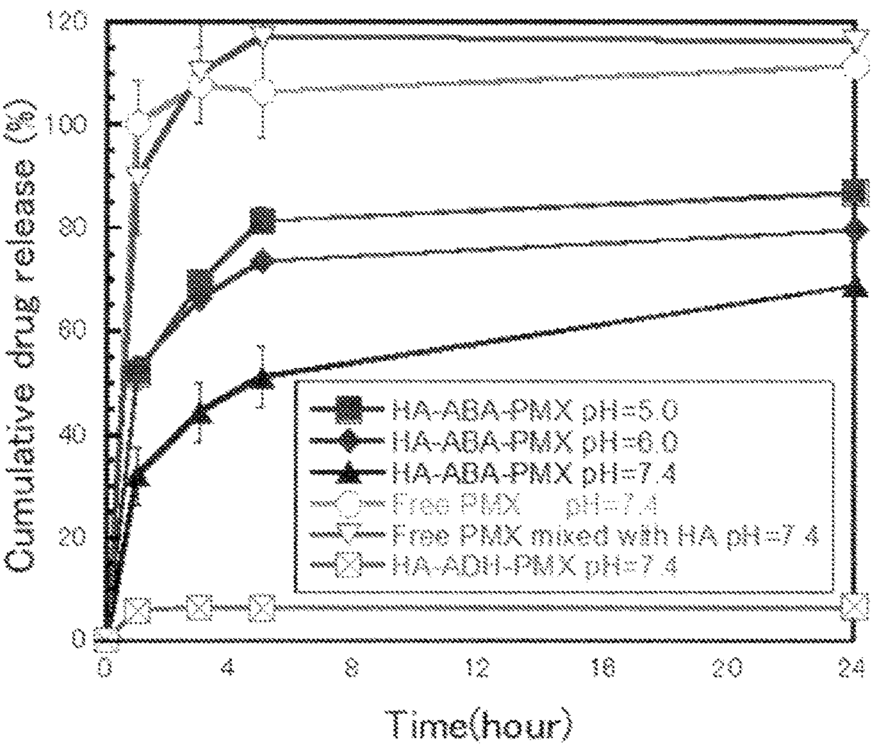
HA-ABA-PMX pH=5.0
HA-ABA-PMX pH=6.0
HA-ABA-PMX pH=7.4
Free PMX pH=7.4
Free PMX mixed with HA pH=7.4
HA-ADH-PMX pH=7.4
Cumulative drug release (%)
Time(hour)
0
20
40
60
80
100
120
0
4
8
12
16
20
24

Fig. 58

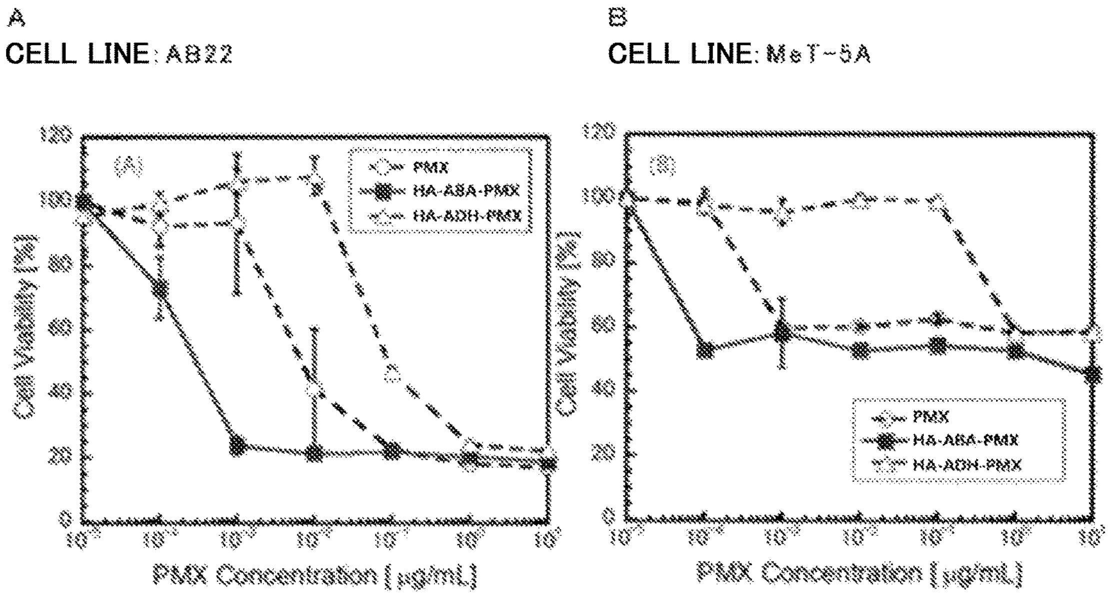
A
CELL LINE: AB22
B
CELL LINE: MeT-5A
Cell Viability [%]
PMX Concentration [μg/mL]
PMX
HA-ASA-PMX
HA-ADH-PMX

Fig. 59

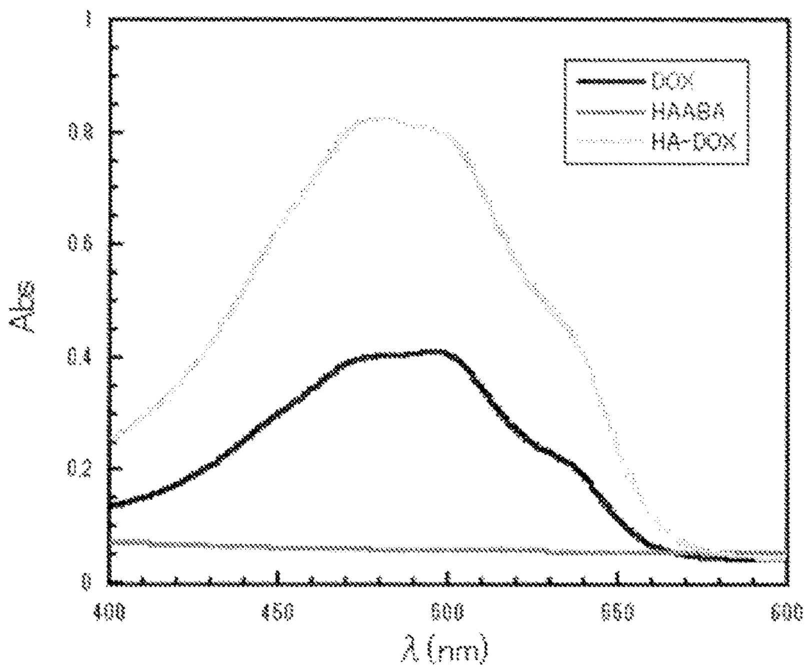
DOX
HAASA
HA-DOX
Abs
λ (nm)

Fig. 60

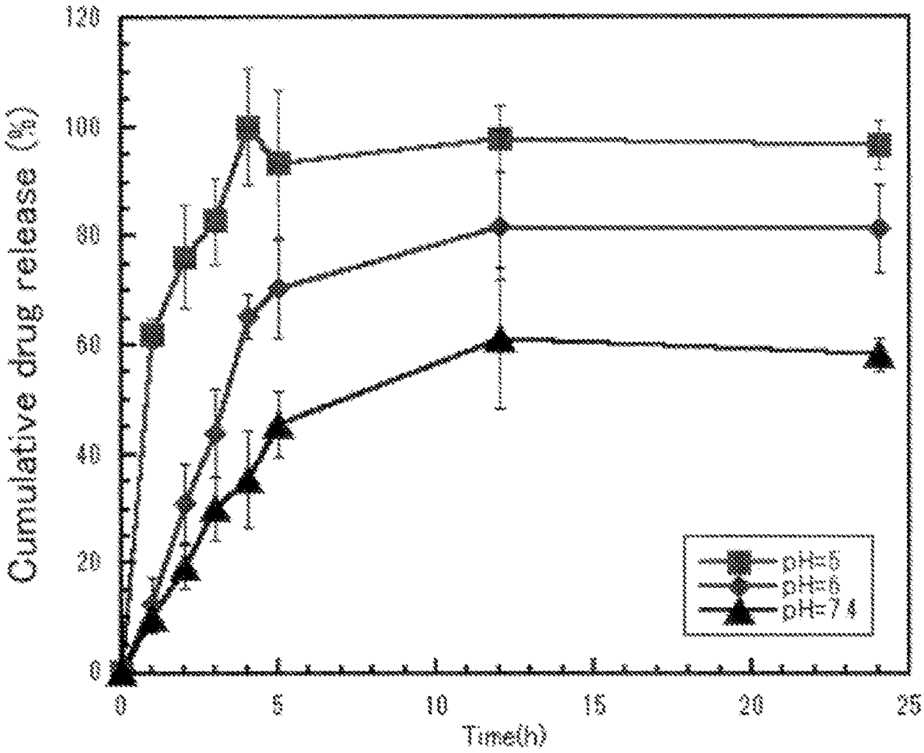
Cumulative drug release (%)
Time(h)
pH=5
pH=6
pH=7.4

Fig. 61

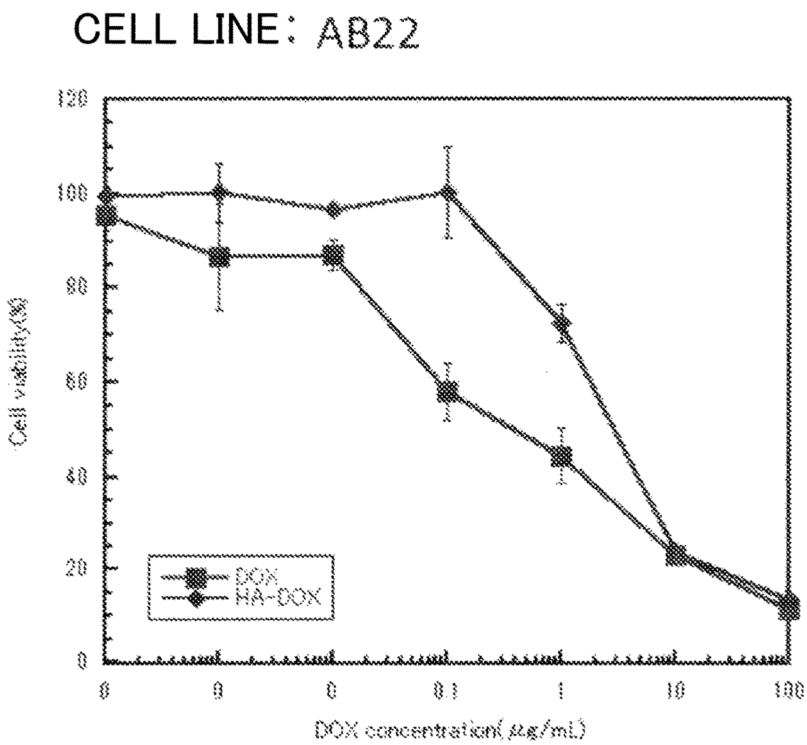
CELL LINE：AB22
Cell viability(%)
DOX concentration(μg/mL)
DOX
HA-DOX

Fig. 62

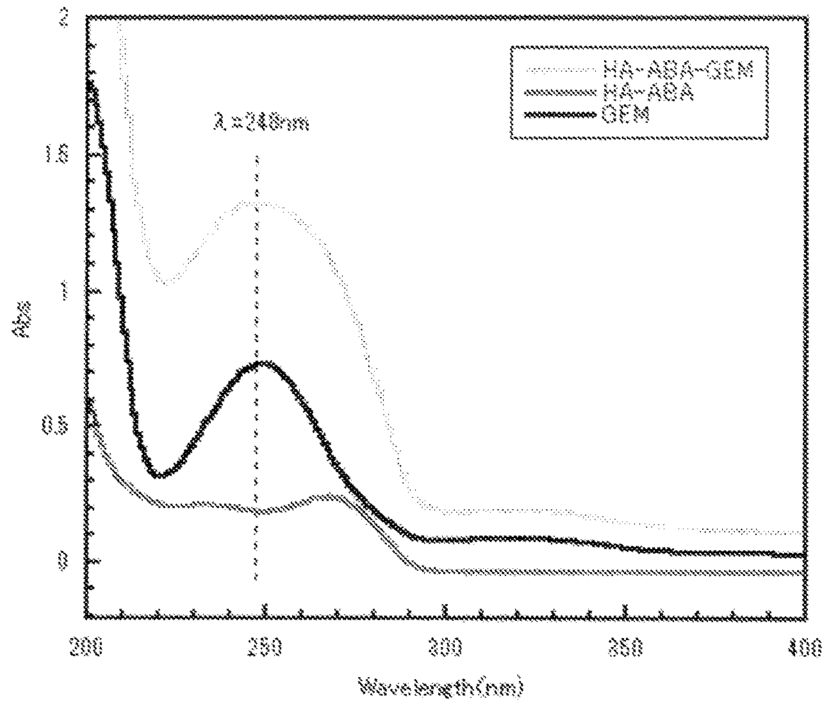
HA-ABA-GEM
HA-ABA
GEM
λ=248nm
Abs
2
1.5
1
0.5
0
200
250
300
350
400
Wavelength(nm)

Fig. 63

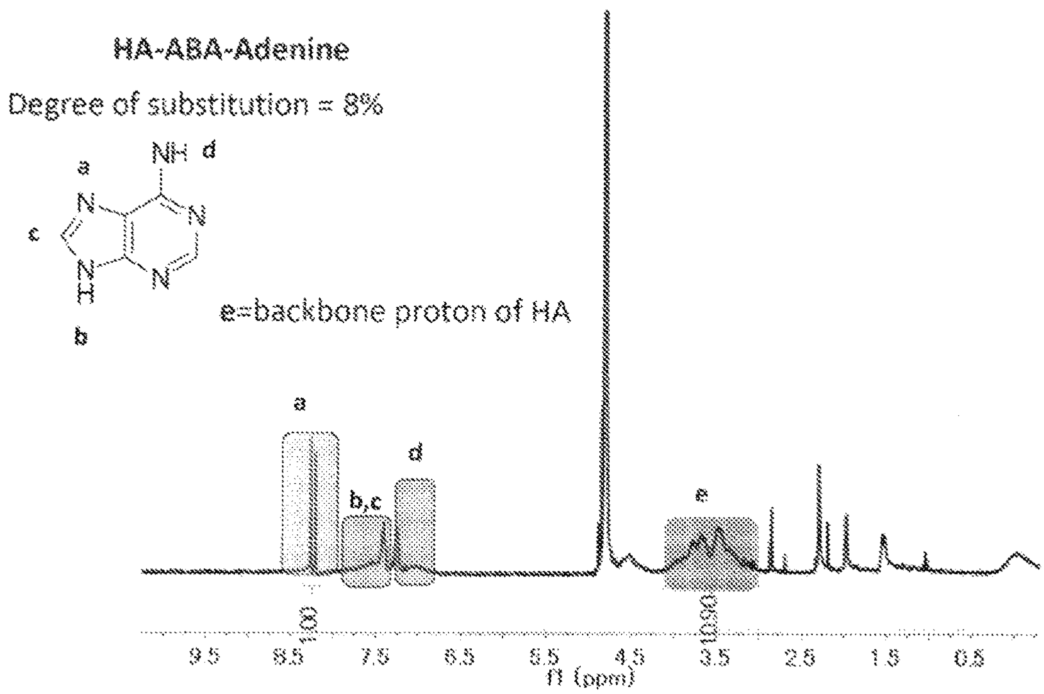
HA-ABA-Adenine
Degree of substitution = 8%
e=backbone proton of HA
a
b,c
d
e
1.00
10.50
9.5
8.5
7.5
6.5
5.5
4.5
3.5
2.5
1.5
0.5
f1 (ppm)

Fig. 64

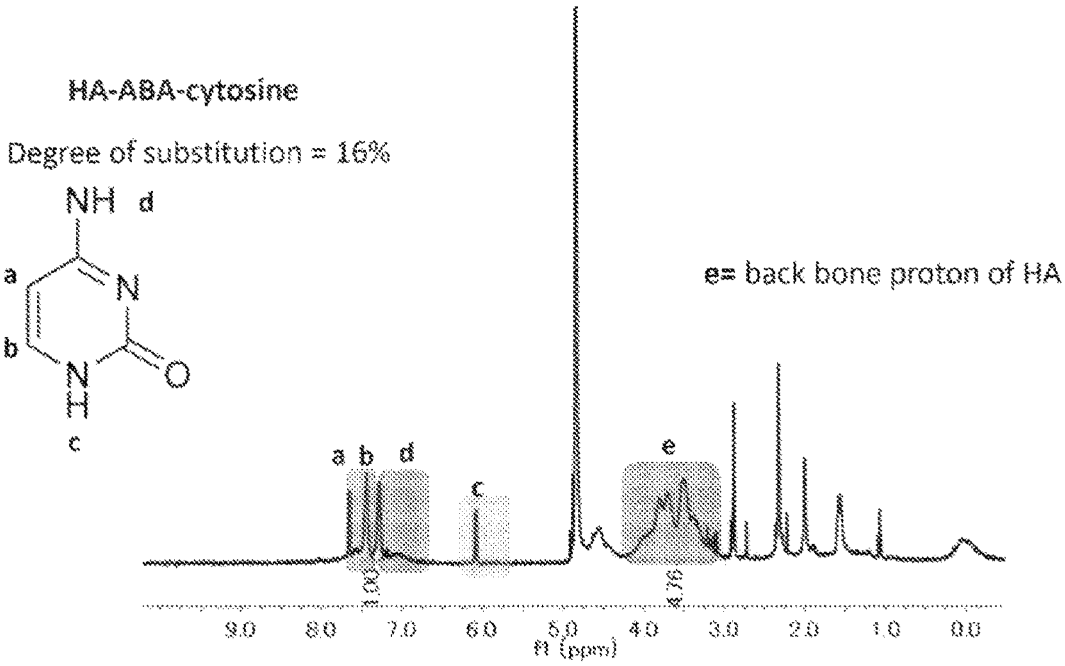
HA-ABA-cytosine
Degree of substitution = 16%
e= back bone proton of HA
a b d
c
e
1.00
4.76
9.0 8.0 7.0 6.0 5.0 4.0 3.0 2.0 1.0 0.0
f1 (ppm)

Fig. 65

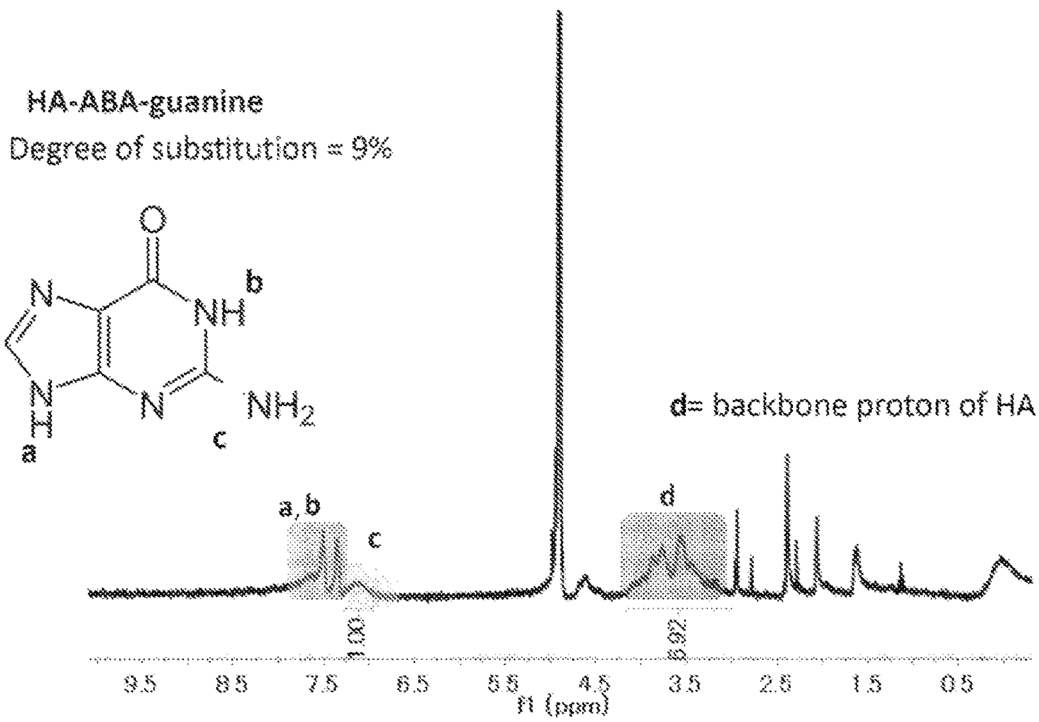
HA-ABA-guanine
Degree of substitution = 9%
d= backbone proton of HA
a, b
c
d
1.00
5.92
9.5 8.5 7.5 6.5 5.5 4.5 3.5 2.5 1.5 0.5
f1 (ppm)

Fig. 66

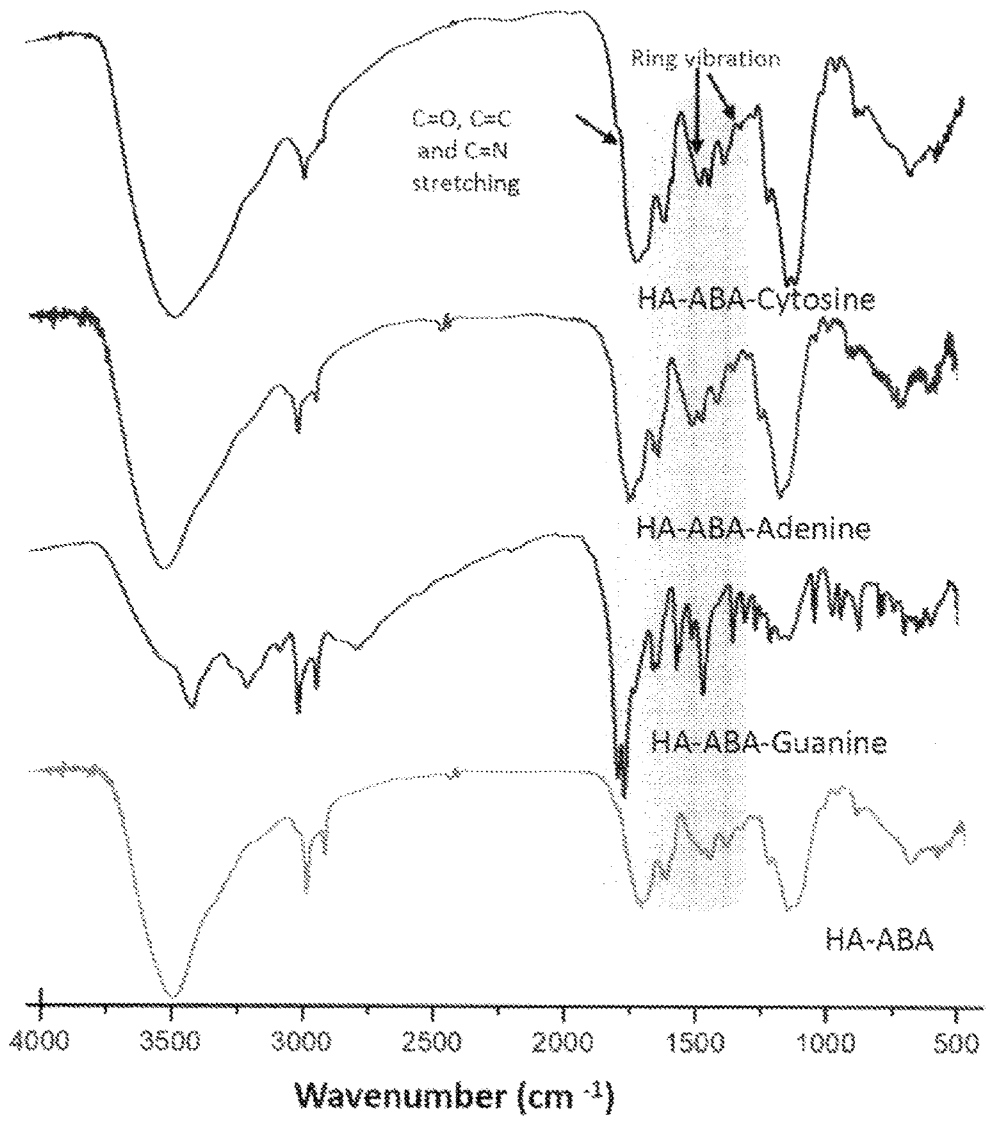
Ring vibration
C=O, C=C
and C=N
stretching
HA-ABA-Cytosine
HA-ABA-Adenine
HA-ABA-Guanine
HA-ABA
4000
3500
3000
2500
2000
1500
1000
500
Wavenumber (cm -1)

Fig. 67

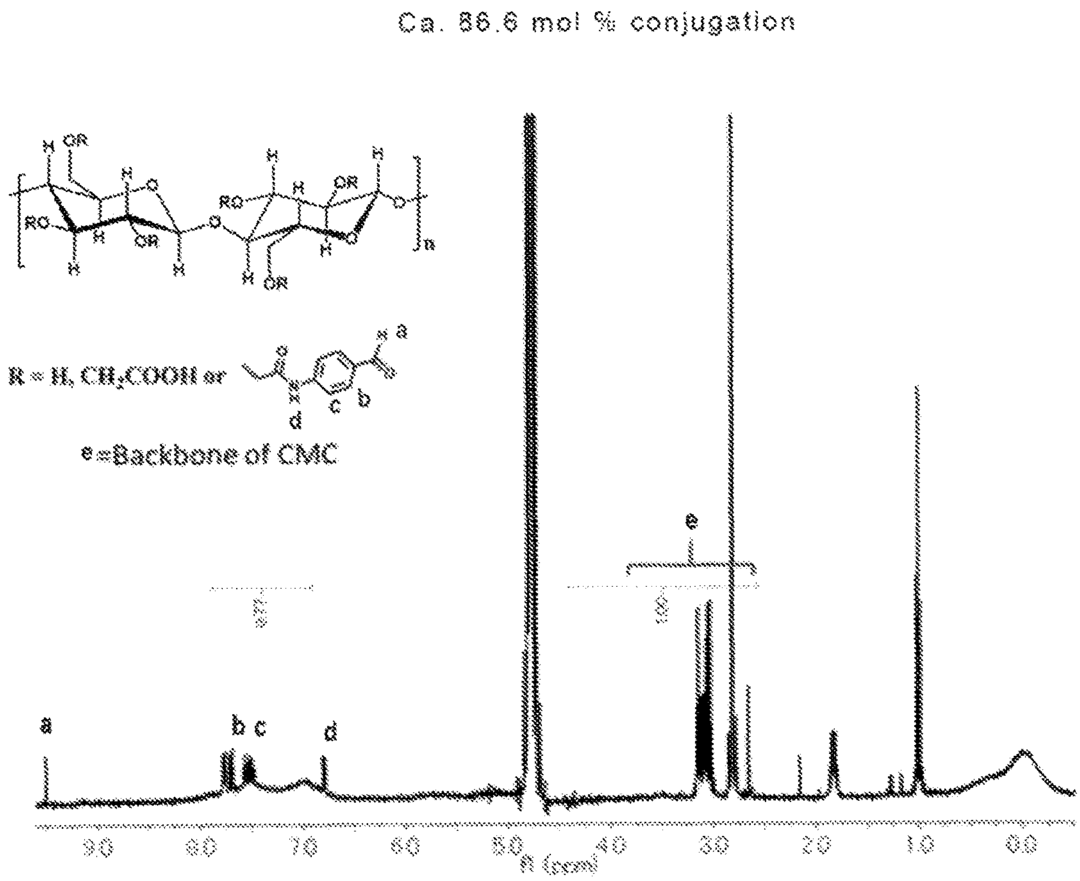
Ca. 86.6 mol % conjugation
R = H, CH2COOH or
e =Backbone of CMC
a
b c
d
e
9.0
8.0
7.0
6.0
5.0
4.0
3.0
2.0
1.0
0.0

Fig. 68

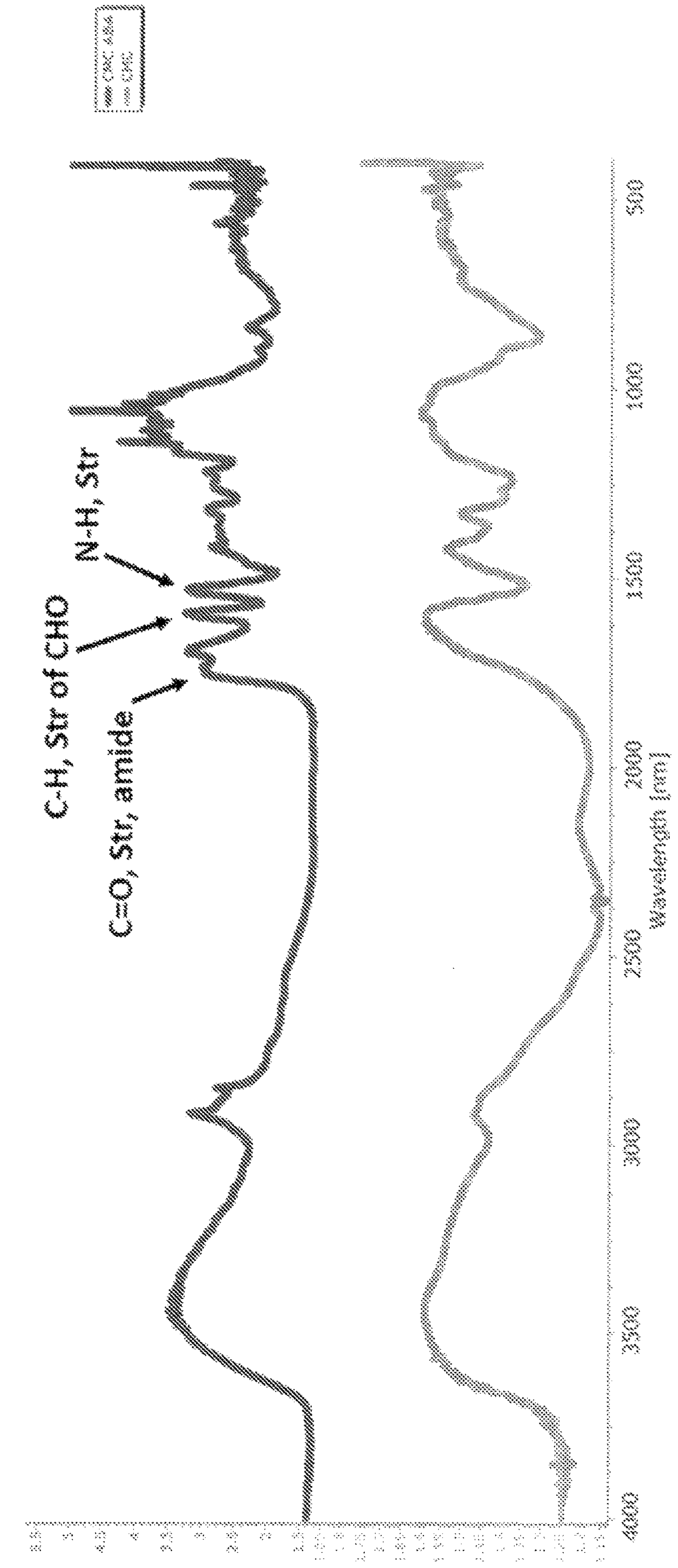
C-H, Str of CHO
N-H, Str
C=O, Str, amide
500
1000
1500
2000
2500
3000
3500
4000
Wavelength [nm]

Fig. 69

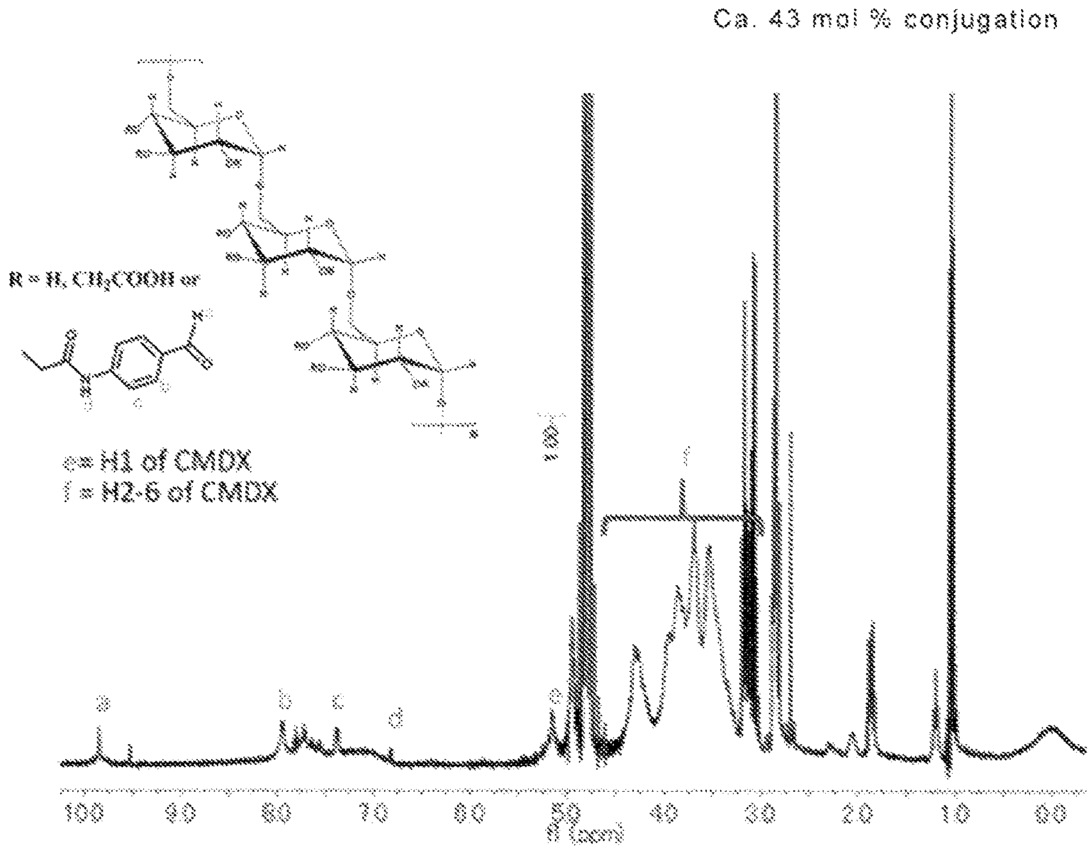
Ca. 43 mol % conjugation
R = H, CH2COOH or
e = H1 of CMDX
f = H2-6 of CMDX
a
b
c
d
e
f
10.0
9.0
8.0
7.0
6.0
5.0
4.0
3.0
2.0
1.0
0.0
f1 (ppm)

Fig. 70

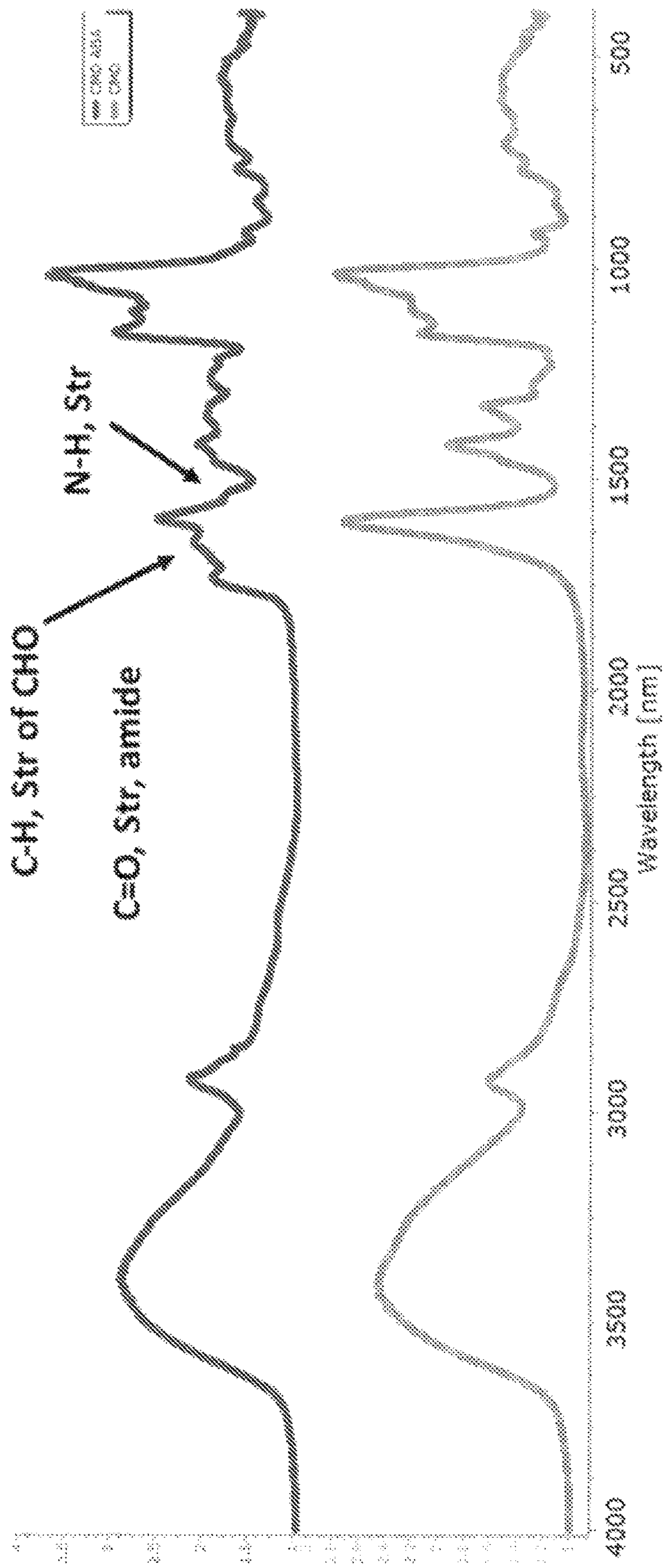
C-H, Str of CHO
C=O, Str, amide
N-H, Str
Wavelength [nm]
500
1000
1500
2000
2500
3000
3500
4000

Fig. 71

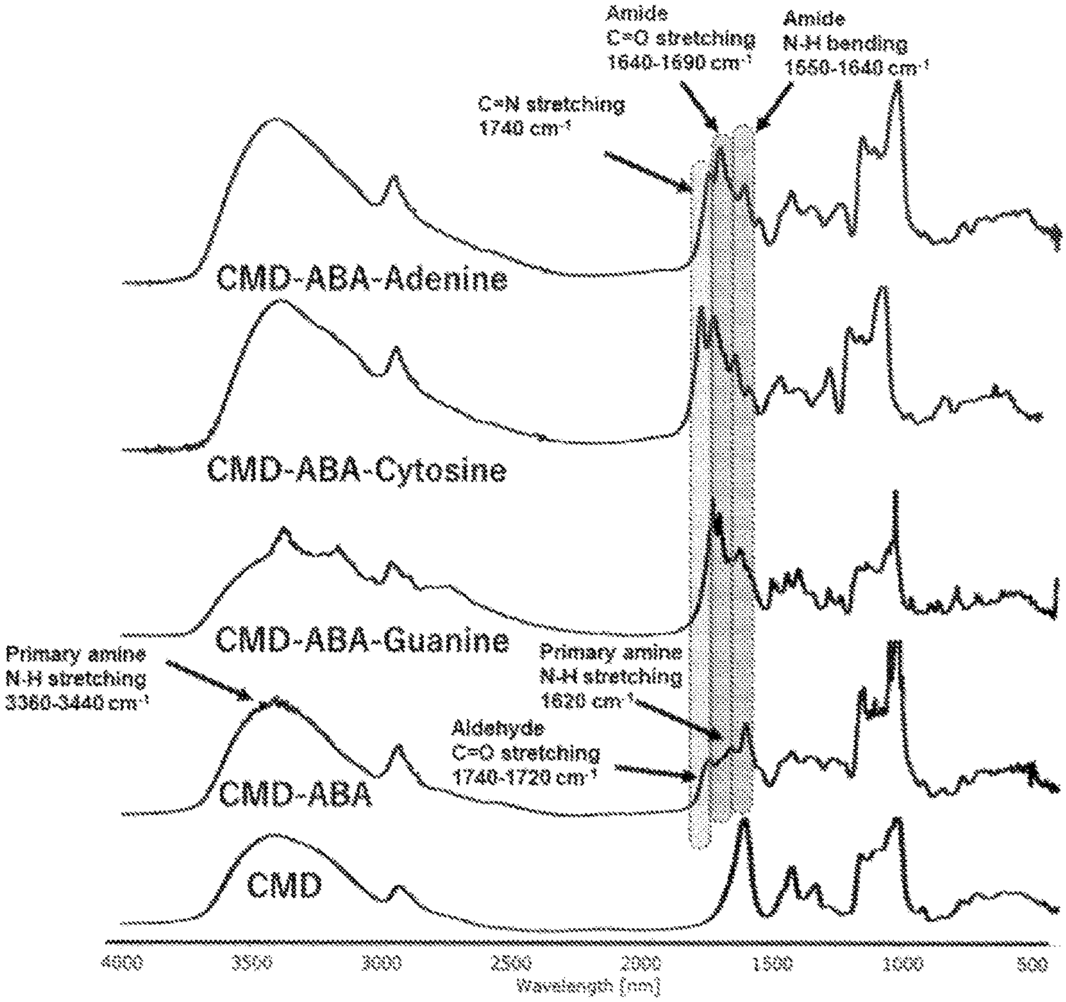
Amide
C=O stretching
1640-1690 $cm^{-1}$
Amide
N-H bending
1550-1640 $cm^{-1}$
C=N stretching
1740 $cm^{-1}$
CMD-ABA-Adenine
CMD-ABA-Cytosine
CMD-ABA-Guanine
Primary amine
N-H stretching
3360-3440 $cm^{-1}$
Primary amine
N-H stretching
1620 $cm^{-1}$
Aldehyde
C=O stretching
1740-1720 $cm^{-1}$
CMD-ABA
CMD
4000
3500
3000
2500
2000
1500
1000
500
Wavelength [nm]

Fig. 72

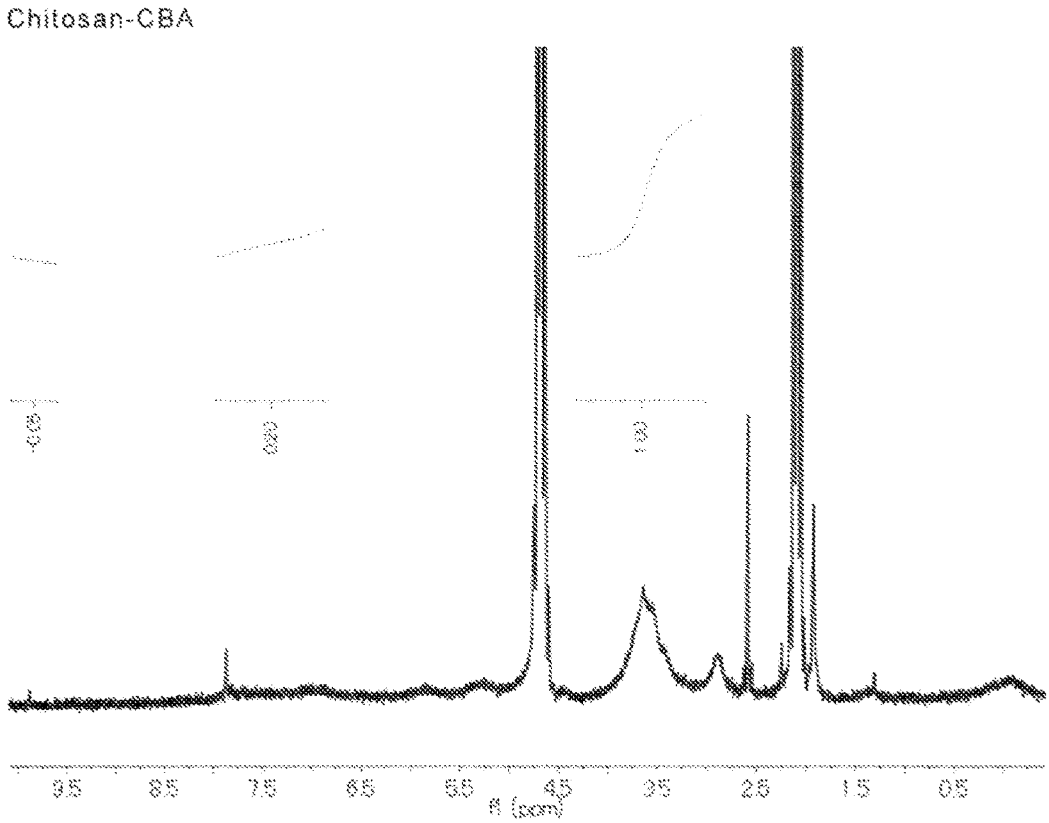
Chitosan-CBA
9.5
8.5
7.5
6.5
5.5
4.5
3.5
2.5
1.5
0.5
f1 (ppm)

Fig. 73

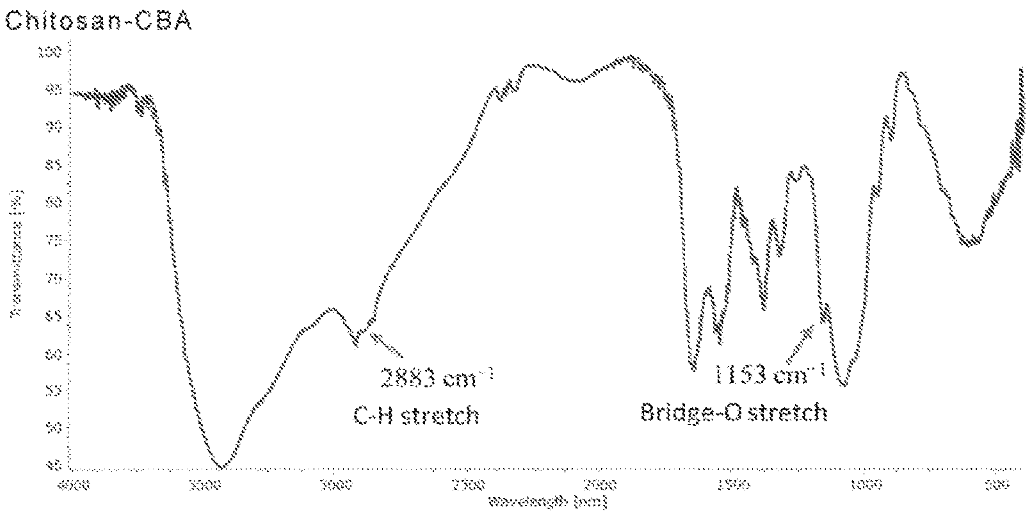
Chitosan-CBA
2883 cm⁻¹
C-H stretch
1153 cm⁻¹
Bridge-O stretch

## INTERNATIONAL SEARCH REPORT

International application No.
**PCT/JP2021/029573**

**A. CLASSIFICATION OF SUBJECT MATTER**

***C08B 11/15***(2006.01)i; ***A61K 9/06***(2006.01)i; ***A61K 9/48***(2006.01)i; ***A61K 45/00***(2006.01)i; ***A61K 47/61***(2017.01)i; ***C08B 37/02***(2006.01)i; ***C08B 37/04***(2006.01)i; ***C08B 37/08***(2006.01)i
FI: C08B11/15; A61K9/06; A61K9/48; A61K45/00; A61K47/61; C08B37/02; C08B37/04; C08B37/08 Z

According to International Patent Classification (IPC) or to both national classification and IPC

**B. FIELDS SEARCHED**

Minimum documentation searched (classification system followed by classification symbols)

C08B11/15; A61K9/06; A61K9/48; A61K45/00; A61K47/61; C08B37/02; C08B37/04; C08B37/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | DELLACHERIE, E. et al., A new approach to aldehydic dextrans, Polymer Bulletin, 1993, vol. 31, no. 2, pp. 145-149, DOI 10.1007/BF00329959<br>in particular, summary, introduction, fig. 1 | 1-3, 9-12, 20, 23 |
| X<br><br><br><br>Y | LIU, C. et al., Multifunctional PEG-grafted chitosan copolymer possessing amino and carboxyl (or formyl) groups, Central European Journal of Chemistry, 2010, vol. 8, no. 3, pp. 576-581, DOI 10.2478/s11532-010-0013-3<br>in particular, abstract, scheme 1, p. 580, right column, paragraph 2 | 1-12, 20-23<br><br><br><br>1-23 |
| Y | BUFFA, R. et al., Conjugates of modified hyaluronic acid with amino compounds for biomedical applications, Carbohydrate Polymers, 2018, vol.189, pp. 273-279, DOI 10.1016/j.carbpol.2018.02.048<br>in particular, abstract, scheme 1, tables 1, 2 | 1-23 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 September 2021** | **28 September 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br><br>Telephone No. |

Form PCT/ISA/210 (second sheet) (January 2015)

INTERNATIONAL SEARCH REPORT

International application No.
PCT/JP2021/029573

C. DOCUMENTS CONSIDERED TO BE RELEVANT

| Category* | Citation of document, with indication, where appropriate, of the relevant passages | Relevant to claim No. |
|---|---|---|
| Y | LI, N. et al., Using casein and oxidized hyaluronic acid to form biocompatible composite hydrogels for controlled drug release, Materials Science & Engineering, C, 2014, vol. 36, pp. 287-293, DOI 10.1016/j.msec.2013.12.025 in particular, abstract, fig. 1 | 1-23 |

Form PCT/ISA/210 (second sheet) (January 2015)

**INTERNATIONAL SEARCH REPORT**
**Information on patent family members**

International application No.
**PCT/JP2021/029573**

| Patent document cited in search report | Publication date (day/month/year) | Patent family member(s) | Publication date (day/month/year) |
|---|---|---|---|

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019189330 A
- JP 2001233786 A
- JP 2020137010 A

### Non-patent literature cited in the description


- *Materials Science and Engineering: C,* 2014, vol. 36, 287-293
- *Carbohydrate Polymers,* 2015, vol. 134, 293-299
- *Carbohydrate Polymers,* 2018, vol. 189, 273-279
- *Carbohydrate Polymers,* 2019, vol. 216, 63-71
- *Materials Chemistry Frontiers,* 2018, vol. 2 (10), 1765-1778
- *J. Biomed. Nanotechnol.,* 2017, vol. 13, 1647-1659
- *Polymer Bulletin,* 1993, vol. 31, 145-149
- *Carbohydrate Polymers,* 2013, vol. 92, 2163-2170
- *Journal of Controlled Release,* 2016, vol. 236, 79-89
- *European Journal of Pharmaceutical Sciences,* 2019, vol. 138, 105008
- *Materials Chemistry Frontiers,* 2018, vol. 2, 1765-1778
- **AKIRA TAKAHASHI et al.** *Biomacromolecules,* 2013, vol. 10 (14), 3581-3588
- **NIMMO, CHELSEA M. et al.** *Biomacromolecules,* 2011, vol. 12 (3), 824-830
- *RSC Adv.,* 2018, vol. 8, 11036-11042
- Japanese Pharmacopoeia
- **CHIKAKO YOMOTA et al.** *Bull. Natl. Health Sci.,* 1999, vol. 117, 135-139
- **CHIKAKO YOMOTA et al.** *Bull. Natl. Inst. Health Sci.,* 2003, vol. 121, 30-33
- Synthesis of Organic Compounds IV, Carboxylic Acids and Derivatives, Esters. Experimental Chemistry Course. vol. 16, 35-70
- *Acid Amides and Acid Imides,* 118-154
- Amino Acids and Peptides. Maruzen, 2007, 258-283
- **AMANO Y.** *European Journal of Pharmaceutical Sciences,* 2019, vol. 138, 105008